(19) Europäisches Patentamt
European Patent Office
Office européen des brevets

(11) EP 4 361 149 A1

(12) # EUROPEAN PATENT APPLICATION
published in accordance with Art. 153(4) EPC

(43) Date of publication:
**01.05.2024 Bulletin 2024/18**

(21) Application number: **22803959.0**

(22) Date of filing: **17.05.2022**

(51) International Patent Classification (IPC):
***C07D 413/14*** (2006.01) ***A61K 31/553*** (2006.01)
***A61P 35/00*** (2006.01) ***A61P 37/02*** (2006.01)

(52) Cooperative Patent Classification (CPC):
**A61K 31/553; A61P 1/00; A61P 17/00; A61P 17/06; A61P 19/02; A61P 29/00; A61P 35/00; A61P 37/02; C07D 413/12; C07D 413/14;** Y02P 20/55

(86) International application number:
**PCT/CN2022/093320**

(87) International publication number:
**WO 2022/242639 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2021 CN 202110546873**

(71) Applicant: **Chengdu Biobel Biotechnology Co., Ltd.**
**Sichuan 610093 (CN)**

(72) Inventors:
- **GUANG, Bing**
  **Chengdu, Sichuan 610093 (CN)**
- **YANG, Tai**
  **Chengdu, Sichuan 610093 (CN)**
- **DONG, Renghan**
  **Chengdu, Sichuan 610093 (CN)**
- **QIN, Chuanjun**
  **Chengdu, Sichuan 610093 (CN)**
- **XIE, Jian**
  **Chengdu, Sichuan 610093 (CN)**
- **PENG, Jian**
  **Chengdu, Sichuan 610093 (CN)**
- **HUANG, Sheng**
  **Chengdu, Sichuan 610093 (CN)**
- **LIU, Jin**
  **Chengdu, Sichuan 610093 (CN)**
- **ZHAN, Wei**
  **Chengdu, Sichuan 610093 (CN)**
- **LAI, Yongxin**
  **Chengdu, Sichuan 610093 (CN)**
- **PENG, Xiangyang**
  **Chengdu, Sichuan 610093 (CN)**
- **XU, Qing**
  **Chengdu, Sichuan 610093 (CN)**
- **ZENG, Yisheng**
  **Chengdu, Sichuan 610093 (CN)**
- **HU, Xiaojun**
  **Chengdu, Sichuan 610093 (CN)**

(74) Representative: **Altmann Stößel Dick Patentanwälte PartG mbB**
**Theodor-Heuss-Anlage 2**
**68165 Mannheim (DE)**

(54) **RECEPTOR-INTERACTING PROTEIN INHIBITOR, PREPARATION METHOD THEREFOR, AND USE THEREOF**

(57) Provided are a compound as a receptor-interacting protein (RIP) inhibitor, a preparation method therefor, and a use thereof, relating to the field of chemical medicines. The compound is a compound as represented by formula (I), or a salt thereof, or a stereoisomer thereof, or a solvate thereof, or a prodrug thereof. The compound has good inhibitory activity on humanized RIP1 kinase, and can be used for preparing drugs for resisting inflammation, resisting tumors and the like.

EP 4 361 149 A1

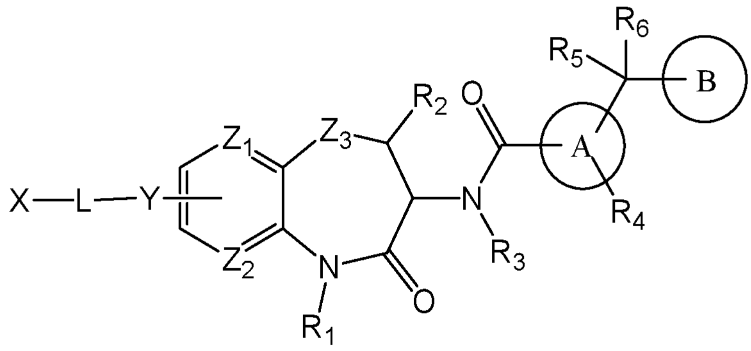

(I)

## Description

### Technical field

The present invention belongs to the field of chemical medicine, and specifically relates to a receptor-interacting protein (RIP) inhibitor, a preparation method therefor, and uses thereof.

### Background technology

Programmed necrosis, also known as necroptosis, is a new and controllable cell death mode with necrotic morphological characteristics. Programmed necrosis plays a key role in embryonic development and the dynamic balance of the adult body, and is widely involved in the pathophysiological processes of many diseases. Receptor-interacting protein 1 (RIP1) is located at a critical position in the programmed necrosis pathway, and is an upstream kinase that has a regulatory effect. As an upstream regulatory molecule of the signaling pathway, its abnormal activation can cause a series of reactions. RIP1 has become the "central controller" determining cell fate in the death receptor signaling pathway.

Numerous studies have shown that cell death and inflammatory responses mediated by the activation of RIP1 and programmed necrosis pathways involve various human diseases, including stroke, myocardial infarction, retinal injury, fatal systemic inflammatory response syndrome, chronic enteritis, and malignant tumors. RIP1 kinase serves as a potential target in the programmed necrosis pathway, and thus suppression of its kinase activity can retard the progression of the disease. RIP1 inhibitors have important application value in treating diseases such as inflammation, cardiovascular and cerebrovascular diseases, neurodegenerative diseases, and tumors. Therefore, RIP inhibitors, especially RIP1 inhibitors, have received great attention from pharmaceutical chemistry researchers.

Philip A. Harris et al. have reported a class of RIP inhibitors with representative structures and found that they have potential applications in treating inflammation or tumors. However, its therapeutic effect still needs to be further improved, and RIP inhibitors with better therapeutic effects are still urgently needed in clinical practice.

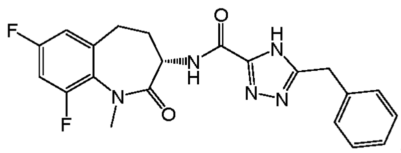,
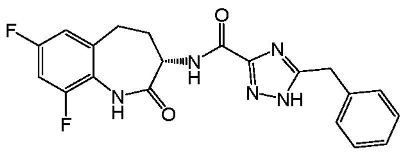,
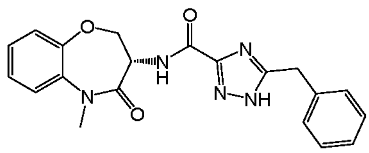.

### Summary of th invention

The present invention aims to provide a receptor-interacting protein (RIP) inhibitor, a preparation method therefor, and uses thereof.

The present invention provides the compound as represented by formula (I), or a salt thereof, or a stereoisomer thereof, or a solvate thereof, or a prodrug thereof:

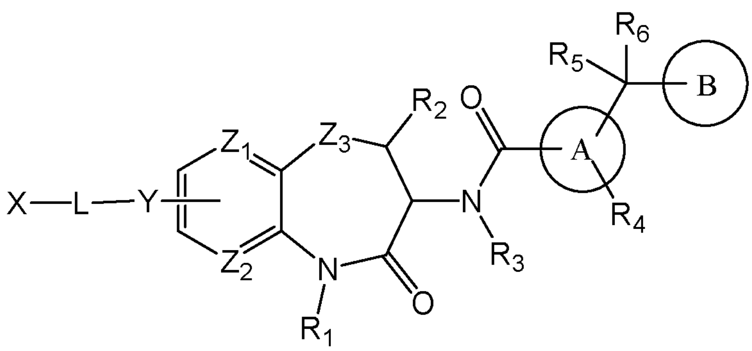

(I) ;

wherein, ring A is a 3-6 membered unsaturated heterocyclyl;

ring B is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino, and hydroxyl;
$Z_1$ and $Z_2$ are each independently selected from CH or N;
$Z_3$ is selected from the group consisting of O, $NR_8$, S or $CR_8R_9$;
$R_1$ is selected from the group consisting of H, $C_1$-$C_6$ alkyl or $C_1$-$C_6$ deuteroalkyl;
$R_3$ is selected from the group consisting of H, $C_1$-$C_6$ alkyl or $C_1$-$C_6$ deuteroalkyl; $R_4$ is selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$ and $R_6$ are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring;
$R_8$, $R_9$ and $R_{10}$ are each independently selected from the group consisting of H, $C_1$-$C_6$ alkyl, amino or hydroxyl;
X is selected from the structure represented by formula (Ib'):

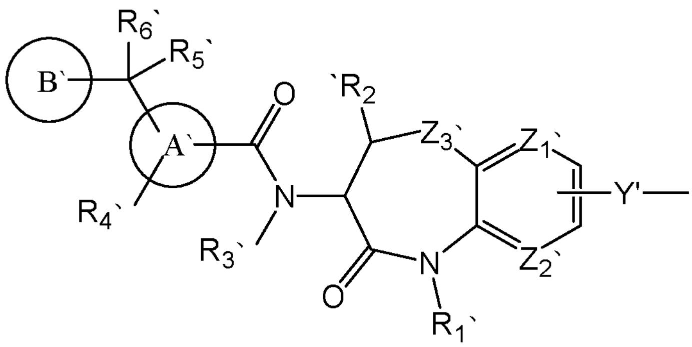

(Ib`)

;

wherein, Y' is selected from the group consisting of , , O, $CH_2$ or

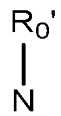 ;

$R_0$' is selected from the group consisting of H, $C_1$-$C_5$ alkyl or cycloalkyl;
ring A' is a 3-6 membered unsaturated heterocyclyl;
ring B' is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino, and hydroxyl;
$Z_1$' and $Z_2$' are each independently selected from CH or N;
$Z_3$' is selected from the group consisting of O, $NR_8$, S or $CR_8R_9$;
$R_1$' and $R_2$' are each independently selected from the group consisting of H, $C_1$-$C_6$ alkyl or $C_1$-$C_6$ deuteroalkyl;
$R_3$' is selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$' and $R_4$' are linked to form a ring; or $R_4$' and $R_5$' are linked to form a ring;
$R_8$, $R_9$ and $R_{10}$ are each independently selected from the group consisting of H, $C_1$-$C_6$ alkyl, amino, and hydroxyl;
L is a chain containing carbon atoms, which can also include any number of heteroatoms N, O, S, and P;
$R_2$ is selected from the group consisting of H, $C_1$-$C_6$ alkyl or $C_1$-$C_6$ deuteroalkyl;
Y' is selected from the group consisting of , , O, $CH_2$ or

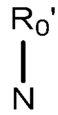 ;

$R_0$ is selected from the group consisting of H, $C_1$-$C_5$ alkyl or cycloalkyl;

alternatively, X is not the structure represented by formula (Ib'), and provided that $R_2$ is methyl, L and X form any group containing carbons; Y is selected from the group consisting of ,  or

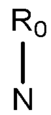 ;

$R_0$ is selected from the group consisting of H, $C_1$-$C_5$ alkyl or cycloalkyl;

alternatively, X is not the structure represented by formula (Ib'), and provided that $R_2$ is H, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cycloalkyl, a substituted or unsubstituted, saturated or unsaturated 3-10 membered heterocyclyl, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group; $R_8$, $R_9$ and $R_{10}$ are each independently selected from the group consisting of H, $C_1$-$C_6$ alkyl, amino or hydroxyl;

L is $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_2$, $n_2'$, $m_1$, $m_1'$, $m_2$, and $m_2'$ are each independently selected from an integer of 1 to 10;

Wherein, W is selected from the group consisting of $-(OCH_2CH_2)_{n3}O-$, a saturated or unsaturated 3-10 membered heterocyclic hydrocarbon group containing N, and a 5-10 membered N-containing heteroaryl; wherein $n_3$ is selected from an integer of 1 to 10;

Yis .

Further, X is a structure represented by formula (Ib'), Y and Y' is , and the compound has a structure represented by following formula (II):

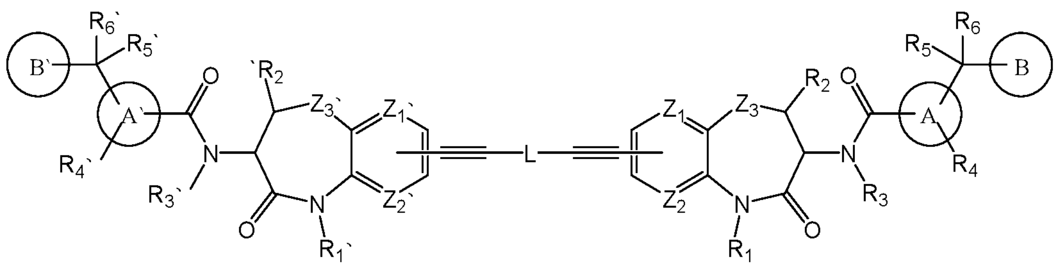

(II)

:

wherein, L is $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2'$, $m_1$, $m_1'$, $m_2$, and $m_2'$ are each independently selected from an integer of 1 to 10;

wherein, W is selected from the group consisting of $-NR_7-$, O, S, 1,3-succinynyl, - $(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (Ia):

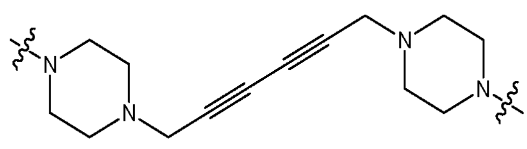

(Ia)

;

wherein $R_7$ is H or $C_1$-$C_3$ alkyl; $n_3$ is selected from an integer of 1 to 10;
wherein, rings A and A' are each independently selected from 3-6 membered unsaturated heterocyclyls;
rings B and B' are each independently selected from a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino, and hydroxyl;
$Z_1$, $Z_2$, $Z_1$' and $Z_2$' are each independently selected from CH or N;
$Z_3$ and $Z_3$' are each independently selected from the group consisting of O, $NR_8$, S or $CR_8R_9$;
$R_1$, $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring; or $R_3$' and $R_4$' are linked to form a ring; or $R_4$' and $R_5$' are linked to form a ring;
$R_8$ and $R_9$ are selected from the group consisting of H or $C_1$-$C_6$ alkyl.

Further, $Z_1$, $Z_2$, $Z_1$', and $Z_2$' are CH; $Z_3$ and $Z_3$' are O;

the compound has the structure as represented by following formula (IIa):

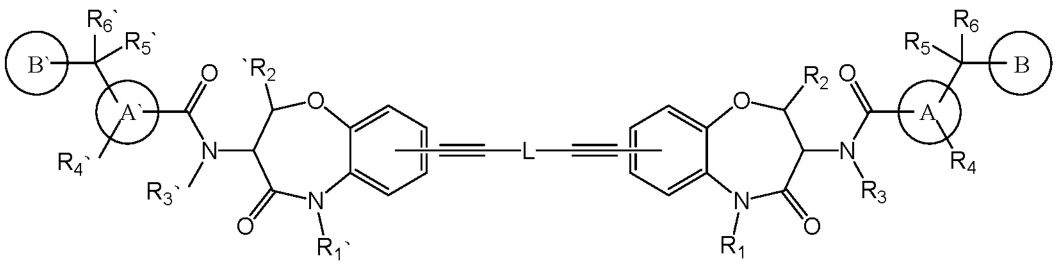

(IIa)

;

wherein, L is selected from the group consisting of $-(CH_2)_{n1}-$, $-(CH_2)_{m1}-W-(CH_2)_{m1'}-$ or $-CH_2-O(CH_2CH_2O)m_2-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;
wherein, W is selected from the group consisting of $-NR_7-$, O, S, 1,3-succinynyl, $-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (Ia):

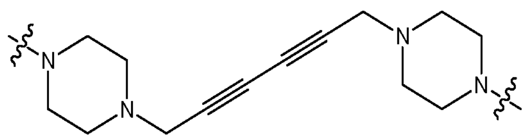

(Ia)

;

wherein $R_7$ is H or $C_1$-$C_3$ alkyl; $n_3$ is selected from an integer of 1 to 4;
rings A and A' are each independently selected from 5-membered unsaturated heterocyclyls; the heteroatoms contained in the heterocyclic group are N, O, or S, and the number of heteroatoms is 1, 2, 3, or 4;
rings B and B' are each independently selected from 5-membered or 6-membered aryl or heteroaryl; the heteroatoms contained in the heteroaryl are N, O, or S, and the number of heteroatoms is 1, 2, 3, or 4;
$R_1$, $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$, $R_6$, $R_5$' and $R_6$' are each independently selected from the group consisting of H, $C_1$-$C_6$ alkyl, $C_1$-$C_6$ deuteroalkyl,

$C_1$-$C_6$ alkoxy, hydroxyl, amino, cyano, or thiol;
or $R_3$ and $R_4$ are linked to form a ring; or $R_4$ and $R_5$ are linked to form a ring; or $R_3$' and $R_4$ are linked to form a ring; or $R_4$' and $R_5$' are linked to form a ring.

Further, $R_3$, $R_3$', $R_4$, $R_4$', $R_5$, $R_5$', $R_6$, and $R_6$' are H; rings B and B' are phenyl; the compound has the structure as represented by following formula (IIb):

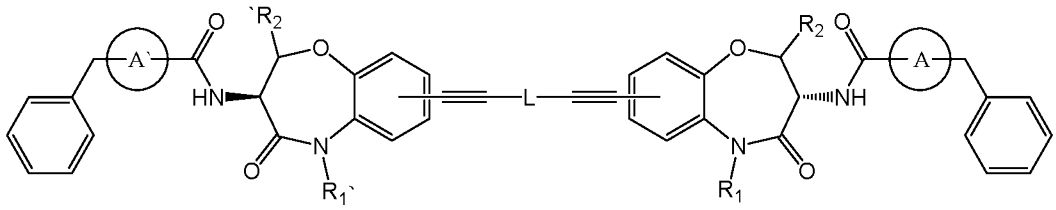

(IIb)

;

wherein, L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;
wherein, W is selected from the group consisting of imino, $-N(CH_3)-$, 1,3-succinynyl, $-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (Ia):

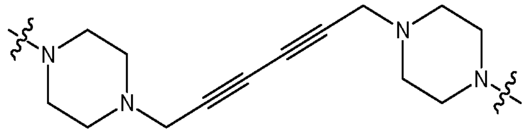

(Ia)

;

wherein, $n_3$ is selected from an integer of 1 to 4;
rings A and A' are each independently selected from the group consisting of

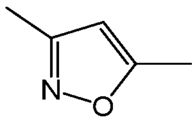

,

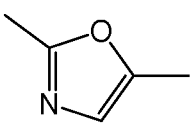

,

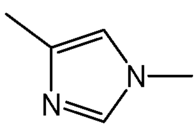

,

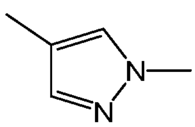

,

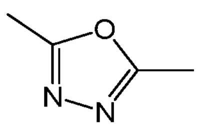

,

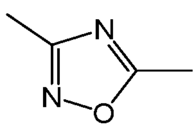

,

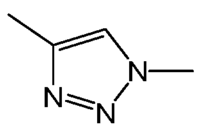

,

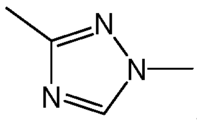

,

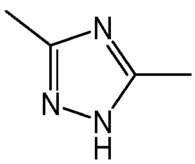

or

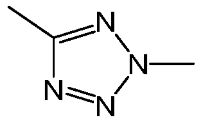

;

$R_1$, $R_2$, $R_1$' and $R_2$' are each independently selected from the group consisting of H, $C_1$-$C_6$ alkyl or $C_1$-$C_6$ deuteroalkyl.

Further, L is the following structures:

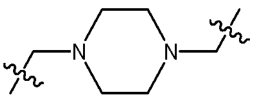

,

$-CH_2-(OCH_2CH_2)_{n3}O-CH_2-$,

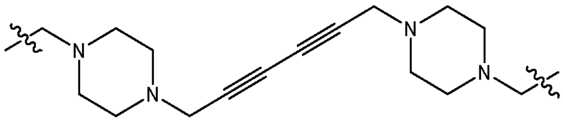

or

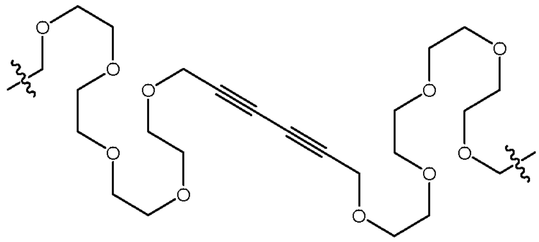

,

wherein, $n_3$ is selected from an integer of 1 to 4.

Further, X is a structure represented by formula (Ib'), Y and Y' are $CH_2$, and the compound has a structure represented by following formula (III):

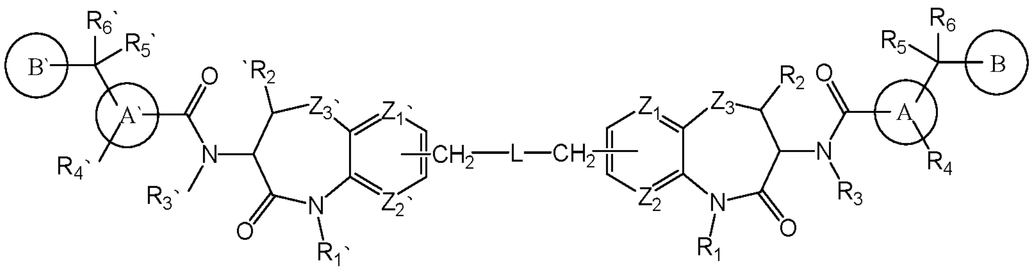

(III)

;

wherein, L is selected from the group consisting of $-(CH_2)_{m1}-W'-(CH_2)_{m1'}-$ or $-CH_2-O(CH_2CH_2O)_{m2}-(CH_2)_{n2}-W'-(CH_2)_{n2'}-(OCH_2CH_2)_{m2'}O-CH_2-$; $n_2$, $n_2'$, $m_1$, $m_1'$, $m_2$, and $m_2'$ are each independently selected from an integer of 1 to 10;

wherein, W' is selected from the group consisting of $-(CH_2)_{n4}-$, $-NR_7-$, O, S, 1,3-succinynyl, $-(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (IIIc):

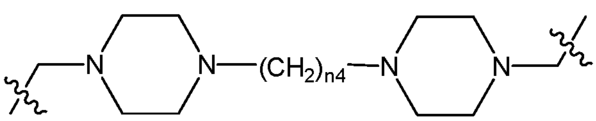

(IIIc)

;

wherein $R_7$ is H or $C_1$-$C_3$ alkyl; $n_3$ is selected from an integer of 1 to 10; $n_4$ is selected from an integer of 1 to 6;

wherein, rings A and A' are each independently selected from 3-6 membered unsaturated heterocyclyls;

rings B and B' are each independently selected from a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered

heteroaryl; the substituted substituents are halogen, alkyl, cyano, amino, and hydroxyl;

$Z_1$, $Z_2$, $Z_1$' and $Z_2$' are each independently selected from CH or N;

$Z_3$ and $Z_3$' are each independently selected from the group consisting of O, $NR_8$, S or $CR_8R_9$;

$R_1$, $R_2$, $R_1$' and $R_2$' are each independently selected from the group consisting of H, $C_1$-$C_6$ alkyl or $C_1$-$C_6$ deuteroalkyl;

$R_3$ and $R_3$' are each independently selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

or $R_3$ and $R_4$ are linked to form a ring; or $R_4$ and $R_5$ are linked to form a ring; or $R_3$' and $R_4$' are linked to form a ring; or $R_4$' and $R_5$' are linked to form a ring;

$R_8$ and $R_9$ are selected from the group consisting of H or $C_1$-$C_6$ alkyl.

Further, $Z_1$, $Z_2$, $Z_1$', and $Z_2$' are CH; $Z_3$ and $Z_3$' are O;

the compound has the structure as represented by following formula (IIIa):

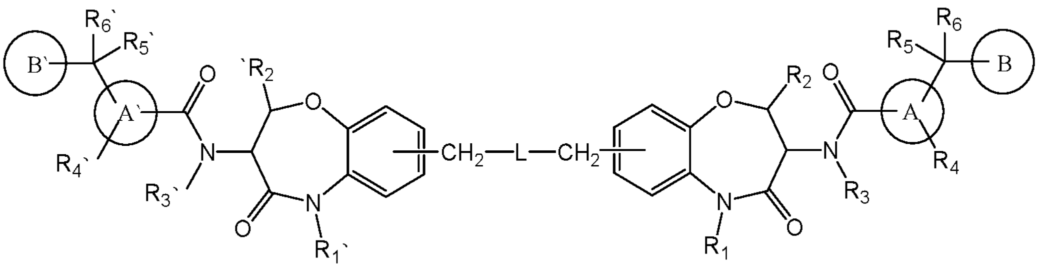

(IIIa) ;

wherein, L is selected from the group consisting of $-(CH_2)_{m1}-W'-(CH_2)_{m1'}-$ or $-CH_2-O(CH_2CH_2O)_{m2}-(CH_2)_{n2}-W'-(CH_2)_{n2'}-(OCH_2CH_2)_{m2'}O-CH_2-$; $n_2$, $n_2$', $m_1$, $m_1$', $m_2$, and $m_2$' are each independently selected from an integer of 1 to 10;

wherein, W' is selected from the group consisting of $-(CH_2)_{n4}-$, $-NR_7-$, O, S, 1,3-succinynyl, $-(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (IIIc):

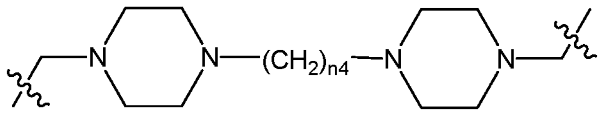

(IIIc) ;

wherein $R_7$ is H or $C_1$-$C_3$ alkyl; $n_3$ is selected from an integer of 1 to 10; $n_4$ is selected from an integer of 1 to 6;

wherein, rings A and A' are each independently selected from 5-membered unsaturated heterocyclyls; the heteroatoms contained in the heterocyclic group are N, O, or S, and the number of heteroatoms is 1, 2, 3, or 4;

rings B and B' are each independently selected from 5-membered or 6-membered aryl or heteroaryl; the heteroatoms contained in the heteroaryl are N, O, or S, and the number of heteroatoms is 1, 2, 3, or 4;

$R_1$, $R_2$, $R_1$' and $R_2$' are each independently selected from the group consisting of H, $C_1$-$C_6$ alkyl or $C_1$-$C_6$ deuteroalkyl;

$R_3$ and $R_3$' are each independently selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

or $R_3$ and $R_4$ are linked to form a ring; or $R_4$ and $R_5$ are linked to form a ring; or $R_3$' and $R_4$' are linked to form a ring; or $R_4$ and $R_5$' are linked to form a ring.

Further, $R_3$, $R_3$', $R_4$, $R_4$', $R_5$, $R_5$', $R_6$, and $R_6$' are H; rings B and B' are phenyl; the compound has the structure

as represented by following formula (IIIb):

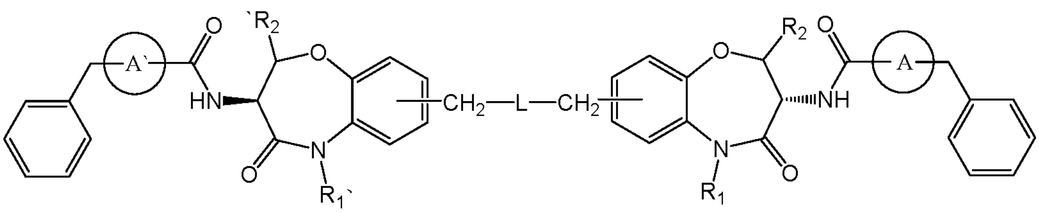

(IIIb)

;

wherein, L is selected from the group consisting of $-(CH_2)_{m1}-W'-(CH_2)_{m1'}-$ or $-CH_2-O(CH_2CH_2O)_{m2}-(CH_2)_{n2}-W'-(CH_2)_{n2'}-(OCH_2CH_2)_{m2'}O-CH_2-$; $n_2$, $n_2'$, $m_1$, $m_1'$, $m_2$, and $m_2'$ are each independently selected from an integer of 1 to 10;

wherein, W is selected from the group consisting of $-(CH_2)_{n4}-$, imino, $-N(CH_3)-$, 1,3-succinynyl, $-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (IIIc):

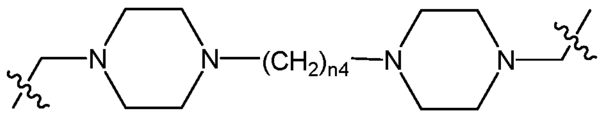

(IIIc)

;

$n_3$ is selected from an integer of 1 to 4; $n_4$ is selected from an integer of 1 to 6;

rings A and A' are each independently selected from the group consisting of:

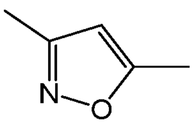 , 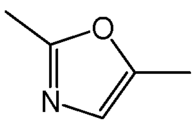 , 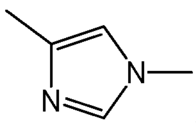 , 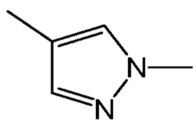 , 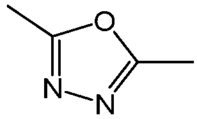 ,

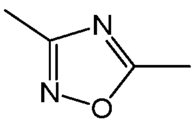 , 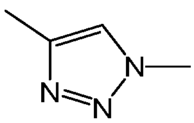 , 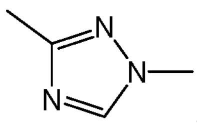 , 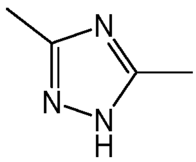 or 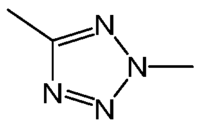 ;

$R_1$, $R_2$, $R_1'$ and $R_2'$ are each independently selected from the group consisting of H, $C_1$-$C_6$ alkyl or $C_1$-$C_6$ deuteroalkyl.

Further, L is the following structures:

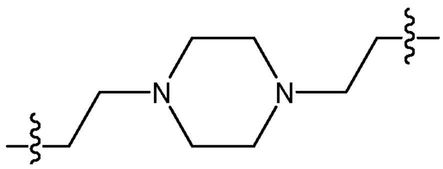

,

$-CH_2-(OCH_2CH_2)_{n4}O-CH_2-$,

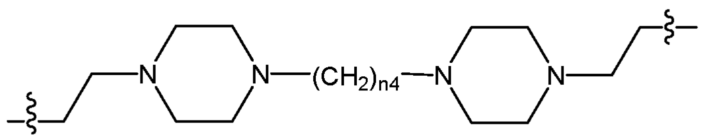

or

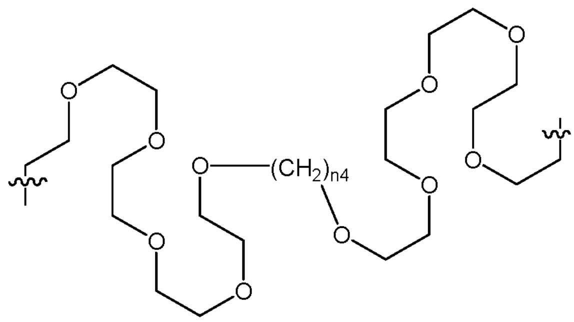

;

wherein, $n_4$ is selected from an integer of 1 to 6.

Further, X is not a structure represented by formula (Ib'), $R_2$ is methyl, Y is $\equiv$, and the compound has a structure represented by following formula (IV):

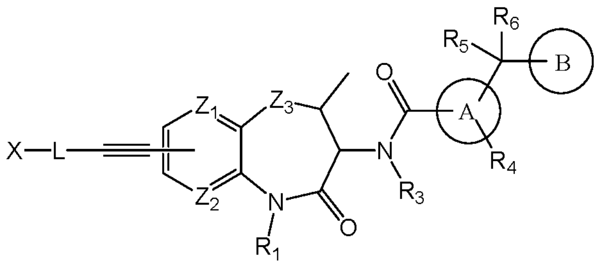

(IV)

;

wherein, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cyclic hydrocarbon group, a substituted or unsubstituted, saturated or unsaturated 3-10 membered heterocyclic hydrocarbon group, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;

L is absent or L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2'$, $m_1$, $m_1'$, $m_2$, and $m_2'$ are each independently selected from an integer of 1 to 10;

wherein, W is selected from the group consisting of $-NR_7-$, O, S, 1,3-succinynyl, $-(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a 5-10 membered aryl, and a 5-10 membered heteroaryl;

wherein, $R_7$ is selected from H or $C_1$-$C_3$ alkyl; $n_3$ is selected from an integer of 1 to 10;

ring A is a 3-6 membered unsaturated heterocyclyl;

ring B is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino, and hydroxyl;

$Z_1$ and $Z_2$ are each independently selected from CH or N;

$Z_3$ is selected from the group consisting of O, $NR_8$, S or $CR_8R_9$;

$R_1$ is selected from the group consisting of hydrogen, $C_1$-$C_6$ alkyl or $C_1$-$C_6$ deuteroalkyl;

$R_3$ is selected from the group consisting of hydrogen, $C_1$-$C_6$ alkyl or $C_1$-$C_6$ deuteroalkyl; $R_4$ is selected from the

group consisting of hydrogen, $C_1$-$C_6$ alkyl, $C_1$-$C_6$ deuteroalkyl, $C_1$-$C_6$ alkoxy, hydroxyl, amino, cyano, or thiol;

$R_5$ and $R_6$ are each independently selected from the group consisting of hydrogen, $C_1$-$C_6$ alkyl, $C_1$-$C_6$ deuteroalkyl, $C_1$-$C_6$ alkoxy, hydroxyl, amino, cyano, or thiol;

or $R_3$ and $R_4$ are linked to form a ring; or $R_4$ and $R_5$ are linked to form a ring;

$R_8$, $R_9$ and $R_{10}$ are each independently selected from the group consisting of H, $C_1$-$C_6$ alkyl, amino or hydroxyl.

Further, $Z_1$ and $Z_2$ are CH, $Z_3$ is O; the compound has a structure as represented by following formula (IVa):

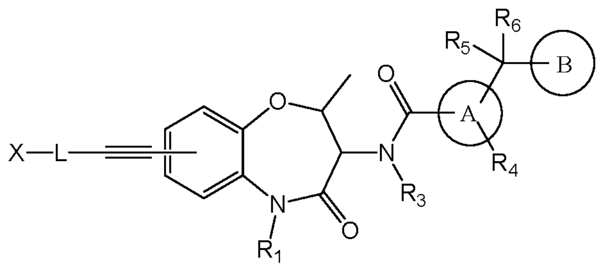

(IVa) .

wherein, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cyclic hydrocarbon group, a substituted or unsubstituted, saturated or unsaturated 3-10 membered heterocyclic hydrocarbon group, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;

L is absent or L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;

wherein, W is selected from the group consisting of $-NR_7-$, O, S, 1,3-succinynyl, - $(OCH_2CH_2)_{n3}O-$, a saturated or unsaturated $C_4$-$C_6$ cyclic hydrocarbon group, a saturated or unsaturated 4-6 membered heterocyclic hydrocarbon group, a 5-10 membered aryl, and a 5-10 membered heteroaryl;

wherein, $R_7$ is selected from H or $C_1$-$C_3$ alkyl; $n_3$ is selected from an integer of 1 to 4;

ring A is selected from 5-membered unsaturated heterocyclyl; the heteroatoms contained in the heterocyclic group are N, O, or S, and the number of heteroatoms is 1, 2, 3, or 4;

ring B is selected from 5-membered or 6-membered substituted aryl or heteroaryl; the heteroatoms contained in the heteroaryl are N, O, or S, and the number of heteroatoms is 1, 2, 3, or 4; the substituted substituents are selected from the group consisting of halogen, alkyl, cyano, amino, and hydroxyl;

$R_1$ is selected from the group consisting of H, $C_1$-$C_6$ alkyl, and $C_1$-$C_6$ deuteroalkyl;

$R_3$ is selected from the group consisting of H, $C_1$-$C_6$ alkyl or $C_1$-$C_6$ deuteroalkyl; $R_4$ is selected from the group consisting of H, $C_1$-$C_6$ alkyl, $C_1$-$C_6$ deuteroalkyl, $C_1$-$C_6$ alkoxy, hydroxyl, amino, cyano, or thiol;

$R_5$ and $R_6$ are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

or $R_3$ and $R_4$ are linked to form a ring; or $R_4$ and $R_5$ are linked to form a ring;

$R_8$, $R_9$ and $R_{10}$ are each independently selected from the group consisting of H, $C_1$-$C_6$ alkyl, amino or hydroxyl.

Further, $R_3$, $R_4$, $R_5$, and $R_6$ are H; ring B is phenyl; the compound has a structure as represented by following formula (IVb):

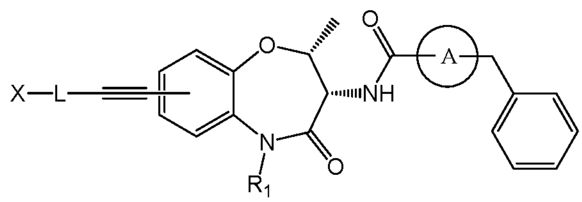

(IVb) ;

wherein, X is selected from the group consisting of H, vinyl, ethynyl, hydroxyl, thiol, aldehyde, carboxyl, sulfo group,

$-NR_8R_9$, $-CR_8R_9R_{10}$, a substituted or unsubstituted, saturated or unsaturated $C_3$-$C_{10}$ cyclic hydrocarbon group, a substituted or unsubstituted, saturated or unsaturated 3-10 membered heterocyclic hydrocarbon group, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;

L is absent or L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;

wherein, W is selected from the group consisting of imino, $-N(CH_3)-$, 1,3-succinynyl, - $(OCH_2CH_2)_{n3}O-$, a saturated or unsaturated $C_4$-$C_6$ cyclic hydrocarbon group, a saturated or unsaturated 4-6 membered heterocyclic hydrocarbon group, a 5-10 membered aryl, and a 5-10 membered heteroaryl;

wherein $n_3$ is selected from an integer of 1 to 4;

ring A is selected from the group consisting of

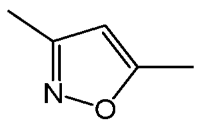 , 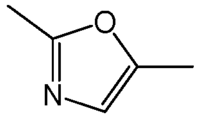 ,

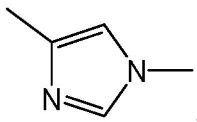 , 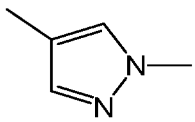 , 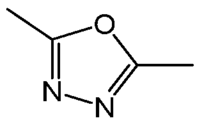 , 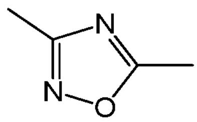 , 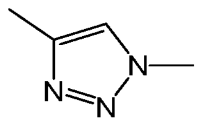 , 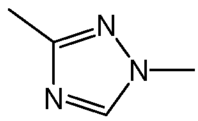 ,

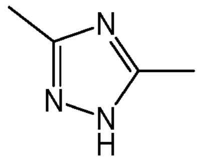 or 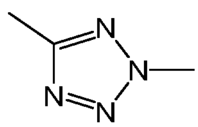 ;

$R_1$ is selected from the group consisting of H, $C_1$-$C_6$ alkyl or $C_1$-$C_6$ deuteroalkyl;

$R_8$, $R_9$ and $R_{10}$ are each independently selected from the group consisting of H, $C_1$-$C_6$ alkyl, amino or hydroxyl.

Further, X is not a structure represented by formula (Ib'), $R_2$ is H, Y is ≡, and the compound has a structure represented by following formula (V):

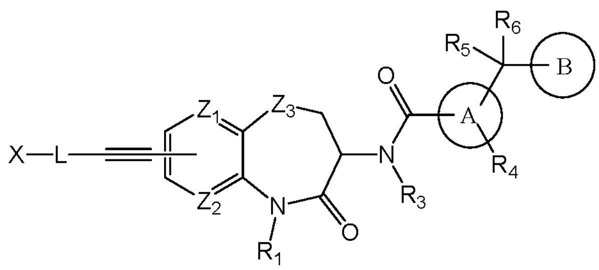

(V) ;

wherein, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cyclic hydrocarbon group, a substituted or unsubstituted, saturated or unsaturated 3-10 membered heterocyclic hydrocarbon group, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;

L is selected from the group consisting of $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_2$, $n_2$', $m_1$, $m_1$', $m_2$, and $m_2$' are each independ-

ently selected from an integer of 1 to 10;
wherein, W is selected from the group consisting of $-(OCH_2CH_2)_{n3}O-$, a saturated or unsaturated 3-10 membered heterocyclic hydrocarbon group containing N, or a 5-10 membered N-containing heteroaryl;
wherein $n_3$ is selected from an integer of 1 to 10;
ring A is a 3-6 membered unsaturated heterocyclyl;
ring B is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are alkyl, cyano, amino, and hydroxyl;
$Z_1$ and $Z_2$ are each independently selected from CH or N;
$Z_3$ is selected from the group consisting of O, $NR_8$, S or $CR_8R_9$;
$R_1$ is selected from the group consisting of hydrogen, $C_1$-$C_6$ alkyl or $C_1$-$C_6$ deuteroalkyl;
$R_3$ is selected from the group consisting of H, $C_1$-$C_6$ alkyl or $C_1$-$C_6$ deuteroalkyl; $R_4$ is selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$ and $R_6$ are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring;
$R_8$, $R_9$ and $R_{10}$ are each independently selected from the group consisting of H, $C_1$-$C_6$ alkyl, amino or hydroxyl.

Further, $Z_1$ and $Z_2$ are CH, $Z_3$ is O; the compound has a structure as represented by following formula (Va):

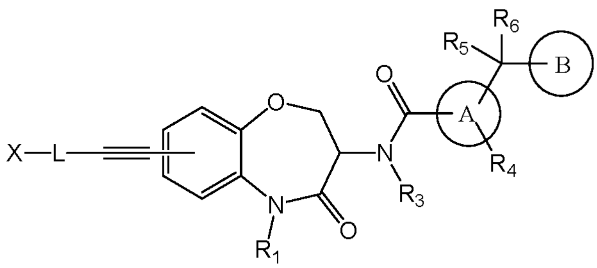

(Va) ;

wherein, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cycloalkyl, a substituted or unsubstituted, saturated or unsaturated 3-10 membered heterocycloalkyl, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;
L is selected from the group consisting of $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_2$, $n_2$', $m_1$, $m_1$', $m_2$, and $m_2$' are each independently selected from an integer of 1 to 4;
wherein, W is selected from the group consisting of $-(OCH_2CH_2)_{n3}O-$, a saturated or unsaturated 4-6 membered N-containing heterocycloalkyl, or a 5-10 membered N-containing heteroaryl;
$n_3$ is selected from an integer of 1 to 4;
ring A is selected from 5-membered unsaturated heterocyclyl; the heteroatoms contained in the heterocyclic group are N, O, or S, and the number of heteroatoms is 1, 2, 3, or 4;
ring B is selected from 5-membered or 6-membered substituted aryl or heteroaryl; the heteroatoms contained in the heteroaryl are N, O, or S, and the number of heteroatoms is 1, 2, 3, or 4; the substituted substituents are selected from the group consisting of halogen, alkyl, cyano, amino, and hydroxyl;
$R_1$ is selected from the group consisting of H, $C_1$-$C_6$ alkyl or $C_1$-$C_6$ deuteroalkyl;
$R_3$ is selected from the group consisting of H, $C_1$-$C_6$ alkyl or $C_1$-$C_6$ deuteroalkyl; $R_4$ is selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$ and $R_6$ are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring;
$R_8$, $R_9$ and $R_{10}$ are each independently selected from the group consisting of H, $C_1$-$C_6$ alkyl, amino or hydroxyl.

Further, $R_3$, $R_4$, $R_5$, and $R_6$ are H; ring B is phenyl; the compound has a structure as represented by following

formula (Vb):

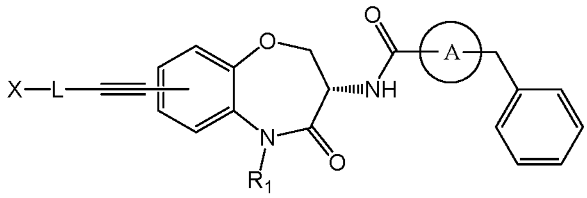

(Vb) ;

wherein, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cycloalkyl, a substituted or unsubstituted, saturated or unsaturated 3-10 membered heterocycloalkyl, a substituted or unsubstituted $C_5$-$C_{10}$ aryl, a substituted or unsubstituted 5-10 membered heteroaryl or halogen; the substituted substituents are preferably selected from the group consisting of alkyl, cyano, amino, hydroxyl, nitro, and sulfo group;

L is selected from the group consisting of $-(CH_2)_{m1}$-W-$(CH_2)_{m1'}$- or $-CH_2-O(CH_2CH_2O)_{m2}-(CH_2)_{n2}$-W-$(CH_2)_{n2'}-(OCH_2CH_2)_{m2'}O-CH_2-$; $n_2$, $n_2'$, $m_1$, $m_1'$, $m_2$, and $m_2'$ are each independently selected from an integer of 1 to 4;

wherein, W is selected from the group consisting of $-(OCH_2CH_2)_{n3}O-$, a saturated or unsaturated 4-6 membered N-containing heterocycloalkyl, and a 5-10 membered N-containing heteroaryl;

$n_3$ is selected from an integer of 1 to 4;

ring A is selected from the group consisting of

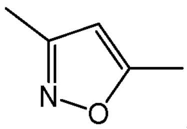 , 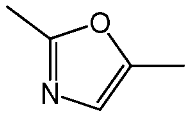 , 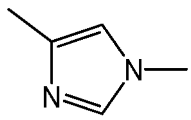

, 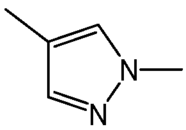 , 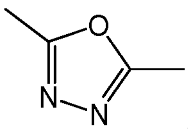 , 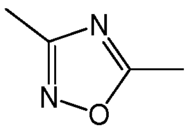 , 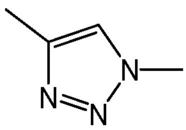 , 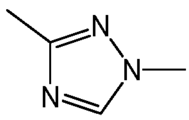 , 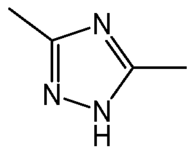

or

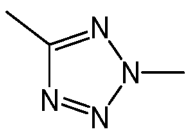 ;

$R_1$ is selected from the group consisting of H, $C_1$-$C_6$ alkyl or $C_1$-$C_6$ deuteroalkyl;

$R_8$, $R_9$ and $R_{10}$ are each independently selected from the group consisting of H, $C_1$-$C_6$ alkyl, amino or hydroxyl.

Further, the salt is hydrochloride, sulfate, citrate, benzenesulfonate, hydrobromate, hydrofluorate, phosphate, acetate, propionate, succinate, oxalate, lactate, malate, succinate, fumarate, maleate, tartrate, trifluoroacetate, sodium salt or potassium salt.

Further, the compound is one of the following compounds:

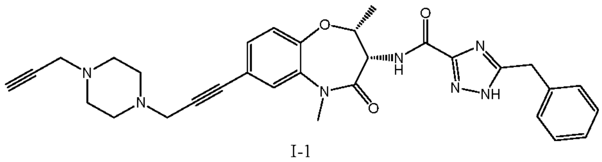

I-1

,

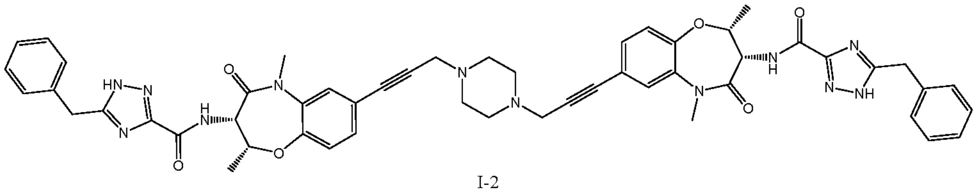

I-2

,

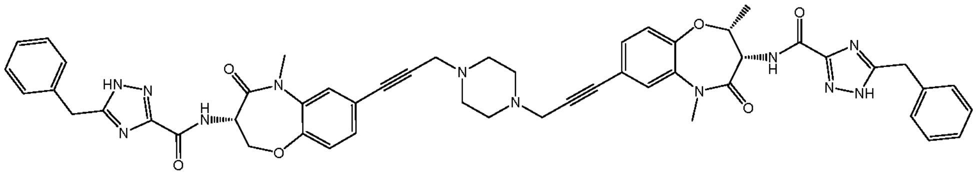

I-3

,

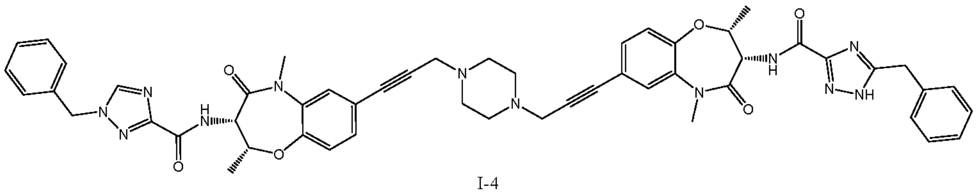

I-4

,

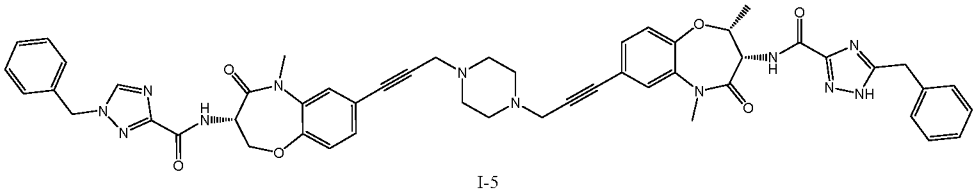

I-5

,

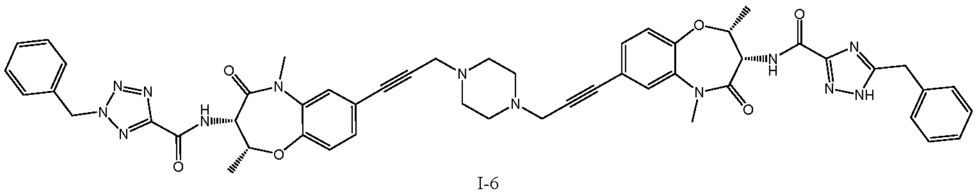

I-6

,

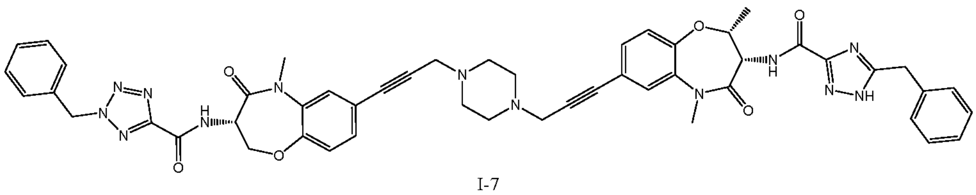

I-7

,

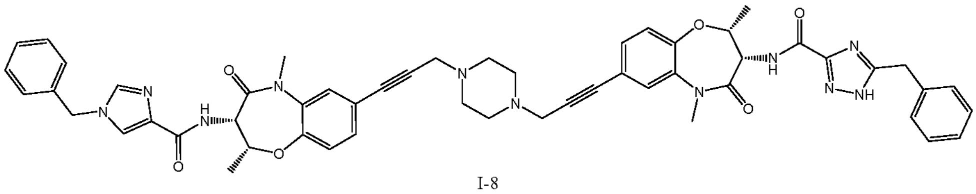

I-8

,

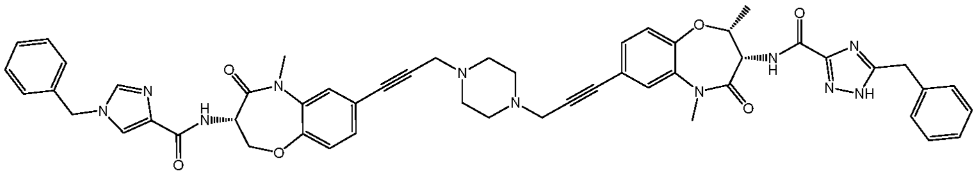

I-9

,

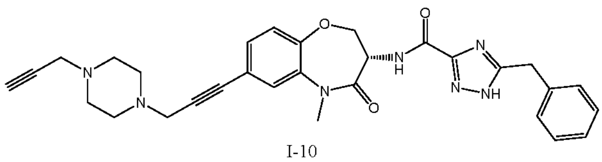

I-10

,

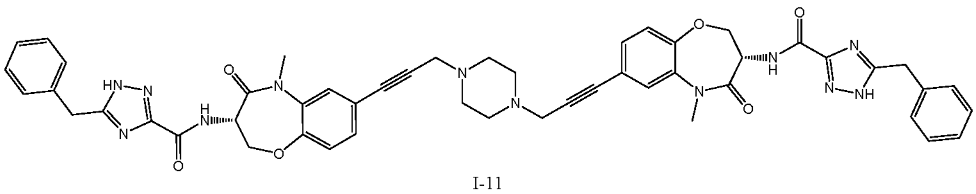

I-11

,

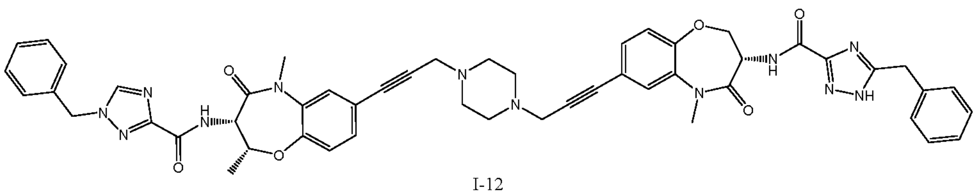

I-12

,

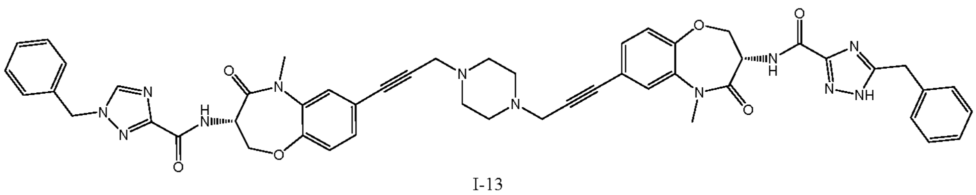

I-13

,

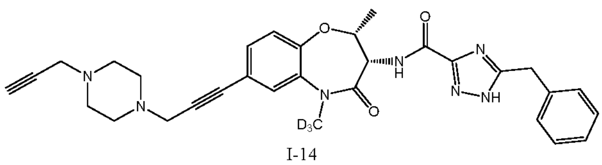

I-14

,

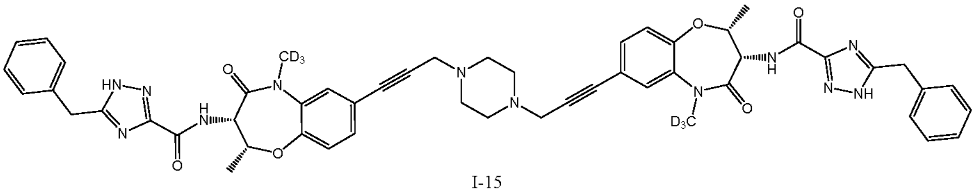

I-15

,

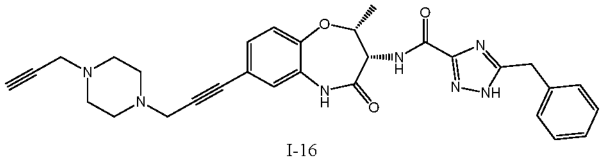

I-16

,

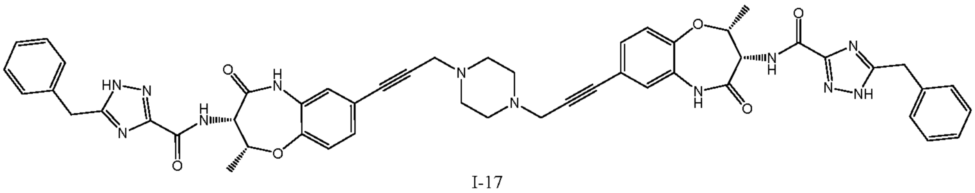

I-17

,

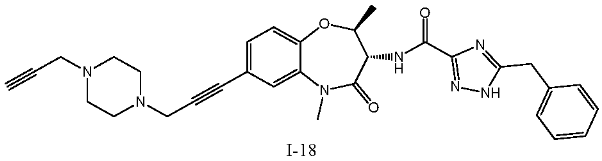

I-18

,

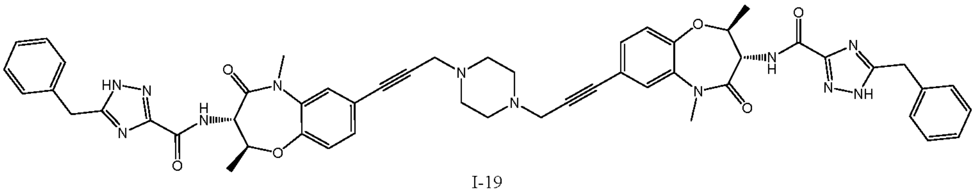

I-19

,

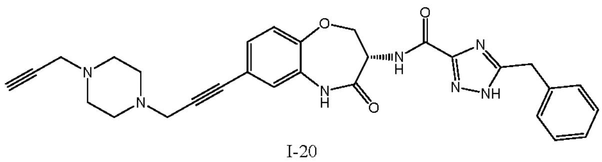

I-20

,

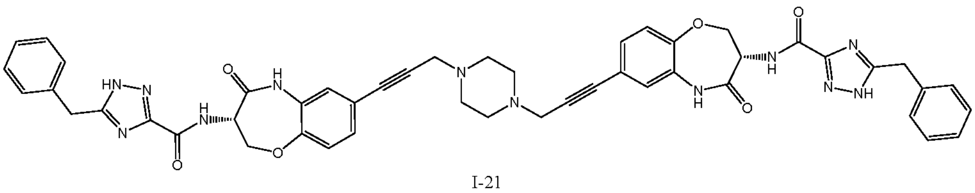

I-21

,

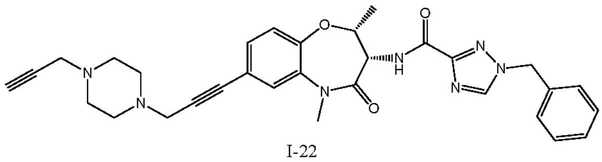

I-22

,

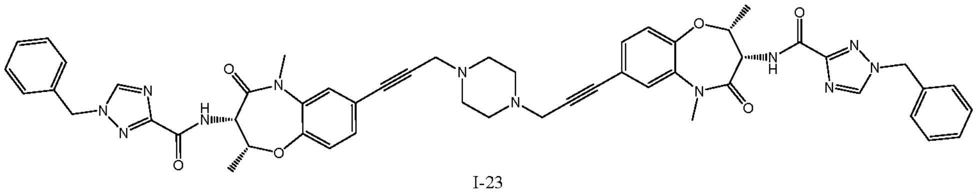

I-23

,

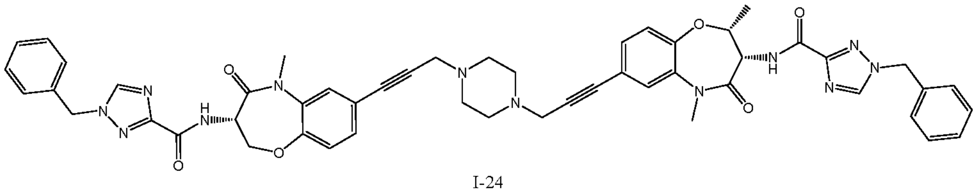

I-24

,

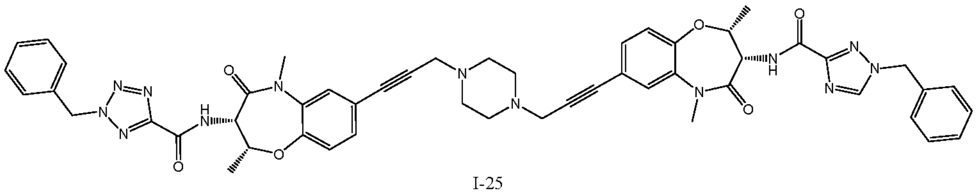

I-25

,

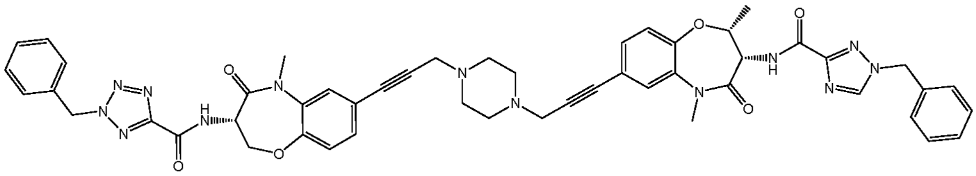

I-26

,

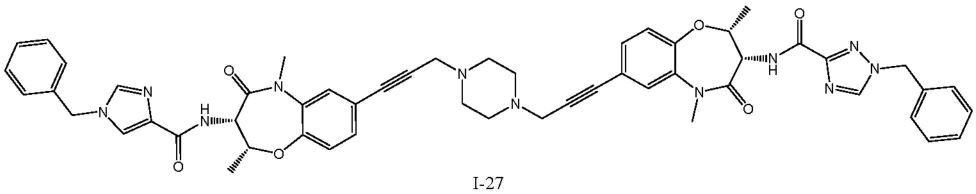

I-27

,

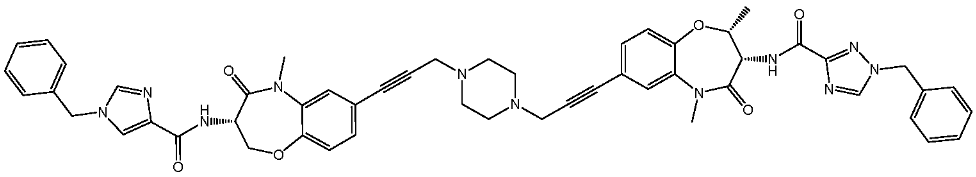

I-28

,

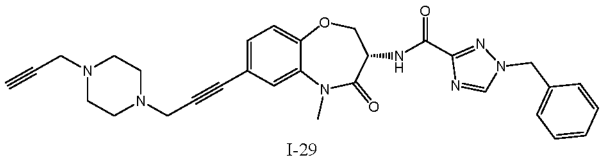

I-29

,

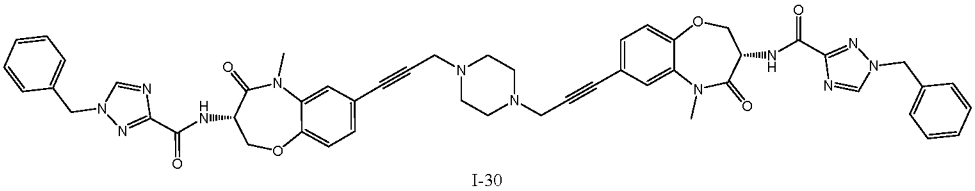

I-30

,

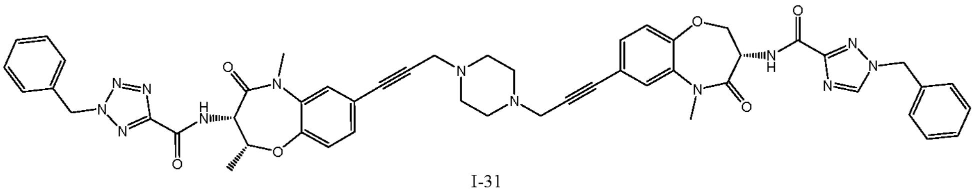

I-31

,

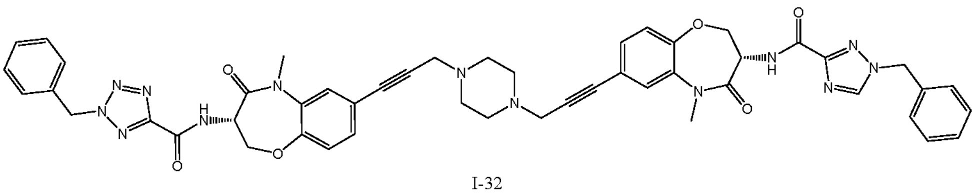

I-32

,

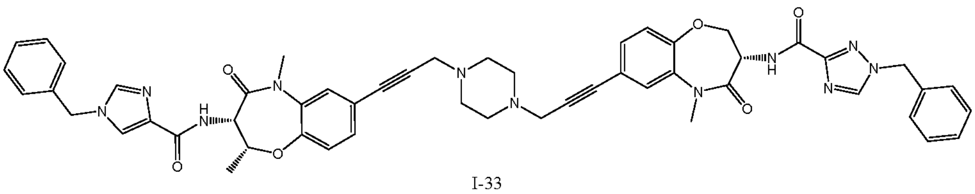

I-33

,

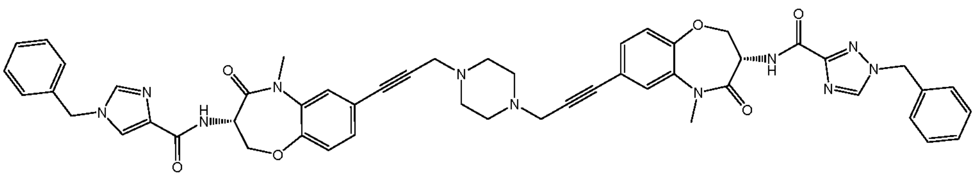

I-34

,

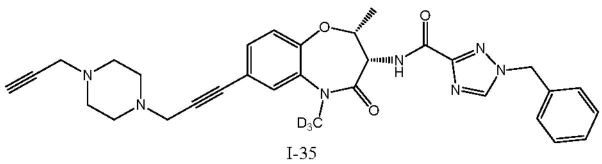

I-35

,

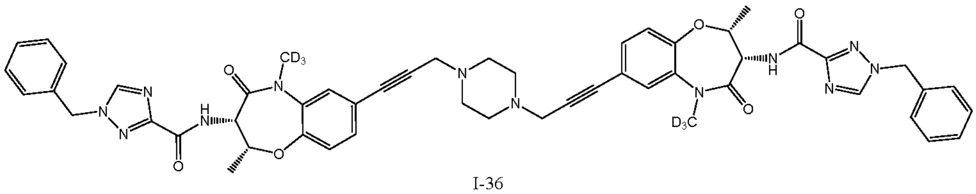

I-36

,

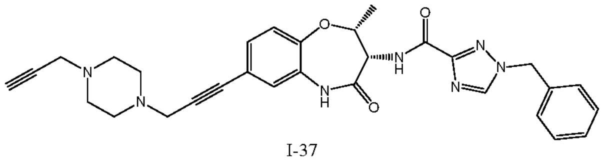

I-37

,

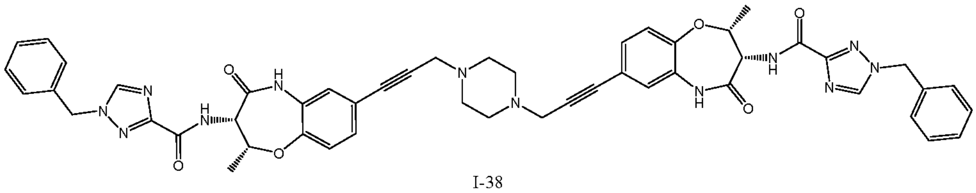

I-38

,

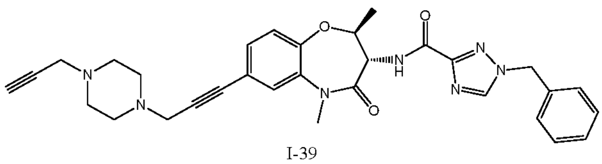

I-39

,

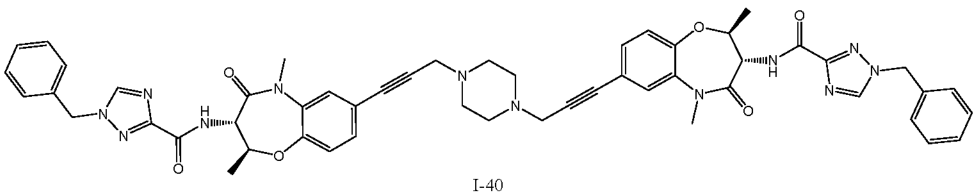

I-40

,

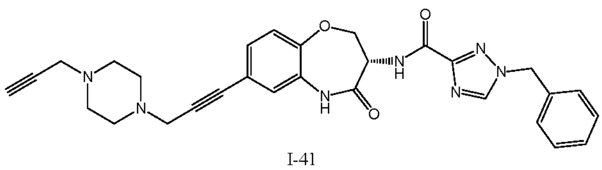

I-41

,

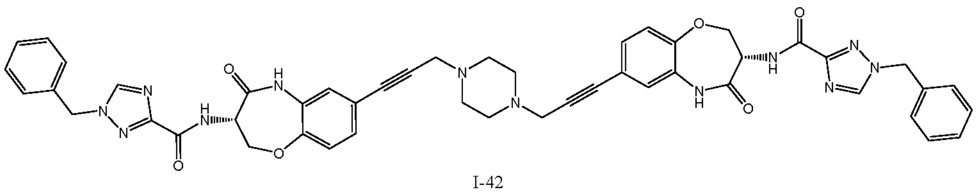

I-42

,

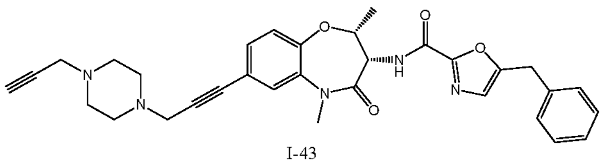

I-43

,

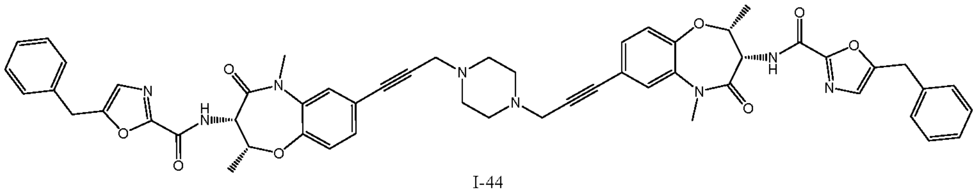

I-44

,

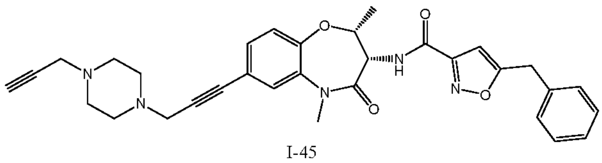

I-45

,

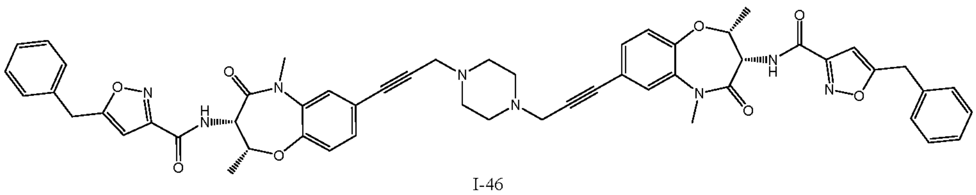

I-46

,

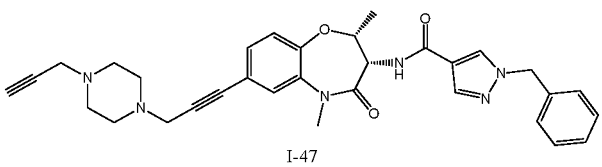

I-47

,

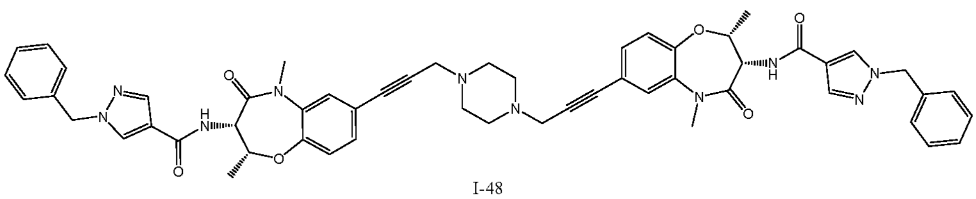

I-48

,

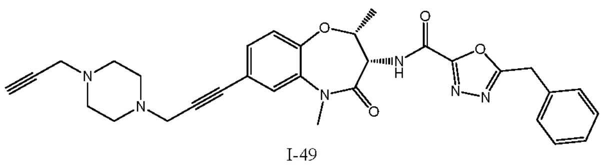

I-49

,

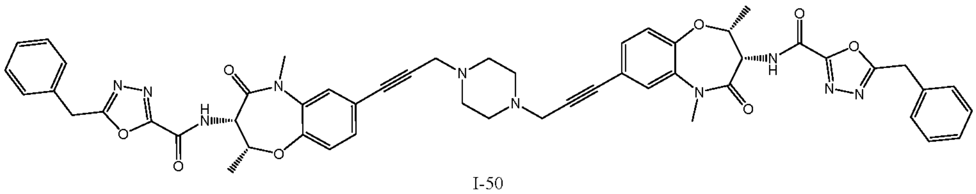

I-50

,

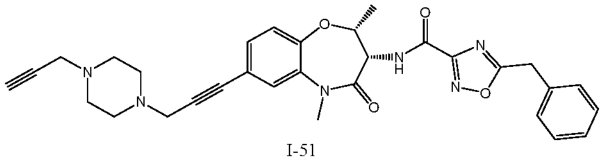

I-51

,

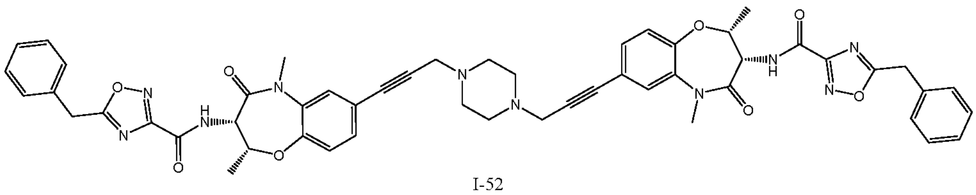

I-52

,

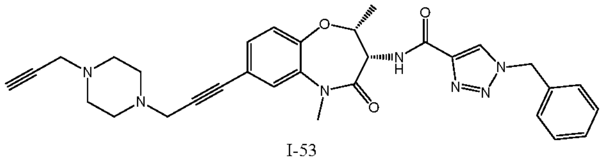

I-53

,

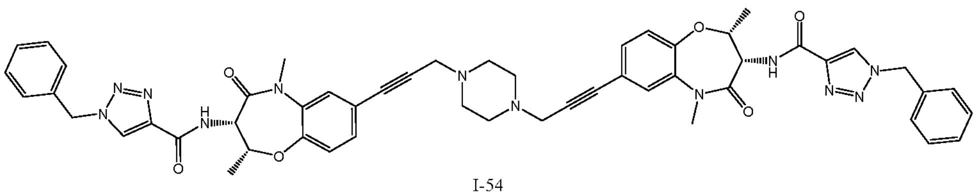

I-54

,

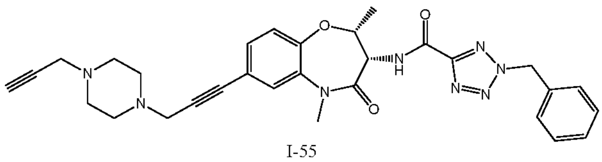

I-55

,

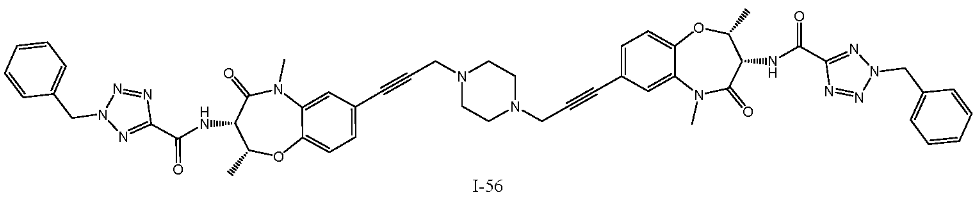

I-56

,

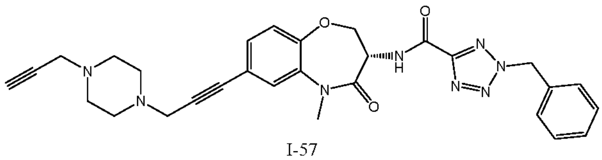

I-57

,

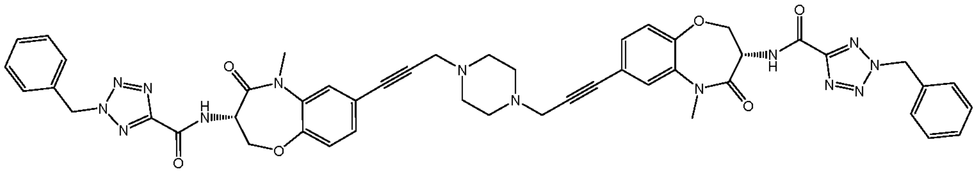

I-58

,

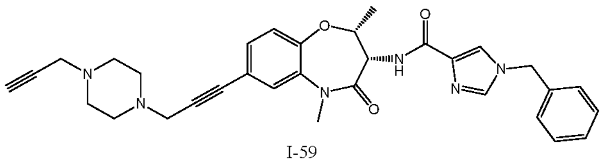

I-59

,

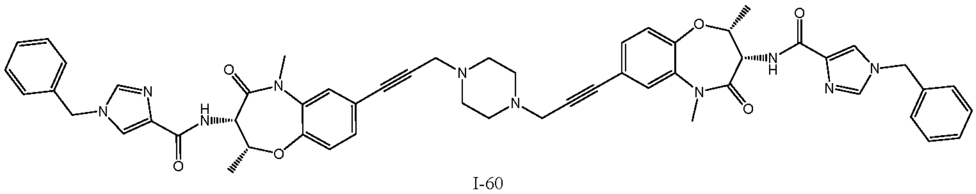

I-60

,

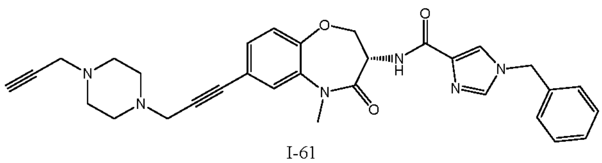

I-61

,

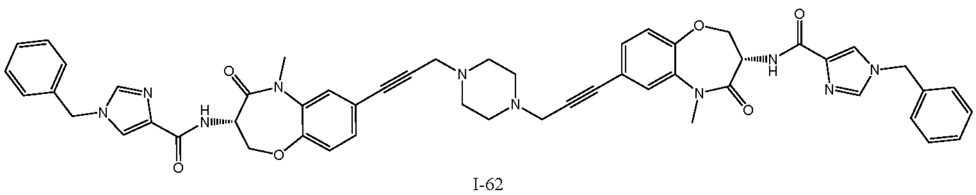

I-62

,

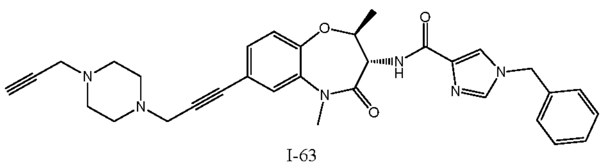

I-63

,

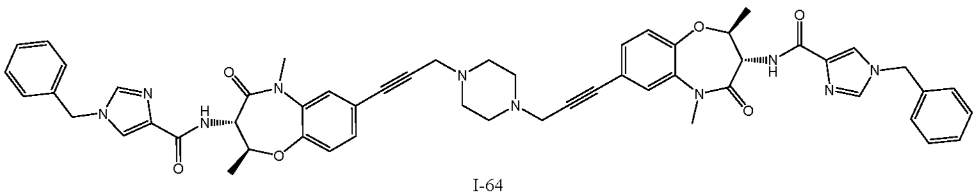

I-64

,

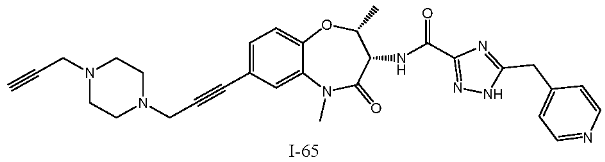

I-65

,

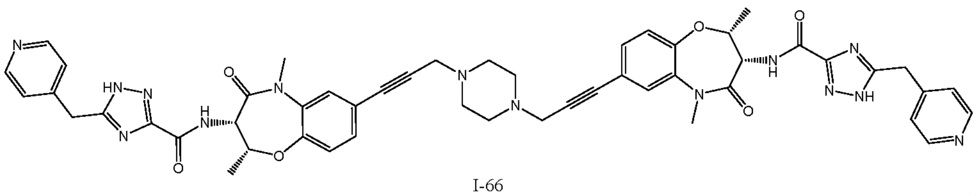

I-66

,

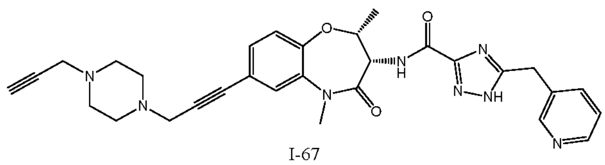

I-67

,

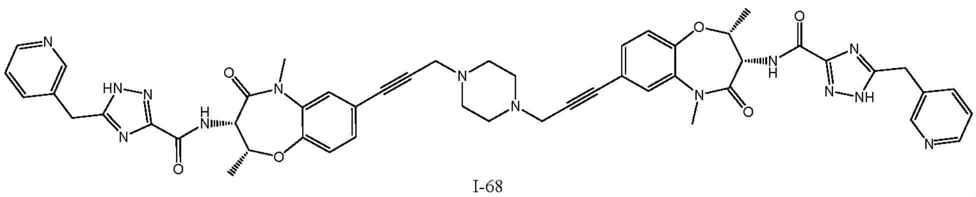

I-68

,

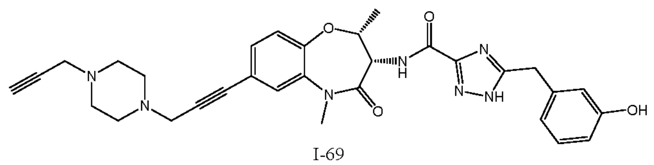

I-69

,

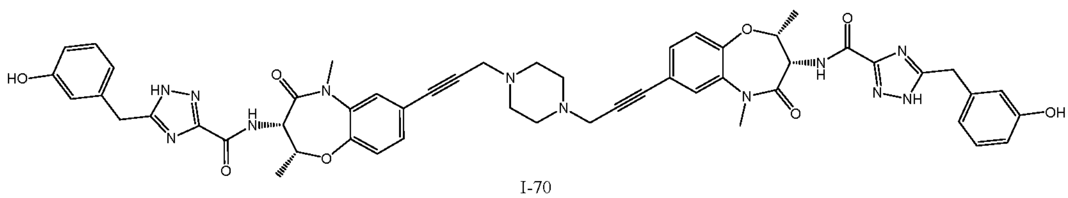

I-70

,

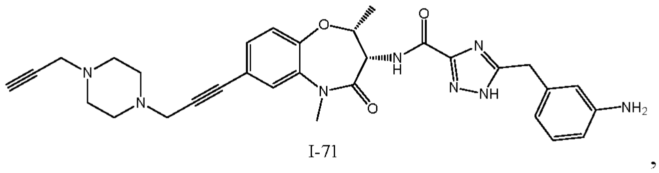

I-71

,

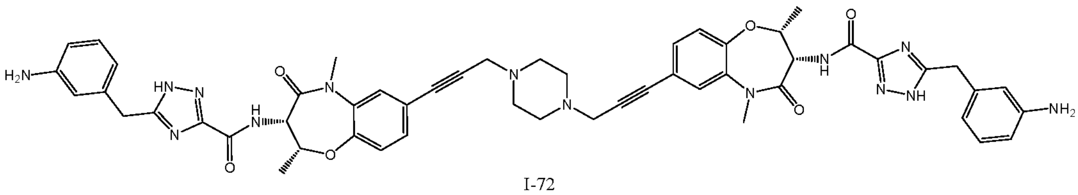

I-72

,

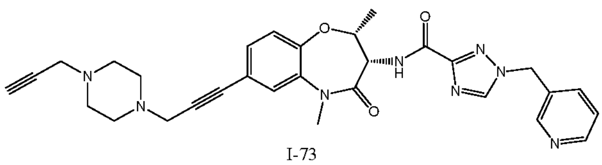

I-73

,

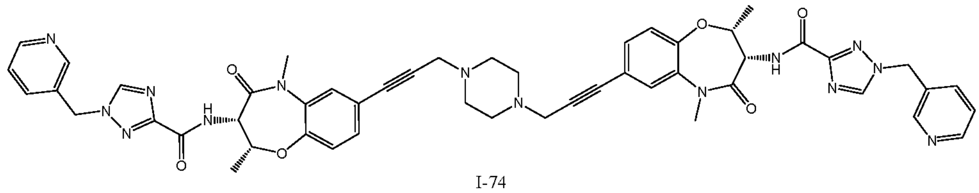

I-74

,

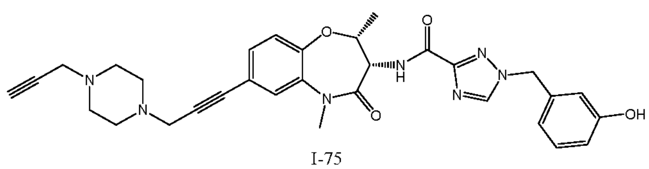

I-75

,

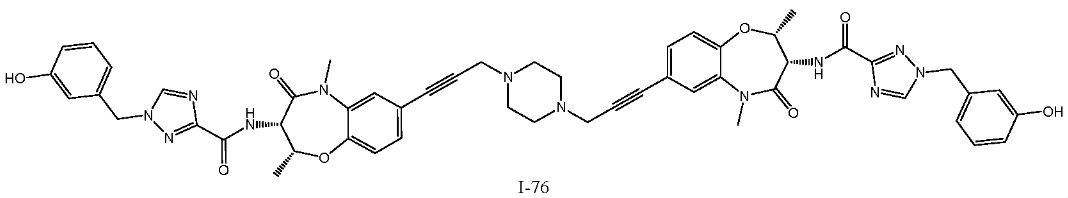

I-76

,

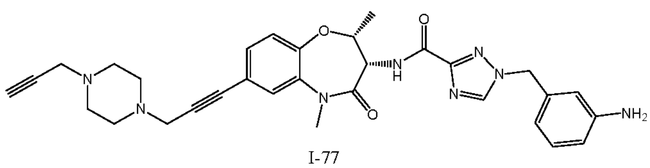

I-77

,

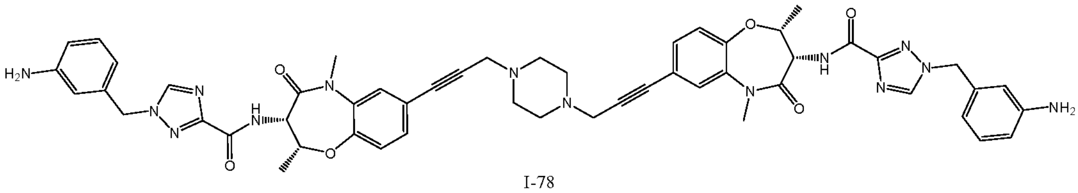

I-78

,

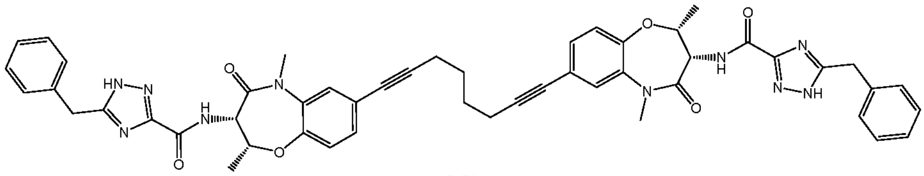

I-79

,

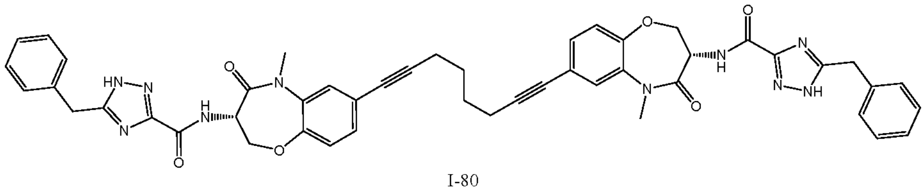

I-80

,

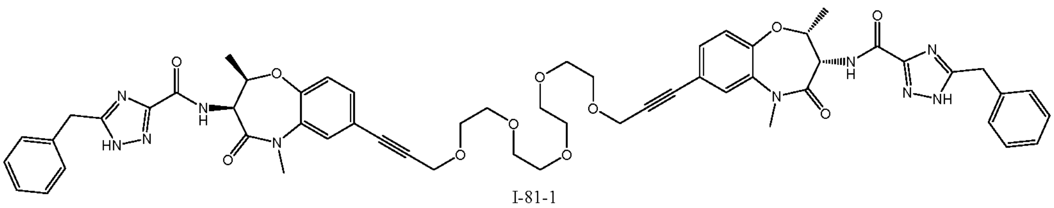

I-81-1

,

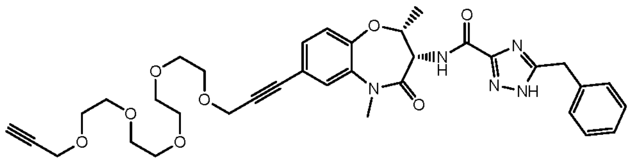

I-81-2

,

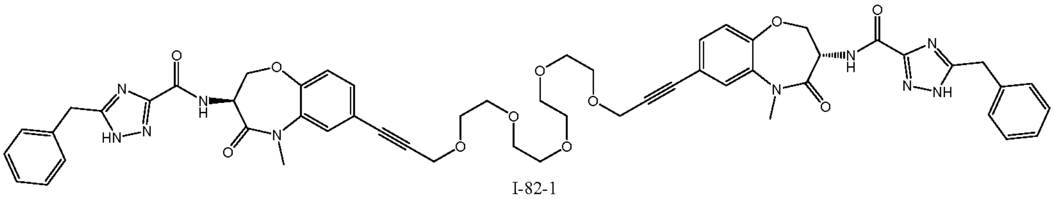

I-82-1

,

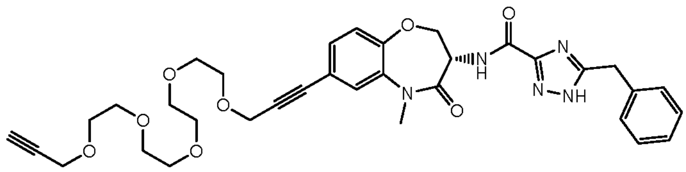

I-82-2

,

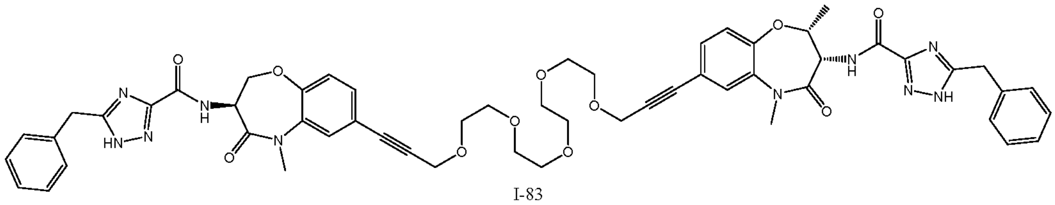

I-83

,

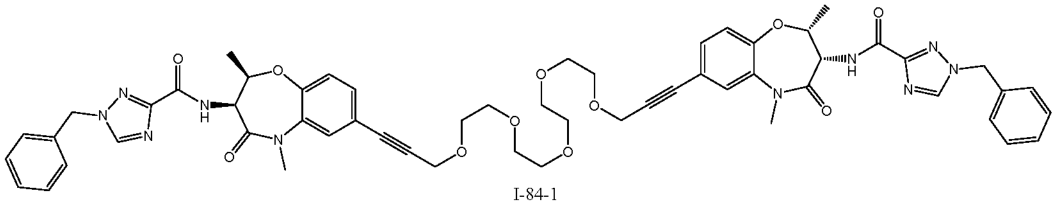

I-84-1

,

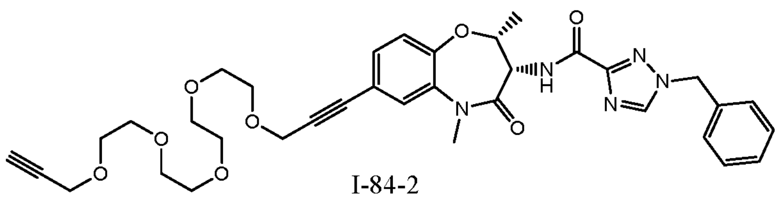

I-84-2

,

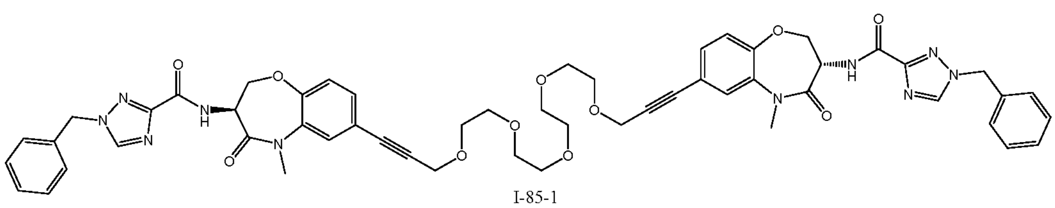

I-85-1

,

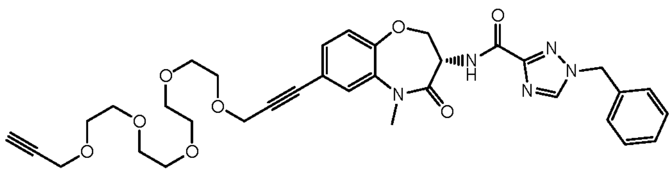

I-85-2

,

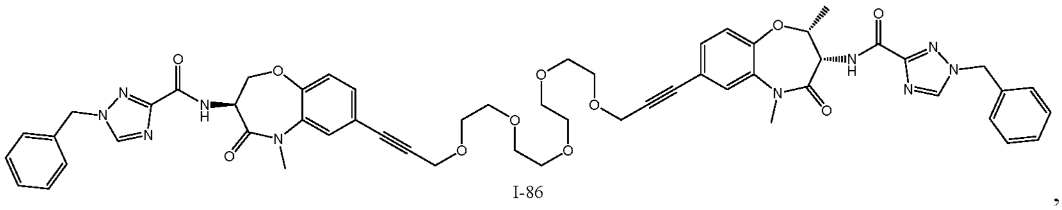

I-86

,

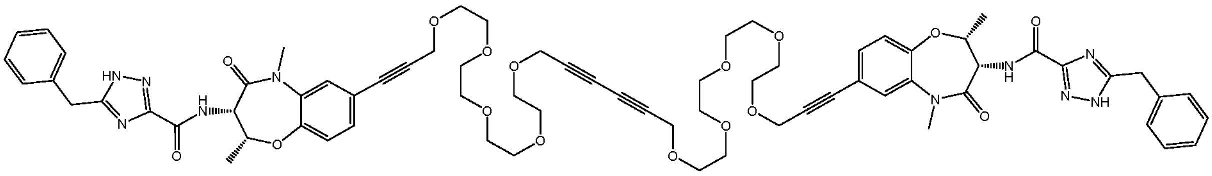

I-87

,

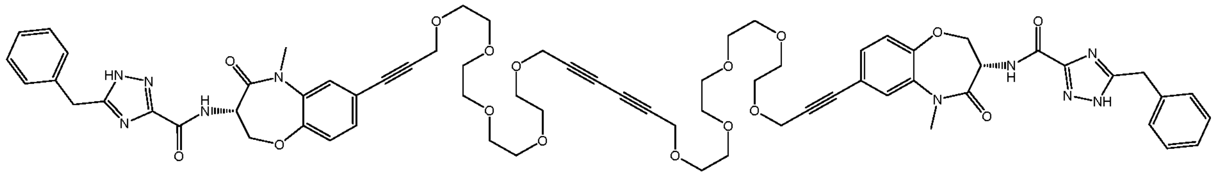

I-88

,

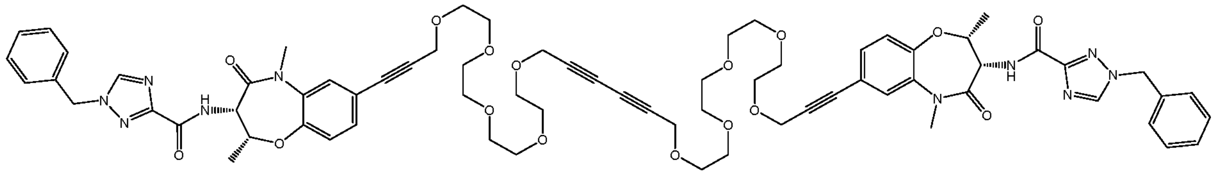

I-89

,

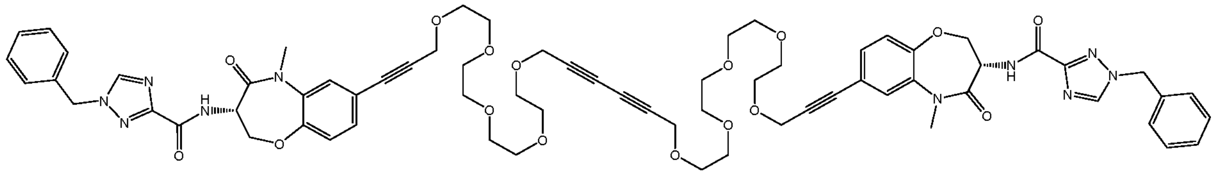

I-90

,

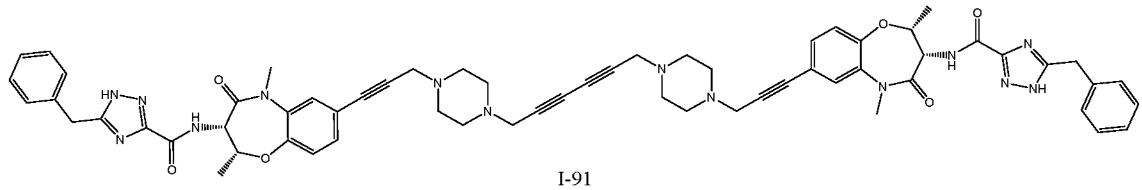

I-91

,

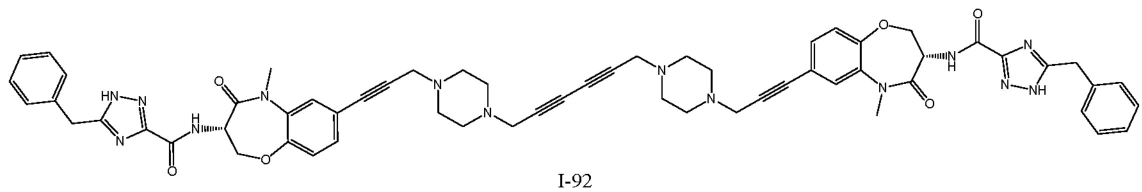

I-92

,

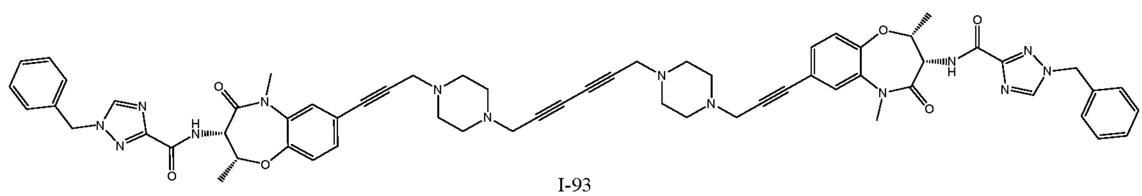

I-93

,

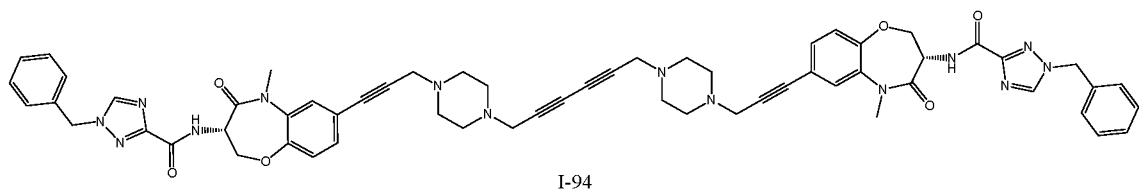

I-94

,

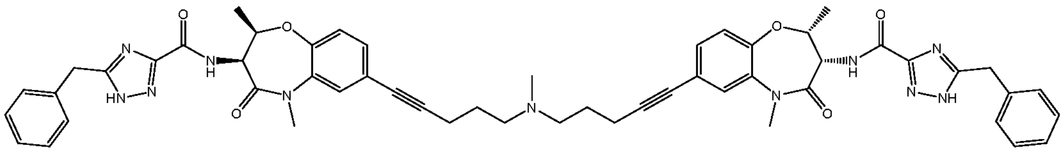

I-95

,

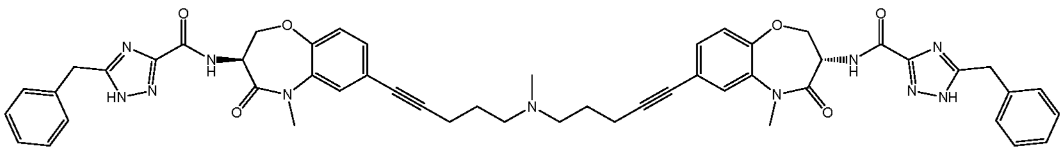

I-96

,

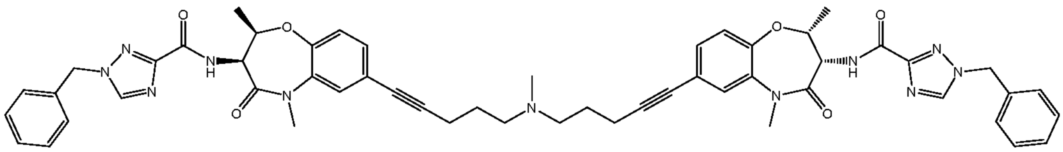

I-97

,

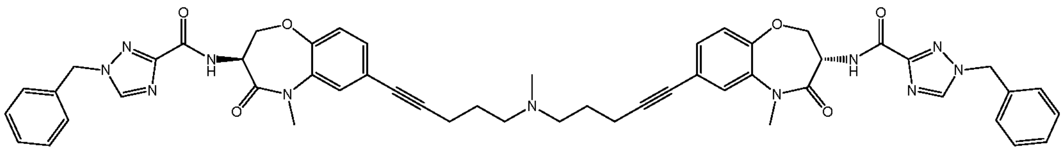

I-98

,

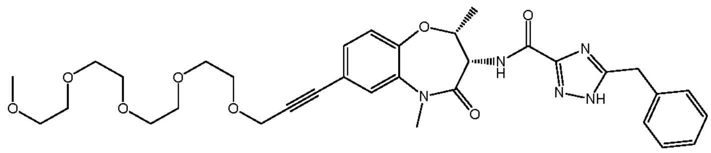

I-99

,

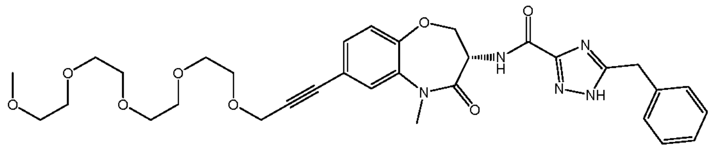

I-100

,

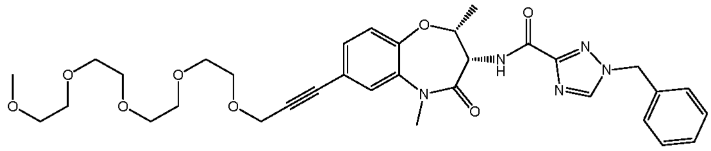

I-101

,

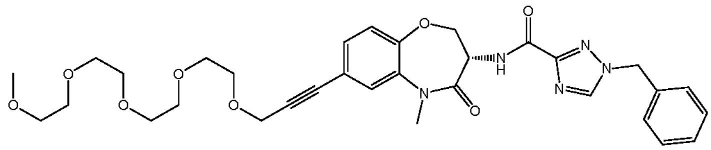

I-102

,

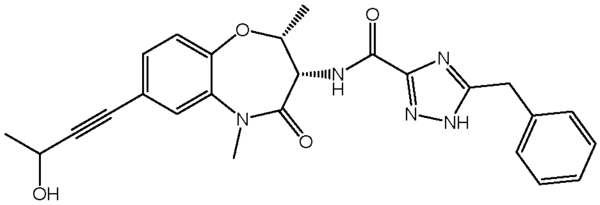

I-103

,

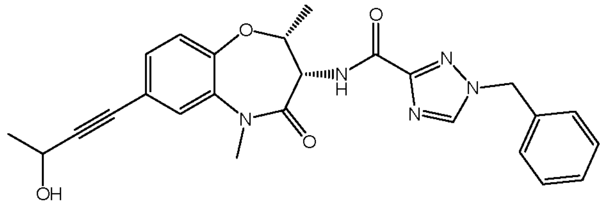

I-104

,

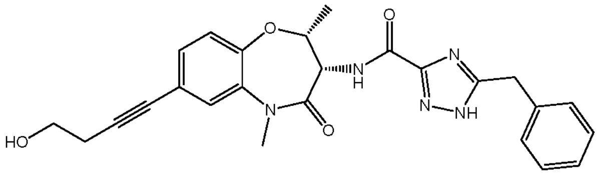

I-105

,

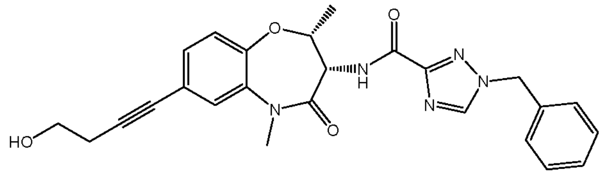

I-106

,

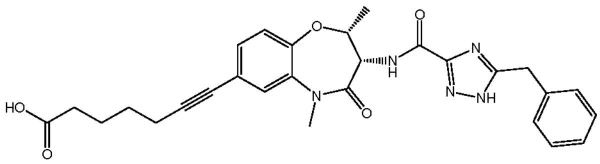

I-107

,

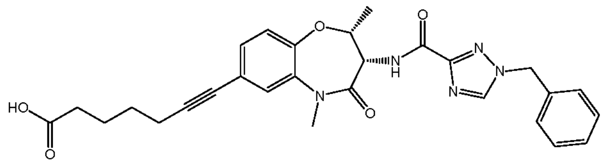

I-108

,

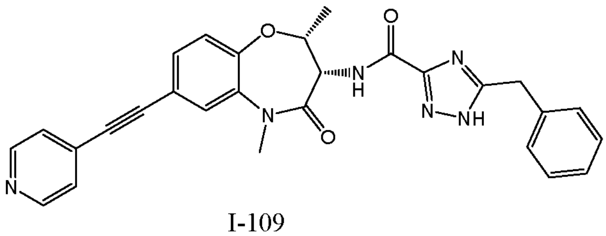

I-109

,

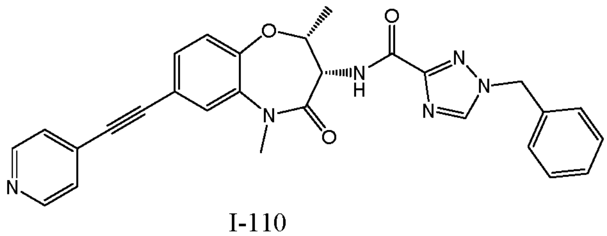

I-110

,

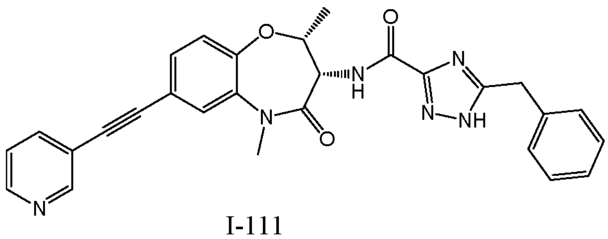

I-111

,

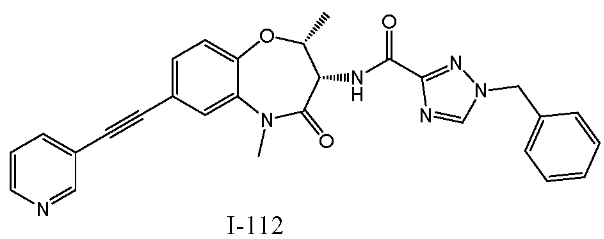

I-112

,

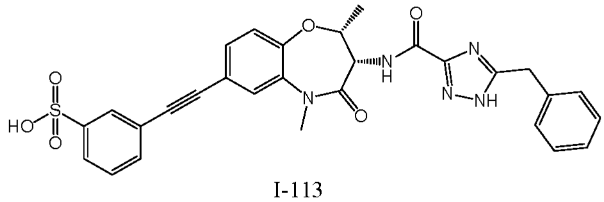

I-113 ,

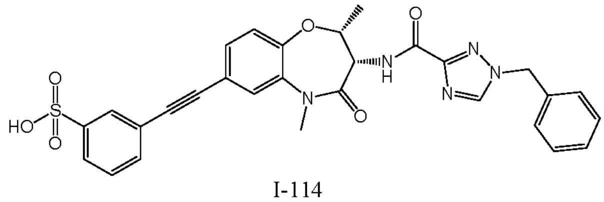

I-114 ,

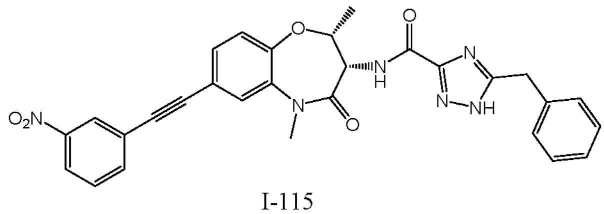

I-115 ,

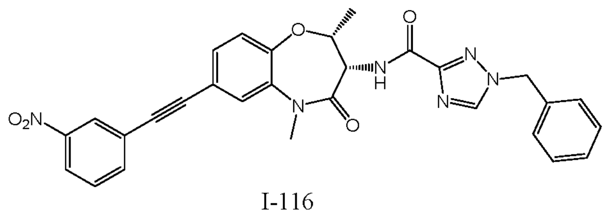

I-116 ,

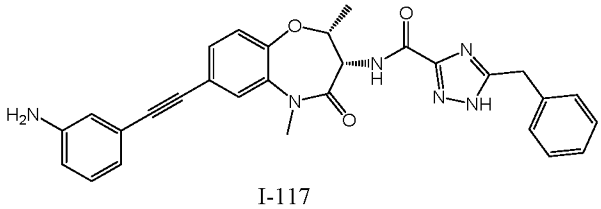

I-117 ,

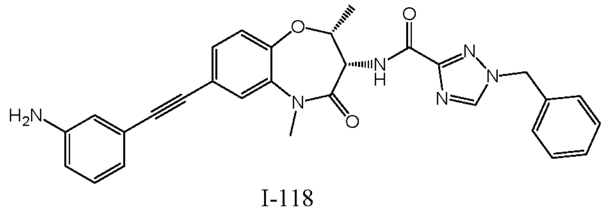

I-118 ,

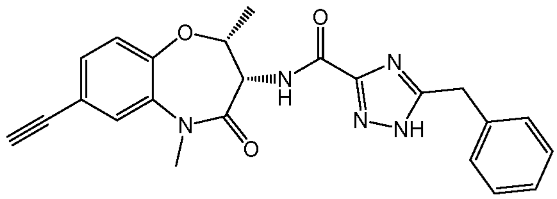

I-119 ,

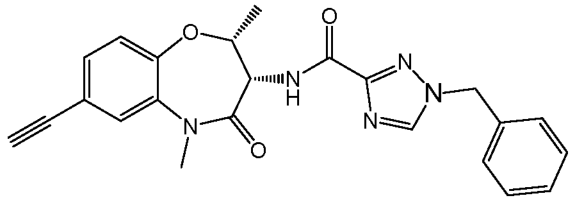

I-120 ,

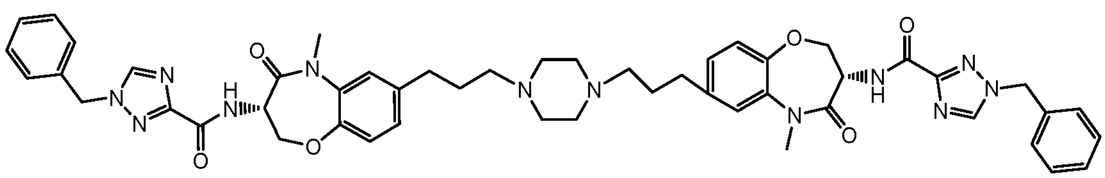

I-121 ,

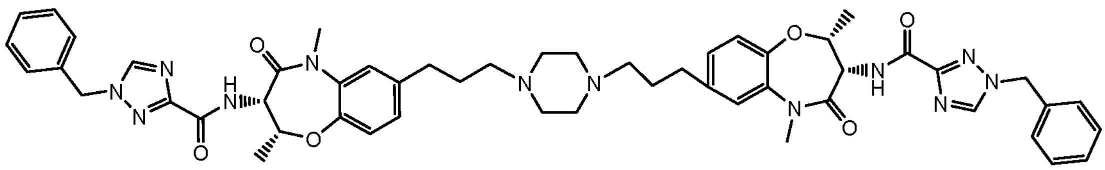

I-122 ,

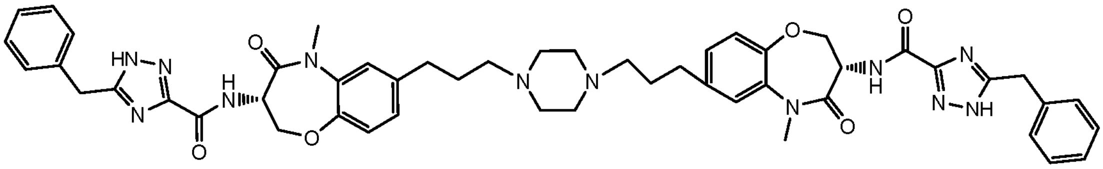

I-123

,

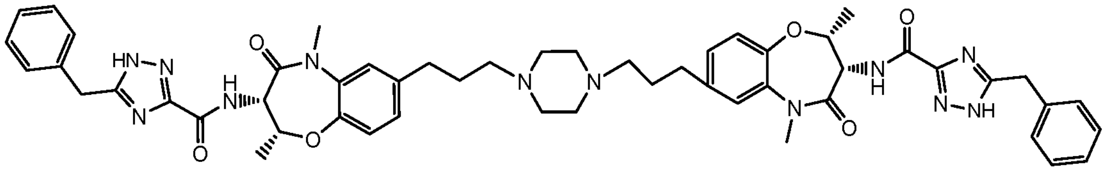

I-124

.

The present invention also provides above compounds, or a salt thereof, or a stereoisomer thereof, or a solvate thereof, or a prodrug thereof for use in the manufacture of RIP inhibitors;
preferably, the RIP inhibitor is receptor-interacting protein 1 (RIP1) inhibitors.

The present invention also provides the use of above compounds, or a salt thereof, or a stereoisomer thereof, or a solvate thereof, or a prodrug thereof in the manufacture of medicaments for anti-inflammation, anti-tumor, prevention and/or treatment of psoriasis;

preferably, the anti-tumor medicaments are those for the treatment of pancreatic cancer, colon cancer, lung cancer, liver cancer, breast cancer, and lymphatic cancer;
and/or, the anti-inflammatory medicaments are those for preventing and/or treating rheumatoid arthritis, ulcerative colitis, and atopic dermatitis.

The present invention also provides a medicament, which is manufactured from a compound according to any one of claims 1 to 17, or a salt thereof, or a stereoisomer thereof, as active ingredients, in combination with pharmaceutically acceptable excipients or auxiliary ingredients.

The definition of terms in the present invention:
In the present invention, the minimum and maximum values of the carbon numbers in hydrocarbon groups are represented by prefixes. For example, the $C_1$-$C_8$ alkyls or $C_{1-8}$ alkyls refer to $C_1$, $C_2$, $C_3$, $C_4$, $C_5$, $C_6$, $C_7$, $C_8$ alkyls, i.e. straight or branched alkyls having 1-8 carbons, such as methyl, ethyl, propyl, isopropyl, butyl, isobutyl, tert-butyl, sec-butyl, pentyl, hexyl, heptyl, octyl, etc. Similarly, the $C_1$-$C_3$ alkoxys refer to $C_1$, $C_2$, $C_3$ alkoxys; $C_3$-$C_8$ cycloalkyls refer to the cycloalkyls having 3, 4, 5, 6, 7, and 8 carbons.

The term "substitution" refers to substituting one, two, or more hydrogens in a molecule with other different atoms, molecules, or groups, including one, two, or more substitutions at the same or defferent atoms in the molecule.

The term "halogen" refers to fluorine, chlorine, bromine, or iodine; the term "alkyl" refers to the hydrocarbon group formed by lossing one hydrogen from a straight or branched alkane molecule;

The term "cyclic hydrocarbon group" refers to a saturated or unsaturated cyclic monovalent substituent group only containing carbons and hydrogens, which can be a group formed by removal of one H atom from saturated alicyclic hydrocarbons, cycloalkenes, or cycloalkynes, and can be further substituted. The $C_3$-$C_6$ cyclic hydrocarbon groups refer to that having 3-6 carbons.

The term "heterocyclic hydrocarbon group" refers to a cyclic hydrocarbon group in which at least one carbon is substituted with a heteroatom, and the heteroatom is O, N, or S.

The term "aryl" refers to all-carbon monocyclic or fused polycyclic (i.e., the rings sharing adjacent carbon atom pairs) groups with conjugated $\pi$ electron systems, such as phenyl and naphthyl. The aromatic ring can condense with other cyclic groups (including saturated and unsaturated rings), but cannot contain heteroatoms such as O, N, or S, and the point connecting the parent must be the carbon atom in the ring with a conjugated $\pi$ electron system.

The term "heteroaryl" refers to an aryl in which at least one carbon atom in the ring with conjugated $\pi$ electron system is substituted with a heteroatom, and the heteroatom is O, N, or S.

The term "heterocyclyl" refers to both heterocyclic hydrocarbon groups and heteroaromatic groups, and the heteroatom is O, N, or S.

Obviously, based on the above content of the present invention, according to the common technical knowledge

and the conventional means in the field, other various modifications, alternations, or changes can further be made, without department from the above basic technical spirits.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

**Description of Figures**

Figure 1. The weight change curve of each group of mice in Experimental Example 5.
Figure 2. The percentage change curve of body weight for each group of mice in Experiment Example 5.
Figure 3. The disease index (DAI) score for each group of mice in Experiment Example 5.
Figure 4. The colon length for each group of mice in Experiment Example 5.
Figure 5. Pathological scores of colon tissue for each group of mice in Experiment Example 5.

**Examples**

The raw materials and equipment used in the specific examples of the present invention were known products obtained by purchasing those commercially available.

**Example 1. Synthesis of A series intermediates**

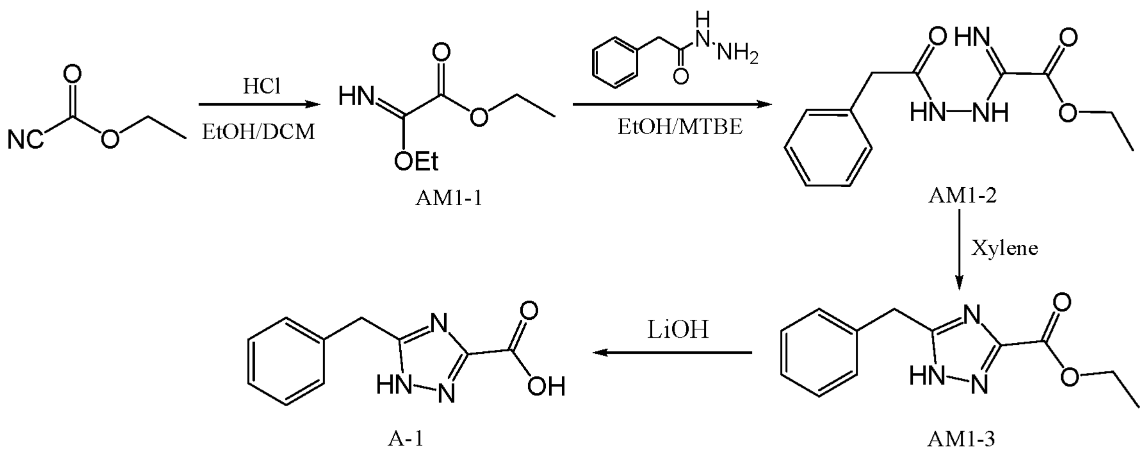


**Step 1:** 20 mL of ethanol was cooled to -5 °C, to which was added 10 g of acetyl chloride (0.13 mol) dropwise, and after addition, the mixture was allowed to react at low temperature (0-5 °C) for half an hour, to obtain freshly prepared HCl gas in ethanol. The mixed solution of ethyl cyanoformate (12.5 g, 0.13 mol) and dichloromethane (30 mL) was added dropwise at low temperature (0-5 °C), and then the reaction was stirred overnight at 0-5 °C, followed by filtering to collect the solids formed in the reaction, that is the imine intermediate hydrochloride. The hydrochloride was suspended in 50 mL of methyl tert-butyl ether, to which was added 20 g of triethylamine, and then the reaction was stirred at room temperature for 5 h. The reaction solution was filterred to remove triethylamine hydrochloride, and then concentrated under reduced pressure to remove methyl tert-butyl ether and obtain 12.3 g of intermediate **AM1-1.**

**Step 2:** 9.6 g of intermediate **AM1-1** (66.1 mmol) prepared in step 1 was dissolved in a mixed solution of ethanol (40 mL) and methyl tert-butyl ether (120 mL), to which was added 10 g of phenylacetic hydrazide (66.6 mmol), and then the reaction was stirred overnight at room temperature. The reaction solution was filtered, and the collected solids were washed with ethanol twice, followed by drying, to obtain 8.7 g of intermediate **AM1-2,** which was used in the next reaction.

**Step 3:** 5 g of intermediate **AM1-2** (20.0 mmol) was suspended in 100 mL of xylene, and reacted under reflux at 170 °C for 24 h. Then, the reaction solution was cooled to room temperature, and filtered to obtain 4.3 g of intermediate **AM1-3**.

**Step 4: 4** g of intermediate **AM1-3** (17.3 mmol) was suspended in 40 mL of water, to which was added 1.2 g

of LiOH (50.1 mmol), and then the mixture was stirred at room temperature for 3 h. The reaction solution was adjusted to pH 2 with 2M dilute hydrochloric acid, and then the solids precipitated, which were collected by filtration. The solids were washed twice with water, and then dried, to obtain 3.3 g of intermediate **A-1**.

**2. Synthesis of intermediate A-2**

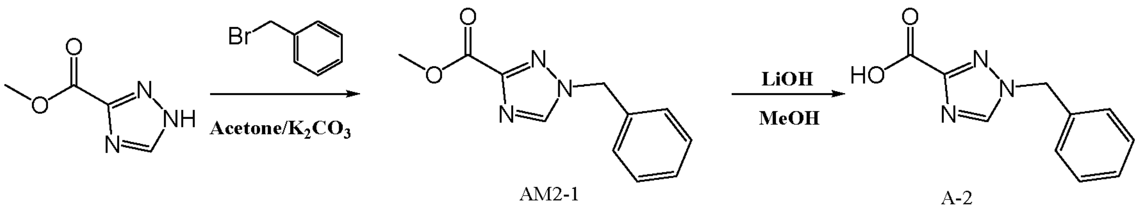


AM2-1 A-2

**Step 1:** 5 g of commercially available methyl 1,2,4-triazol-3-carboxylate (39.3 mmol) was dissolved in 100 mL of acetone, to which were added 8.1 g of potassium carbonate (58.6 mmol) and 6.7 g of benzyl bromide (39.1 mmol), and then the mixture was stirred overnight at room temperature. The complete reaction of the raw material was detected by TLC. Then, 100 mL of water was added, and the resultant solution was extracted three times with 150 mL of ethyl acetate. The organic layers were combined, washed once with saturated NaCl aqueous solution, and dried over 50 g of anhydrous sodium sulfate. Sodium sulfate was removed by filtration. The filtrate was concentrated under reduced pressure to dry, and then 50 mL of petroleum ether was added to crystallize. 6.6 g of wet product was obtained, which was recrystallized in 30 mL mixed solution of ethyl acetate and petroleum ether (EA:PE = 1:1). The crystals were collected by filtration and dried to obtain a total of 4.1 g of intermediate **AM2-1.**

**Step 2:** 2 g of intermediate **AM2-1** (9.2 mmol) was dissolved in 20 mL of methanol, to which was added the solution of LiOH (0.7 g, 29.2 mmol) in 20 mL of water, and the mixture was stirred at room temperature for 5 h. TLC indicated the complete reaction of the raw material. The reaction solution was adjusted to pH 2-3 with 2 M dilute hydrochloric acid. Solids precipitated, were collected by filtration, washed with water to neutral, and dried to obtain 1.7 g of intermediate **A-2.**

**3. Synthesis of intermediate A-10**

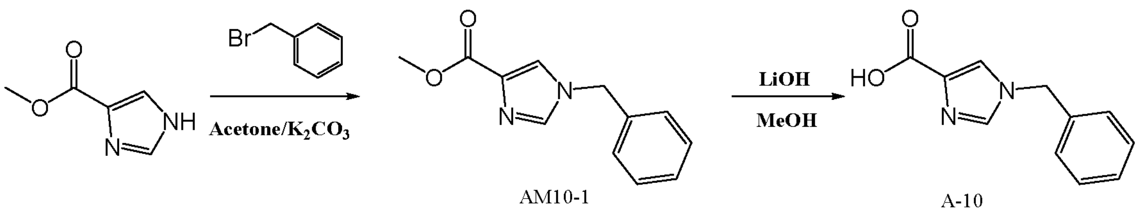


AM10-1 A-10

Using methyl 1H-imidazol-4-carboxylate as the raw material, the intermediate A-10 was obtained by the synthesis method similar to that of intermediate **A-2.**

The other intermediates **A-3, A-4, A-5, A-6, A-7, A-8,** and **A-9** were commercially available and had the following structures:

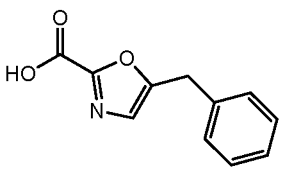
A-3 ,
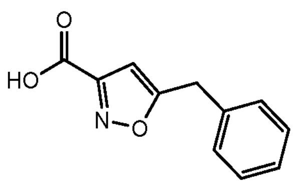
A-4 ,
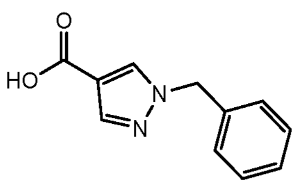
A-5 ,
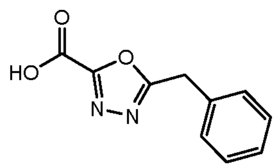
A-6 ,

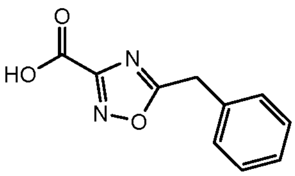
A-7 ,

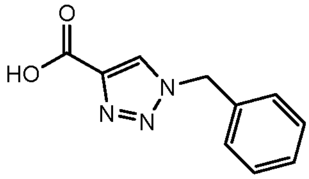
A-8 ,

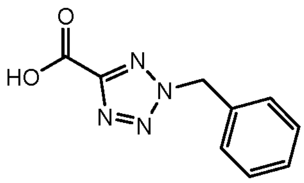
A-9 .

### 4. Synthesis of intermediate A-11

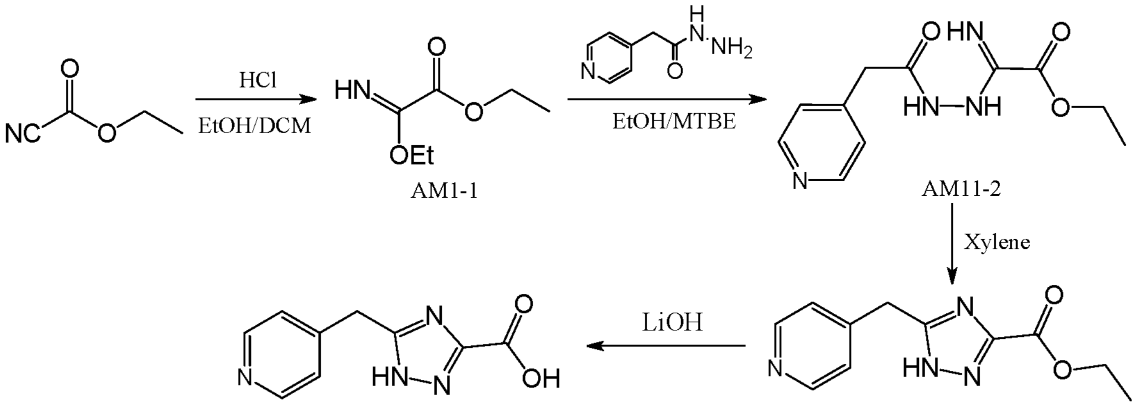

AM1-1, AM11-2, AM11-3, A-11

Substituting phenylacetic hydrazide in the method of synthesizing **A-1** with pyridine-4-carbohydrazide, the intermediate **A-11** was prepared by the synthesis method similar to that of intermediate **A-1**.

### 5. Synthesis of other intermediates

Similar to the above method, using pyridine-3-carbohydrazide, 3-hydroxyphenylacetic hydrazide, and 3-aminophenylacetic hydrazide as raw materials, intermediates **A-12, A-13, A-14, A-15,** and **A-16** were synthesized, with the following structures:

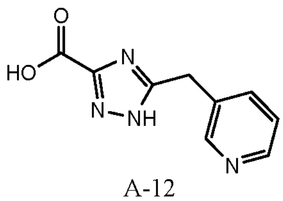
A-12 ,

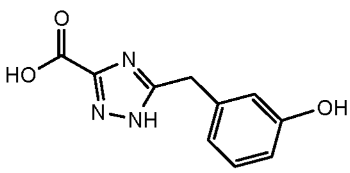
A-13 ,

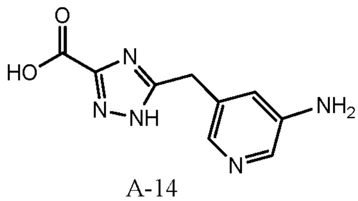
A-14 ,

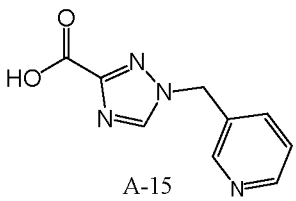
A-15 ,

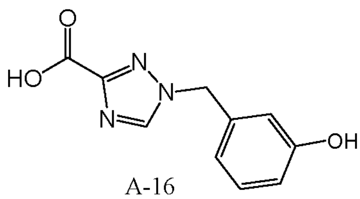
A-16 ,

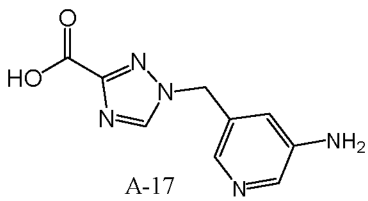
A-17 .

**Example 2. Synthesis of M series intermediates**

**1. Synthesis of intermediate M-1**

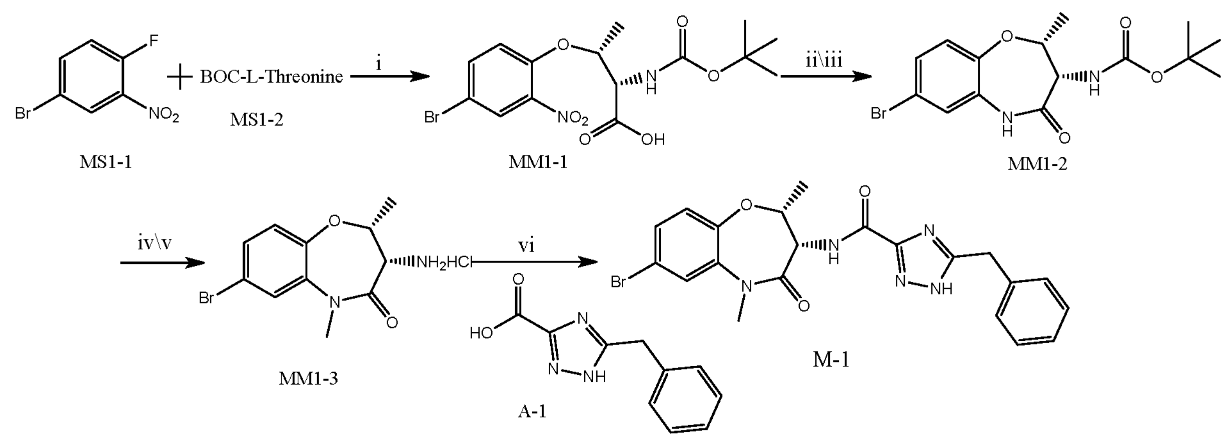


i: NaH/DMF; ii: $H_2$, Pd/C, MeOH; iii: TBTU, DIPEA, DMSO; iv: MeI, $Cs_2CO_3$, DMF; v: HCl, MTBE; vi: TBTU, DIPEA, DCM

The specific procedures were as follows:

Step 1: Synthesis of intermediate **MM1-1**

5 g of BOC-L-threonine (MS1-2, CAS: 2592-18-9, 22.8 mmol) was dissolved in 50 mL of DMF, and then the reaction solution was cooled to 0 °C in an ice salt bath. 1.4 g of NaH (57.0 mmol) was suspended in 10 mL of DMF, and added to the reaction solution dropwise. The ice salt bath was removed, and the reaction solution was stirred at room temperature until bubbles disappeared, to which was added 5.0 g of 4-bromo-1-fluoro-2-nitrobenzene (MS-1-1, 22.7 mmol) dropwise, and the resultant solution was stirred at room temperature for 4 h. Ice water was added to quench the reaction. 2M dilute hydrochloric acid was added to the reaction system, and the pH value of the system was adjusted to be 2-3. The reaction solution was extracted three times with 50 mL of ethyl acetate. The organic layers were combined, washed with water, dried, and concentrated under reduced pressure. The residue was purified by column chromatography to obtain 7.6 g of intermediate **MM1-1** (molecular weight 417.25) as yellow semi-solids.

Step 2: Synthesis of intermediate **MM1-2**

The above intermediate **MM1-1** was dissolved in 60 mL of methanol, and then subjected to the catalytic hydrogenation in the presence of the catalyst Pd/C. After completion of the reaction, the reaction solution was filtered to remove Pd/C, and the filtrate was concentrated under reduced pressure. The residue was stirred in methyl tert-butyl ether to crystallize. The solid was filtered, dried, and then dissolved in 25 mL of DMSO, to which were added 6.4 g of TBTU (19.9 mmol) and 2.6 g of DIPEA (20.1 mmol). The reaction solution was stirred overnight at room temperature, and then the reaction was quenched with 25 mL of ice water. The reaction solution was extracted three times with 30 mL of ethyl acetate. The organic layers were combined, washed with water, dried, and concentrated under reduced pressure. The residue was crystallized in petroleum ether, and then the crystals were filtered and dried, to obtain 5.1 g of intermediate **MM1-2** (molecular weight 371.23) as white solids.

Step 3: Synthesis of intermediate **MM1-3**

2.5 g of intermediate **MM1-2** (6.7 mmol) was dissolved in 50 mL of DMF, to which were added 3.3 g of cesium carbonate (10.2 mmol) and 1.4 g of iodomethane (10.2 mmol), and then the mixture was stirred at room temperature until the reaction was completed. The reaction was quenched with 50 mL of ice water. The reaction solution was extracted three times with 80 mL of ethyl acetate. The organic layers were combined, washed with water, dried, and concentrated under reduced pressure. The residue was crystallized in petroleum ether, and then filtered to collect the solids, to which was added 20 mL of methyl tert-butyl ether saturated with hydrochloric acid gas. The resultant solution was stirred at room temperature for 6 h, and then concentrated under reduced pressure to remove the solvent. The residue was

crystallized in petroleum ether to obtain 1.9 g of intermediate **MM1-3** (molecular weight 321.60) as light yellow solids.

Step 4: Synthesis of intermediate **M-1**

1.7 g of intermediate **MM1-3** (5.3 mmol), 3.4 g of TBTU (10.6 mmol), and 1.0 g of DIPEA (7.7 mmol) were dissolved in 20 mL of DCM, to which was added 1.3 g of intermediate **A-1** (5-benzyl-4H-1,2,4-triazol-3-carboxylic acid, 6.4 mmol), and then the mixture was stirred overnight at room temperature. Ice water was added to quench the reaction. The organic layer was washed with water, dried, and concentrated under reduced pressure. The residue was separated by column chromatography to obtain 1.8 g of intermediate **M-1**.

**2. Synthesis of intermediate M-2**

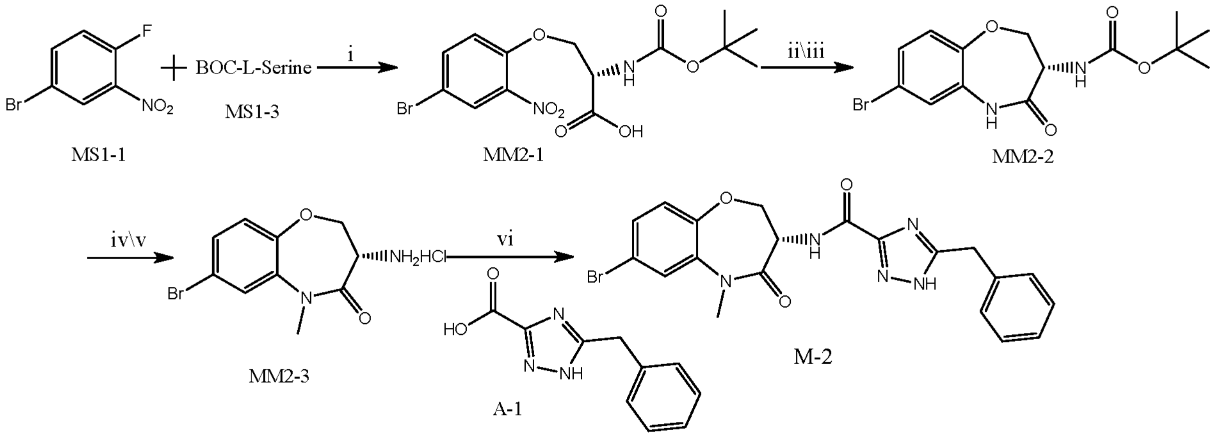

MS1-1 MS1-3 MM2-1 MM2-2 MM2-3 A-1 M-2

i: NaH/DMF; ii: $H_2$, Pd/C, MeOH; iii: TBTU, DIPEA, DMSO; iv: MeI, $Cs_2CO_3$, DMF; v: HCl, MTBE; vi: TBTU, DIPEA, DCM

Using 4-bromo-1-fluoro-2-nitrobenzene and BOC-L-serine as raw materials, the intermediate **M-2** was obtained by the synthesis method similar to that of intermediate **M-1.**

**3. Synthesis of intermediate M-3**

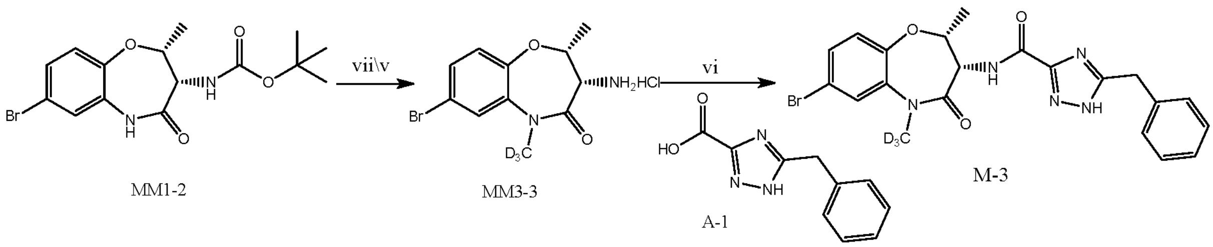

MM1-2 MM3-3 A-1 M-3

vii: $CD_3I$, $Cs_2CO_3$, DMF; v: HCl, MTBE; vi: TBTU, DIPEA, DCM

Using **MM1-2** as the raw material and substituting iodomethane with deuterated iodomethane, the intermediate **M-3** was prepared by the synthesis method similar to that of steps 3 and 4 for intermediate **M-1**.

**4. Synthesis of intermediate M-4**

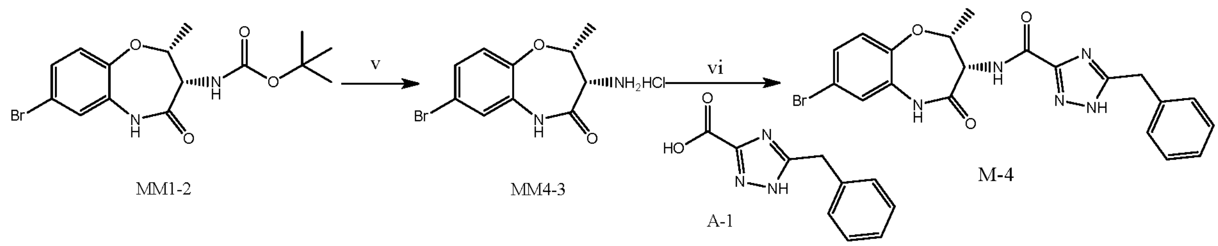


MM1-2 MM4-3 A-1 M-4

v: HCl, MTBE; vi: TBTU, DIPEA, DCM

**MM1-2** was used as the raw material, to which was added methyl tert-butyl ether saturated with hydrochloric acid gas, and then the solution was stirred at room temperature for 6 h. The solvent was removed by concentration under reduced pressure. The residue was crystallized in petroleum ether to obtain the intermediate **MM4-3** (molecular weight 307.57).

Using above intermediate **MM4-3** and intermediate **A-1** as raw materials, compound **M-4** was obtained by the synthesis method similar to that of step 4 for intermediate **M-1.**

**5. Synthesis of intermediate M-5**

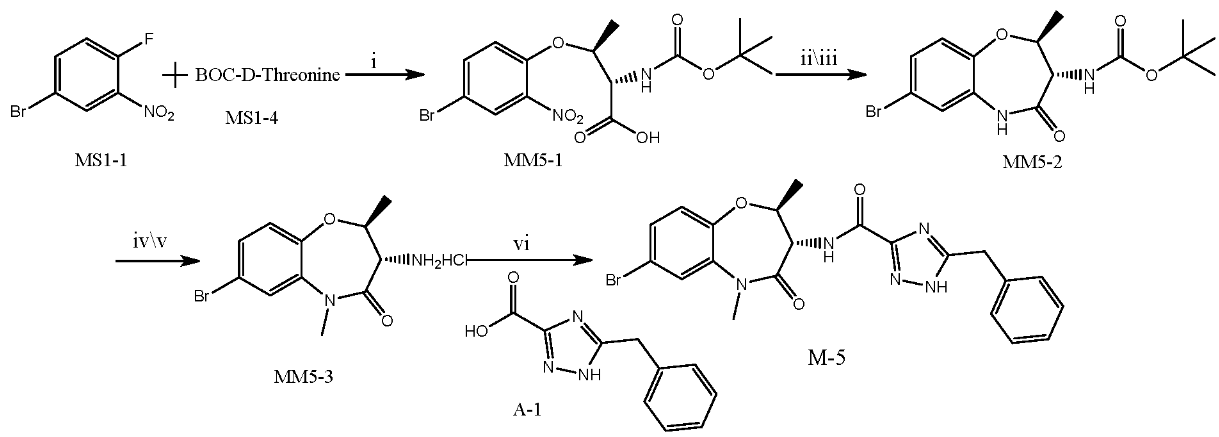


MS1-1 MS1-4 MM5-1 MM5-2 MM5-3 A-1 M-5

Using BOC-D-threonine as raw material, compound **M-5** was obtained by the synthesis method similar to that of intermediate **M-1.**

**6. Synthesis of other intermediates**

Similar to the above synthesis method, corresponding intermediates were synthesized from different raw materials, and the intermediates have the following structures:

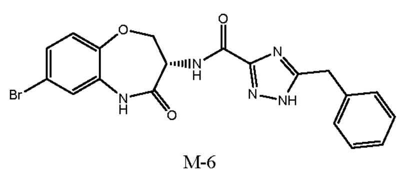

M-6 ,

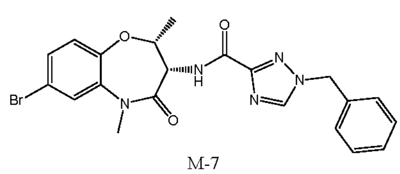

M-7 ,

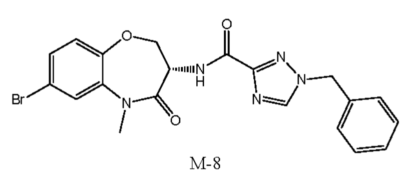

M-8 ,

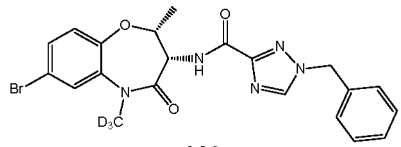

M-9 ,

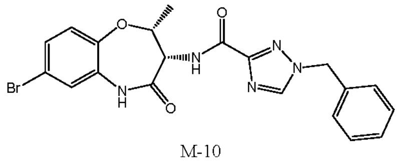

M-10 ,

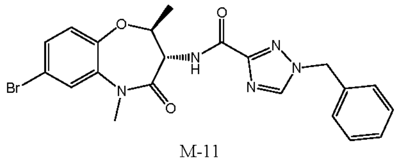

M-11 ,

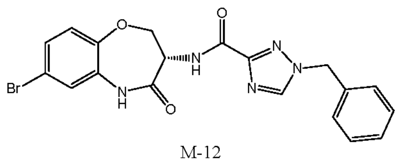

M-12 ,

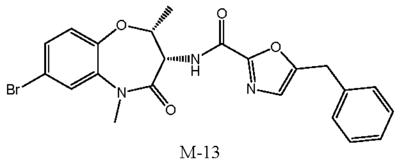

M-13 ,

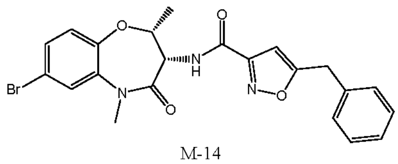

M-14 ,

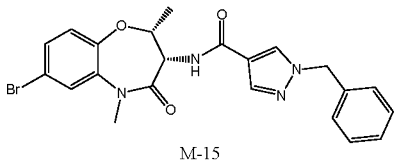

M-15 ,

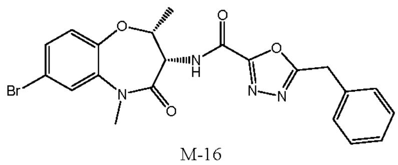

M-16 ,

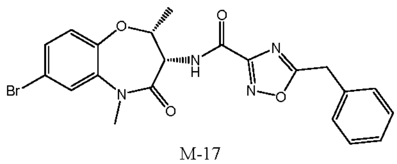

M-17 ,

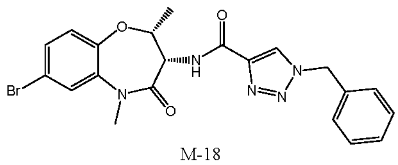

M-18 ,

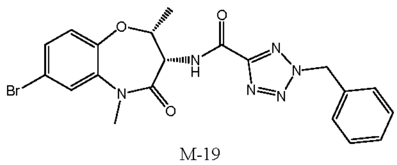

M-19 ,

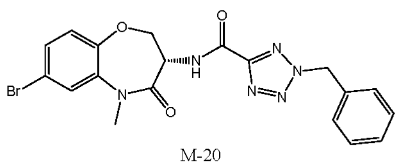

M-20 ,

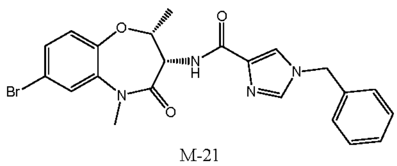

M-21 ,

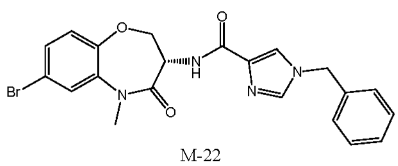

M-22 ,

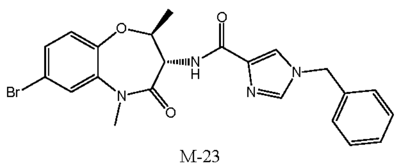

M-23 ,

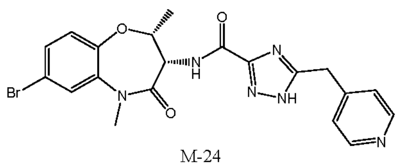

M-24 ,

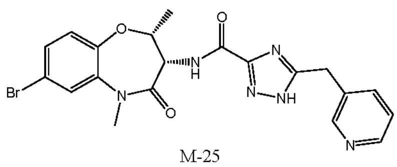

M-25 ,

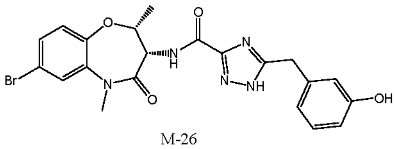
M-26 ,
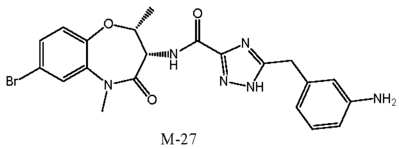
M-27 ,

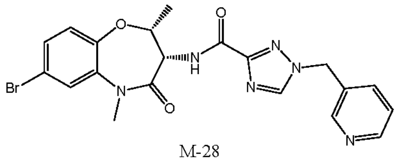
M-28 ,
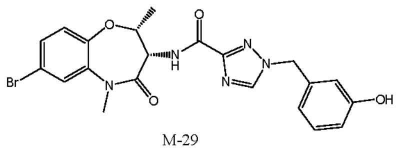
M-29 ,

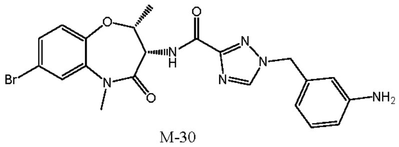
M-30 .

**Example 3. Synthesis of compound I-1 of the present invention**

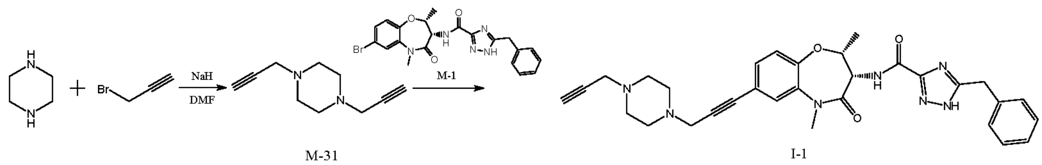

M-31 I-1

15 g of piperazine (174.1 mmol) was added to a flask, to which was added 50 mL of DMF to dissolve, and then the flask was cooled to 0 °C in an ice salt bath. 10.4 g of NaH (433.3 mmol) was slowly added, and after addition, the mixture was stirred at 0 °C for 5 min. 80% solution of propargyl bromide (60 g) in toluene was slowly added. After that, the ice salt bath was removed. Then, the mixture was stirred at room temperature for 5 h. TLC detection showed that the raw material disappeared. The reaction solution was slowly added to 200 mL of water, and then extracted twice with 150 mL of ethyl acetate. The ethyl acetate layers were combined, dried with 10 g of anhydrous sodium sulfate for 30 minutes, and then filtered to remove sodium sulfate. The filtrate was concentrated to dry, and then the intermediate **M-31** was obtained as sheet-like solids.

400 mg of intermediate **M-1** (0.85 mmol) was added to a flask, to which were added 20 mg of palladium acetate, 20 mg of CuI, 40 mg of 2-dicyclohexylphosphino-2,4,6-triisopropylbiphenyl, 400 mg of triphenylphosphine, 151 mg of intermediate **M-31** (0.93 mmol), 5 mL of triethylamine, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-1**.
ESI-MS *m/z*: 550.26 $[M-1]^-$

**Example 4. Synthesis of compound I-2 of the present invention**

Compound **I-2** could be obtained by the following method 1:

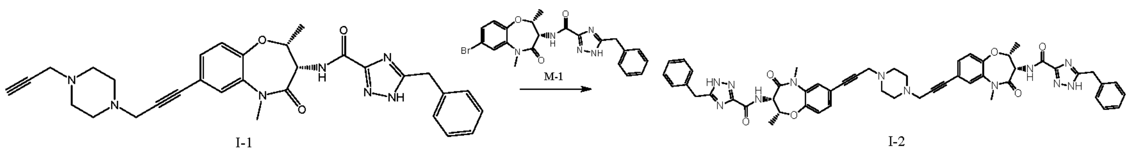

I-1 I-2

100 mg of intermediate **I-1** (0.18 mmol) was added to a flask, to which were added 5 mg of palladium acetate, 5 mg of CuI, 8 mg of 2-dicyclohexylphosphino-2,4,6-triisopropylbiphenyl, 100 mg of triphenylphosphine, 85 mg of intermediate **M-1** (0.18 mmol), 3 mL of triethylamine, and 10 mL of acetonitrile; the mixture was allowed to react overnight in an oil bath at 80 °C under nitrogen protection. TLC indicated the disappearance of raw materials. 50 mL of water was slowly added, and then the resultant solution was extracted twice with 50 mL of ethyl acetate. The ethyl acetate layers were combined, washed twice with 30 mL of saturated NaCl aqueous solution, dried with 5 g of anhydrous sodium sulfate for 30 min, and then filtered to remove sodium sulfate. The filtrate was concentrated to dry, and the residue was purified over column chromatography, to provide compound **I-2**.
ESI-MS *m/z*: 939.41 [M-1]$^-$

Compound **I-2** could be obtained by the following method 2:

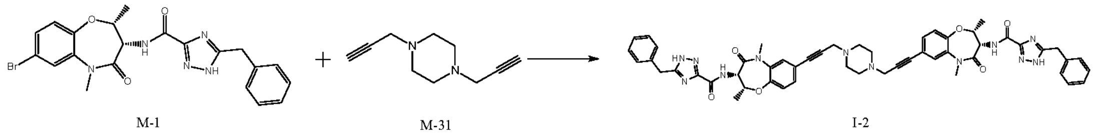


400 mg of intermediate **M-1** (0.85 mmol) was added to a flask, to which were added 20 mg of palladium acetate, 20 mg of CuI, 40 mg of 2-dicyclohexylphosphino-2,4,6-triisopropylbiphenyl, 400 mg of triphenylphosphine, 76 mg of intermediate **M-31** (0.47 mmol), 5 mL of triethylamine, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-2**.

**Example 5. Synthesis of compound I-3 of the present invention**

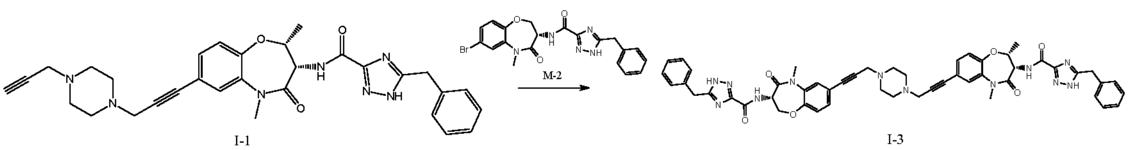


Compound **I-1** and intermediate **M-2** were used as raw materials, to obtain compound **I-3**, by the synthesis method similar to Method 1 of Example 4.
ESI-MS m/z: 925.40 [M-1]$^-$

**Example 6. Synthesis of compound I-4 of the present invention**

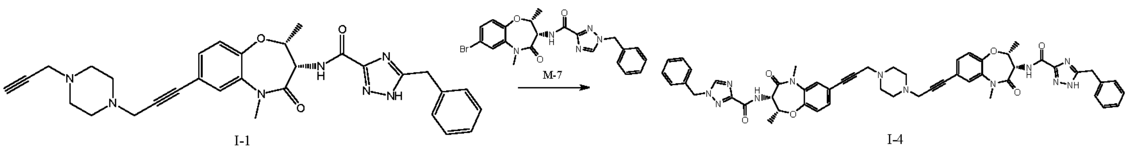


Compound **I-1** and intermediate **M-7** were used as raw materials, to obtain compound **I-4,** by the synthesis method similar to Method 1 of Example 4.
ESI-MS *m/z*: 939.41 [M-1]$^-$

**Example 7. Synthesis of compound I-5 of the present invention**

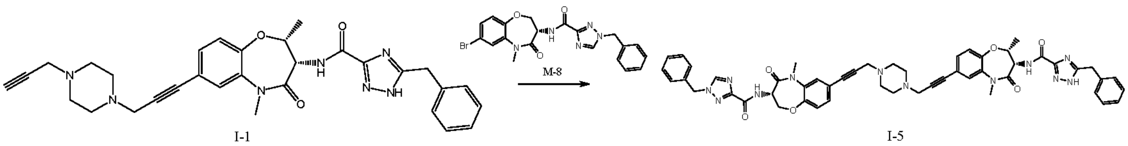


I-1 I-5

Compound **I-1** and intermediate **M-8** were used as raw materials, to obtain compound **I-5**, by the synthesis method similar to Method 1 of Example 4.
ESI-MS *m/z:* 925.40 $[M-1]^-$

**Example 8. Synthesis of compound I-6 of the present invention**

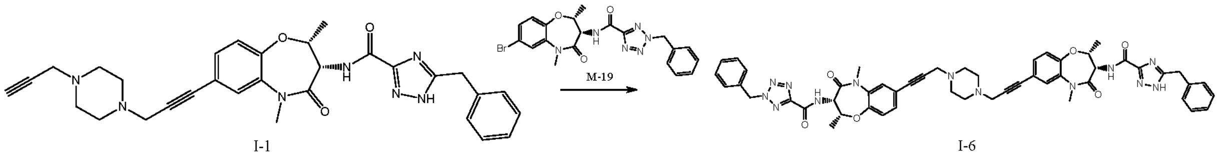


I-1 I-6

Compound **I-1** and intermediate **M-19** were used as raw materials, to obtain compound **I-6**, by the synthesis method similar to Method 1 of Example 4.
ESI-MS *m/z*: 940.41 $[M-1]^-$

**Example 9. Synthesis of compound I-7 of the present invention**

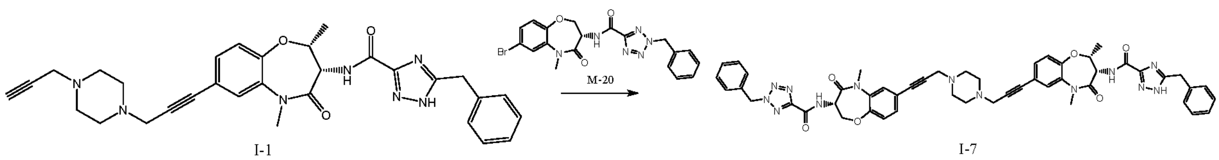


I-1 I-7

Compound **I-1** and intermediate **M-20** were used as raw materials, to obtain compound **I-7**, by the synthesis method similar to Method 1 of Example 4.
ESI-MS *m/z:* 926.39 $[M-1]^-$

**Example 10. Synthesis of compound I-8 of the present invention**

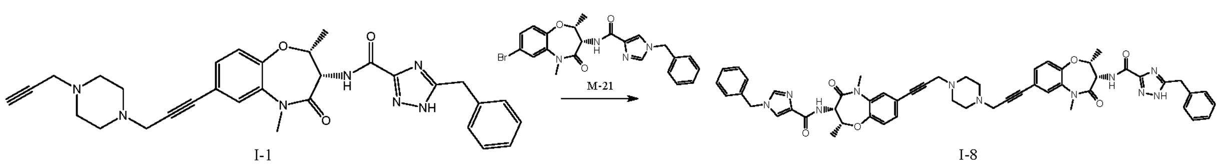


I-1 I-8

Compound **I-1** and intermediate **M-21** were used as raw materials, to obtain compound **I-8**, by the synthesis method similar to Method 1 of Example 4.
ESI-MS m/z: 938.42$[M-1]^-$

**Example 11. Synthesis of compound I-9 of the present invention**

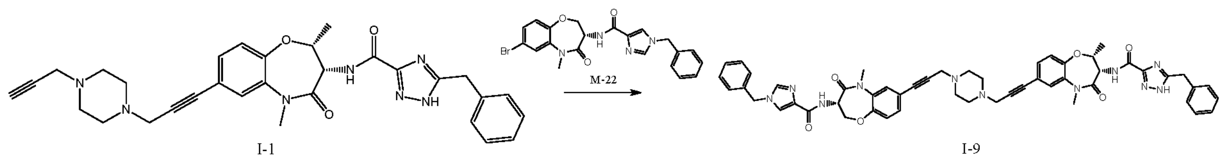


I-1 I-9

Compound **I-1** and intermediate **M-22** were used as raw materials, to obtain compound **I-9**, by the synthesis method similar to Method 1 of Example 4.
ESI-MS *m/z:* 924.40 [M-1]⁻

**Example 12. Synthesis of compound I-10 of the present invention**

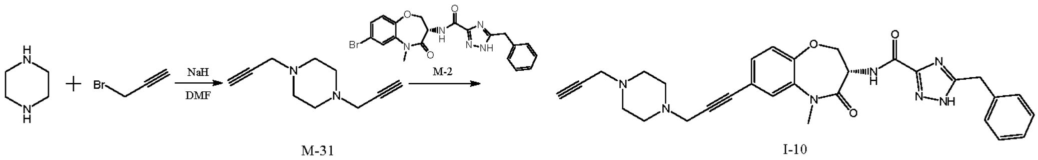


M-31 I-10

Intermediate **M-2** was used as raw material, to obtain compound **I-10**, by the synthesis method similar to that of Example 1.
ESI-MS *m*/*z*: 536.25 [M-1]⁻

**Example 13. Synthesis of compound I-11 of the present invention**

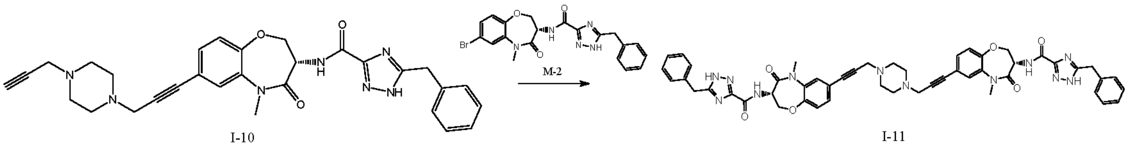


I-10 I-11

Compound **I-10** and intermediate **M-2** were used as raw materials, to obtain compound **I-11,** by the synthesis method similar to Method 1 of Example 4.

$^1$HNMR (400 MHz, DMSO-$d_6$) $\delta$: 8.40 (d, *J*= 7.8 Hz, 2H), 7.55-7.50 (m, 2H), 7.38-7.20 (m, 16H), 4.91-4.83 (m, 4H), 4.65-4.55 (m, 2H), 4.45-4.35 (m, 4H), 3.49 (s, 4H), 3.35 (s, 6H), 2.60-2.40 (m, 8H).
ESI-MS m/z: 911.38[M-1]⁻

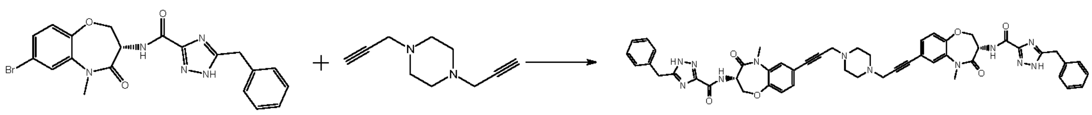

M-2 M-31 I-11

Alternatively, intermediates **M-2** and **M-31** were used as raw materials, to obtain compound **1-11,** by the synthesis method similar to Method 2 of Example 4.

**Example 14. Synthesis of compound I-12 of the present invention**

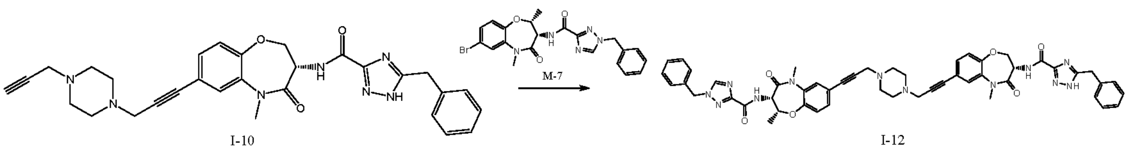


I-10 I-12

Compound **I-10** and intermediate **M-7** were used as raw materials, to obtain compound **I-12,** by the synthesis method similar to Method 1 of Example 4.
ESI-MS *m/z:* 925.40 [M-1]$^-$

**Example 15. Synthesis of compound I-13 of the present invention**

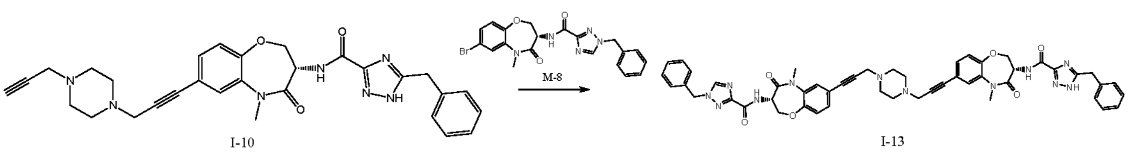


I-10 I-13

Compound **I-10** and intermediate **M-8** were used as raw materials, to obtain compound **I-13,** by the synthesis method similar to Method 1 of Example 4.
ESI-MS *m/z*: 911.38 [M-1]$^-$

**Example 16. Synthesis of compound I-14 of the present invention**

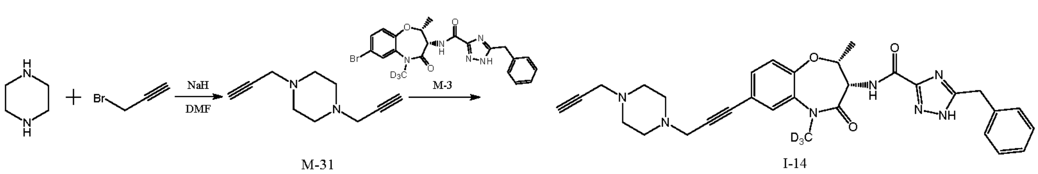


M-31 I-14

Intermediate **M-3** was used as raw material, to obtain compound **I-14**, by the synthesis method similar to that of Example 1.
ESI-MS *m/z*: 553.28 [M-1]$^-$

**Example 17. Synthesis of compound I-15 of the present invention**

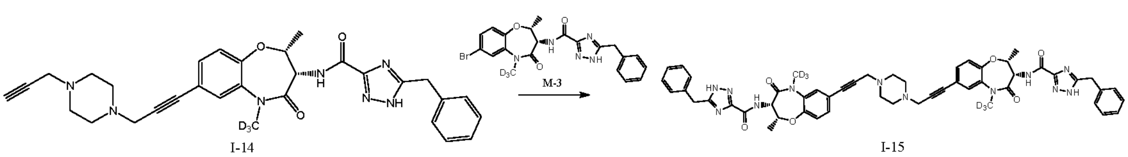


I-14 I-15

Compound **I-10** and intermediate **M-2** were used as raw materials, to obtain compound **I-15,** by the synthesis method similar to Method 1 of Example 4.
ESI-MS *m/z*: 945.45 [M-1]$^-$

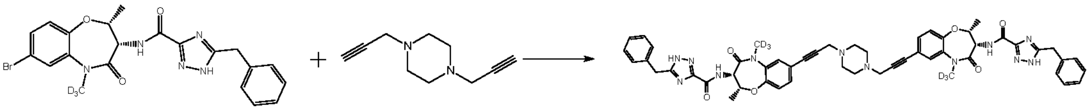

M-3 M-31 I-15

Alternatively, intermediates **M-3** and **M-31** were used as raw materials, to obtain compound **I-15**, by the synthesis method similar to Method 2 of Example 4.

**Example 18. Synthesis of compound I-16 of the present invention**

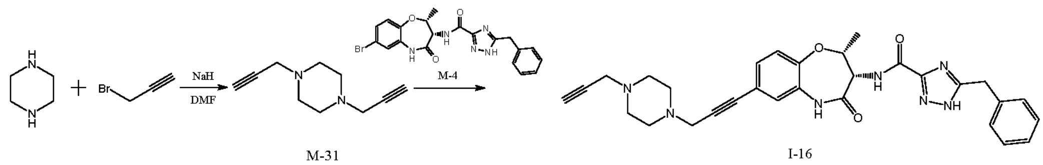


M-31 I-16

Intermediate **M-4** was used as raw material, to obtain compound **I-16**, by the synthesis method similar to that of Example 1.
ESI-MS *m/z*: 536.25 $[M-1]^-$

**Example 19. Synthesis of compound I-17 of the present invention**

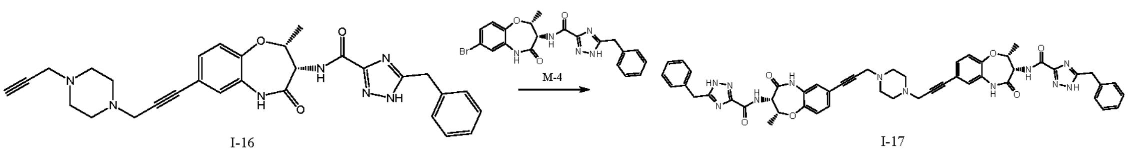


I-16 I-17

Compound **I-16** and intermediate **M-4** were used as raw materials, to obtain compound **I-17,** by the synthesis method similar to Method 1 of Example 4.
ESI-MS *m/z*: 911.38 $[M-1]^-$

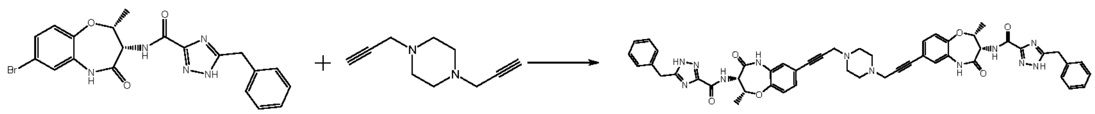

M-4 M-31 I-17

Alternatively, intermediates **M-4** and **M-31** were used as raw materials, to obtain compound **I-17**, by the synthesis method similar to Method 2 of Example 4.

**Example 20. Synthesis of compound I-18 of the present invention**

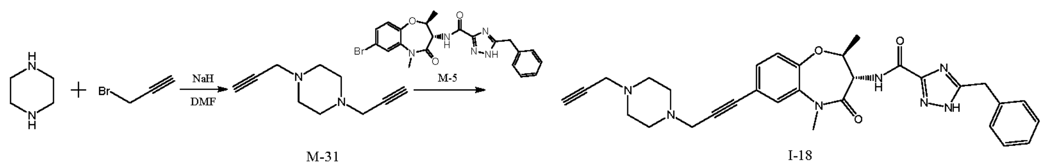


M-31 I-18

Intermediate **M-5** was used as raw material, to obtain compound **I-18**, by the synthesis method similar to that of Example 1.
ESI-MS *m/z*: 550.26 $[M-1]^-$

**Example 21. Synthesis of compound I-19 of the present invention**

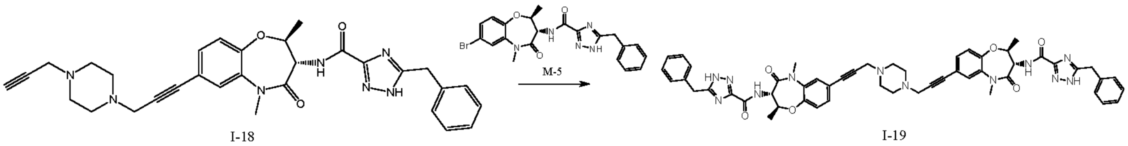


I-18 I-19

Compound **I-18** and intermediate **M-5** were used as raw materials, to obtain compound **I-19,** by the synthesis method similar to Method 1 of Example 4.
ESI-MS *m/z*: 939.41 [M-1]$^-$

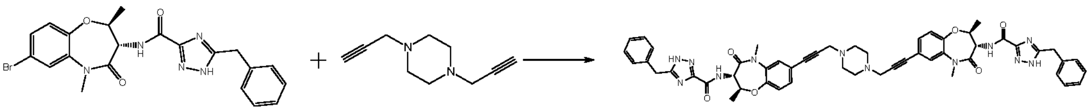

M-5 M-31 I-19

Alternatively, intermediates **M-5** and **M-31** were used as raw materials, to obtain compound **I-19**, by the synthesis method similar to Method 2 of Example 4.

**Example 22. Synthesis of compound I-20 of the present invention**

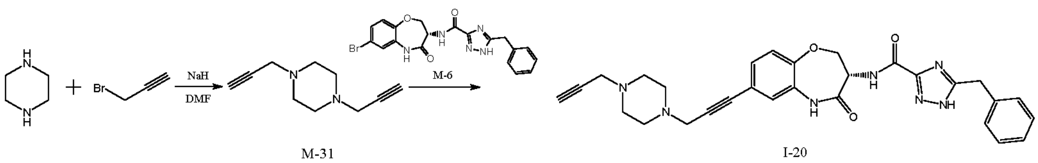


M-31 I-20

Intermediate **M-6** was used as raw material, to obtain compound **I-20**, by the synthesis method similar to that of Example 1.
ESI-MS *m/z*: 522.23 [M-1]$^-$

**Example 23. Synthesis of compound I-21 of the present invention**

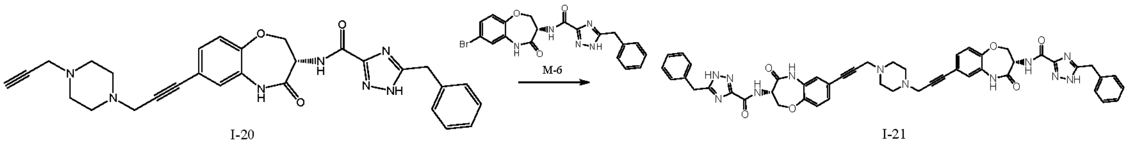


I-20 I-21

Compound **I-20** and intermediate **M-6** were used as raw materials, to obtain compound **I-21,** by the synthesis method similar to Method 1 of Example 4.
ESI-MS *m/z*: 883.35 [M-1]$^-$

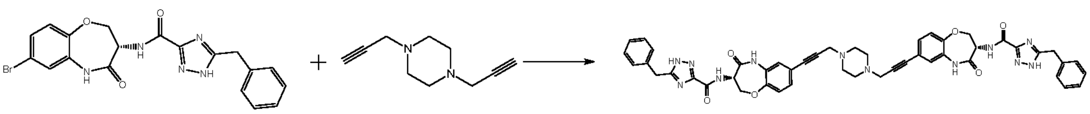

M-6 M-31 I-21

Alternatively, intermediates **M-6** and **M-31** were used as raw materials, to obtain compound **I-21**, by the synthesis method similar to Method 2 of Example 4.

**Example 24. Synthesis of compound I-22 of the present invention**

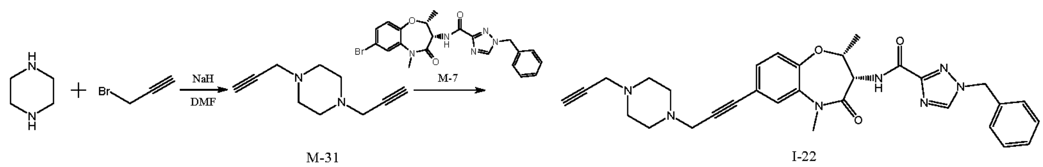


M-31 I-22

Intermediate **M-7** was used as raw material, to obtain compound **I-22** by the synthesis method similar to that of Example 1.

$^1$HNMR (400 MHz, DMSO-$d_6$) $\delta$: 8.83 (s, 1H), 7.95-7.90 (m, 1H), 7.55-7.50 (m, 1H), 7.40-7.22 (m, 7H), 5.54-5.41 (m, 2H), 4.90-4.78 (m, 2H), 3.49 (s, 2H), 3.33 (s, 3H), 3.26-3.20 (m, 2H), 3.16-3.09 (m, 1H), 2.60-2.40 (m, 8H), 1.28-1.23 (m, 3H).
ESI-MS *m/z*: 550.26 [M-1]$^-$

**Example 25. Synthesis of compound I-23 of the present invention**

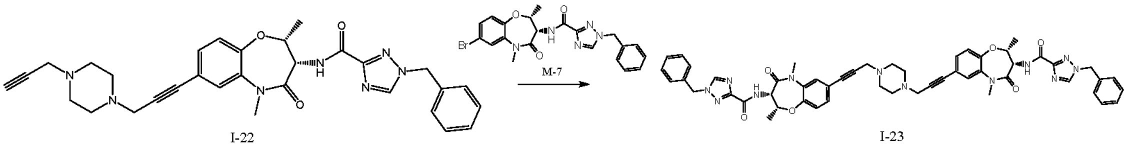


I-22 I-23

Compound **I-22** and intermediate **M-7** were used as raw materials, to obtain compound **I-23,** by the synthesis method similar to Method 1 of Example 4.

$^1$HNMR (400 MHz, $CDCl_3$) $\delta$: 8.09 (d, *J* = 7.0 Hz, 2H), 7.99 (s, 2H), 7.43-7.33 (m, 6H), 7.33-7.23 (m, 8H), 7.12 (d, *J* = 8.2 Hz, 2H), 5.37 (s, 4H), 5.10 (t, *J* = 6.7 Hz, 2H), 5.05-4.93 (m, 2H), 3.54 (s, 4H), 3.40 (s, 6H), 2.90-2.60 (m, 8H), 1.40 (d, *J*= 6.3 Hz, 6H).
ESI-MS *m/z*: 939.41 [M-1]$^-$

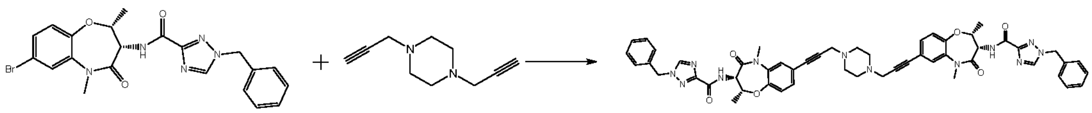

M-7 M-31 I-23

Alternatively, intermediates **M-7** and **M-31** were used as raw materials, to obtain compound **I-23**, by the synthesis method similar to Method 2 of Example 4.

**Example 26. Synthesis of compound I-24 of the present invention**

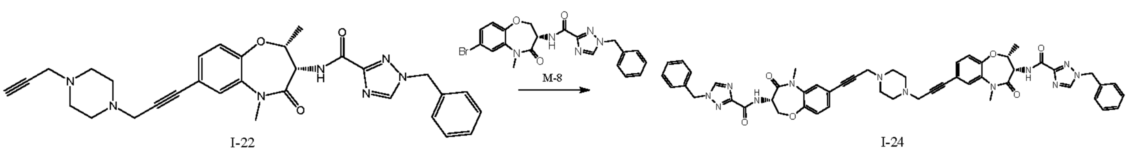


I-22 I-24

Compound **I-22** and intermediate **M-8** were used as raw materials, to obtain compound **I-24,** by the synthesis method similar to Method 1 of Example 4.
ESI-MS *m/z:* 925.40 [M-1]$^-$

**Example 27. Synthesis of compound I-25 of the present invention**

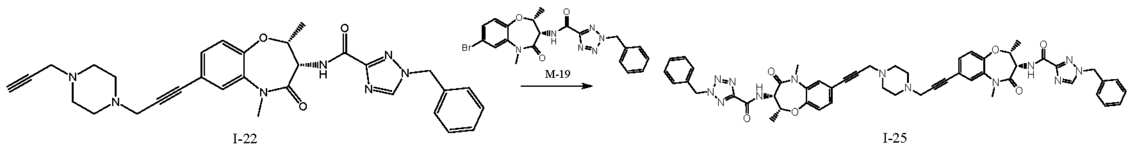


I-22 I-25

Compound **I-22** and intermediate **M-19** were used as raw materials, to obtain compound **I-25,** by the synthesis method similar to Method 1 of Example 4.
ESI-MS *m/z*: 940.41 [M-1]$^-$

**Example 28. Synthesis of compound I-26 of the present invention**

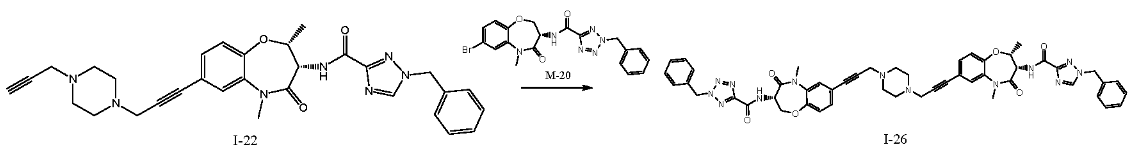


I-22 I-26

Compound **I-22** and intermediate **M-20** were used as raw materials, to obtain compound **I-26,** by the synthesis method similar to Method 1 of Example 4.
ESI-MS *m/z:* 926.39 [M-1]$^-$

**Example 29. Synthesis of compound I-27 of the present invention**

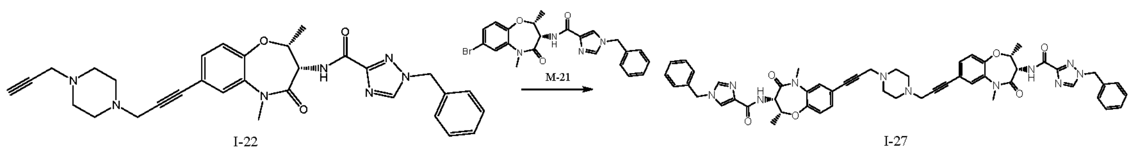


I-22 I-27

Compound **I-22** and intermediate **M-21** were used as raw materials, to obtain compound **I-27,** by the synthesis method similar to Method 1 of Example 4.
ESI-MS *m/z:* 938.42 [M-1]$^-$

**Example 30. Synthesis of compound I-28 of the present invention**

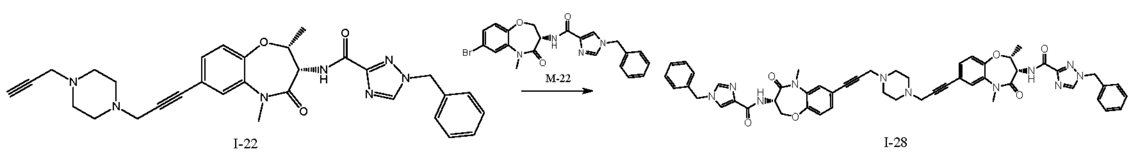


I-22 I-28

Compound **I-22** and intermediate **M-22** were used as raw materials, to obtain compound **I-28,** by the synthesis method similar to Method 1 of Example 4.

ESI-MS *m/z:* 924.40 [M-1]$^-$

**Example 31. Synthesis of compound I-29 of the present invention**

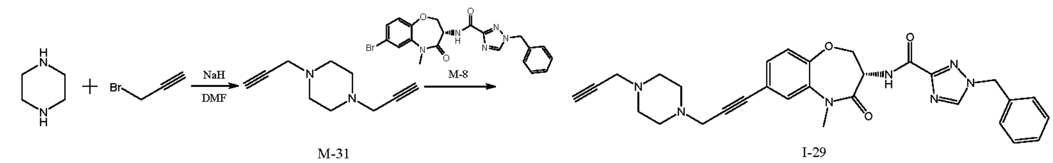


M-31 I-29

Intermediate **M-8** was used as raw material, to obtain compound **I-29** by the synthesis method similar to that of Example 1.
ESI-MS *m/z*: 536.25 [M-1]$^-$

**Example 32. Synthesis of compound I-30 of the present invention**

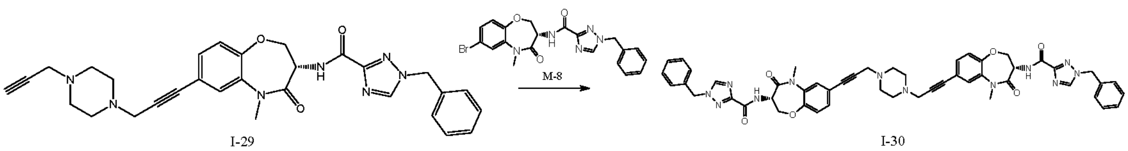


I-29 I-30

Compound **I-29** and intermediate **M-8** were used as raw materials, to obtain compound **I-30,** by the synthesis method similar to Method 1 of Example 4.

$^1$HNMR (400 MHz, DMSO-$d_6$) $\delta$: 8.84 (s, 2H), 8.47 (d, *J*= 8.0 Hz, 2H), 7.70-7.50 (m, 2H), 7.49-7.25 (m, 12H), 7.21 (d, *J*= 8.0 Hz, 2H), 5.48 (s, 4H), 4.86-4.79 (m, 2H), 4.62-4.56 (m, 2H), 4.44-4.39 (m, 2H), 3.52 (s, 4H), 3.40 (s, 6H), 2.78-2.48 (m, 8H).
ESI-MS *m/z*: 911.38 [M-1]$^-$

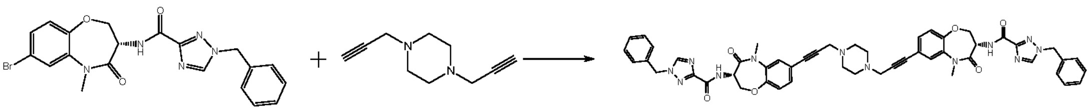

M-8 M-31 I-30

Alternatively, intermediates **M-8** and **M-31** were used as raw materials, to obtain compound **I-30**, by the synthesis method similar to Method 2 of Example 4.

**Example 33. Synthesis of compound I-31 of the present invention**

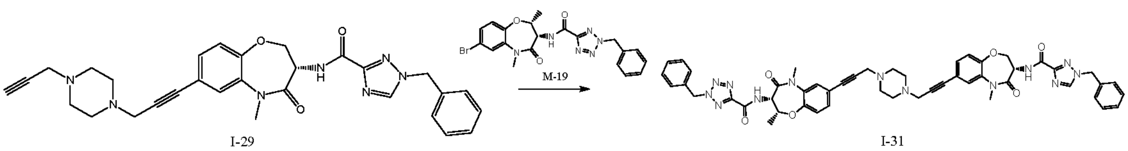


I-29 I-31

Compound **I-29** and intermediate **M-19** were used as raw materials, to obtain compound **I-31,** by the synthesis method similar to Method 1 of Example 4.
ESI-MS *m/z:* 926.39 [M-1]$^-$

**Example 34. Synthesis of compound I-32 of the present invention**

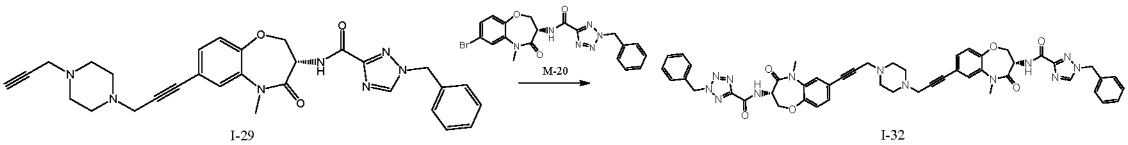


I-29 I-32

Compound **I-29** and intermediate **M-20** were used as raw materials, to obtain compound **I-32,** by the synthesis method similar to Method 1 of Example 4.
ESI-MS *m/z*: 912.38 [M-1]$^-$

**Example 35. Synthesis of compound I-33 of the present invention**

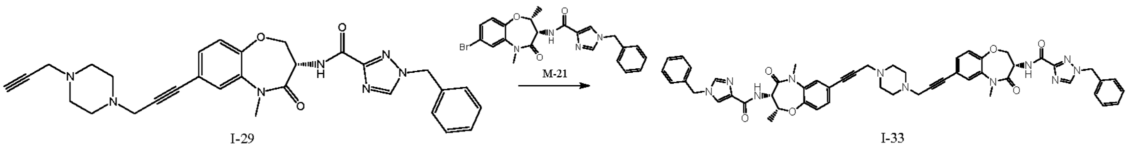


I-29 I-33

Compound **I-29** and intermediate **M-21** were used as raw materials, to obtain compound **I-33,** by the synthesis method similar to Method 1 of Example 4.
ESI-MS *m/z:* 924.40 [M-1]$^-$

**Example 36. Synthesis of compound I-34 of the present invention**

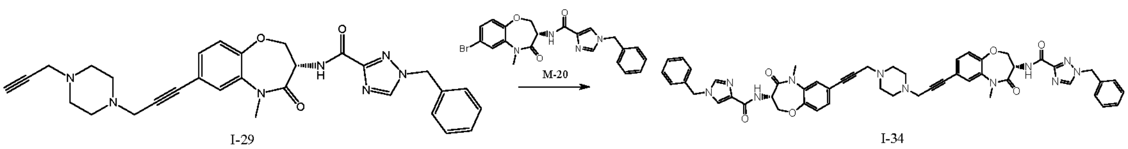


I-29 I-34

Compound **I-29** and intermediate **M-22** were used as raw materials, to obtain compound **I-34,** by the synthesis method similar to Method 1 of Example 4.
ESI-MS *m/z:* 910.39 [M-1]$^-$

**Example 37. Synthesis of compound I-35 of the present invention**

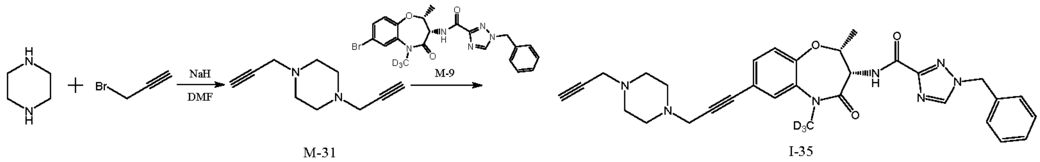


M-31 I-35

Intermediate **M-9** was used as raw material, to obtain compound **I-35** by the synthesis method similar to that of Example 1.
ESI-MS *m/z:* 553.28 [M-1]$^-$

**Example 38. Synthesis of compound I-36 of the present invention**

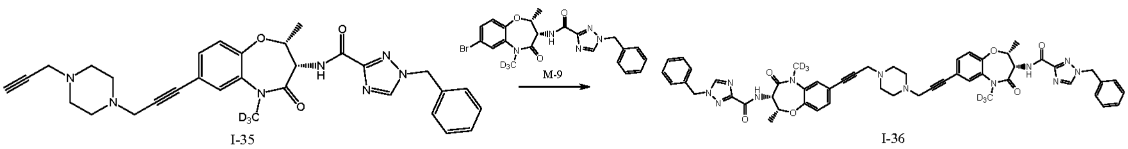


I-35 I-36

Compound **I-35** and intermediate **M-9** were used as raw materials, to obtain compound **I-36,** by the synthesis method similar to Method 1 of Example 4.

$^1$HNMR (400 MHz, $CDCl_3$) $\delta$: 8.10 (d, $J$ = 7.0 Hz, 2H), 8.00 (s, 2H), 7.43-7.34 (m, 6H), 7.34-7.23 (m, 8H), 7.14-7.10 (m, 2H), 5.37 (s, 4H), 5.11 (t, $J$ = 6.7 Hz, 2H), 5.05-4.93 (m, 2H), 3.55 (s, 4H), 2.88-2.68 (m, 8H), 1.41 (d, $J$ = 6.3 Hz, 6H). ESI-MS $m/z$: 945.45 $[M-1]^-$

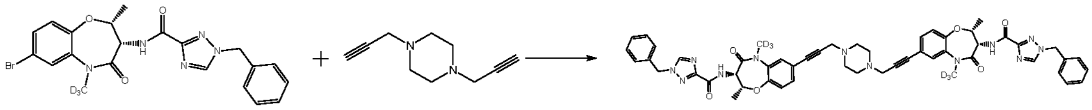

M-9 M-31 I-36

Alternatively, intermediates **M-9** and **M-31** were used as raw materials, to obtain compound **I-36**, by the synthesis method similar to Method 2 of Example 4.

**Example 39. Synthesis of compound I-37 of the present invention**

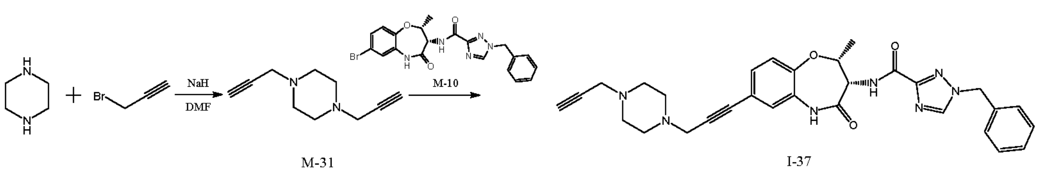


M-31 I-37

Intermediate **M-10** was used as raw material, to obtain compound **I-37** by the synthesis method similar to that of Example 1.
ESI-MS $m/z$: 536.25 $[M-1]^-$

**Example 40. Synthesis of compound I-38 of the present invention**

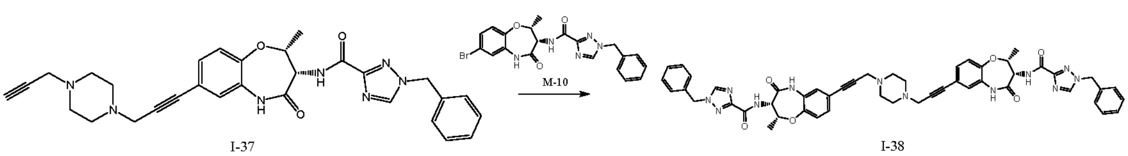


I-37 I-38

Compound **I-37** and intermediate **M-10** were used as raw materials, to obtain compound **I-38,** by the synthesis method similar to Method 1 of Example 4.
ESI-MS $m/z$: 911.38 $[M-1]^-$

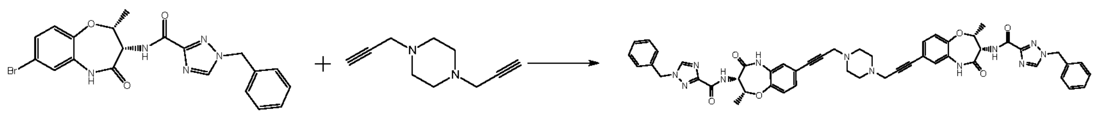

M-10 M-31 I-38

Alternatively, intermediates **M-10** and **M-31** were used as raw materials, to obtain compound **I-38**, by the synthesis method similar to Method 2 of Example 4.

**Example 41. Synthesis of compound I-39 of the present invention**

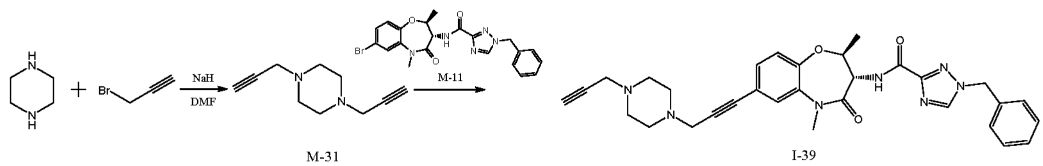


M-31 I-39

Intermediate **M-11** was used as raw material, to obtain compound **I-39** by the synthesis method similar to that of Example 1.
ESI-MS *m/z*: 550.26 $[M-1]^-$

**Example 42. Synthesis of compound I-40 of the present invention**

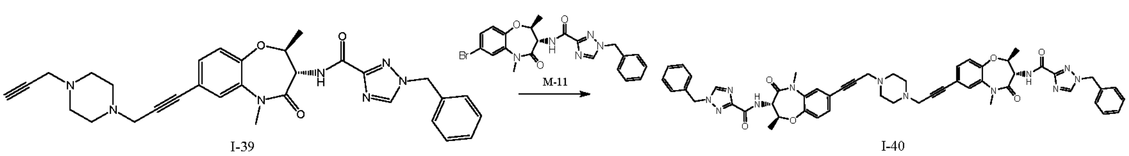


I-39 I-40

Compound **I-39** and intermediate **M-11** were used as raw materials, to obtain compound **I-40,** by the synthesis method similar to Method 1 of Example 4.
ESI-MS *m/z*: 939.41 $[M-1]^-$

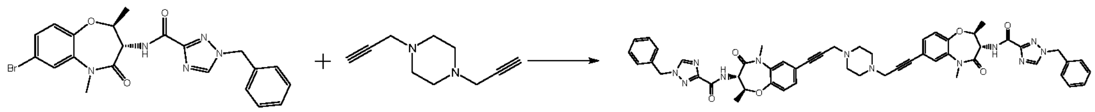

M-11 M-31 I-40

Alternatively, intermediates **M-11** and **M-31** were used as raw materials, to obtain compound **I-40**, by the synthesis method similar to Method 2 of Example 4.

**Example 43. Synthesis of compound I-41 of the present invention**

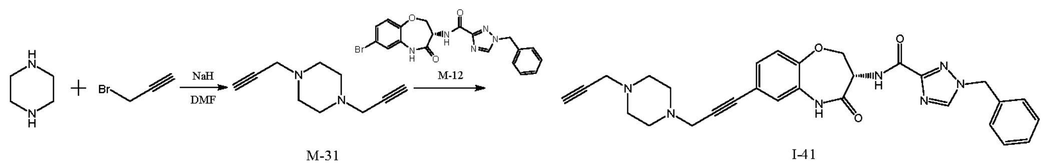


M-31 I-41

Intermediate **M-12** was used as raw material, to obtain compound **I-41** by the synthesis method similar to that of Example 1.
ESI-MS *m/z*: 522.23 [M-1]$^-$

**Example 44. Synthesis of compound I-42 of the present invention**

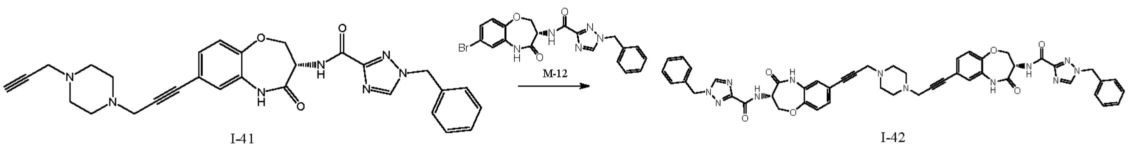


I-41 I-42

Compound **I-41** and intermediate **M-12** were used as raw materials, to obtain compound **I-42,** by the synthesis method similar to Method 1 of Example 4.
ESI-MS *m/z*: 883.35 [M-1]$^-$

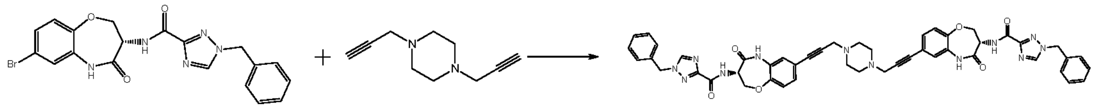

M-12 M-31 I-42

Alternatively, intermediates **M-12** and **M-31** were used as raw materials, to obtain compound **I-42,** by the synthesis method similar to Method 2 of Example 4.

**Example 45. Synthesis of compound I-43 of the present invention**

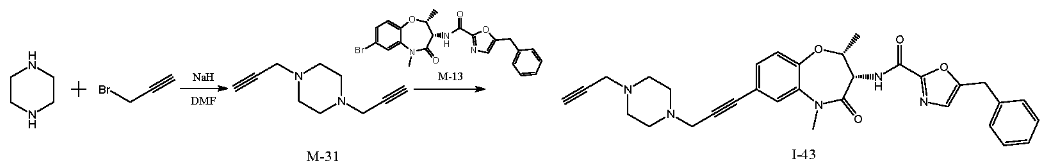


M-31 I-43

Intermediate **M-13** was used as raw material, to obtain compound **I-43** by the synthesis method similar to that of Example 1.
ESI-MS *m/z*: 550.25 [M-1]$^-$

**Example 46. Synthesis of compound I-44 of the present invention**

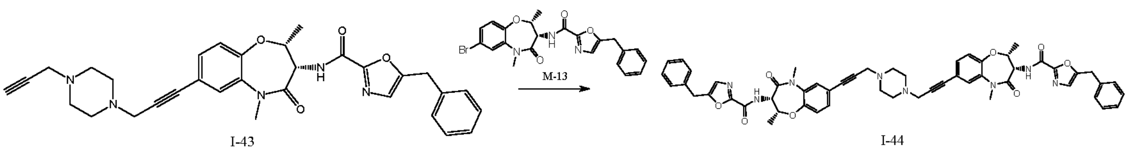


I-43 I-44

Compound **I-43** and intermediate **M-13** were used as raw materials, to obtain compound **I-44,** by the synthesis method similar to Method 1 of Example 4.
ESI-MS *m/z*: 939.39 [M-1]$^-$

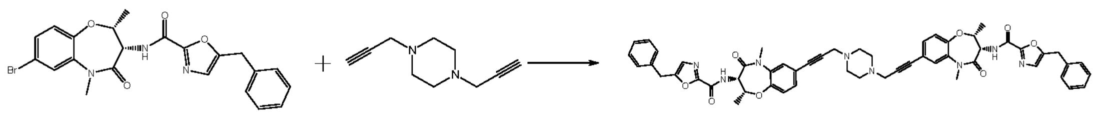

M-13 M-31 I-44

Alternatively, intermediates **M-13** and **M-31** were used as raw materials, to obtain compound **I-44,** by the synthesis method similar to Method 2 of Example 4.

**Example 47. Synthesis of compound I-45 of the present invention**

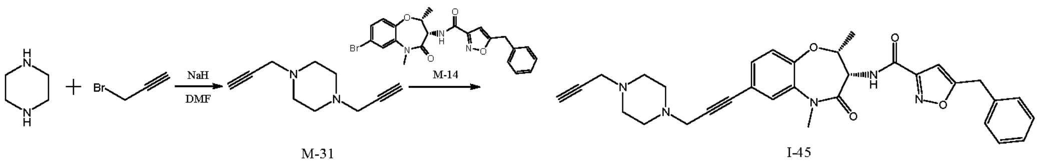


M-31 I-45

Intermediate **M-14** was used as raw material, to obtain compound **I-45** by the synthesis method similar to that of Example 1.
ESI-MS *m/z*: 550.25 $[M-1]^-$

**Example 48. Synthesis of compound I-46 of the present invention**

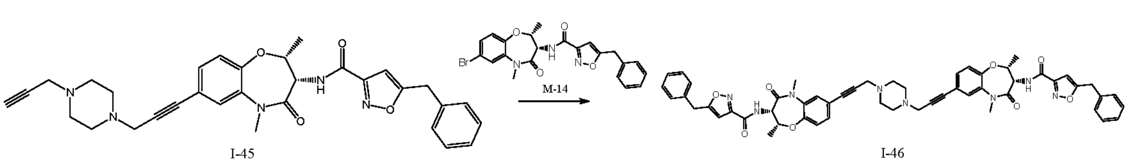


I-45 I-46

Compound **I-45** and intermediate **M-14** were used as raw materials, to obtain compound **I-46,** by the synthesis method similar to Method 1 of Example 4.
ESI-MS *m/z*: 939.39 $[M-1]^-$

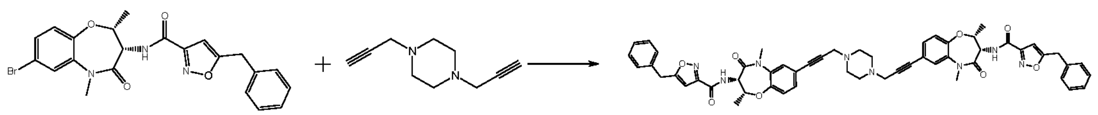

M-14 M-31 I-46

Alternatively, intermediates **M-14** and **M-31** were used as raw materials, to obtain compound **I-46**, by the synthesis method similar to Method 2 of Example 4.

**Example 49. Synthesis of compound I-47 of the present invention**

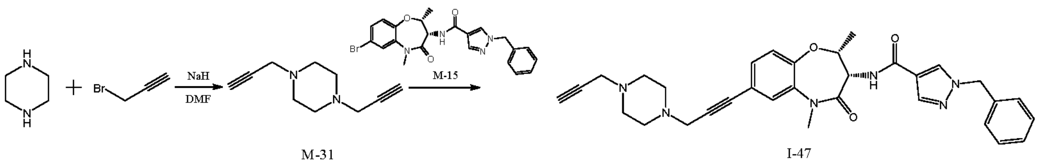


M-31 I-47

Intermediate **M-15** was used as raw material, to obtain compound **I-47** by the synthesis method similar to that of Example 1.
ESI-MS *m/z:* 549.27 $[M-1]^-$

**Example 50. Synthesis of compound I-48 of the present invention**

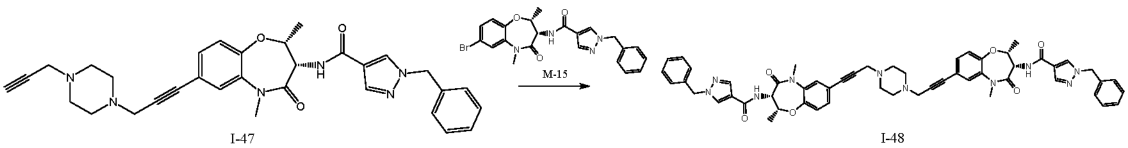


I-47 I-48

Compound **I-47** and intermediate **M-15** were used as raw materials, to obtain compound **I-48,** by the synthesis method similar to Method 1 of Example 4.
ESI-MS *m/z:* 937.42 $[M-1]^-$

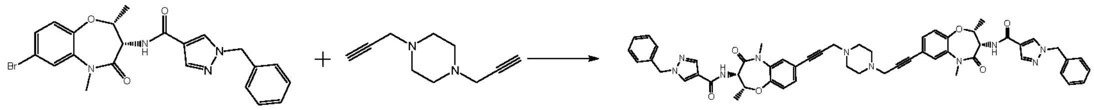

M-15 M-31 I-48

Alternatively, intermediates **M-15** and **M-31** were used as raw materials, to obtain compound **I-48**, by the synthesis method similar to Method 2 of Example 4.

**Example 51. Synthesis of compound I-49 of the present invention**

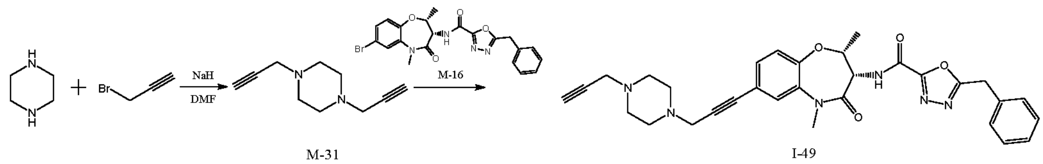


M-31 I-49

Intermediate **M-16** was used as raw material, to obtain compound **I-49** by the synthesis method similar to that of Example 1.
ESI-MS *m/z*: 551.25 $[M-1]^-$

**Example 52. Synthesis of compound I-50 of the present invention**

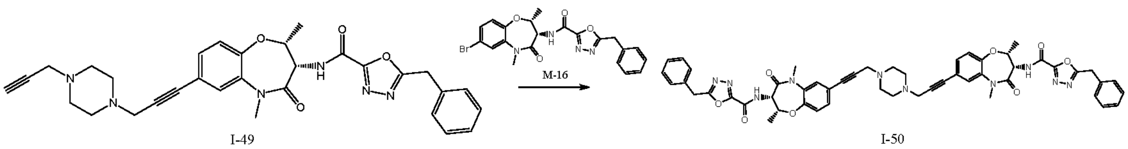


I-49 I-50

Compound **I-49** and intermediate **M-16** were used as raw materials, to obtain compound **I-50,** by the synthesis method similar to Method 1 of Example 4.
ESI-MS *m/z*: 941.38 $[M-1]^-$

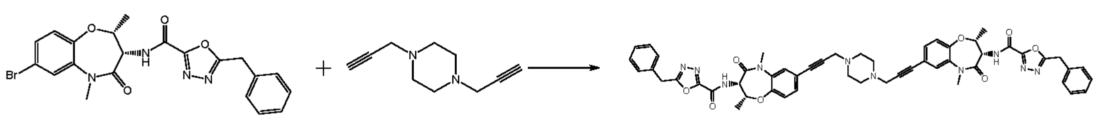

M-16 M-31 I-50

Alternatively, intermediates **M-16** and **M-31** were used as raw materials, to obtain compound **I-50,** by the synthesis method similar to Method 2 of Example 4.

**Example 53. Synthesis of compound I-51 of the present invention**

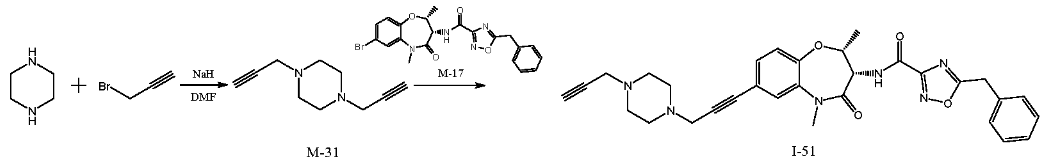


M-31 I-51

Intermediate **M-17** was used as raw material, to obtain compound **I-51** by the synthesis method similar to that of Example 1.
ESI-MS *m/z*: 551.25 [M-1]$^-$

**Example 54. Synthesis of compound I-52 of the present invention**

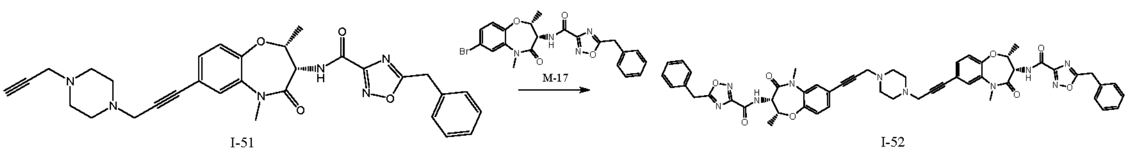


I-51 I-52

Compound **I-51** and intermediate **M-17** were used as raw materials, to obtain compound **I-52,** by the synthesis method similar to Method 1 of Example 4.
ESI-MS *m/z*: 941.38 [M-1]$^-$

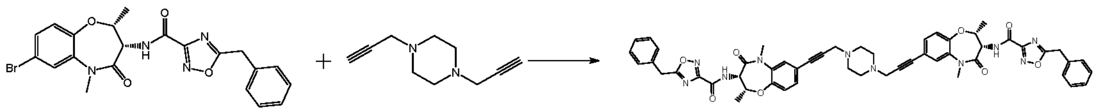

M-17 M-31 I-52

Alternatively, intermediates **M-17** and **M-31** were used as raw materials, to obtain compound **I-52**, by the synthesis method similar to Method 2 of Example 4.

**Example 55. Synthesis of compound I-53 of the present invention**

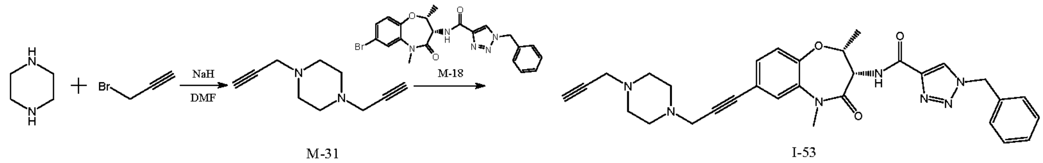


M-31 I-53

Intermediate **M-18** was used as raw material, to obtain compound **I-53** by the synthesis method similar to that of Example 1.
ESI-MS *m/z*: 550.26 [M-1]$^-$

**Example 56. Synthesis of compound I-54 of the present invention**

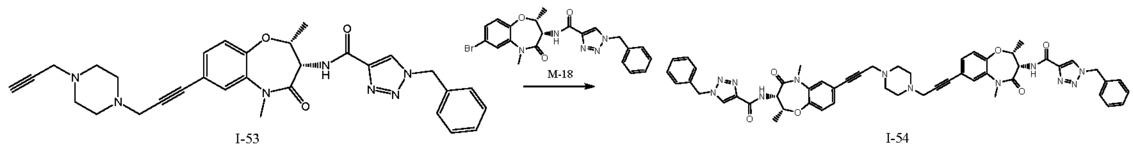

I-53 I-54

Compound **I-53** and intermediate **M-18** were used as raw materials, to obtain compound **I-54,** by the synthesis method similar to Method 1 of Example 4.
ESI-MS *m/z*: 939.41 [M-1]$^-$

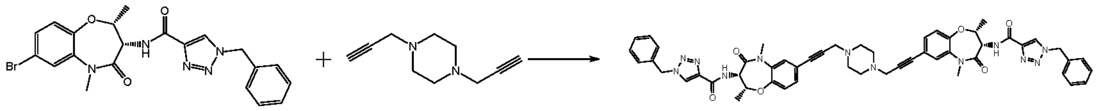
M-18 M-31 I-54

Alternatively, intermediates **M-18** and **M-31** were used as raw materials, to obtain compound **I-54**, by the synthesis method similar to Method 2 of Example 4.

**Example 57. Synthesis of compound I-55 of the present invention**

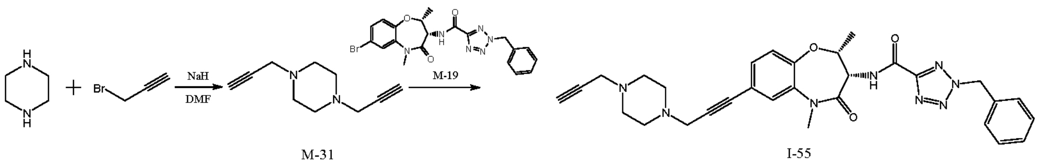

M-31 I-55

Intermediate **M-19** was used as raw material, to obtain compound **I-55** by the synthesis method similar to that of Example 1.
ESI-MS *m/z*: 551.26 [M-1]$^-$

**Example 58. Synthesis of compound I-56 of the present invention**

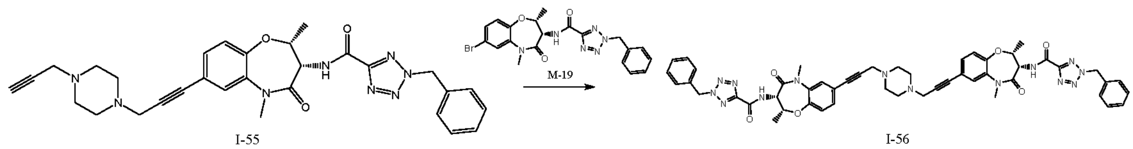

I-55 I-56

Compound **I-55** and intermediate **M-19** were used as raw materials, to obtain compound **I-56,** by the synthesis method similar to Method 1 of Example 4.
ESI-MS *m/z*: 941.40 [M-1]$^-$

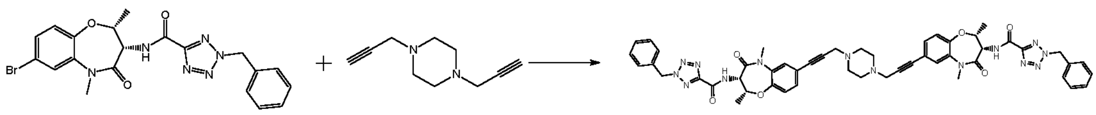

M-19 M-31 I-56

Alternatively, intermediates **M-19** and **M-31** were used as raw materials, to obtain compound **I-56,** by the synthesis method similar to Method 2 of Example 4.

**Example 59. Synthesis of compound I-57 of the present invention**

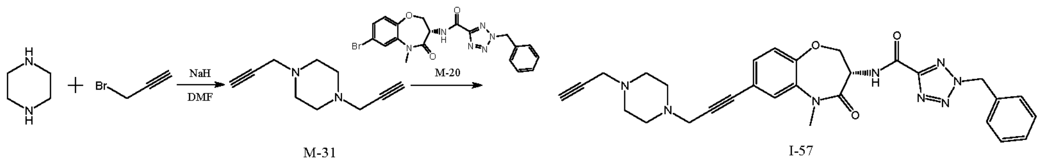


M-31 I-57

Intermediate **M-20** was used as raw material, to obtain compound **I-57** by the synthesis method similar to that of Example 1.
ESI-MS *m/z*: 537.24 $[M-1]^-$

**Example 60. Synthesis of compound I-58 of the present invention**

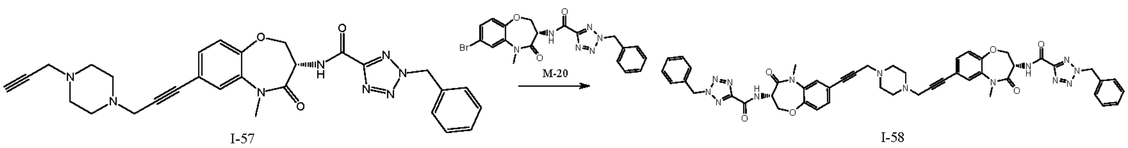


I-57 I-58

Compound **I-57** and intermediate **M-20** were used as raw materials, to obtain compound **I-58,** by the synthesis method similar to Method 1 of Example 4.
ESI-MS *m/z*: 913.37 $[M-1]^-$

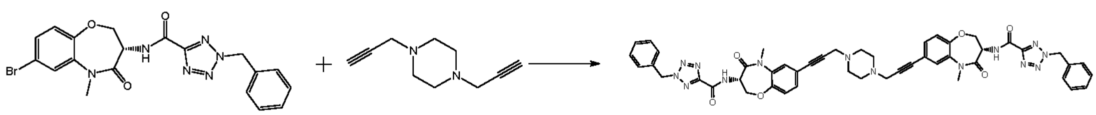

M-20 M-31 I-58

Alternatively, intermediates **M-20** and **M-31** were used as raw materials, to obtain compound **I-58,** by the synthesis method similar to Method 2 of Example 4.

**Example 61. Synthesis of compound I-59 of the present invention**

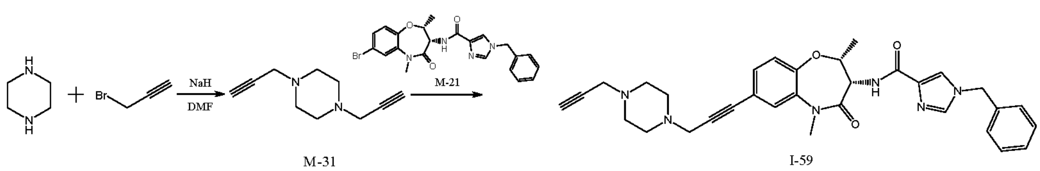


M-31 I-59

Intermediate **M-21** was used as raw material, to obtain compound **I-59** by the synthesis method similar to that of Example 1.
ESI-MS *m/z:* 549.27 [M-1]$^-$

**Example 62. Synthesis of compound I-60 of the present invention**

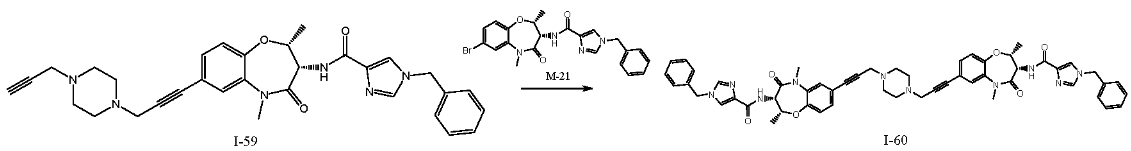


I-59 I-60

Compound **I-59** and intermediate **M-21** were used as raw materials, to obtain compound **I-60,** by the synthesis method similar to Method 1 of Example 4.
ESI-MS *m/z:* 937.42 [M-1]$^-$

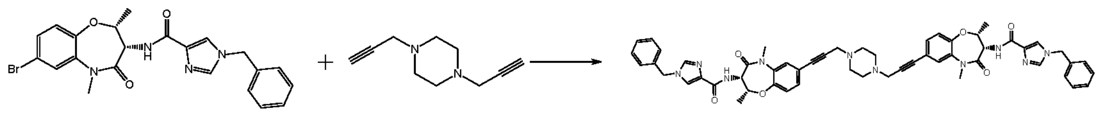

M-21 M-31 I-60

Alternatively, intermediates **M-21** and **M-31** were used as raw materials, to obtain compound **I-60**, by the synthesis method similar to Method 2 of Example 4.

**Example 63. Synthesis of compound I-61 of the present invention**

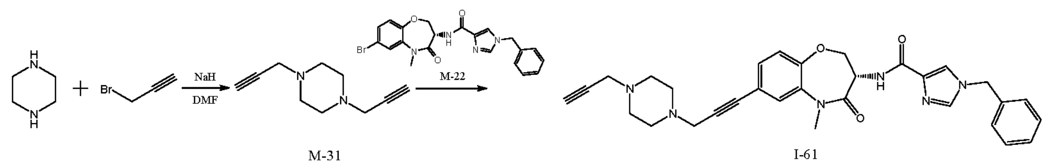


M-31 I-61

Intermediate **M-22** was used as raw material to obtain compound **I-61** by the synthesis method similar to that of Example 1.
ESI-MS *m/z*: 535.25 [M-1]$^-$

**Example 64. Synthesis of compound I-62 of the present invention**

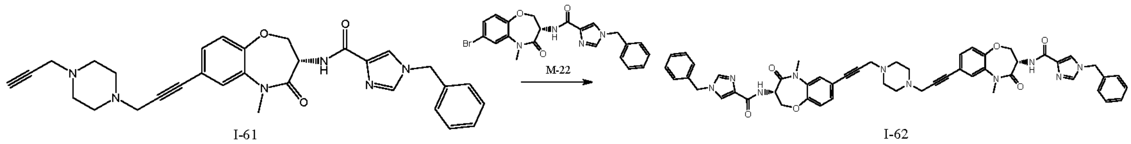


I-61 I-62

Compound **I-61** and intermediate M-22 were used as raw materials, to obtain compound I-62, by the synthesis method similar to Method 1 of Example 4.
ESI-MS *m/z:* 909.39 [M-1]$^-$

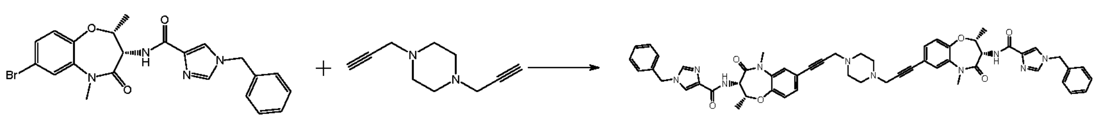

M-21 M-31 I-60

Alternatively, intermediates **M-22** and **M-31** were used as raw materials, to obtain compound **I-62**, by the synthesis method similar to Method 2 of Example 4.

**Example 65. Synthesis of compound I-63 of the present invention**

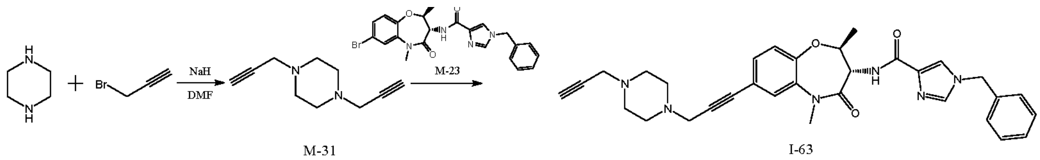


M-31 I-63

Intermediate **M-23** was used as raw material to obtain compound **I-63** by the synthesis method similar to that of Example 1.
ESI-MS *m/z:* 549.27 [M-1]$^-$

**Example 66. Synthesis of compound I-64 of the present invention**

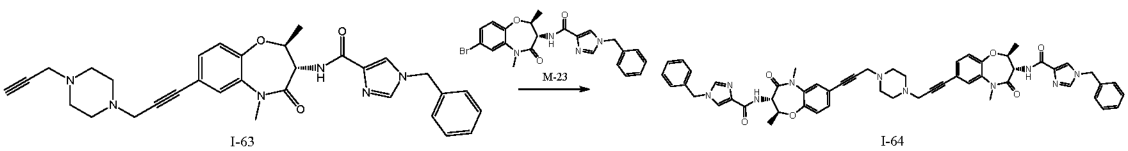


I-63 I-64

Compound **I-63** and intermediate **M-23** were used as raw materials, to obtain compound **I-64,** by the synthesis method similar to Method 1 of Example 4.
ESI-MS *m/z:* 937.42 [M-1]$^-$

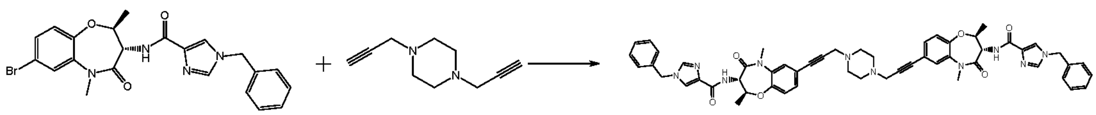

M-23 M-31 I-64

Alternatively, intermediates **M-23** and **M-31** were used as raw materials, to obtain compound **I-64**, by the synthesis method similar to Method 2 of Example 4.

**Example 67. Synthesis of compound I-65 of the present invention**

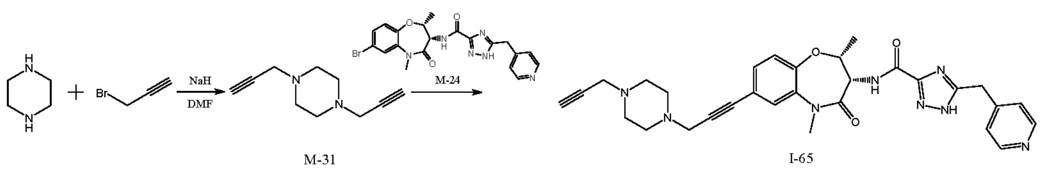


M-31 I-65

Intermediate **M-24** was used as raw material to obtain compound **I-65** by the synthesis method similar to that

of Example 1.
ESI-MS *m/z:* 551.26 [M-1]$^-$

**Example 68. Synthesis of compound I-66 of the present invention**

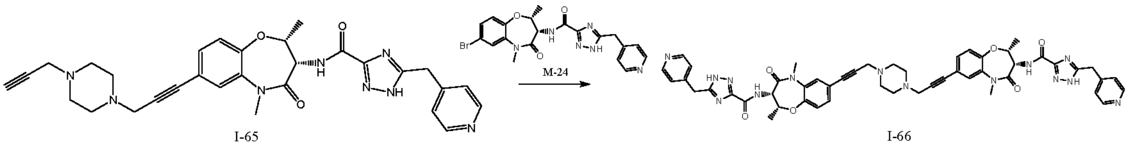


I-65 I-66

Compound **I-65** and intermediate **M-24** were used as raw materials, to obtain compound **I-66,** by the synthesis method similar to Method 1 of Example 4.
ESI-MS *m/z:* 941.40 [M-1]$^-$

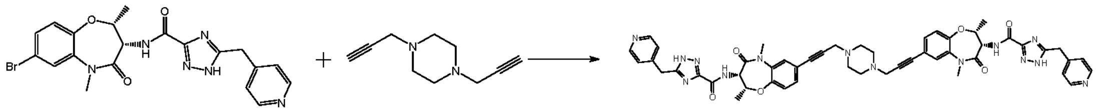

M-24 M-31 I-66

Alternatively, intermediates **M-24** and **M-31** were used as raw materials, to obtain compound **I-66**, by the synthesis method similar to Method 2 of Example 4.

**Example 69. Synthesis of compound I-67 of the present invention**

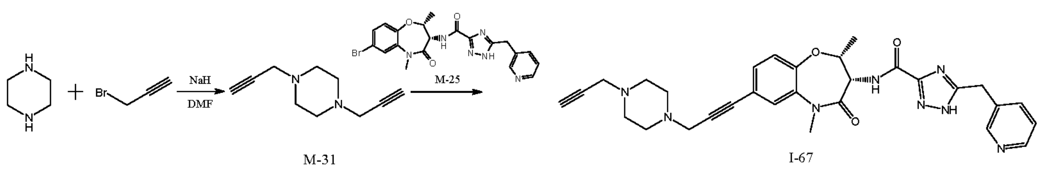


M-31 I-67

Intermediate **M-25** was used as raw material to obtain compound **I-67** by the synthesis method similar to that of Example 1.
ESI-MS *m/z*: 551.26 [M-1]$^-$

**Example 70. Synthesis of compound I-68 of the present invention**

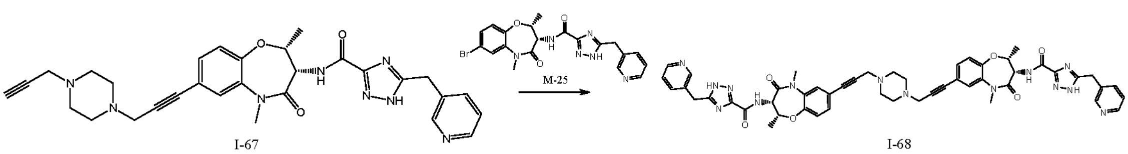


I-67 I-68

Compound **I-67** and intermediate **M-25** were used as raw materials, to obtain compound **I-68,** by the synthesis method similar to Method 1 of Example 4.
ESI-MS *m/z*: 941.40 [M-1]$^-$

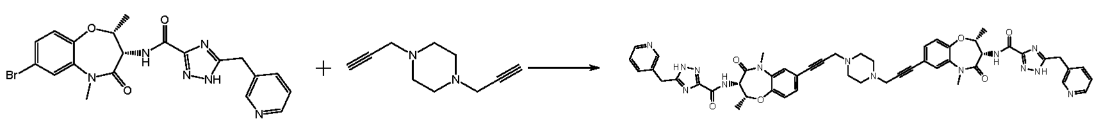

M-25 M-31 I-68

Alternatively, intermediates **M-25** and **M-31** were used as raw materials, to obtain compound **I-68,** by the synthesis method similar to Method 2 of Example 4.

**Example 71. Synthesis of compound I-69 of the present invention**

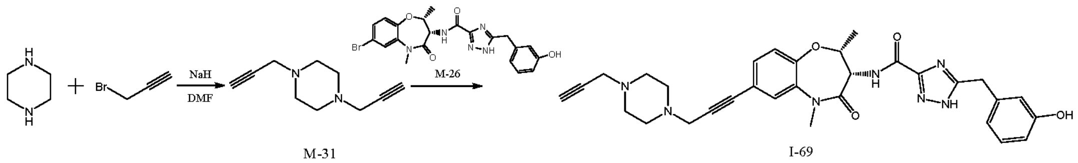


M-31 I-69

Intermediate **M-26** was used as raw material to obtain compound **I-69** by the synthesis method similar to that of Example 1.
ESI-MS *m/z:* 566.26 [M-1]$^-$

**Example 72. Synthesis of compound I-70 of the present invention**

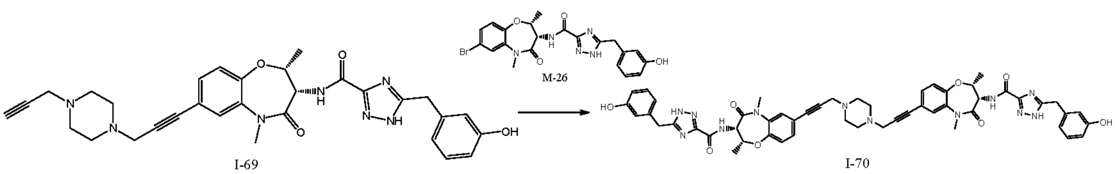


I-69 I-70

Compound **I-69** and intermediate **M-26** were used as raw materials, to obtain compound **I-70,** by the synthesis method similar to Method 1 of Example 4.
ESI-MS *m/z*: 971.40 [M-1]$^-$

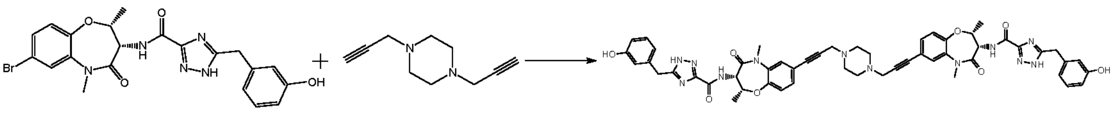

M-26 M-31 I-70

Alternatively, intermediates **M-26** and **M-31** were used as raw materials, to obtain compound **I-70**, by the synthesis method similar to Method 2 of Example 4.

**Example 73. Synthesis of compound I-71 of the present invention**

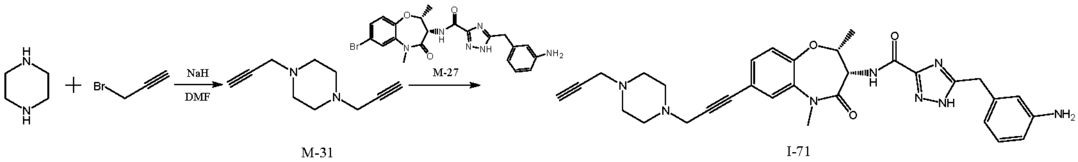


M-31 I-71

Intermediate **M-27** was used as raw material to prepare compound **I-69** by the synthesis method similar to that of Example 1.
ESI-MS *m/z*: 565.28 [M-1]⁻

**Example 74. Synthesis of compound I-72 of the present invention**

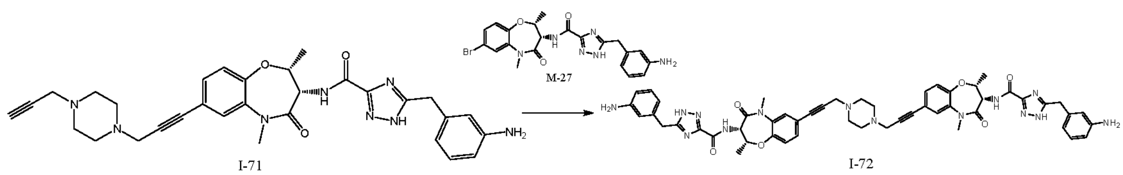

M-27

I-71

I-72

Compound **I-71** and intermediate **M-27** were used as raw materials, to prepare compound **I-72**, by the synthesis method similar to Method 1 of Example 4.
ESI-MS *m/z:* 969.44 [M-1]⁻

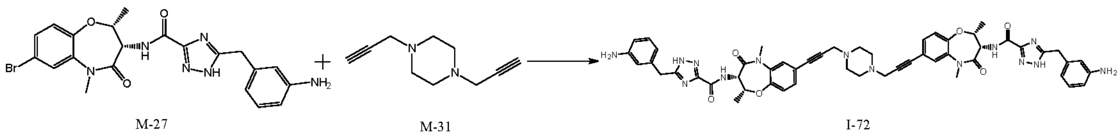

M-27

M-31

I-72

Alternatively, intermediates **M-27** and **M-31** were used as raw materials, to prepare compound **I-72**, by the synthesis method similar to Method 2 of Example 4.

**Example 75. Synthesis of compound I-73 of the present invention**

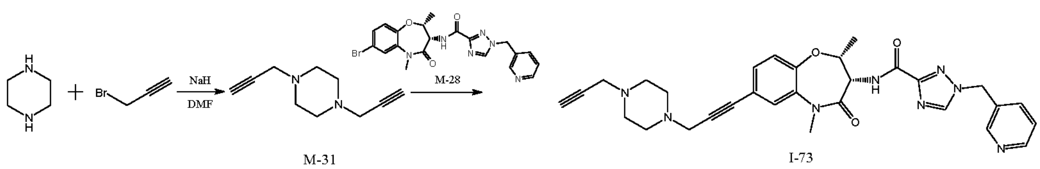


M-28

M-31

I-73

Intermediate **M-28** was used as raw material to prepare compound **I-73** by the synthesis method similar to that of Example 1.
ESI-MS *m/z*: 551.26 [M-1]⁻

**Example 76. Synthesis of compound I-74 of the present invention**

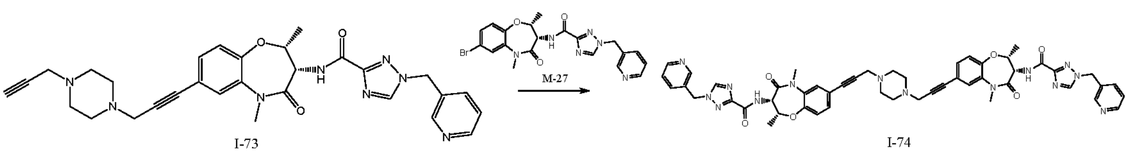

M-27

I-73

I-74

Compound **I-73** and intermediate **M-28** were used as raw materials, to prepare compound **I-74**, by the synthesis method similar to Method 1 of Example 4.
ESI-MS *m/z*: 941.40 [M-1]⁻

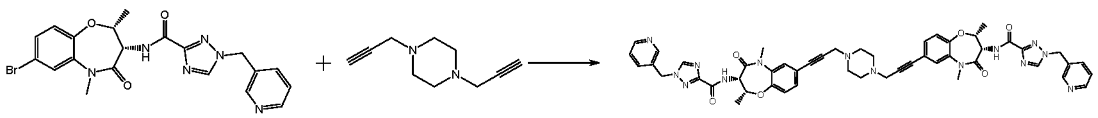

M-27 M-31 I-74

Alternatively, intermediates **M-28** and **M-31** were used as raw materials, to prepare compound **I-74,** by the synthesis method similar to Method 2 of Example 4.

**Example 77. Synthesis of compound I-75 of the present invention**

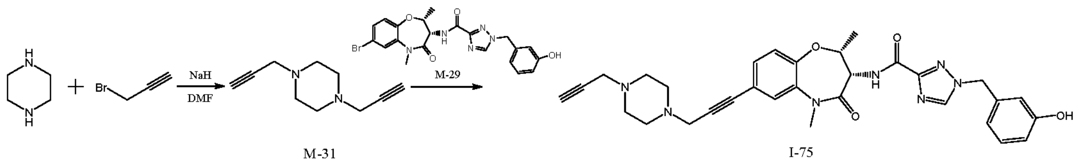


M-31 I-75

Intermediate M-29 was used as raw material to prepare compound **I-75** by the synthesis method similar to that of Example 1.
ESI-MS *m/z:* 566.26 [M-1]⁻

**Example 78. Synthesis of compound I-76 of the present invention**

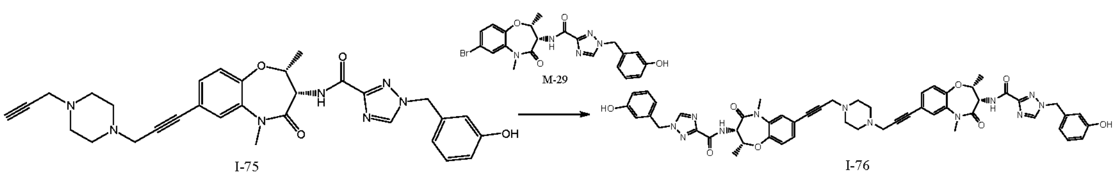


I-75 I-76

Compound **I-75** and intermediate **M-29** were used as raw materials, to prepare compound **I-76**, by the synthesis method similar to Method 1 of Example 4.
ESI-MS *m/z*: 971.40 [M-1]⁻

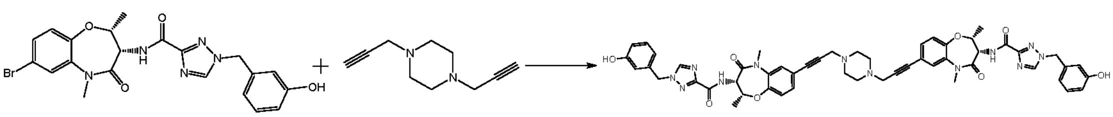

M-29 M-31 I-76

Alternatively, intermediates **M-29** and **M-31** were used as raw materials, to prepare compound **I-76**, by the synthesis method similar to Method 2 of Example 4.

**Example 79. Synthesis of compound I-77 of the present invention**

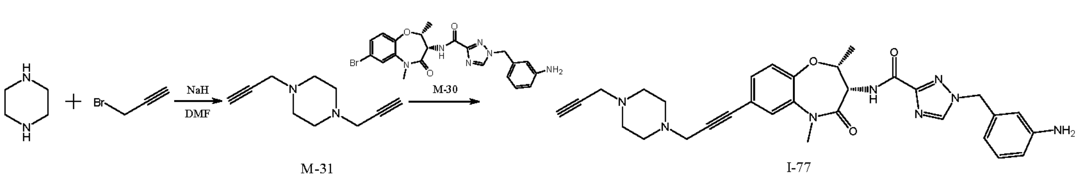


M-31 I-77

Intermediate **M-30** was used as raw material to prepare compound **I-77** by the synthesis method similar to that of Example 1.
ESI-MS *m*/*z*: 565.28 [M-1]$^-$

**Example 80. Synthesis of compound I-78 of the present invention**

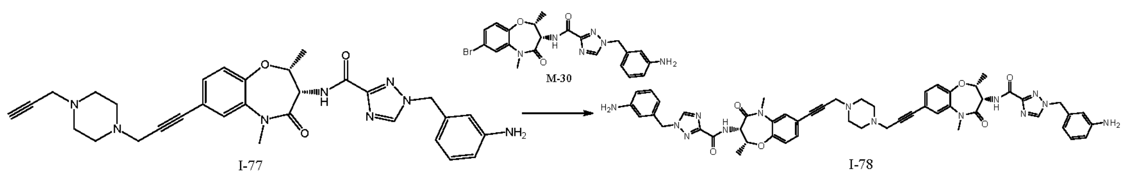

M-30

I-77 I-78

Compound **I-77** and intermediate **M-30** were used as raw materials, to prepare compound **I-78**, by the synthesis method similar to Method 1 of Example 4.
ESI-MS *m*/*z:* 969.44 [M-1]$^-$

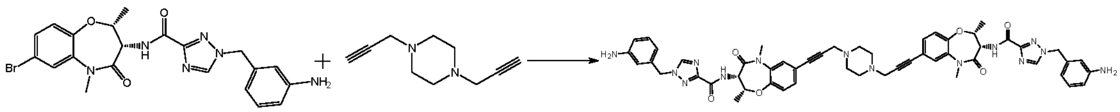

M-30 M-31 I-78

Alternatively, intermediates **M-30** and **M-31** were used as raw materials, to prepare compound **I-78**, by the synthesis method similar to Method 2 of Example 4.

**Example 81. Synthesis of compound I-79 of the present invention**

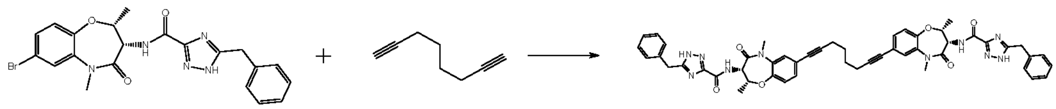

M-1 S-1 I-79

400 mg of intermediate **M-1** (0.85 mmol) was transferred to a flask, to which were added 20 mg of palladium acetate, 20 mg of CuI, 40 mg of 2-dicyclohexylphosphino-2,4,6-triisopropylbiphenyl, 400 mg of triphenylphosphine, 50 mg of 1,7-octanediyne (**S-1**, 0.47 mmol), 5 mL of triethylamine, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-79**.
ESI-MS *m*/*z*: 883.38 [M-1]$^-$

**Example 82. Synthesis of compound I-80 of the present invention**

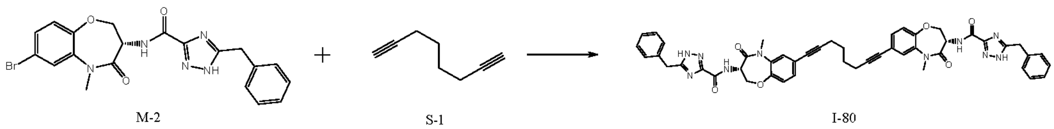

M-2 S-1 I-80

Intermediates **M-30** and **S-1** were used as raw materials, to prepare compound **I-80**, by the synthesis method similar to that of Example 81.
ESI-MS *m/z*: 855.34 [M-1]⁻

**Example 83. Synthesis of compound I-81 of the present invention**

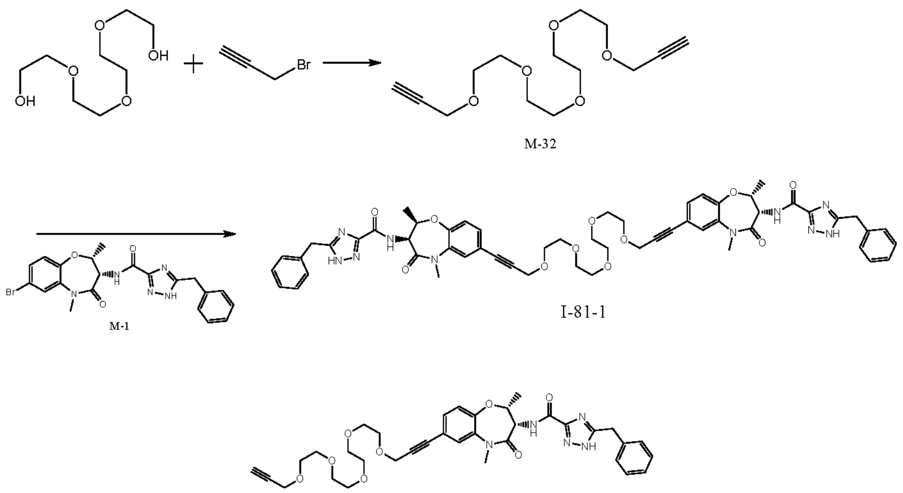

M-32

M-1

I-81-1

I-81-2

10 mL of 50% NaOH aqueous solution was placed in a flask, to which were added 10 mL of toluene, 0.1 g of TBTU, and 1 g of tetra-polyethylene glycol, and then the mixture was stirred to dissolve. Then, 80% solution of propargyl bromide (2 g) in toluene was added, and the reaction solution was allowed to react in an oil bath at 60 °C for 20 h. The reaction system was cooled to room temperature, to which were added 20 mL of ethyl acetate and 20 mL of water, and then the resultant solution was shaken and stood for layering. The ethyl acetate layer was collected, and the water layer was extracted twice with 10 mL of ethyl acetate. The ethyl acetate layers were combined, washed once with saturated NaCl aqueous solution, dried with anhydrous sodium sulfate for 30 min, and filtered to remove anhydrous sodium sulfate. The filtrate was concentrated to dryness to obtain intermediate **M-32.**

400 mg of intermediate **M-1** (0.85 mmol) was transferred to a flask, to which were added 20 mg of palladium acetate, 20 mg of CuI, 40 mg of 2-dicyclohexylphosphino-2,4,6-triisopropylbiphenyl, 400 mg of triphenylphosphine, 127 mg of intermediate M-32 (0.47 mmol), 5 mL of triethylamine, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-81-1**:

ESI-MS *m/z:* 1047.44 [M-1]⁻
and compound **I-81-2:**
ESI-MS *m/z*: 658.30 [M-1]⁻.

**Example 84. Synthesis of compound I-82 of the present invention**

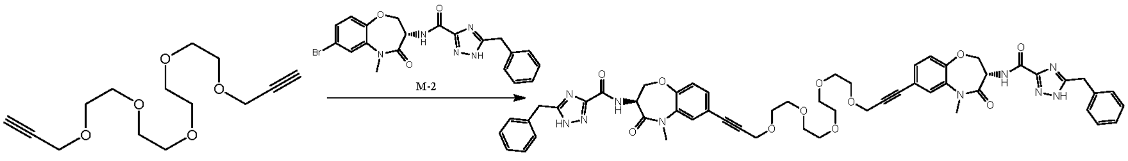

M-2

M-32

I-82-1

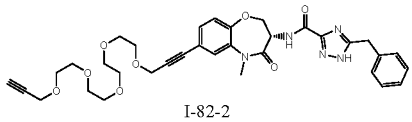

I-82-2

Intermediates **M-2** and **M-32** were used as raw materials, to prepare compound **I-82-1** and **I-82-2**, by the synthesis method similar to that of Example 84.

1-82-1, ESI-MS *m/z:* 1019.40 [M-1]$^-$

1-82-2, ESI-MS *m/z:* 644.23 [M-1]$^-$

**Example 85. Synthesis of compound I-83 of the present invention**

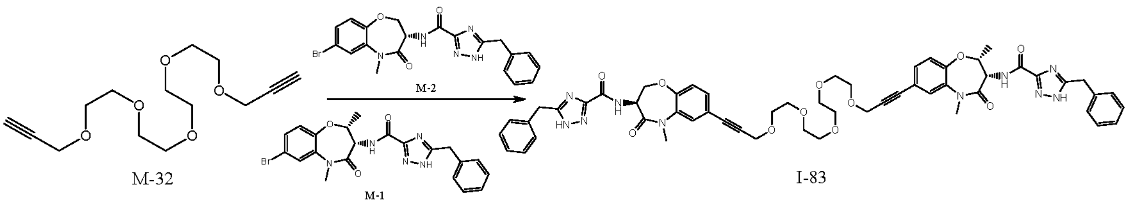

M-2

M-32

M-1

I-83

Intermediates **M-1, M-2** and **M-32** were used as raw materials, to prepare compound **I-83,** by the synthesis method similar to that of Example 84.

ESI-MS *m/z:* 1033.43 [M-1]$^-$

**Example 86. Synthesis of compound I-84 of the present invention**

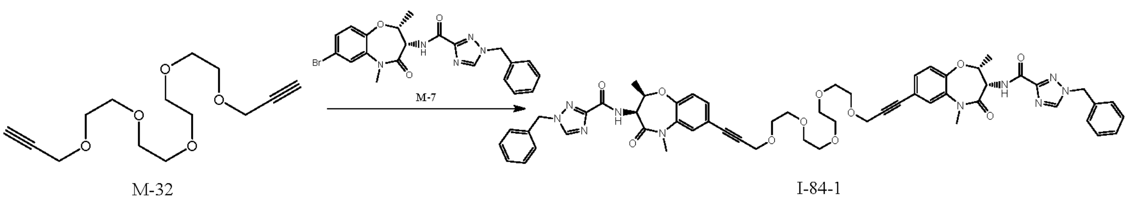

M-7

M-32

I-84-1

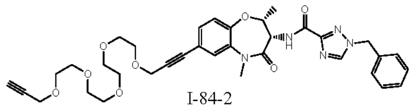

I-84-2

Intermediates **M-7** and **M-32** were used as raw materials, to prepare compounds **I-84-1** and

**I-84-2,** by the synthesis method similar to that of Example 84.

**I-84-1,** ESI-MS *m/z:* 1047.43 [M-1]$^-$

**I-84-2**, ESI-MS *m/z:* 658.28 [M-1]$^-$

**Example 87. Synthesis of compound I-85 of the present invention**

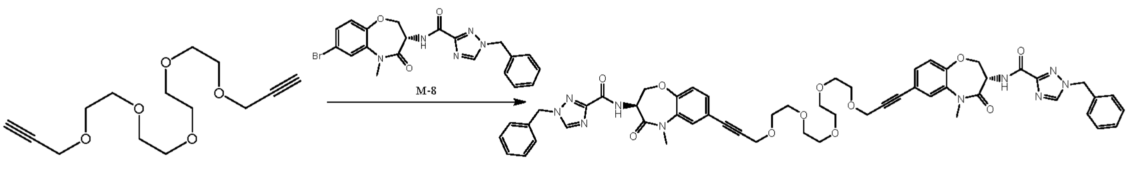


M-32 I-85-1

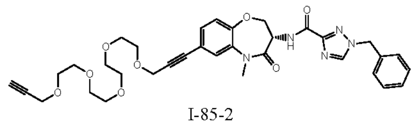

I-85-2

Intermediates **M-8** and **M-32** were used as raw materials, to prepare compounds **I-85-1** and **I-85-2**, by the synthesis method similar to that of Example 84.
$^1$HNMR spectral data of **I-85-1**:

$^1$HNMR (400 MHz, DMSO-$d_6$) $\delta$: 8.84 (s, 2H), 8.47 (d, *J* = 7.8Hz, 2H), 7.66-7.58 (m, 2H), 7.43-7.27 (m, 12H), 7.26-7.20 (m, 2H), 5.49 (s, 4H), 4.90-4.78 (m, 2H), 4.65-4.55 (m, 2H), 4.46-4.38 (m, 4H), 3.68-3.56 (m, 4H), 3.56-3.46 (m, 10H), 3.44-3.39 (m, 2H), 3.31 (s, 6H).
ESI-MS *m/z:* 1019.40 [M-1]$^-$
1-85-2, ESI-MS *m/z:* 644.27 [M-1]$^-$

## Example 88. Synthesis of compound I-86 of the present invention

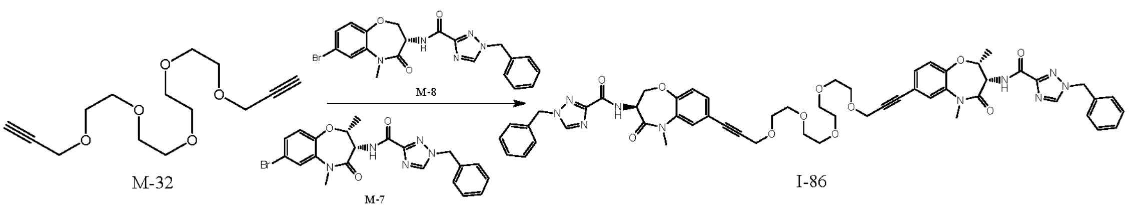


M-32 I-86

Intermediates **M-7, M-8** and **M-32** were used as raw materials, to prepare compound **I-86,** by the synthesis method similar to that of Example 84.
ESI-MS *m/z:* 1033.43 [M-1]$^-$

## Example 89. Synthesis of compound I-87 of the present invention

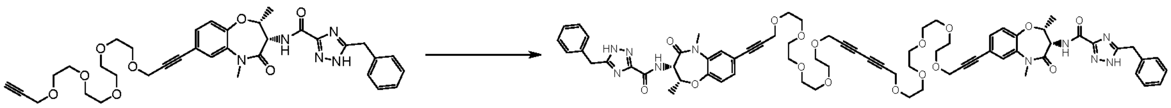

I-81-2 I-87

200 mg of intermediate **I-81-2** (0.30 mmol) was transferred to a flask, to which were added 10 mg of palladium acetate, 10 mg of CuI, 20 mg of 2-dicyclohexylphosphino-2,4,6-triisopropylbiphenyl, 200 mg of triphenylphosphine, 3 mL of triethylamine, and 10 mL of acetonitrile; the mixture was allowed to react overnight in an oil bath at 80 °C under nitrogen protection. TLC indicated the disappearance of raw materials. 70 mL of water was slowly added, and then the resultant solution was extracted twice with 50 mL of ethyl acetate. The ethyl acetate layers were combined, washed twice with 30 mL of saturated NaCl aqueous solution, dried with 5 g of anhydrous sodium sulfate for 30 min, and then filtered to remove sodium sulfate. The filtrate was concentrated to dry, and the residue was purified over column chromatography, to provide compound **I-87**:

ESI-MS *m*/*z*: 1315.58 [M-1]-

**Example 90. Synthesis of compound I-88 of the present invention**

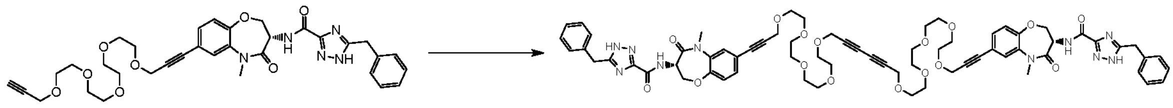

I-82-2 I-88

Intermediate **I-82-2** was used as raw material, to prepare compound **I-88,** by the synthesis method similar to that of Example 89.
ESI-MS *m*/*z:* 1287.54 [M-1]-

**Example 91. Synthesis of compound I-89 of the present invention**

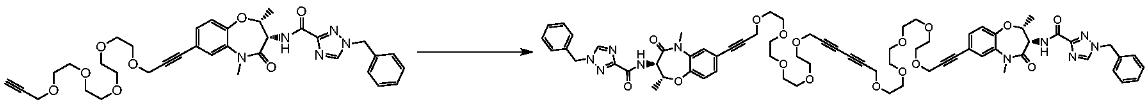

I-84-2 I-89

Compound **I-84-2** was used as raw material, to prepare compound **I-89,** by the synthesis method similar to that of Example 89.

$^1$HNMR (400 MHz, DMSO-$d_6$) $\delta$: 8.85 (s, 2H), 8.66-8.60 (m, 2H), 8.20-8.12 (m, 2H), 7.42-7.24 (m, 14H), 5.56-5.43 (m, 4H), 4.90-4.82 (m, 4H), 4.44-4.38 (m, 2H), 4.36-4.28 (m, 6H), 3.68-3.62 (m, 8H), 3.62-3.48 (m, 20H), 3.38-3.32 (m, 2H), 2.71 (s, 6H), 1.42-1.22 (m, 6H).
ESI-MS *m*/*z*: 1315.58 [M-1]-

**Example 92. Synthesis of compound I-90 of the present invention**

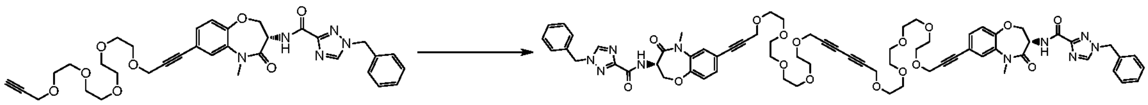

I-85-2 I-90

Compound **I-85-2** was used as raw material, to prepare compound **I-90,** by the synthesis method similar to that of Example 89.
ESI-MS *m*/*z:* 1287.54 [M-1]-

**Example 93. Synthesis of compound I-91 of the present invention**

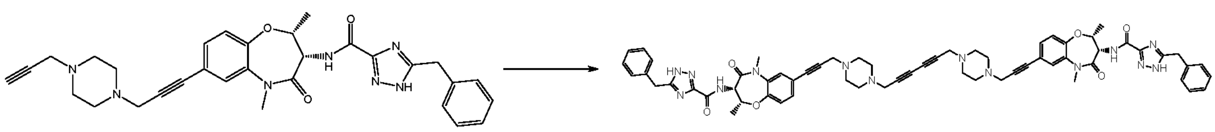

I-1 I-91

Compound **I-1** was used as raw material, to prepare compound **I-91**, by the synthesis method similar to that of Example 89.
ESI-MS *m/z*: 1109.51 [M-1]$^-$

**Example 94. Synthesis of compound I-92 of the present invention**

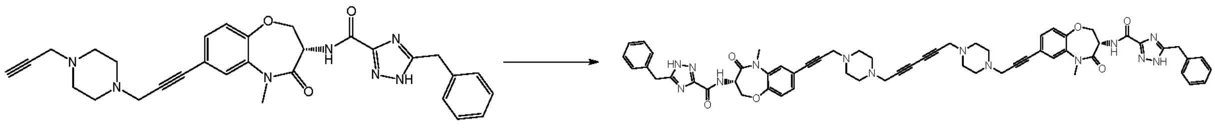

I-10 I-92

Compound **I-10** was used as raw material, to prepare compound **I-92**, by the synthesis method similar to that of Example 89.
ESI-MS *m/z*: 1071.48 [M-1]$^-$

**Example 95. Synthesis of compound I-93 of the present invention**

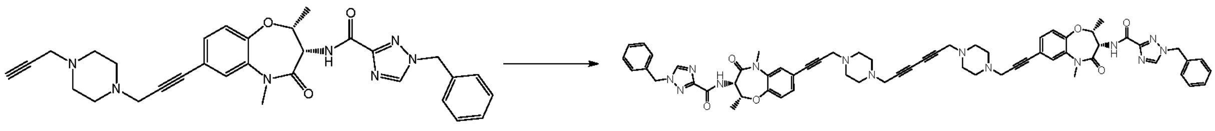

I-22 I-93

Compound **I-22** was used as raw material, to prepare compound **I-93,** by the synthesis method similar to that of Example 89.
ESI-MS *m/z*: 1109.51 [M-1]$^-$

**Example 96. Synthesis of compound I-94 of the present invention**

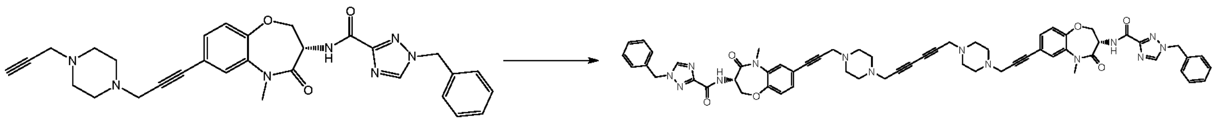

I-29 I-94

Compound **I-29** was used as raw material, to prepare compound **I-94,** by the synthesis method similar to that of Example 89.

$^1$HNMR (400 MHz, DMSO-$d_6$) $\delta$: 8.84 (s, 2H), 8.47 (d, *J* = 8.0 Hz, 2H), 7.58 (s, 2H), 7.39-7.28 (m, 12H), 7.21 (d, *J* = 8.0 Hz, 2H), 5.48 (s, 4H), 4.86-4.79 (m, 2H), 4.61-4.56 (m, 2H), 4.44-4.39 (m, 2H), 3.51-3.47 (m, 8H), 3.43 (s, 6H), 2.66-2.46 (m, 16H).
ESI-MS *m/z*: 1071.48 [M-1]$^-$

**Example 97. Synthesis of compound I-95 of the present invention**

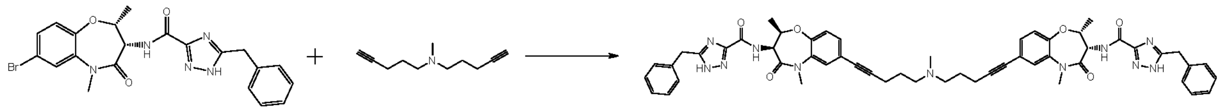

M-1 S-2 I-95

Intermediates **M-1** and **S-2** were used as raw materials, to prepare compound **I-95**, by the synthesis method similar to that of Example 81.
ESI-MS *m/z:* 940.43 $[M-1]^-$

**Example 98. Synthesis of compound I-96 of the present invention**

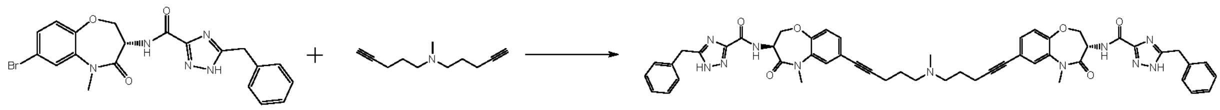

M-2 S-2 I-96

Intermediates **M-2** and **S-2** were used as raw materials, to prepare compound **I-96,** by the synthesis method similar to that of Example 81.
ESI-MS *m/z*: 912.40 $[M-1]^-$

**Example 99. Synthesis of compound I-97 of the present invention**

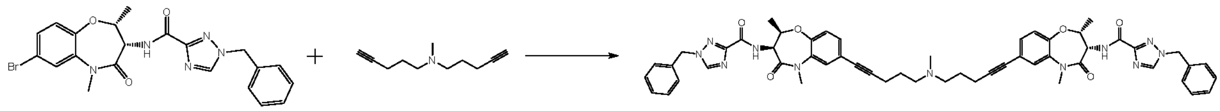

M-7 S-2 I-97

Intermediates **M-7** and **S-2** were used as raw materials, to prepare compound **I-97,** by the synthesis method similar to that of Example 81.
ESI-MS *m/z:* 940.43 $[M-1]^-$

**Example 100. Synthesis of compound I-98 of the present invention**

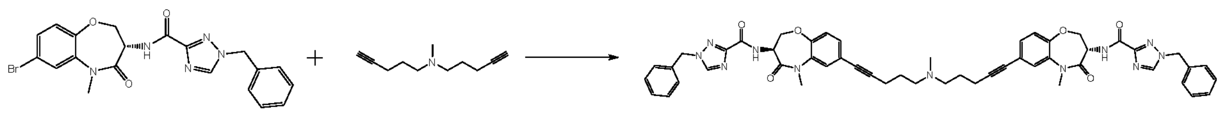

M-8 S-2 I-98

Intermediates **M-8** and **S-2** were used as raw materials, to prepare compound **I-98**, by the synthesis method similar to that of Example 81.
ESI-MS *m/z:* 912.40 $[M-1]^-$

**Example 101. Synthesis of compound I-99 of the present invention**

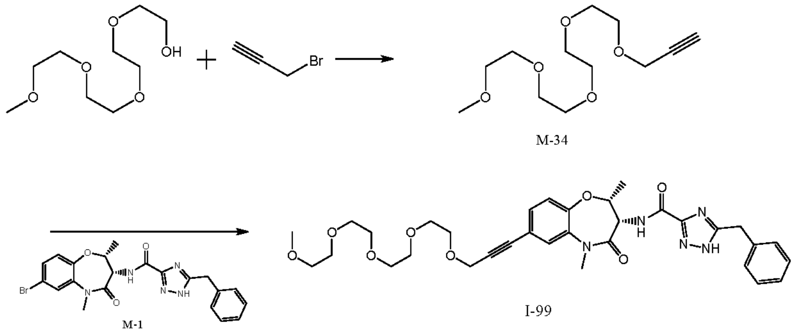

M-34

M-1

I-99

10 mL of 50% NaOH aqueous solution was placed in a flask, to which were added 10 mL of toluene, 0.1 g of TBTU, and 1 g of tetra-polyethylene glycol, and then the mixture was stirred to dissolve. Then, 80% solution of propargyl bromide (1.1 g) in toluene was added, and the reaction solution was allowed to react in an oil bath at 60 °C for 20 h. The reaction system was cooled to room temperature, to which were added 20 mL of ethyl acetate and 20 mL of water, and then the resultant solution was shaken and stood for layering. The ethyl acetate layer was collected, and the water layer was extracted twice with 10 mL of ethyl acetate. The ethyl acetate layers were combined, washed once with saturated NaCl aqueous solution, dried with anhydrous sodium sulfate for 30 min, and filtered to remove anhydrous sodium sulfate. The filtrate was concentrated to dryness to obtain intermediate **M-34.**

400 mg of intermediate **M-1** (0.85 mmol) was transferred to a flask, to which were added 20 mg of palladium acetate, 20 mg of CuI, 40 mg of 2-dicyclohexylphosphino-2,4,6-triisopropylbiphenyl, 400 mg of triphenylphosphine, 209 mg of intermediate **M-34** (0.85 mmol), 5 mL of triethylamine, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-99**:

ESI-MS *m*/*z*: 634.30 [M-1]$^-$

**Example 102. Synthesis of compound I-100 of the present invention**

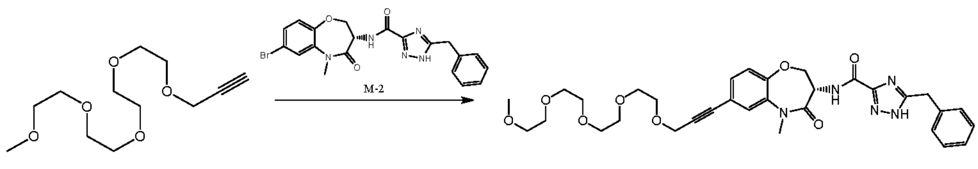

M-2

M-34

I-100

Intermediates **M-2** and **M-34** were used as raw materials, to prepare compound **I-100,** by the synthesis method similar to that of Example 101.

ESI-MS *m*/*z:* 620.28 [M-1]$^-$

**Example 103. Synthesis of compound I-101 of the present invention**

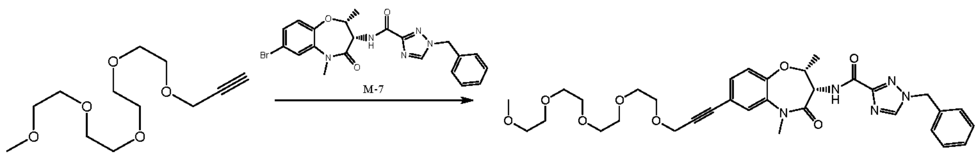

M-7

M-34

I-101

Intermediates **M-7** and **M-34** were used as raw materials, to prepare compound **I-101**, by the synthesis method similar to that of Example 101.

$^1$HNMR (400 MHz, DMSO-$d_6$) $\delta$: 8.85(s, 1H), 7.95(d, $J$ = 5.8 Hz, 1H), 7.68-7.53 (m, 1H), 7.43-7.24 (m, 7H), 5.55-5.45 (m, 2H), 4.93-4.81 (m, 2H), 4.42 (s, 2H), 3.68-3.56 (m, 4H), 3.56-3.46 (m, 10H), 3.44-3.39 (m, 2H), 3.35 (s, 3H), 3.23 (s, 3H), 1.32-1.25 (m, 3H).
ESI-MS m/z: 634.30 [M-1]$^-$

**Example 104. Synthesis of compound I-102 of the present invention**

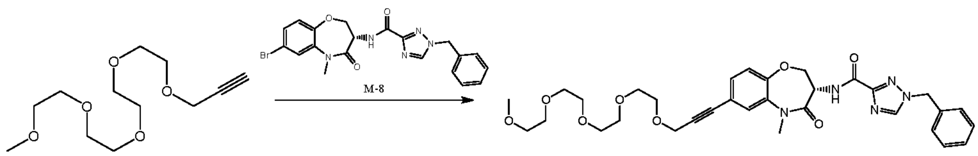


M-34 I-102

Intermediates **M-8** and **M-34** were used as raw materials, to prepare compound **I-102**, by the synthesis method similar to that of Example 101.

$^1$HNMR (400 MHz, DMSO-$d_6$) $\delta$: 8.84 (s, 1H), 8.47 (d, $J$ = 7.9 Hz, 1H), 7.72-7.58 (m, 1H), 7.43-7.20 (m, 7H), 5.49 (s, 2H), 4.66-4.54 (m, 1H), 4.48-4.36 (m, 2H), 3.69-3.56 (m, 4H), 3.56-3.47 (m, 10H), 3.47-3.37 (m, 2H), 3.36-3.27 (m, 3H), 3.23 (s, 3H).
ESI-MS *m/z:* 620.28 [M-1]$^-$

**Example 105. Synthesis of compound I-103 of the present invention**

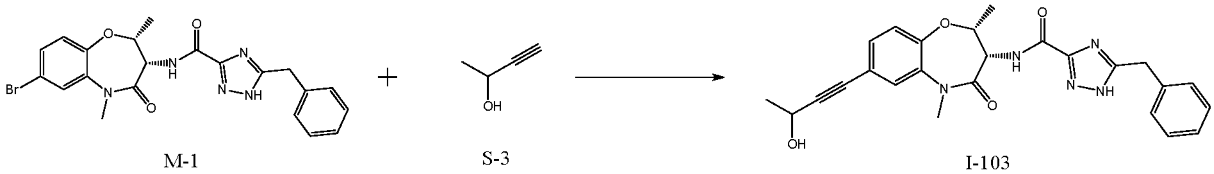

M-1 S-3 I-103

Intermediates **M-1** and **S-3** were used as raw materials, to prepare compound **I-103,** by the synthesis method similar to Method 2 of Example 4.
ESI-MS *m/z:* 458.19 [M-1]$^-$

**Example 106. Synthesis of compound I-104 of the present invention**

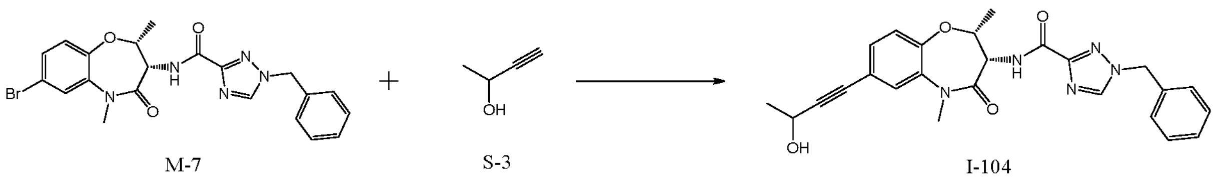

M-7 S-3 I-104

Intermediates **M-1** and **S-3** were used as raw materials, to prepare compound **I-104,** by the synthesis method similar to Method 2 of Example 4.
ESI-MS *m/z*: 458.19 [M-1]$^-$

**Example 107. Synthesis of compound I-105 of the present invention**

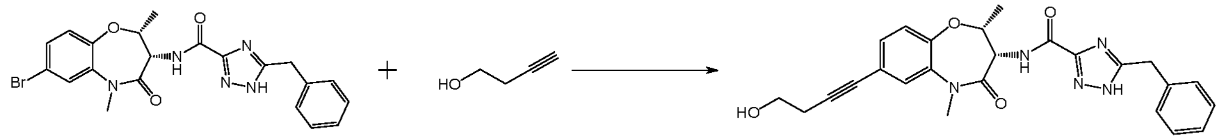

M-1 S-4 I-105

Intermediates **M-1** and **S-4** were used as raw materials, to prepare compound **I-105**, by the synthesis method similar to Method 2 of Example 4.
ESI-MS *m/z*: 458.19 [M-1]$^-$

**Example 108. Synthesis of compound I-106 of the present invention**

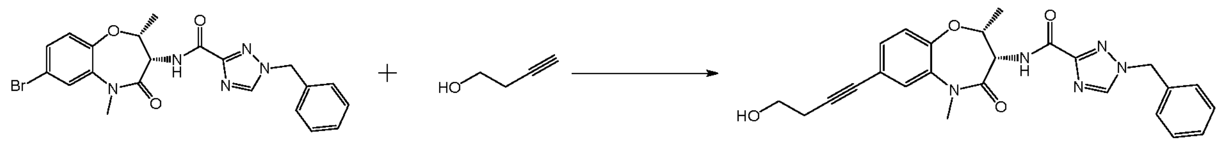

M-7 S-4 I-106

Intermediates **M-1** and **S-4** were used as raw materials, to prepare compound **I-106**, by the synthesis method similar to Method 2 of Example 4.

$^1$HNMR (400MHz, DMSO-$d_6$) $\delta$: 8.85 (s, 1H), 7.98-7.92 (m, 1H), 7.49 (d, $J$ = 2.0 Hz, 1H), 7.43-7.27 (m, 5H), 7.26-7.20 (m, 2H), 5.56-5.43 (m, 2H), 4.94-4.88 (m, 1H), 4.88-4.78 (m, 2H), 3.64-3.54 (m, 2H), 3.23 (s, 3H), 1.25 (d, 3H).
ESI-MS *m/z*: 458.19 [M-1]$^-$

**Example 109. Synthesis of compound I-107 of the present invention**

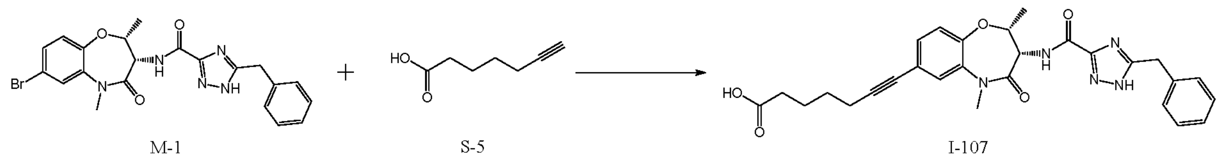

M-1 S-5 I-107

Intermediates **M-1** and **S-5** were used as raw materials, to prepare compounds **I-107**, by the synthesis method similar to Method 2 of Example 4.
ESI-MS *m/z*: 514.22 [M-1]$^-$

**Example 110. Synthesis of compound I-108 of the present invention**

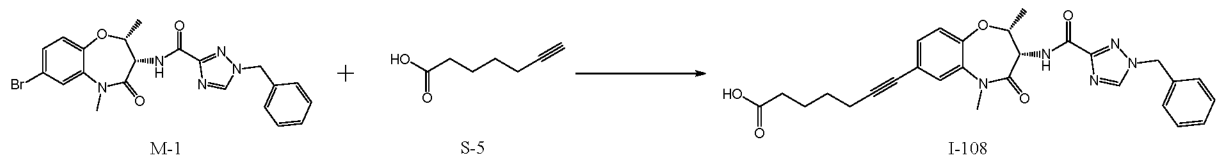

M-1 S-5 I-108

Intermediates **M-7** and **S-5** were used as raw materials, to prepare compound **I-108**, by the synthesis method

similar to Method 2 of Example 4.

$^1$HNMR (400 MHz, DMSO-$d_6$) $\delta$: 11.88 (s, 1H), 8.72 (s, 1H), 7.82 (d, $J$ = 6.0 Hz, 1H), 7.38-7.33 (m, 1H), 7.29-7.14 (m, 6H), 7.13-7.09 (m, 1H), 5.39-5.34 (m, 2H), 4.77-4.68 (m, 2H), 3.21 (s, 3H), 2.32 (t, $J$ = 6.8 Hz, 2H), 2.24-2.04 (m, 2H), 1.60-1.30 (m, 4H), 1.18-1.12 (m, 3H).
ESI-MS $m/z$: 514.22 $[M-1]^-$

**Example 111. Synthesis of compound I-109 of the present invention**

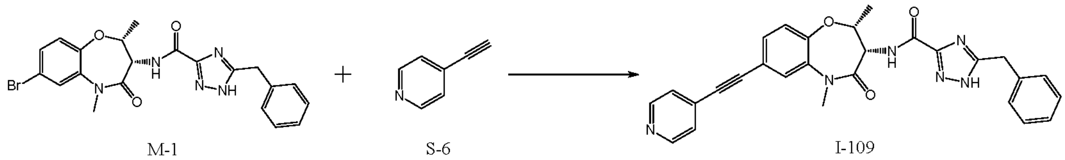
M-1 S-6 I-109

Intermediates **M-1** and **S-6** were used as raw materials, to prepare compound **I-109**, by the synthesis method similar to Method 2 of Example 4.
ESI-MS $m/z$: 491.19 $[M-1]^-$

**Example 112. Synthesis of compound I-110 of the present invention**

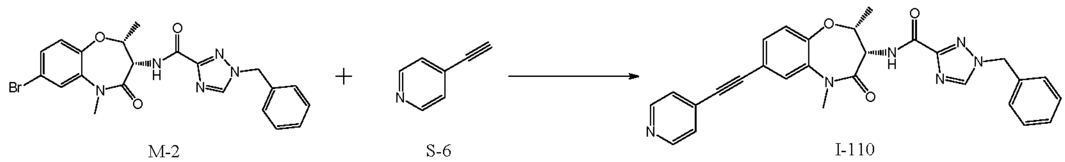
M-2 S-6 I-110

Intermediates **M-2** and **S-6** were used as raw materials, to prepare compound **I-110**, by the synthesis method similar to Method 2 of Example 4.

$^1$HNMR (400 MHz, DMSO-$d_6$) $\delta$: 8.84 (s, 1H), 8.66-8.60 (m, 2H), 7.98-7.92 (m, 1H), 7.76-7.71 (m, 1H), 7.57-7.49 (m, 3H), 7.41-7.26 (m, 6H), 5.54-5.52 (m, 2H), 4.93-4.84 (m, 2H), 3.37 (s, 3H), 1.28 (d, $J$ = 5.2 Hz, 3H)。
ESI-MS $m/z$: 491.19 $[M-1]^-$

**Example 113. Synthesis of compound I-111 of the present invention**

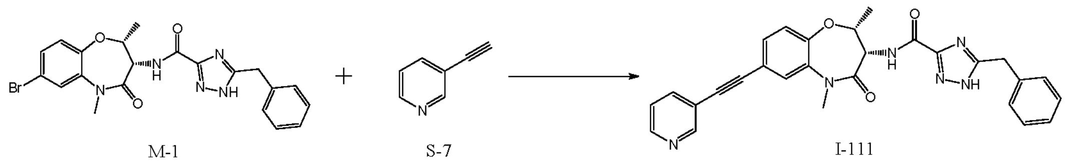
M-1 S-7 I-111

Intermediates **M-1** and **S-7** were used as raw materials, to prepare compound **I-111**, by the synthesis method similar to Method 2 of Example 4.
ESI-MS $m/z$: 491.19 $[M-1]^-$

**Example 114. Synthesis of compound I-112 of the present invention**

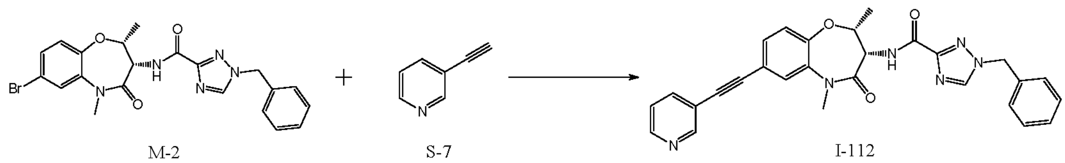

M-2 S-7 I-112

Intermediates M-2 and S-7 were used as raw materials, to prepare compound **I-112,** by the synthesis method similar to Method 2 of Example 4.
ESI-MS *m/z*: 491.19 [M-1]$^-$

**Example 115. Synthesis of compound I-113 of the present invention**

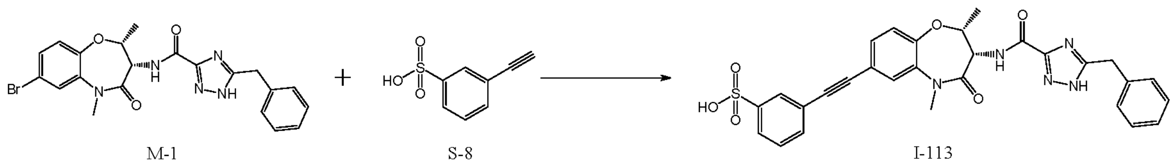

M-1 S-8 I-113

Intermediates **M-1** and **S-8** were used as raw materials, to prepare compound **I-113**, by the synthesis method similar to Method 2 of Example 4.
ESI-MS *m/z*: 570.15 [M-1]$^-$

**Example 116. Synthesis of compound I-114 of the present invention**

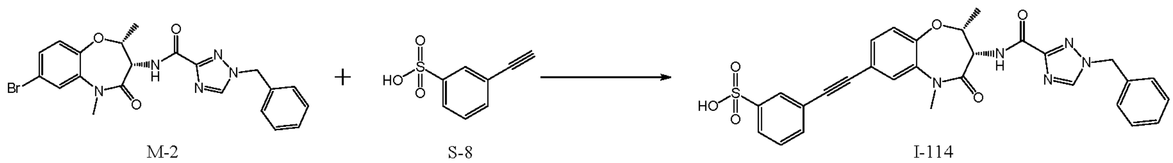

M-2 S-8 I-114

Intermediates **M-2** and **S-8** were used as raw materials, to prepare compound **I-114**, by the synthesis method similar to Method 2 of Example 4.
ESI-MS *m/z*: 570.15 [M-1]$^-$

**Example 117. Synthesis of compound I-115 of the present invention**

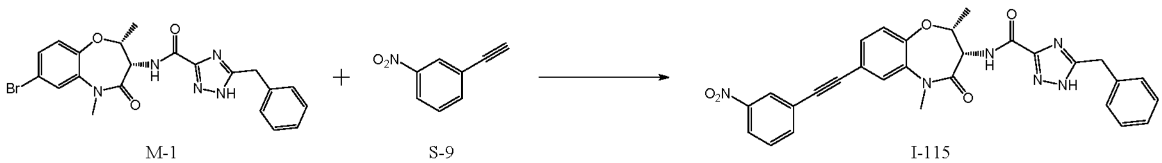

M-1 S-9 I-115

Intermediates **M-1** and **S-9** were used as raw materials, to prepare compound **I-115**, by the synthesis method similar to Method 2 of Example 4.
ESI-MS *m/z*: 535.18 [M-1]$^-$

**Example 118. Synthesis of compound I-116 of the present invention**

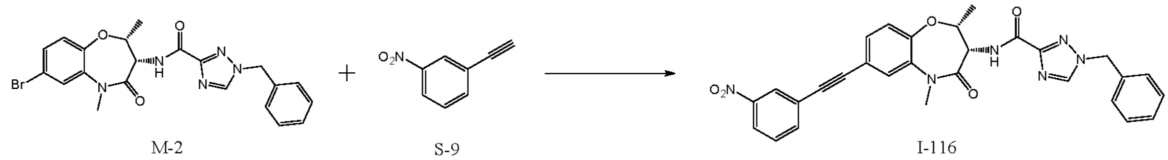

M-2 S-9 I-116

Intermediates **M-2** and **S-9** were used as raw materials, to prepare compound **I-116**, by the synthesis method similar to Method 2 of Example 4.
ESI-MS *m/z*: 535.18 $[M-1]^-$

**Example 119. Synthesis of compound I-117 of the present invention**

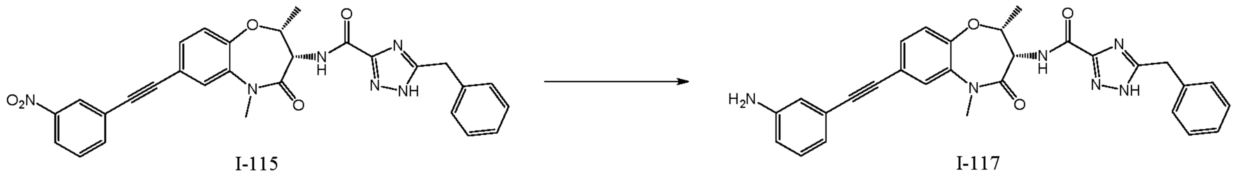

I-115 I-117

Compound **I-115** was used as raw material and dissolved in ethanol/water, to which were added $NH_4Cl$ and iron powder, and then the solution was allowed to react under reflux until the reaction of the raw material was complete. After concentration, the residue was separated by silica gel column chromatography to obtain **I-117**.
ESI-MS *m/z*: 505.21 $[M-1]^-$

**Example 120. Synthesis of compound I-118 of the present invention**

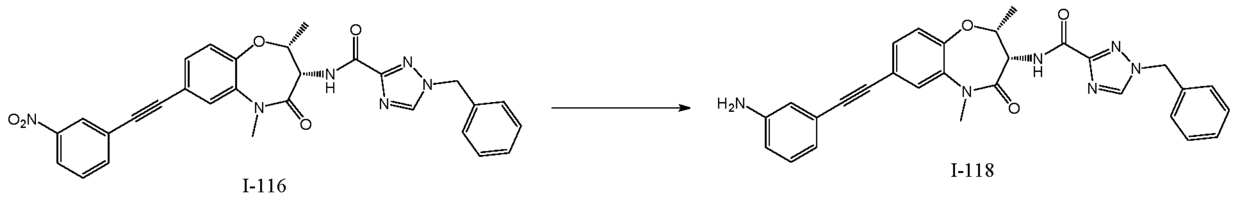

I-116 I-118

Compound **I-116** was used as raw material to prepare compound **I-118**, using the synthesis method similar to that of Example 119.
ESI-MS *m/z*: 535.18$[M-1]^-$

**Example 121. Synthesis of compound I-119 of the present invention**

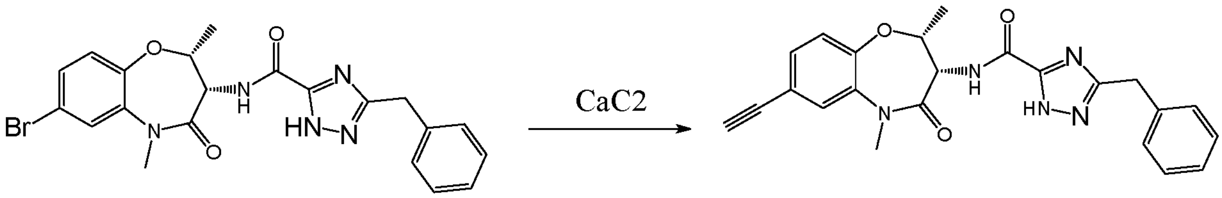


M-1

I-119

400 mg of intermediate **M-1** (0.85 mmol) was transferred to a flask, to which were added 20 mg of palladium acetate, 20 mg of CuI, 40 mg of 2-dicyclohexylphosphino-2,4,6-triisopropylbiphenyl, 400 mg of triphenylphosphine, 250 mg of $CaCO_3$, 5 mL of triethylamine, catalytic amount of water, and 10 mL of acetonitrile; the mixture was allowed to react overnight in an oil bath at 80 °C under nitrogen protection. TLC indicated the disappearance of raw materials. 150 mL of water was slowly added, and then the resultant solution was extracted twice with 100 mL of ethyl acetate. The ethyl acetate layers were combined, washed twice with 70 mL of saturated NaCl aqueous solution, dried with 10 g of anhydrous sodium sulfate for 30 min, and then filtered to remove sodium sulfate. The filtrate was concentrated to dry, and the residue was purified over column chromatography, to provide compound **I-119**.
ESI-MS *m/z*: 414.21 $[M-1]^-$

**Example 122. Synthesis of compound I-120 of the present invention**

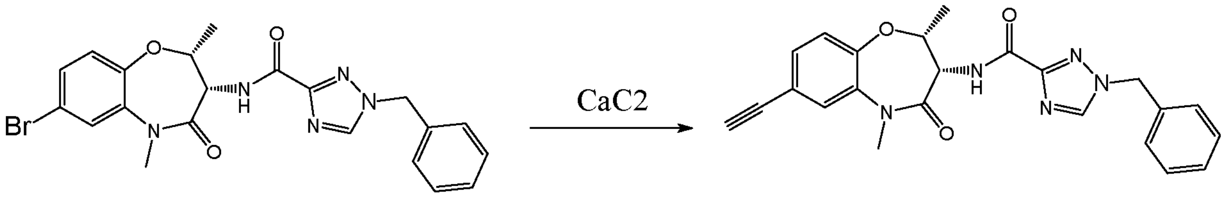


M-7

I-120

Compound M-7 was used as starting material to prepare compound **I-120**, using the synthesis method similar to that of Example 121.
ESI-MS *m/z*: 414.16 $[M-1]^-$

**Example 123. Synthesis of compound I-121 of the present invention**

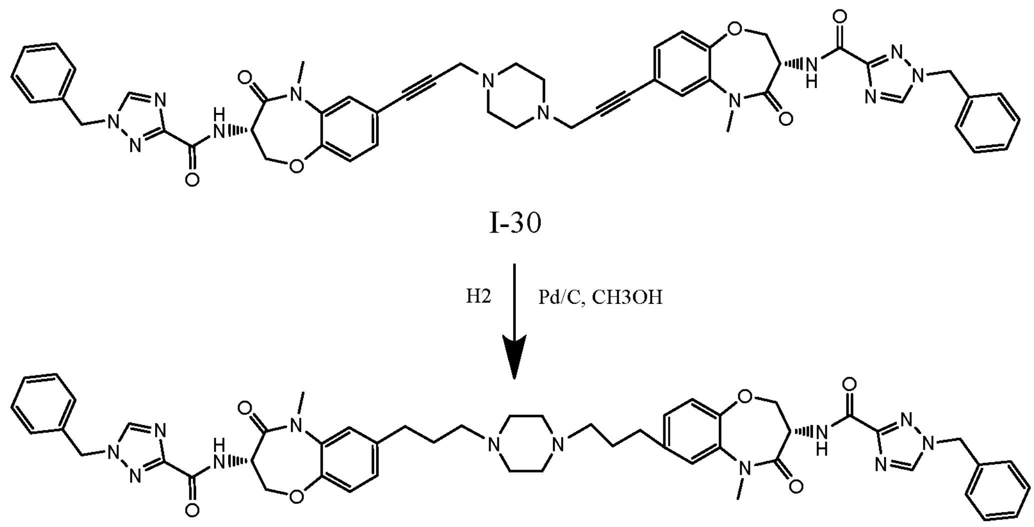


I-30

I-121

50 mg of compound **I-30** was placed into a hydrogenation bottle, and then dissolved by adding 15 mL of methanol. To the solution, was added 5 mg of 10% Pd/C, and then hydrogen gas at atmospheric pressure was introduced and purged three times to completely replace the air in the bottle. The reaction solution was allowed to react for 4 h at room temperature. TLC showed that the reaction of the raw material was complete, and thus the reaction was stopped. The solvent was removed by concentration under reduced pressure. The residue was separated by column chromatography to obtain compound **I-121.**
ESI-MS *m/z*: 919.47 $[M-1]^-$

**Example 124. Synthesis of compound I-122 of the present invention**

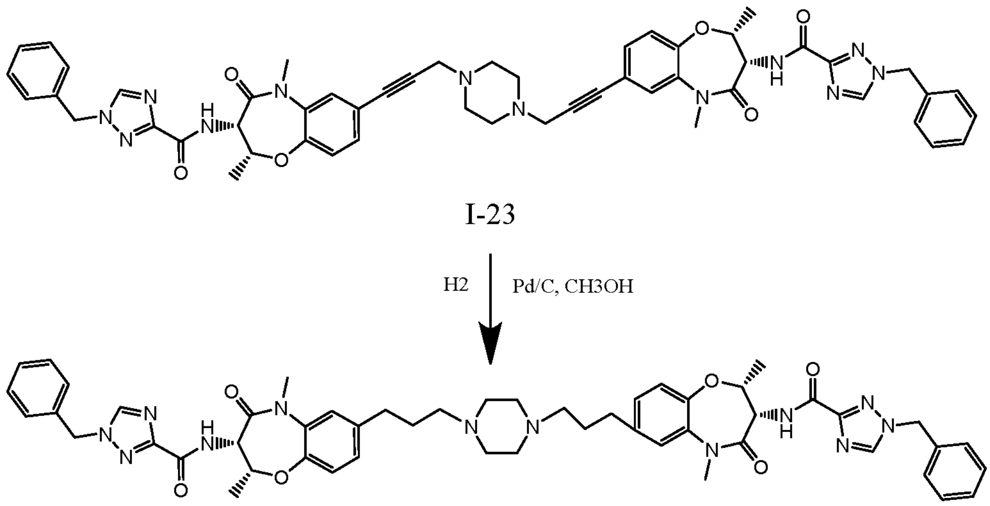


I-23

I-122

Compound **I-23** was used as starting material to prepare compound **I-122**, as described in Example 123.
ESI-MS *m/z:* 947.49 $[M-1]^-$

**Example 125. Synthesis of compound I-123 of the present invention**

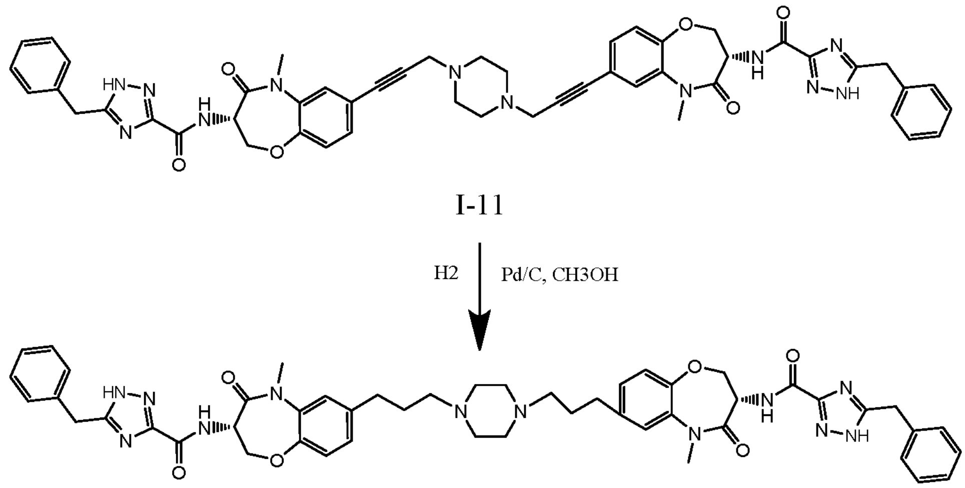


I-11

I-123

Compound **I-11** was used as starting material to prepare compound **I-123,** as described in Example 123. ESI-MS *m/z*: 919.45 [M-1]-

**Example 126. Synthesis of compound I-124 of the present invention**

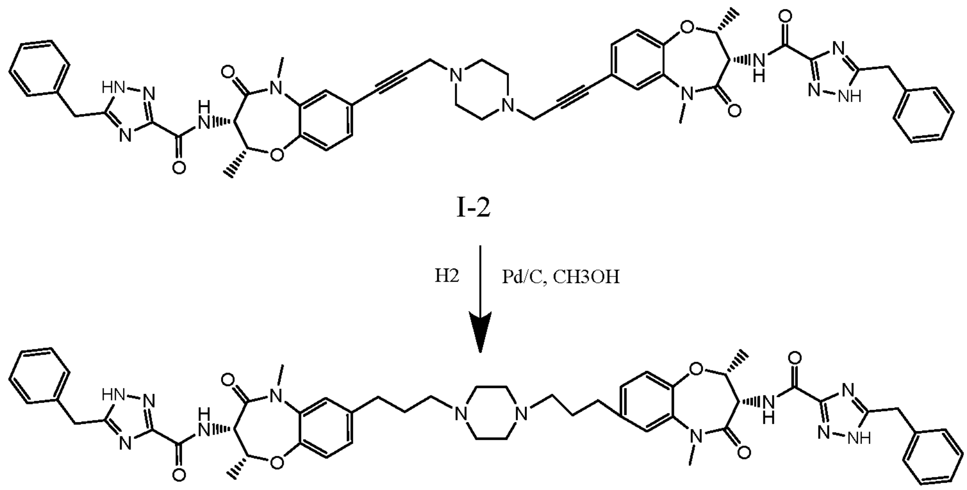


I-2

I-124

Compound **I-2** was used as starting material to prepare compound **I-124,** as described in Example 123. ESI-MS *m/z:* 947.48 [M-1]-

The beneficial effects of the present invention were demonstrated with reference to specific Experimental Examples.

**Example 1. Inhibitory activity of the compound according to the present invention on RIP1 kinase**

The compound of the present invention and the control compound were dissolved in the assay buffer (50 mM Hepes pH 7.5, 50 mM NaCl, 30 mM $MgCl_2$, 1 mM DTT, 0.02% CHAPS, 0.5 mg/mL BSA), and diluted in a ratio of 1:1.5 using a 22-point titration (high concentration 2 μM), and then added to a 384-well plate. Each concentration of inhibitor (3.5 μL) and 3.5 μL of human RIP kinase (25 nM) dissolved in the assay buffer were added to the wells. After warming at 37 °C for 1 h, 3.5 μL of ATP (15 μM to 1.5 mM) in buffer was added to initiate the reaction, and then allowed to react at room temperature for 5 h. After completion of the reaction, 5 μL of ADP-Glo reagent containing 0.02% CHAPS was added to each well and incubated at room temperature for 1 h to terminate the kinase reaction and use up all remaining ATP. Then, 5 μL of ADP-Glo detection solution containing 0.02% CHAPS was added to each well and incubated at

room temperature for 30 min to measure the emittance intensity. For each ATP concentration, the activity data was obtained by the emittance intensity minus the control intensity.

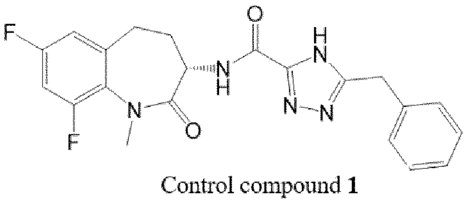

Control compound 1 ,

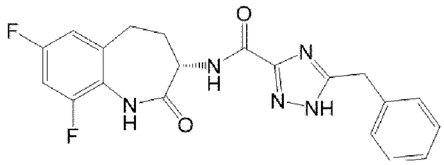

Control compound 2 ,

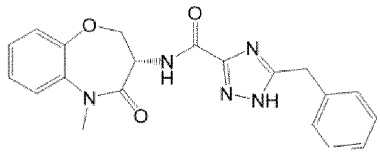

Control compound 3

The $IC_{50}$ value for each ATP concentration was determined by calculation, and the results are shown in Table 1:

**Table 1. Inhibitory activity of the compound according to the present invention on RIP1 kinase ($IC_{50}$).**

| Compounds | $IC_{50}$ (nM) | Compounds | $IC_{50}$ (nM) |
|---|---|---|---|
| **I-1** | 4.79 | **I-2** | 3.81 |
| **I-3** | 7.22 | **I-4** | 6.68 |
| **I-5** | 3.35 | **I-6** | 11.24 |
| **I-7** | 7.81 | **I-8** | 9.50 |
| **I-9** | 8.72 | **I-10** | 3.21 |
| **I-11** | 1.96 | **I-12** | 2.75 |
| **I-13** | 3.22 | **I-14** | 4.52 |
| **I-15** | 4.88 | **I-16** | 5.59 |
| **I-17** | 3.56 | **I-18** | 57.69 |
| **I-19** | 62.31 | **I-20** | 9.44 |
| **I-21** | 9.69 | **I-22** | 4.17 |
| **I-23** | 4.66 | **I-24** | 4.68 |
| **I-25** | 3.39 | **I-26** | 3.73 |
| **I-27** | 4.00 | **I-28** | 4.59 |
| **I-29** | 2.88 | **I-30** | 2.62 |
| **I-31** | 2.95 | **I-32** | 3.08 |
| **I-33** | 3.54 | **I-34** | 4.68 |
| **I-35** | 5.89 | **I-36** | 4.36 |
| **I-37** | 6.68 | **I-38** | 4.18 |
| **I-39** | 79.04 | **I-40** | 66.28 |
| **I-41** | 5.59 | **I-42** | 5.03 |
| **I-43** | 12.68 | **I-44** | 14.89 |
| **I-45** | 14.22 | **I-46** | 10.75 |

(continued)

| Compounds | $IC_{50}$ (nM) | Compounds | $IC_{50}$ (nM) |
|---|---|---|---|
| **I-47** | 13.56 | **I-48** | 13.27 |
| **I-49** | 16.18 | **I-50** | 14.35 |
| **I-51** | 15.94 | **I-52** | 13.30 |
| **I-53** | 15.07 | **I-54** | 14.66 |
| **I-55** | 8.91 | **I-56** | 7.44 |
| **I-57** | 8.09 | **I-58** | 6.16 |
| **I-59** | 10.25 | **I-60** | 9.91 |
| **I-61** | 8.43 | **I-62** | 8.07 |
| **I-63** | 59.24 | **I-64** | 62.17 |
| **I-65** | 20.74 | **I-66** | 18.80 |
| **I-67** | 14.58 | **I-68** | 16.02 |
| **I-69** | 19.44 | **I-70** | 18.23 |
| **I-71** | 23.89 | **I-72** | 22.07 |
| **I-73** | 19.06 | **I-74** | 17.54 |
| **I-75** | 20.97 | **I-76** | 22.36 |
| **I-77** | 28.98 | **I-78** | 23.15 |
| **I-79** | 10.54 | **I-80** | 8.85 |
| **I-81-1** | 7.79 | **I-81-2** | 9.08 |
| **I-81-2** | 6.60 | **I-82-2** | 7.15 |
| **I-81** | 8.18 | **I-84-1** | 8.99 |
| **I-84-2** | 8.03 | **I-85-1** | 7.49 |
| **I-85-2** | 6.63 | **I-82** | 7.91 |
| **I-83** | 6.32 | **I-84** | 6.58 |
| **I-85** | 7.44 | **I-86** | 6.29 |
| **I-87** | 4.36 | **I-88** | 5.89 |
| **I-89** | 5.73 | **I-90** | 6.16 |
| **I-91** | 19.78 | **I-92** | 18.52 |
| **I-93** | 18.33 | **I-94** | 16.99 |
| **I-95** | 2.39 | **I-96** | 1.47 |
| **I-97** | 2.54 | **I-98** | 1.88 |
| **I-99** | 10.25 | **I-100** | 10.25 |
| **I-101** | 4.85 | **I-102** | 6.53 |
| **I-103** | 19.89 | **I-104** | 20.46 |
| **I-105** | 4.26 | **I-106** | 4.73 |
| **I-107** | 6.69 | **I-108** | 7.21 |
| **I-109** | 9.35 | **I-110** | 11.74 |
| **I-111** | 7.35 | **I-112** | 7.99 |
| **I-113** | 12.69 | **I-114** | 13.77 |

(continued)

| Compounds | $IC_{50}$ (nM) | Compounds | $IC_{50}$ (nM) |
|---|---|---|---|
| **I-115** | 9.81 | **I-116** | 10.75 |
| **I-117** | 4.92 | **I-118** | 3.18 |
| **I-119** | 3.17 | **I-120** | 4.84 |
| Control compound **1** | 24.25 | Control compound **2** | 20.63 |
| Control compound **3** | 29.11 | | |

The experimental results showed that the compound of the present invention had significant inhibitory activity against human RIP1 kinase, indicating that the compound of the present invention could be used in the manufacture of RIP inhibitors.

**Experimental Example 2: The inhitory activities of the compound according to the present invention on the necrosis of tumor cells U937 induced by TNF-α**

Human histiocytic lymphoma cells U937 (purchased from ATCC) in logarithmic growth phase were collected and resuspended in 1640 medium, and then the cell concentration was adjusted to 60 cells/μL, to obtain cell suspension, which was seeded in 384-well plate, with 20 μL cell suspension for each well. After the cells were incubated in a 5% $CO_2$ incubator at 37 °C for 24 h, the test compound of the present invention and the control compound were diluted to the corresponding pre-determined concentration using the medium, and added to the 384-well plate at 5 μL/well. The final concentration of the compound ranged from 0 nM to 1000 nM, which was obtained by successive dilution in a ratio of 1:10. Moreover, the necrosis-inducing agent was added to the 384-well plate at 5 μL/well, which was consisted of TNF-α (100 ng/ml), Smac Mimetics (AT-406, 1 μM), and z-VAD-FMK (20 μM). After addition of test compound and the necrosis-inducing agent, the cells were cultured in a 5% $CO_2$ incubator at 37 °C for 24 h. Then, 30 μL of CellTiter-Glo luminescent solution was added to each well, and shaken for 2 min. After standing at room temperature for 10 min, the absorbance was measured using an enzyme-linked immunosorbent assay (ELISA). The necrosis induction rate and the necrosis inhibition rate were calculated by the following formula:

$$\text{The necrosis induction rate } (\%) = 100 \times (1 - B/A);$$

A is the group without necrosis-inducing agents, and B is the group with necrosis-inducing agents.

The inhition rate (%) = 100-(A-X);the inhibition rate $(\%) \quad = 100 - \frac{A - X}{A - B} \times 100$ ; A is the group without necrosis-inducing agents, B is the group with necrosis-inducing agents, and X is the group with compounds and necrosis-inducing agents.

The inhitory activities ($IC_{50}$) of test compounds on the necrosis of U937 cells induced by TNF-α were calculated by GraphPad, and the results are shown in following Table 2 (3 parallel wells were set for each group in the experiment):

**Table 2. The inhitory activities ($IC_{50}$) of the compound according to the present invention on the necrosis of U937 cells induced by TNF-α.**

| Compound | $IC_{50}$ (nM) | Compound | $IC_{50}$ (nM) |
|---|---|---|---|
| **I-1** | 5.52 | **I-2** | 4.47 |
| **I-10** | 2.88 | **I-11** | 2.09 |
| **I-22** | 6.14 | **I-23** | 5.91 |
| **I-29** | 5.27 | **I-30** | 4.89 |
| **I-99** | 3.36 | **I-100** | 3.14 |
| **I-101** | 5.62 | **I-102** | 2.08 |
| **I-119** | 3.19 | **I-120** | 4.05 |

(continued)

| Compound | $IC_{50}$ (nM) | Compound | $IC_{50}$ (nM) |
|---|---|---|---|
| **I-121** | 6.12 | **I-122** | 7.14 |
| **I-123** | 4.45 | **I-124** | 6.75 |
| Control compound **1** | 8.69 | Control compound **2** | 12.77 |
| Control compound **3** | 7.15 | | |

Based on the above experimental results, the inhitory activities ($IC_{50}$) of the compounds according to the present invention on the necrosis of U937 cells induced by TNF-$\alpha$ were significant.

**Experimental Example 3. Inhibitory activities of the compounds according to the present invention on the necrosis of human colon cancer cells HT-29 and human pancreatic cancer cells PANC-1 induced by TNF-$\alpha$**

Human colon cancer cells HT-29 and human pancreatic cancer cells PANC-1 (purchased from ATCC) in logarithmic growth phase were collected and resuspended in 1640 medium, and then the cell concentration was adjusted to 20 cells/$\mu$L, to obtain cell suspension, which was seeded in 384-well plate, with 20 $\mu$L cell suspension for each well. After the cells were incubated in a 5% $CO_2$ incubator at 37 °C for 24 h, the test compound of the present invention and the control compound were diluted to the corresponding pre-determined concentration using the medium, and added to the 384-well plate at 5 $\mu$L/well. The final concentration of the compound ranged from 0 nM to 1000 nM, which was obtained by successive dilution in a ratio of 1:10. Moreover, the necrosis-inducing agent was added to the 384-well plate at 5 $\mu$L/well, which was consisted of TNF-$\alpha$ (100 ng/mL), Smac Mimetics (AT-406, 1 $\mu$M), and z-VAD-FMK (20 $\mu$M). After addition of test compound and the necrosis-inducing agent, the cells were cultured in a 5% $CO_2$ incubator at 37 °C for 24 h. Then, 30 $\mu$L of CellTiter-Glo luminescent solution was added to each well, and shaken for 2 min. After standing at room temperature for 10 min, the absorbance was measured using ELISA. The necrosis induction rate and the necrosis inhibition rate were calculated by the following formula:

$$\text{The necrosis induction rate } (\%) = 100 \times (1 - \text{B}/\text{A});$$

A is the group without necrosis-inducing agents, and B is the group with necrosis-inducing agents.

The inhition rate (%) = 100-(A-X);the inhibition rate $(\%) = 100 - \frac{\text{A} - \text{X}}{\text{A} - \text{B}} \times 100$; A is the group without necrosis-inducing agents, B is the group with necrosis-inducing agents, and X is the group with compounds and necrosis-inducing agents.

The inhitory activities ($IC_{50}$) of test compounds on the necrosis of human colon cancer cells and human pancreatic cancer cells induced by TNF-$\alpha$ were calculated by GraphPad, and the results are shown in following Table 3 (3 parallel wells were set for each group in the experiment):

**Table 3. The inhitory activities ($IC_{50}$) of the compound according to the present invention on the necrosis of HT-29 and PANC-1 cells induced by TNF-$\alpha$.**

| Compound | HT-29 $IC_{50}$ (nM) | PANC-1 $IC_{50}$ (nM) |
|---|---|---|
| **I-1** | 19.17 | 28.74 |
| **I-2** | 12.99 | 24.35 |
| **I-10** | 15.30 | 26.89 |
| **I-11** | 10.85 | 20.44 |
| **I-22** | 26.91 | 20.47 |
| **I-23** | 26.38 | 17.84 |
| **I-29** | 19.68 | 17.39 |
| **I-30** | 16.62 | 15.97 |

(continued)

| Compound | HT-29 $IC_{50}$ (nM) | PANC-1 $IC_{50}$ (nM) |
|---|---|---|
| **I-99** | 11.88 | 16.27 |
| **I-100** | 8.65 | 12.52 |
| **I-101** | 10.07 | 13.69 |
| **I-102** | 7.92 | 13.77 |
| **I-109** | 13.12 | 12.56 |
| **I-110** | 10.94 | 11.08 |
| **I-111** | 13.65 | 12.44 |
| **I-112** | 10.01 | 11.31 |
| **I-119** | 15.32 | 17.11 |
| **I-120** | 14.58 | 15.73 |
| Control compound **1** | >100 | 34.95 |
| Control compound **2** | >100 | 48.27 |
| Control compound **3** | >100 | 51.26 |

From the above experimental results, the inhitory activities of the compounds according to the present invention on the necrosis of human colon cancer cells HT-29 and human pancreatic cancer cells PANC-1 induced by TNF-α were significant.

**Experimental Example 4. Inhibitory activities of the compounds according to the present invention on the necrosis of HacaT cells induced by TNF-α**

HacaT cells (purchased from ATCC) in logarithmic growth phase were collected and resuspended in 1640 medium, and then the cell concentration was adjusted to 20 cells/μL, to obtain cell suspension, which was seeded in 384-well plate, with 20 μL cell suspension for each well. After the cells were cultured in a 5% $CO_2$ incubator at 37 °C for 24 h, the test compound of the present invention and the control compound were diluted to the corresponding predetermined concentration using the medium, and added to the 384-well plate at 5 μL/well. The final concentration of the compound ranged from 0 nM to 1000 nM, which was obtained by successive dilution in a ratio of 1:10. Moreover, the necrosis-inducing agent was added to the 384-well plate at 5 μL/well, which was consisted of TNF-α (100 ng/mL), Smac Mimetics (AT-406, 1 μM), and z-VAD-FMK (20 μM). After addition of test compound and the necrosis-inducing agent, the cells were cultured in a 5% $CO_2$ incubator at 37 °C for 24 h. Then, 30 μL of CellTiter-Glo luminescent solution was added to each well, and shaken for 2 min. After standing at room temperature for 10 min, the absorbance was measured using ELISA. The necrosis induction rate and the necrosis inhibition rate were calculated by the following formula:

$$\text{The necrosis induction rate } (\%) = 100 \times (1 - \mathrm{B}/\mathrm{A});$$

A is the group without necrosis-inducing agents, and B is the group with necrosis-inducing agents.

The inhition rate (%) = 100-(A-X);the inhibition rate $(\%) \quad = 100 - \frac{\mathrm{A} - \mathrm{X}}{\mathrm{A} - \mathrm{B}} \times 100$ ; A is the group without necrosis-inducing agents, B is the group with necrosis-inducing agents, and X is the group with compounds and necrosis-inducing agents.

The inhitory activities ($IC_{50}$) of test compounds on the necrosis of HacaT cells induced by TNF-α were calculated by GraphPad, and the results are shown in following Table 4 (3 parallel wells were set for each group in the experiment):

**Table 4. The inhitory activities ($IC_{50}$) of the compound according to the present invention on the necrosis of HacaT cells induced by TNF-α.**

| Compound | $IC_{50}$ (nM) |
|---|---|
| **I-1** | 3.85 |
| **I-2** | 3.89 |
| **I-10** | 2.47 |
| **I-11** | 2.97 |
| **I-22** | 2.44 |
| **I-23** | 2.68 |
| **I-29** | 3.89 |
| **I-30** | 4.07 |
| **I-99** | 2.56 |
| **I-100** | 2.39 |
| **I-101** | 2.95 |
| **I-102** | 3.58 |
| Control compound **1** | 12.84 |
| Control compound **2** | 18.57 |
| Control compound **3** | 9.92 |

Based on the experimental results, the compound of the present invention had significant inhibitory activity on the necrosis of HacaT cells induced by TNF, and could be used for the prevention and treatment of psoriasis and other diseases.

**Experimental Example 5: The therapeutic effect of the compound according the present invention on DSS-induced inflammatory bowel disease model in mice**

28 8-week-old C57BL/6J male mice were purchased from Chengdu Dossy Experimental Animal Co., Ltd. After one week of adaptive feeding, the mice were randomly divided into 4 groups, including blank group, model group, test group, and positive control group, with 7 mice in each group. The blank group and the model group received 100 μL RO water per day, the test group was given compound **I-30** of the present invention (50 mg/kg/day) dispersed in 100 μL of RO water, and the positive control group was given control compound 3 (Comp3) (50 mg/kg/d) in Experimental Example 1 every day, which was also dispersed in 100 μL RO water. The blank group had normal drinking water every day, while the model group, **I-30** compound group, and control compound 3 (Comp3) group were given 3% DSS aqueous solution for 5 days, and then 3% DSS solution was changed to normal drinking water. The mice were euthanized on day 9 to collect colon tissues. The body weight, the water intake, and the disease index score (DAI) were observed daily for each mouse, and the results were recorded. The scoring method is shown in the table below:

Table 5. DAI score of DSS inflammatory bowel disease model in mice.

| Score | The percentage of weight loss | Fecal viscosity | Fecal occult blood |
|---|---|---|---|
| 0 | 0 | Normal | Negative |
| 1 | 1-5% | Soft stool | Light blue |
| 2 | 5-10% | Mucoid stool | Blue |
| 3 | 10-20% | Dilute liquid stool | Dark blue |
| 4 | >20% | | Gross blood stool |

After measuring the length of the colon tissue collected, the samples were taken and subjected to HE staining for pathological scoring, and the results were recorded. The scoring method is shown in the table below:

Table 6. Pathological scoring table for colon tissues of DSS inflammatory bowel disease model in mice.

| Crypt damage | Ulcer | Inflammation |
|---|---|---|
| 0) No crypt damage; | 0) No ulcer | 0) No obvious inflammation; |
| 1) Loss of 1/3 of basal recesses; | 1) 1-2 or fewer ulcer lesions; | 1) Inflammation of epithelial cells or neutrophils in the lamina propria; |
| 2) Loss of 2/3 of basal recesses; | 2) 1-2 or 4 ulcer lesions; | 2) Inflammatory cells extend to the submucosa; |
| 3) Loss of full layer recesses; | 3) Diffuse/confluence | 3) Transmural inflammation. |
| | ulcer | |
| 4) Loss of full thickness recess and surface erosion; | | |
| 5) Diffuse/converging surface erosion | | |

The weight records of mice during the experiment are shown in Figures 1 and 2. The results showed that compound **I-30** had effects on alleviating weight loss, with a much smaller weight loss compared to the model group as well as a slightly smaller weight loss compared to the positive control group. The record of disease index (DAI) score is shown in Figure 3. the DAI score of compound **I-30** was much lower than that of the model group and the positive control group, indicating a significant therapeutic effect. The record of colon length is shown in Figure 4. The colon length of compound **I-30** group was greater than that of the model group and the positive control group, and was significantly different from that of the positive control group, indicating compound **I-30** had a significant protective effect on the colon. The pathological score of colon tissue is shown in Figure 5. The score of compound **I-30** was better than that of the model group and the positive control group, indicating an obvious protective effect on the colon.

The experimental results showed that in a DSS-induced inflammatory bowel disease model in mice, compound **I-30** significantly alleviated the weight loss, significantly reduced the disease index (DAI) score, and significantly protected the colon tissue compared to the model group and th positive drug group, indicating a significant therapeutic effect of compound **I-30**.

In summary, the compound of the present invention had good inhibitory activity against human RIP1 kinase, whose effect was superior to that of compounds in the prior art, and thereby could be used in the manufacture of RIP inhibitors; moreover, the compound of the present invention had significant inhibitory activity against TNF-$\alpha$ induced necrosis in various cells, and so it could be used in the manufacture ofmedicaments for preventing and treating pancreatic cancer, colon cancer, lymphatic cancer, psoriasis and other diseases, as well as be better used in the treatment of inflammatory diseases, such as ulcerative colitis, atopic dermatitis, etc.

## Claims

1. A compound as represented by formula (I), or a salt thereof, or a stereoisomer thereof, or a solvate thereof, or a prodrug thereof:

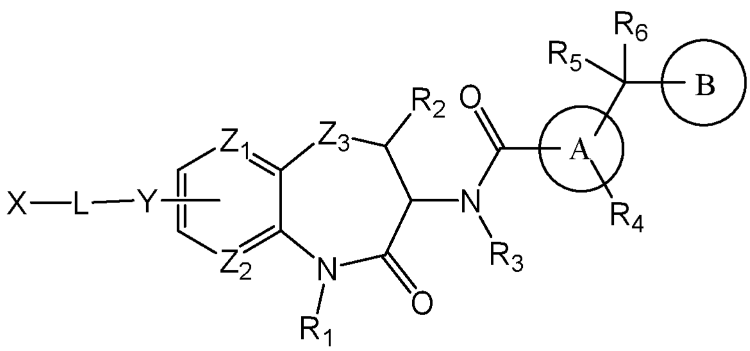

(I) ;

wherein, ring A is a 3-6 membered unsaturated heterocyclyl;

ring B is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino or hydroxyl;
$Z_1$ and $Z_2$ are each independently selected from CH or N;
$Z_3$ is selected from the group consisting of O, $NR_8$, S or $CR_8R_9$;
$R_1$ is selected from the group consisting of H, $C_1$-$C_6$ alkyl or $C_1$-$C_6$ deuteroalkyl;
$R_3$ is selected from the group consisting of H, $C_1$-$C_6$ alkyl or $C_1$-$C_6$ deuteroalkyl; $R_4$ is selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$ and $R_6$ are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring;
$R_8$, $R_9$ and $R_{10}$ are each independently selected from the group consisting of H, $C_1$-$C_6$ alkyl, amino or hydroxyl;
X is selected from the structure represented by formula (Ib'):

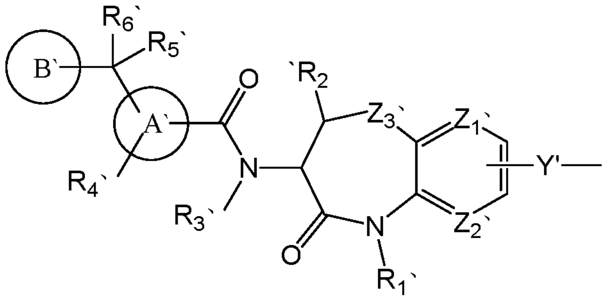

(Ib`)

;

wherein, Y' is selected from the group consisting of ≡, =, O, $CH_2$ or

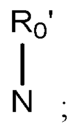

;

$R_0$' is selected from the group consisting of H, $C_1$-$C_5$ alkyl or cycloalkyl;
ring A' is a 3-6 membered unsaturated heterocyclyl;
ring B' is a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino or hydroxyl;
$Z_1$' and $Z_2$' are each independently selected from CH or N;
$Z_3$' is selected from the group consisting of O, $NR_8$, S or $CR_8R_9$;
$R_1$' and $R_2$' are each independently selected from the group consisting of H, $C_1$-$C_6$ alkyl or $C_1$-$C_6$ deuteroalkyl;
$R_3$' is selected from the group consisting of H, $C_1$-$C_6$ alkyl or $C_1$-$C_6$ deuteroalkyl; $R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$' and $R_4$' are linked to form a ring; or $R_4$' and $R_5$' are linked to form a ring;
$R_8$, $R_9$ and $R_{10}$ are each independently selected from the group consisting of H, $C_1$-$C_6$ alkyl, amino or hydroxyl;
L is a chain containing carbon atoms, which can also include any number of heteroatoms N, O, S, and P;
$R_2$ is selected from the group consisting of H, $C_1$-$C_6$ alkyl or $C_1$-$C_6$ deuteroalkyl;
Y' is selected from the group consisting of ≡, =, O, $CH_2$ or

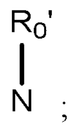

$R_0$ is selected from the group consisting of H, $C_1$-$C_5$ alkyl or cycloalkyl;

alternatively, X is not the structure represented by formula (Ib'), and provided that $R_2$ is methyl, L and X form any group containing carbons; Y is selected from the group consisting of ≡, = or

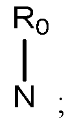

$R_0$ is selected from the group consisting of H, $C_1$-$C_5$ alkyl or cycloalkyl;

alternatively, X is not the structure represented by formula (Ib'), and provided that $R_2$ is H, X is selected from the group consisting of H, vinyl, ethynyl, hydroxyl, thiol, aldehyde, carboxyl, sulfo group, $-NR_8R_9$, $-CR_8R_9R_{10}$, a substituted or unsubstituted, saturated or unsaturated $C_3$-$C_{10}$ cycloalkyl, a substituted or unsubstituted, saturated or unsaturated 3-10 membered heterocyclyl, a substituted or unsubstituted $C_5$-$C_{10}$ aryl, a substituted or unsubstituted 5-10 membered heteroaryl or halogen; the substituted substituents are selected from the group consisting of alkyl, cyano, amino, hydroxyl, nitro or sulfo group;

L is $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}-O-CH_2-$; $n_2$, $n_2'$, $m_1$, $m_{1'}$, $m_2$, and $m_2'$ are each independently selected from an integer of 1 to 10;

wherein, W is selected from the group consisting of $-(OCH_2CH_2)_{n3}O-$, a saturated or unsaturated 3-10 membered heterocyclic hydrocarbon group containing N, or a 5-10 membered N-containing heteroaryl; wherein $n_3$ is selected from an integer of 1 to 10;

Y is.

2. A compound according to claim 1, or a salt thereof, or a stereoisomer thereof, or a solvate thereof, or a prodrug thereof, **characterized in that** X is a structure represented by formula (Ib'), Y and Y' is ≡, and the compound has a structure represented by following formula (II):

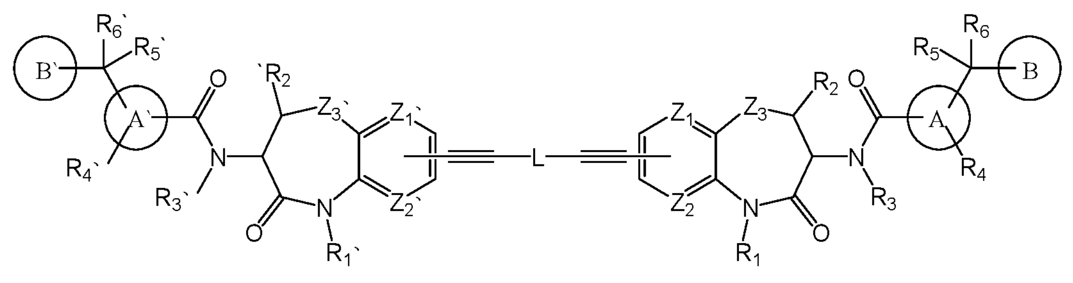

(II)

;

wherein, L is $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2'$, $m_1$, $m_1'$, $m_2$, and $m_2'$ are each independently selected from an integer of 1 to 10;

wherein, W is selected from the group consisting of $-NR_7-$, O, S, 1,3-succinynyl,$-(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (Ia):

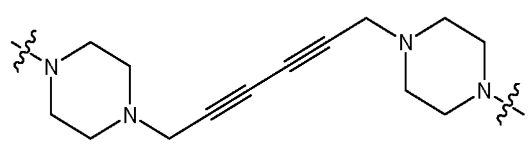

(Ia)

;

wherein $R_7$ is H or $C_1$-$C_3$ alkyl; $n_3$ is selected from an integer of 1 to 10;
wherein, rings A and A' are each independently selected from 3-6 membered unsaturated heterocyclyls;
rings B and B' are each independently selected from a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino or hydroxyl;
$Z_1$, $Z_2$, $Z_1$' and $Z_2$' are each independently selected from CH or N;
$Z_3$ and $Z_3$' are each independently selected from the group consisting of O, $NR_8$, S or $CR_8R_9$;
$R_1$, $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl;
$R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring; or $R_3$' and $R_4$' are linked to form a ring; or $R_4$' and $R_5$' are linked to form a ring;
$R_8$ and $R_9$ are selected from the group consisting of H or $C_1$-$C_6$ alkyl.

3. A compound according to claim 2, or a salt thereof, or a stereoisomer thereof, or a solvate thereof, or a prodrug thereof, **characterized in that** $Z_1$, $Z_2$, $Z_1$', and $Z_2$' are CH; $Z_3$ and $Z_3$' are O; the compound has the structure as represented by following formula (IIa):

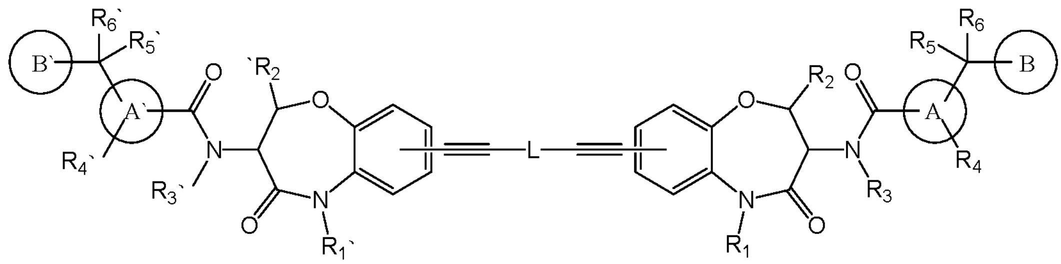

(IIa) ;

wherein, L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;

wherein, W is selected from the group consisting of $-NR_7-$, O, S, 1,3-succinynyl,$-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (Ia):

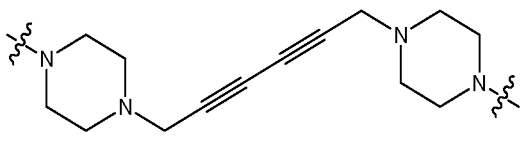

(Ia) ;

wherein $R_7$ is H or $C_1$-$C_3$ alkyl; $n_3$ is selected from an integer of 1 to 4;
rings A and A' are each independently selected from 5-membered unsaturated heterocyclyls; the heteroatoms contained in the heterocyclic group are N, O, or S, and the number of heteroatoms is 1, 2, 3, or 4;
rings B and B' are each independently selected from 5-membered or 6-membered aryl or heteroaryl; the heteroatoms contained in the heteroaryl are N, O, or S, and the number of heteroatoms is 1, 2, 3, or 4;
$R_1$, $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl;
$R_4$ and $R_4$' are each independently selected from the group consisting of H, $C_1$-$C_6$ alkyl, $C_1$-$C_6$ deuteroalkyl,

$C_1$-$C_6$ alkoxy, hydroxyl, amino, cyano, or thiol;

$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;

or $R_3$ and $R_4$ are linked to form a ring; or $R_4$ and $R_5$ are linked to form a ring; or $R_3$' and $R_4$' are linked to form a ring; or $R_4$' and $R_5$' are linked to form a ring.

**4.** A compound according to claim 3, or a salt thereof, or a stereoisomer thereof, or a solvate thereof, or a prodrug thereof, **characterized in that** $R_3$, $R_3$', $R_4$, $R_4$', $R_5$, $R_5$', $R_6$, and $R_6$' are H; rings B and B' ar phenyl; the compound has the structure as represented by following formula (IIb):

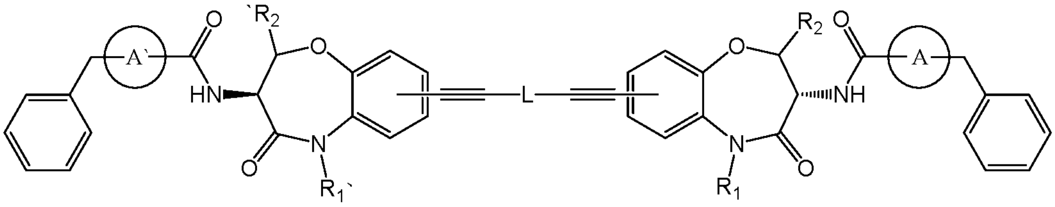

(IIb) ;

wherein, L is selected from the group consisting of $-(CH_2)_{n1}-$, $-(CH_2)_{m1}-W-(CH_2)_{m1'}-$ or $-CH_2-O(CH_2CH_2O)_{m2}-(CH_2)_{n2}-W-(CH_2)_{n2'}-(OCH_2CH_2)_{m2'}O-CH_2-$; $n_1$, $n_2$, $n_2$', $m_1$, $m_1$', $m_2$, and $m_2$' are each independently selected from an integer of 1 to 4;

wherein, W is selected from the group consisting of imino, $-N(CH_3)-$, 1,3-succinynyl, $-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (Ia):

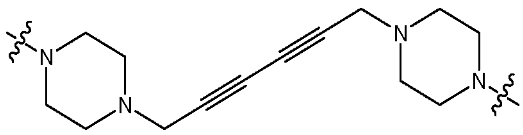

(Ia) ;

wherein, $n_3$ is selected from an integer of 1 to 4;

rings A and A' are each independently selected from the group consisting of

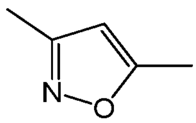
,

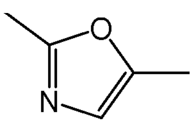
,
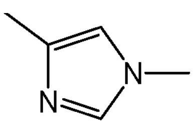
,
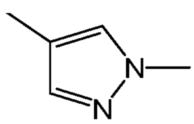
,
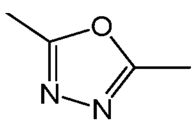
,
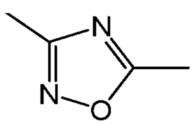
,
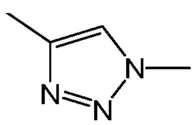
,

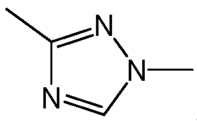
,
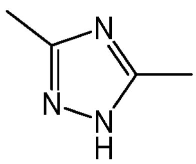
or
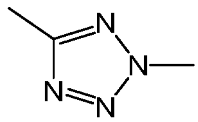
;

$R_1$, $R_2$, $R_1$' and $R_2$' are each independently selected from the group consisting of H, $C_1$-$C_6$ alkyl or $C_1$-$C_6$ deuteroalkyl.

5. A compound according to claim 4, or a salt thereof, or a stereoisomer thereof, or a solvate thereof, or a prodrug thereof, **characterized in that** L is the following structures:

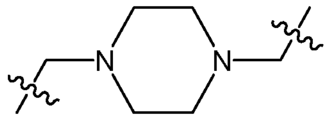

,

$-CH_2-(OCH_2CH_2)_{n3}O-CH_2-$,

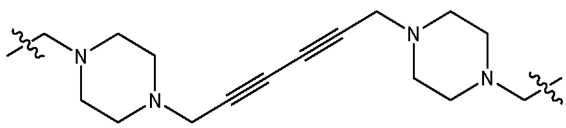

or

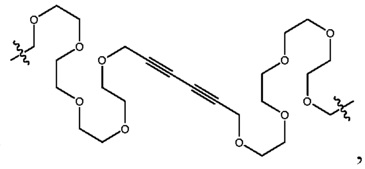

,

wherein, $n_3$ is selected from an integer of 1 to 4.

6. A compound according to claim 1, or a salt thereof, or a stereoisomer thereof, or a solvate thereof, or a prodrug thereof, **characterized in that** X is a structure represented by formula (Ib'), Y and Y' is $CH_2$, and the compound has a structure represented by following formula (III):

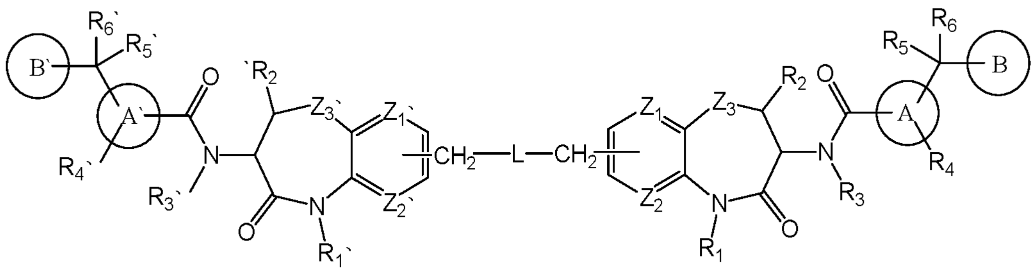

(III)

:

wherein, L is selected from the group consisting of $-(CH_2)_{m1}-W'-(CH_2)_{m1'}-$ or $-CH_2-O(CH_2CH_2O)_{m2}-(CH_2)_{n2}-W'-(CH_2)_{n2'}-(OCH_2CH_2)_{m2'}O-CH_2-$; $n_2$, $n_2$', $m_1$, $m_1$', $m_2$, and $m_2$' are each independently selected from an integer of 1 to 10;

wherein, W' is selected from the group consisting of $-(CH_2)_{n4}-$, $-NR_7-$, O, S, 1,3-succinynyl, $-(OCH_2CH_2)_{n3}O-$, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (IIIc):

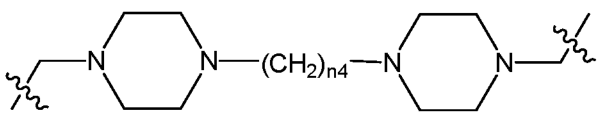

(IIIc)

;

wherein $R_7$ is H or $C_1$-$C_3$ alkyl; $n_3$ is selected from an integer of 1 to 10; $n_4$ is selected from an integer of 1 to 6;
wherein, rings A and A' are each independently selected from 3-6 membered unsaturated heterocyclyls;
rings B and B' are each independently selected from a substituted or unsubstituted, saturated or unsaturated $C_3$-$C_6$ cyclic hydrocarbon group, a substituted or unsubstituted, saturated or unsaturated 3-6 membered heterocyclic hydrocarbon group, a substituted or unsubstituted $C_5$-$C_{10}$ aryl, or a substituted or unsubstituted 5-10 membered heteroaryl; the substituted substituents are halogen, alkyl, cyano, amino or hydroxyl;

$Z_1$, $Z_2$, $Z_1$' and $Z_2$' are each independently selected from CH or N;
$Z_3$ and $Z_3$' are each independently selected from the group consisting of O, $NR_8$, S or $CR_8R_9$;
$R_1$ $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl;
$R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring; or $R_3$' and $R_4$' are linked to form a ring; or $R_4$' and $R_5$' are linked to form a ring;
$R_8$ and $R_9$ are selected from the group consisting of H or $C_1$-$C_6$ alkyl.

7. A compound according to claim 6, or a salt thereof, or a stereoisomer thereof, or a solvate thereof, or a prodrug thereof, **characterized in that** $Z_1$, $Z_2$, $Z_1$', and $Z_2$' are CH; $Z_3$ and $Z_3$' are O; the compound has the structure as represented by following formula (IIIa):

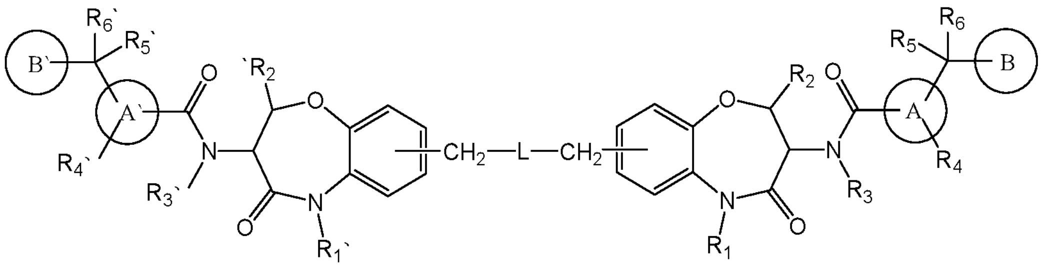

(IIIa) ;

wherein, L is selected from the group consisting of $-(CH_2)_{m1}$-W'-$(CH_2)_{m1'}$- or $-CH_2-O(CH_2CH_2O)_{m2}-(CH_2)_{n2}$-W'-$(CH_2)_{n2'}-(OCH_2CH_2)_{m2}-O-CH_2-$; $n_2$, $n_2$', $m_1$, $m_1$', $m_2$, and $m_2$' are each independently selected from an integer of 1 to 10;

wherein, W' is selected from the group consisting of $-(CH_2)_{n4}$-, $-NR_7$-, O, S, 1,3-succinynyl,$-(OCH_2CH_2)_{n3}O$-, a saturated or unsaturated $C_3$-$C_{10}$ cyclic hydrocarbon group, a saturated or unsaturated 3-10 membered heterocyclic hydrocarbon group, a $C_5$-$C_{10}$ aryl, a 5-10 membered heteroaryl or a structure as represented by formula (IIIc):

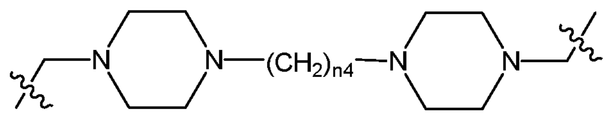

(IIIc) ;

wherein $R_7$ is H or $C_1$-$C_3$ alkyl; $n_3$ is selected from an integer of 1 to 10; $n_4$ is selected from an integer of 1 to 6; wherein, rings A and A' are each independently selected from 5-membered unsaturated heterocyclyls; the heteroatoms contained in the heterocyclic group are N, O, or S, and the number of heteroatoms is 1, 2, 3, or 4; rings B and B' are each independently selected from 5-membered or 6-membered aryl or heteroaryl; the heteroatoms contained in the heteroaryl are N, O, or S, and the number of heteroatoms is 1, 2, 3, or 4;
$R_1$, $R_2$, $R_1$' and $R_2$' are each independently selected from the group consisting of H, $C_1$-$C_6$ alkyl or $C_1$-$C_6$ deuteroalkyl;
$R_3$ and $R_3$' are each independently selected from the group consisting of H, $C_1$-$C_6$ alkyl or $C_1$-$C_6$ deuteroalkyl;
$R_4$ and $R_4$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
$R_5$, $R_6$, $R_5$' and $R_6$' are each independently selected from the group consisting of H, $C_1$-$C_6$ alkyl, $C_1$-$C_6$ deuteroalkyl, $C_1$-$C_6$ alkoxy, hydroxyl, amino, cyano, or thiol;
or $R_3$ and $R_4$ are linked to form a ring; or $R_4$ and $R_5$ are linked to form a ring; or $R_3$' and $R_4$' are linked to form

a ring; or $R_4$' and $R_5$' are linked to form a ring.

**8.** A compound according to claim 7, or a salt thereof, or a stereoisomer thereof, or a solvate thereof, or a prodrug thereof, **characterized in that** $R_3$, $R_3$', $R_4$, $R_4$, $R_5$, $R_5$', $R_6$, and $R_6$' are H; rings B and B' are phenyl; the compound has the structure as represented by following formula (IIIb):

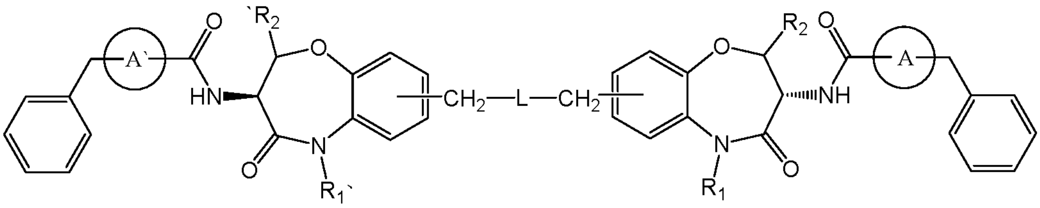

(IIIb) ;

wherein, L is selected from the group consisting of $-(CH_2)_{m1}-W'-(CH_2)_{m1'}-$ or $-CH_2-O(CH_2CH_2O)_{m2}-(CH_2)_{n2}-W'-(CH_2)_{n2'}-(OCH_2CH_2)_{m2}-O-CH_2-$; $n_2$, $n_2$', $m_1$, $m_1$', $m_2$, and $m_2$' are each independently selected from an integer of 1 to 10;

wherein, W is selected from the group consisting of $-(CH_2)_{n4}-$, imino, $-N(CH_3)-$, 1,3-succinynyl, $-(OCH_2CH_2)_{n3}O-$, a saturated or unsaturated $C_4$-$C_6$ cyclic hydrocarbon group, a saturated or unsaturated 4-6 membered heterocyclic hydrocarbon group, a $C_5$-$C_{10}$ aryl, a 5-10 membered heteroaryl or a structure as represented by formula (IIIc):

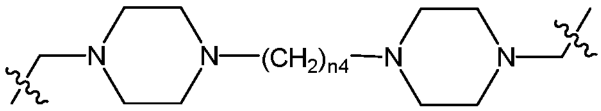

(IIIc) ;

$n_3$ is selected from an integer of 1 to 4; $n_4$ is selected from an integer of 1 to 6;
rings A and A' are each independently selected from the group consisting of:

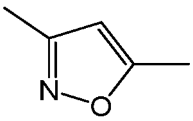

,

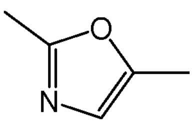

,

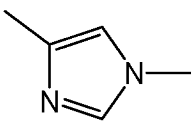

,

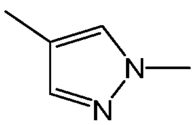

,

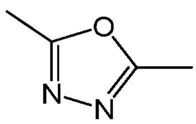

,

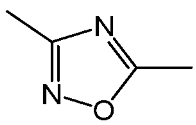

,

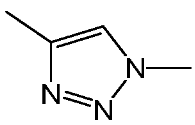

,

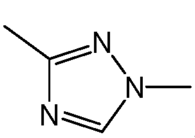

,

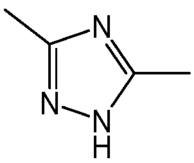

or

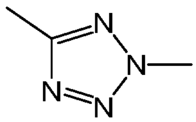

;

$R_1$, $R_2$, $R_1$' and $R_2$' are each independently selected from the group consisting of H, $C_1$-$C_6$ alkyl or $C_1$-$C_6$ deuteroalkyl.

**9.** A compound according to claim 8, or a salt thereof, or a stereoisomer thereof, or a solvate thereof, or a prodrug

thereof, **characterized in that** L is the following structures:

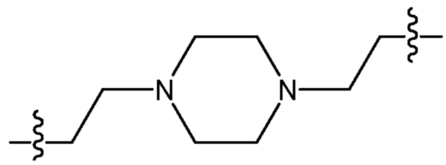

,

$-CH_2-(OCH_2CH_2)_{n4}O-CH_2-$,

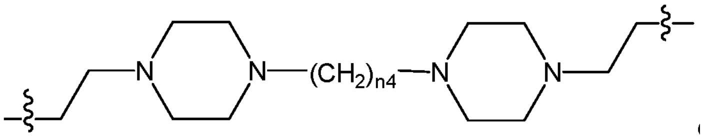

,

or

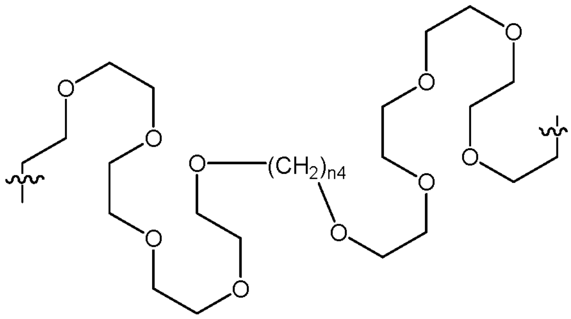

;

wherein, $n_4$ is selected from an integer of 1 to 6.

**10.** A compound according to any one of claims 1 to 9, or a salt thereof, or a stereoisomer thereof, or a solvate thereof, or a prodrug thereof, **characterized in that** the salt is hydrochloride, sulfate, citrate, benzenesulfonate, hydrobromate, hydrofluorate, phosphate, acetate, propionate, succinate, oxalate, lactate, malate, succinate, fumarate, maleate, tartrate, trifluoroacetate, sodium salt or potassium salt.

**11.** A compound according to any one of claims 1 to 9, or a salt thereof, or a stereoisomer thereof, or a solvate thereof, or a prodrug thereof, **characterized in that** the compound is one of the following compounds:

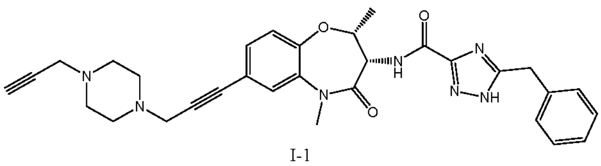

I-1

,

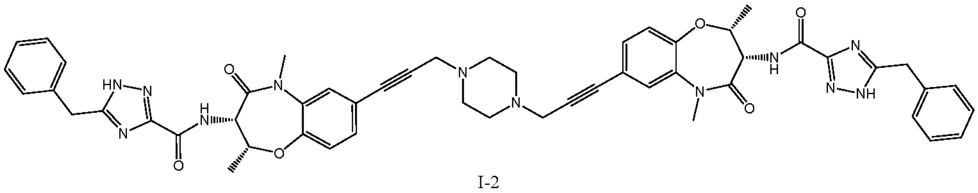

I-2

,

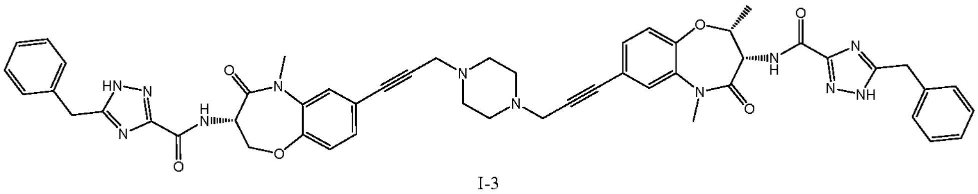

I-3

,

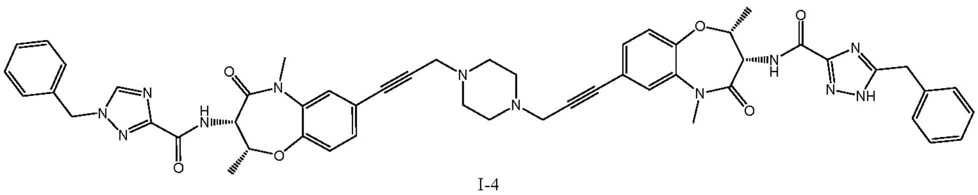

I-4

,

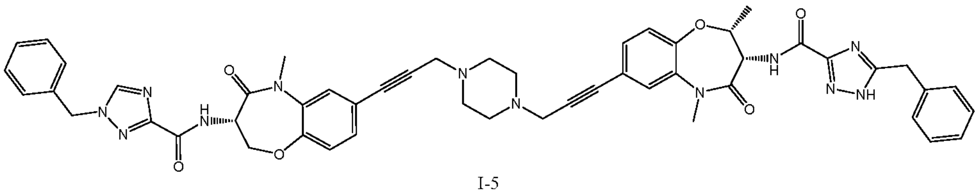

I-5

,

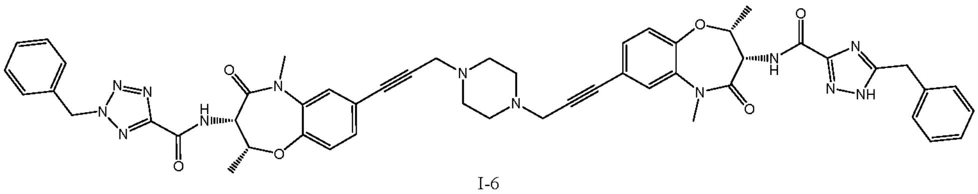

I-6

,

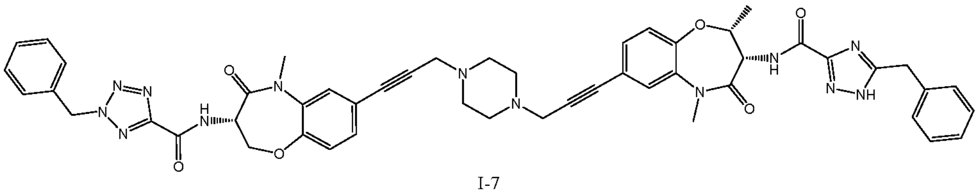

I-7

,

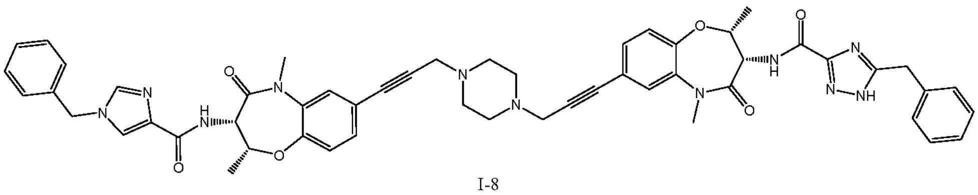

I-8

,

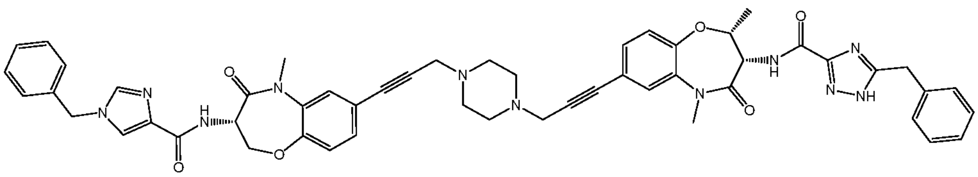

I-9

,

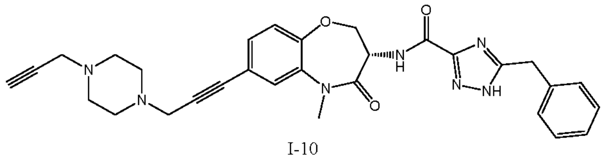

I-10

,

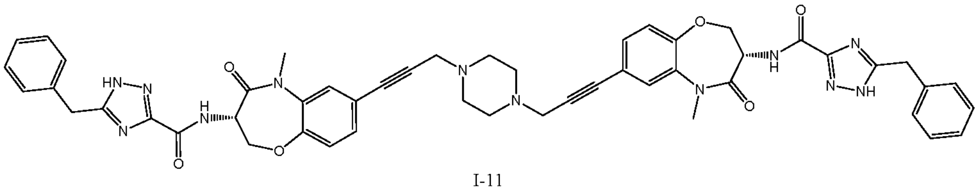

I-11

,

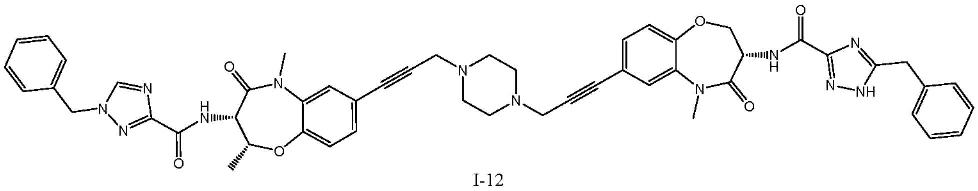

I-12

,

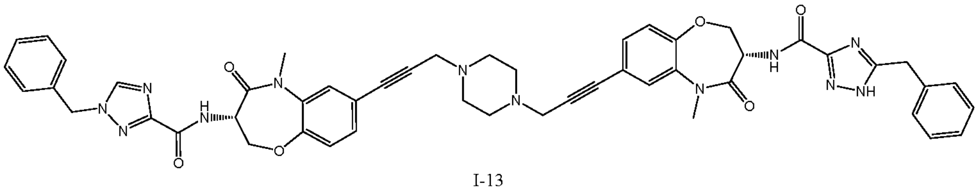

I-13

,

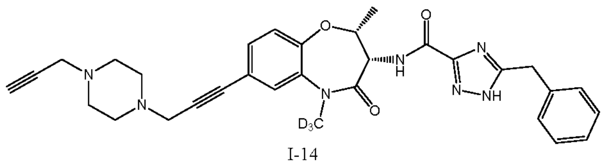

I-14

,

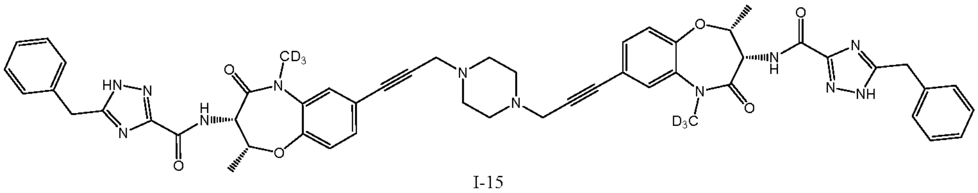

I-15

,

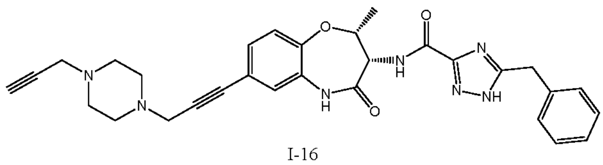

I-16

,

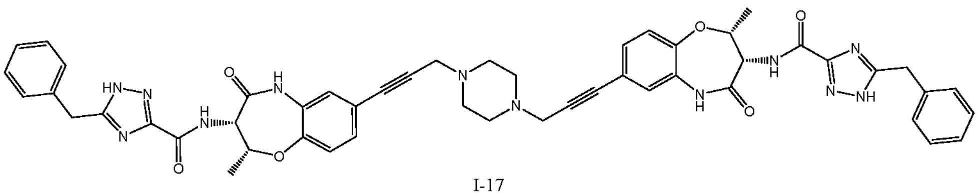

I-17

,

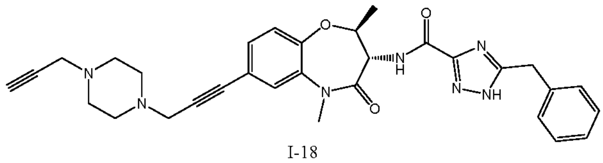

I-18

,

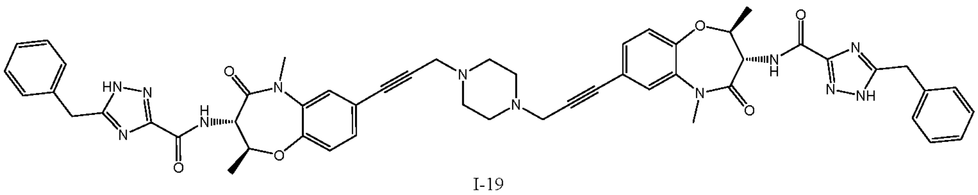

I-19

,

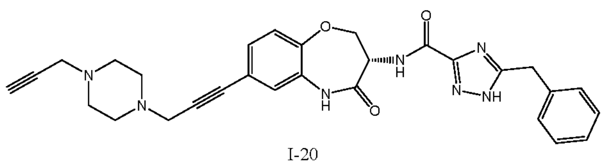

I-20

,

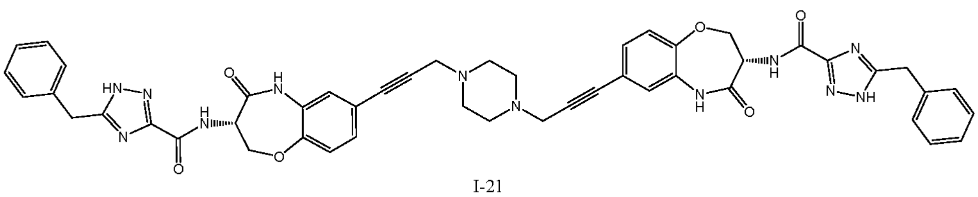

I-21

,

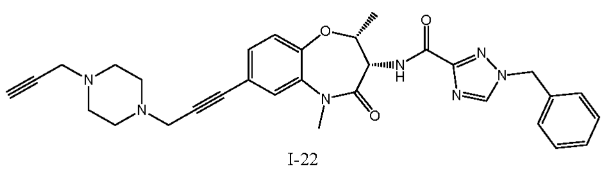

I-22

,

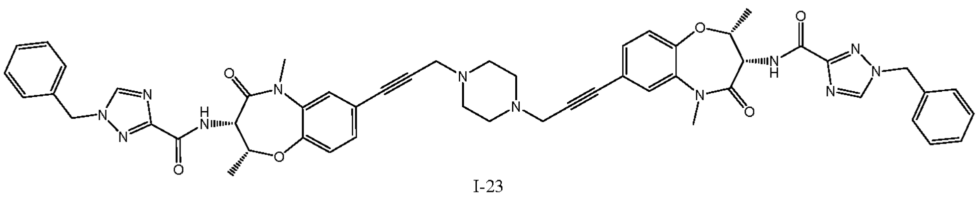

I-23

,

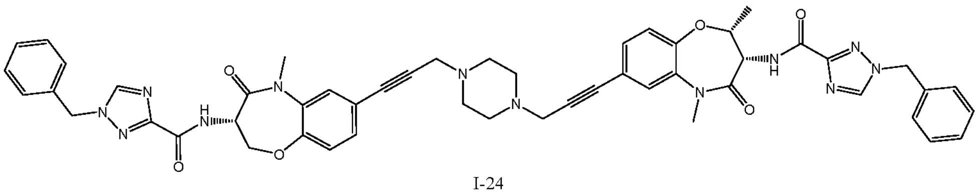

I-24

,

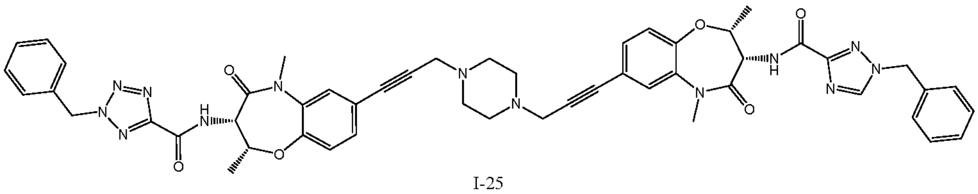

I-25

,

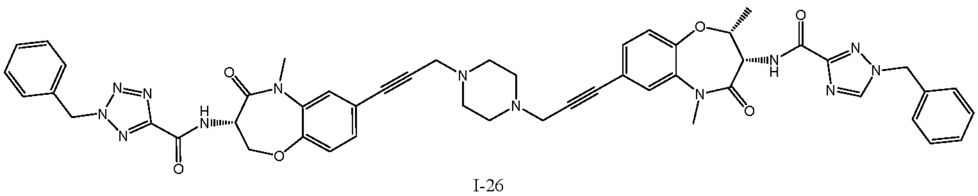

I-26

,

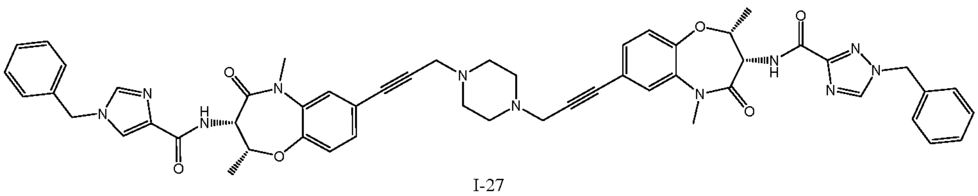

I-27

,

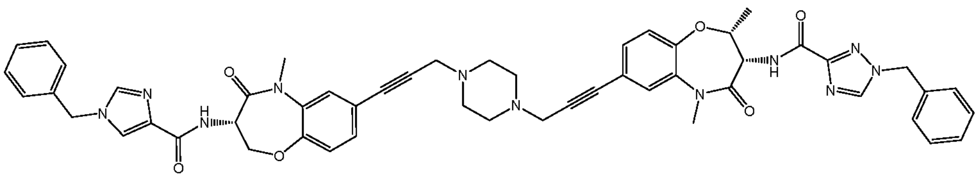

I-28

,

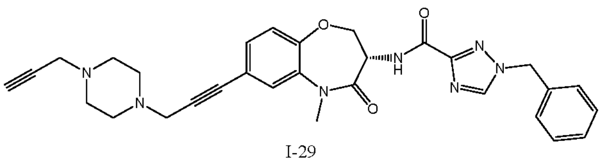

I-29

,

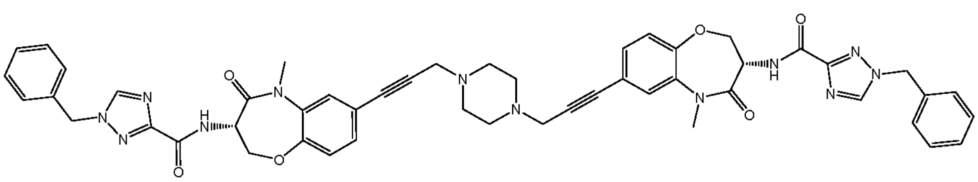

I-30

,

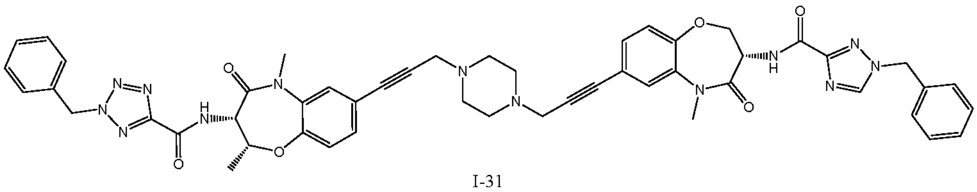

I-31

,

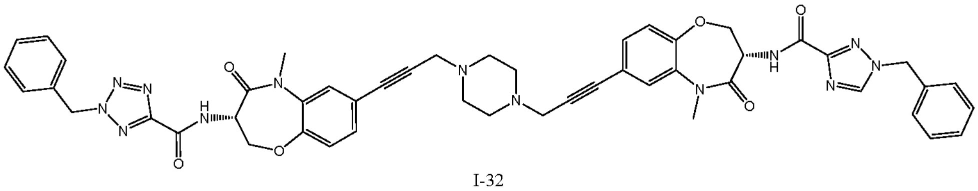

I-32

,

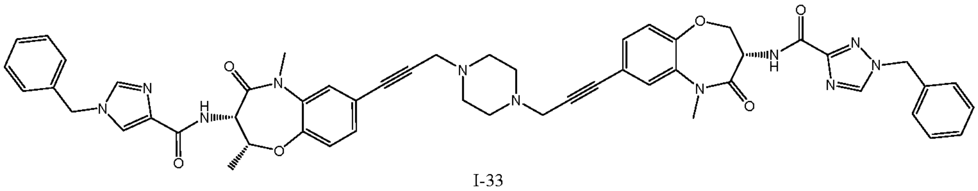

I-33

,

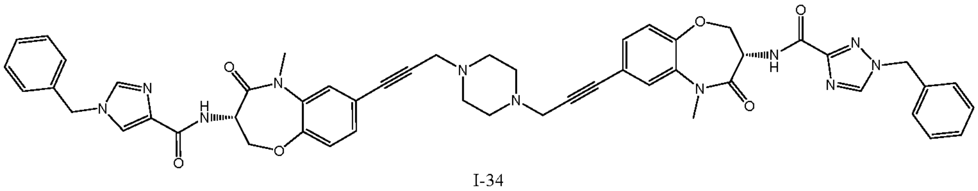

I-34

,

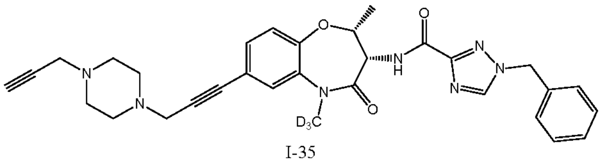

I-35

,

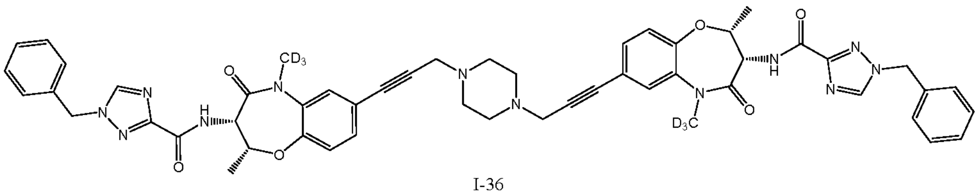

I-36

,

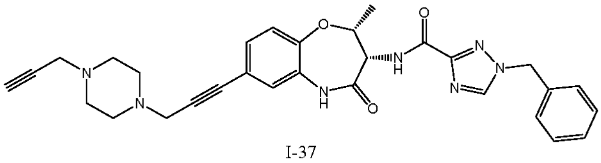

I-37

,

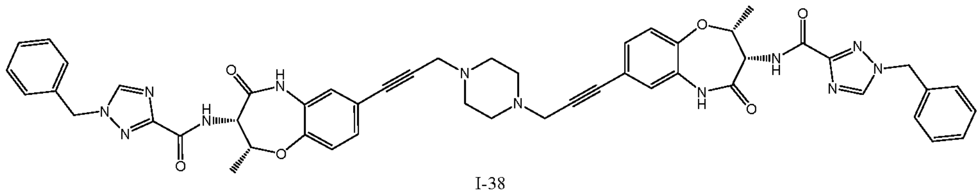

I-38

,

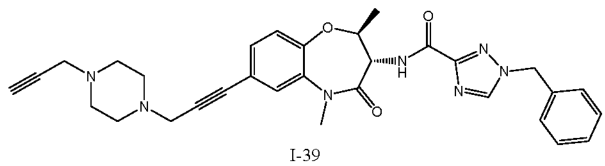

I-39

,

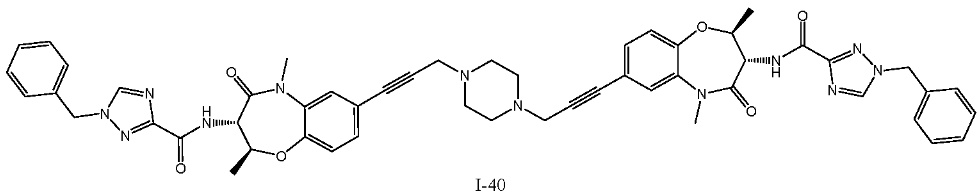

I-40

,

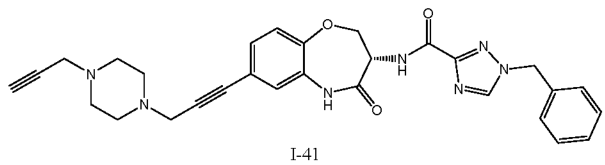

I-41

,

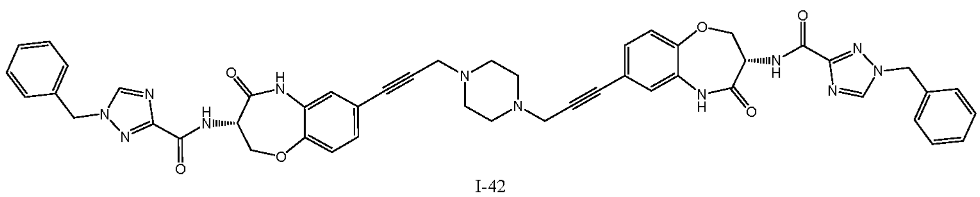

I-42

,

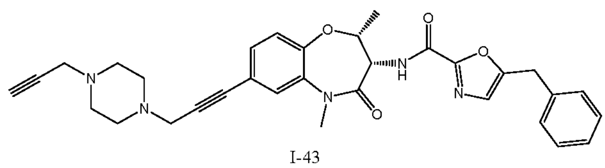

I-43

,

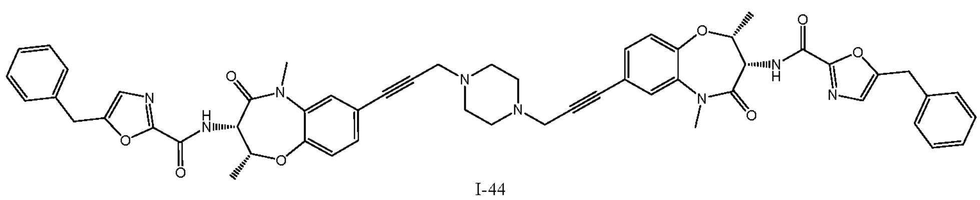

I-44

,

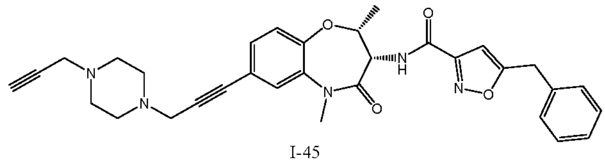

I-45

,

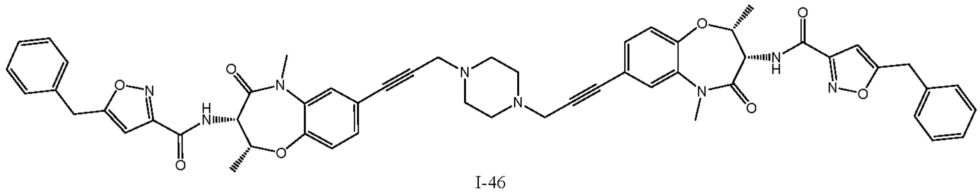

I-46

,

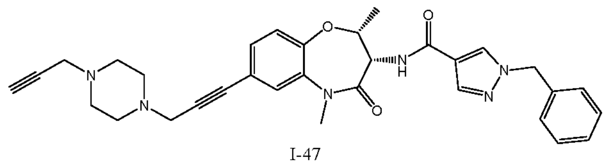

I-47

,

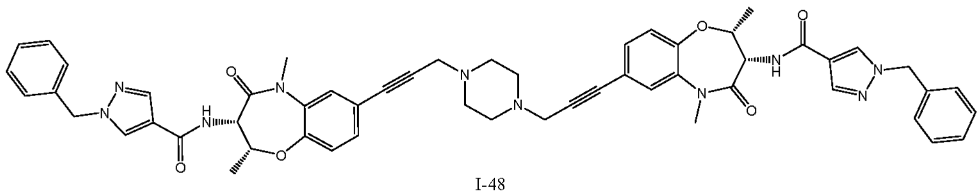

I-48

,

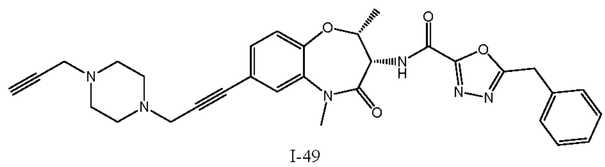

I-49

,

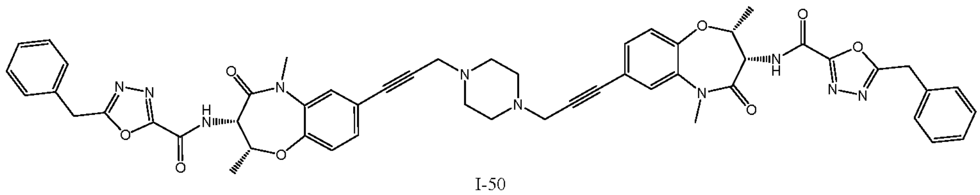

I-50

,

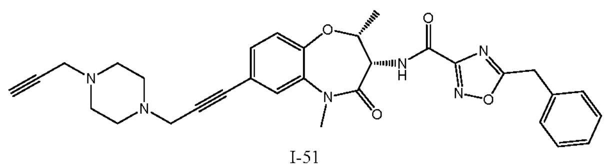

I-51

,

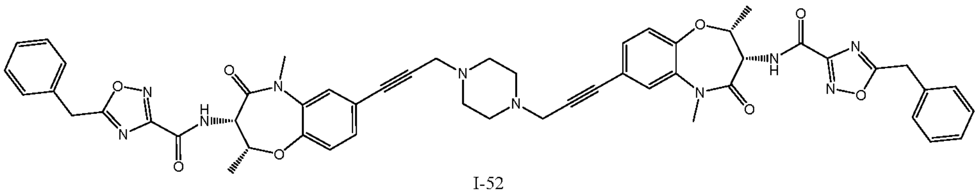

I-52

,

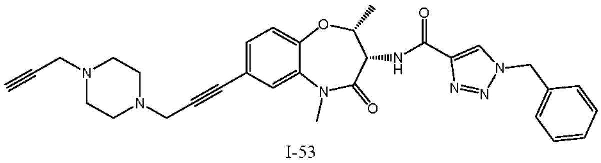

I-53

,

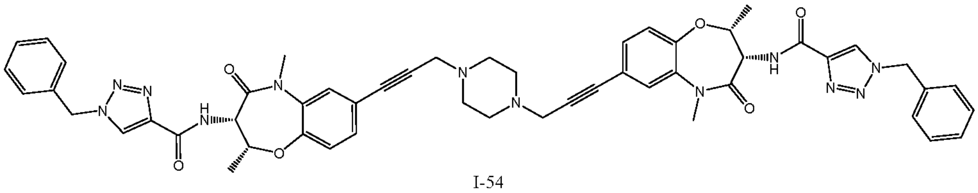

I-54

,

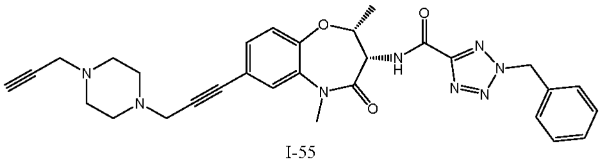

I-55

,

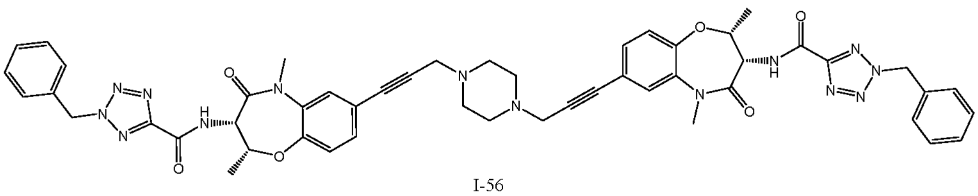

I-56

,

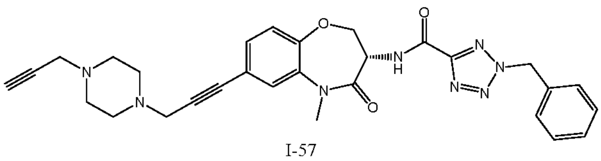

I-57

,

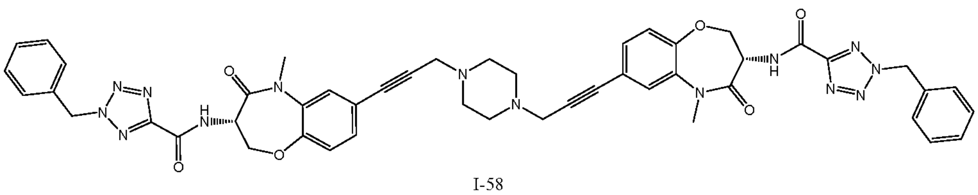

I-58

,

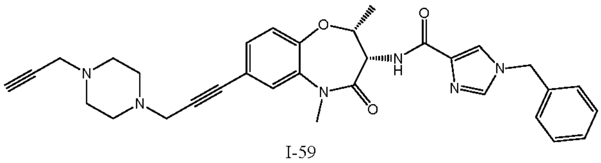

I-59

,

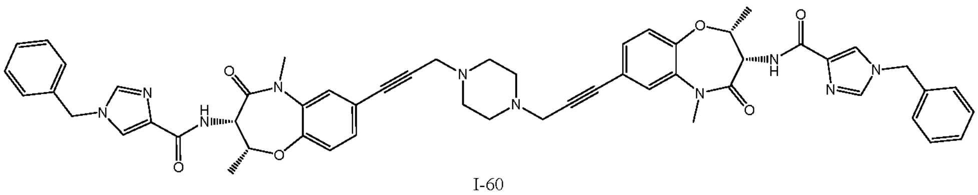

I-60

,

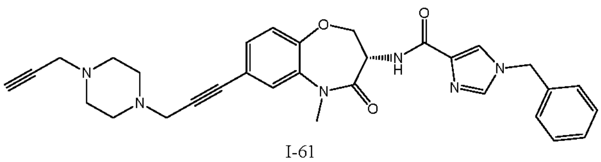

I-61

,

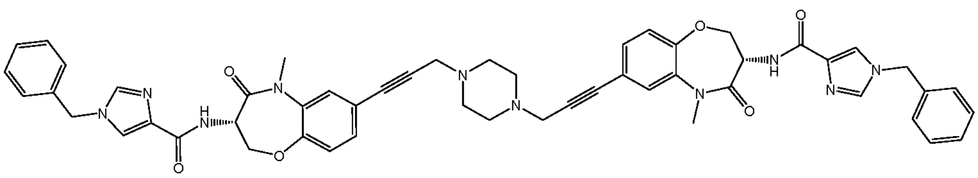

I-62

,

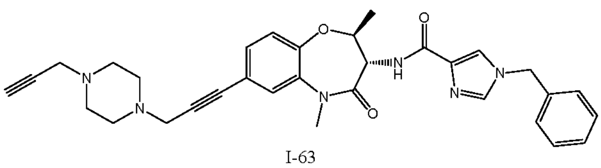

I-63

,

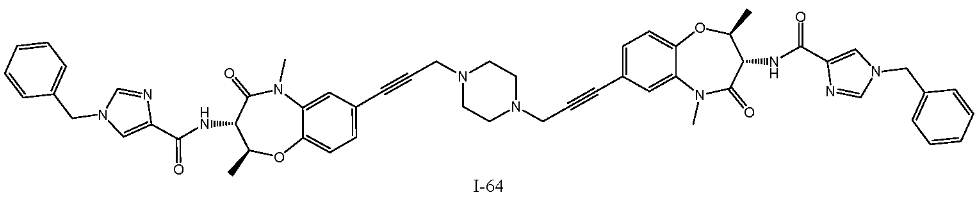

I-64

,

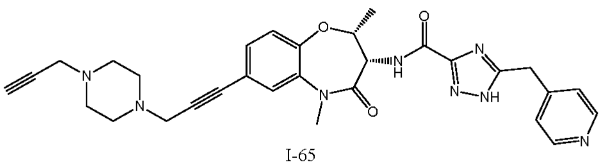

I-65

,

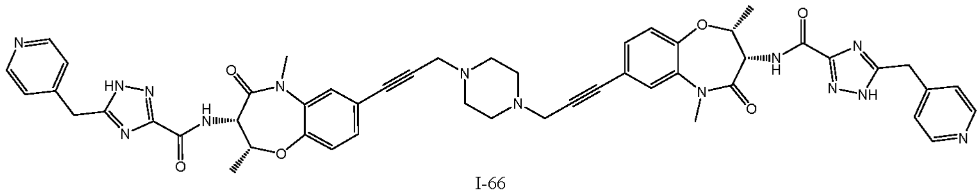

I-66

,

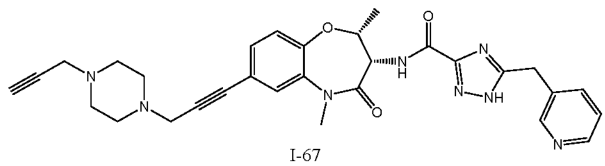

I-67

,

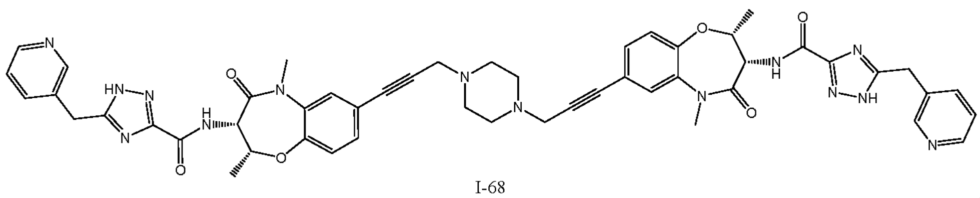

I-68

,

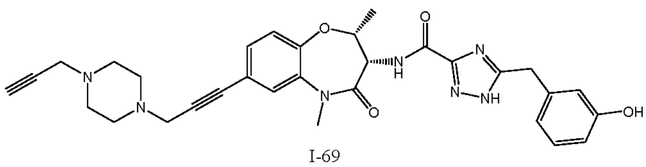

I-69

,

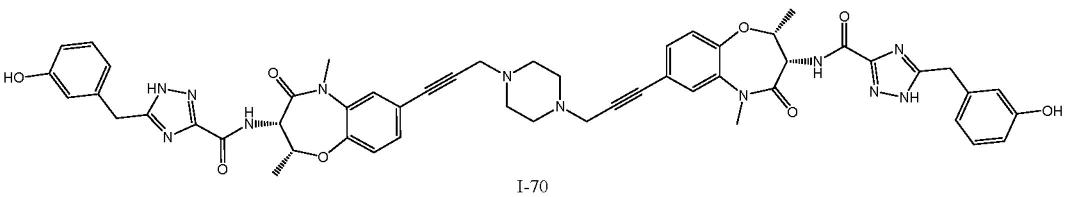

I-70

,

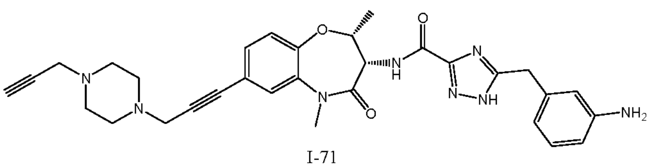

I-71

,

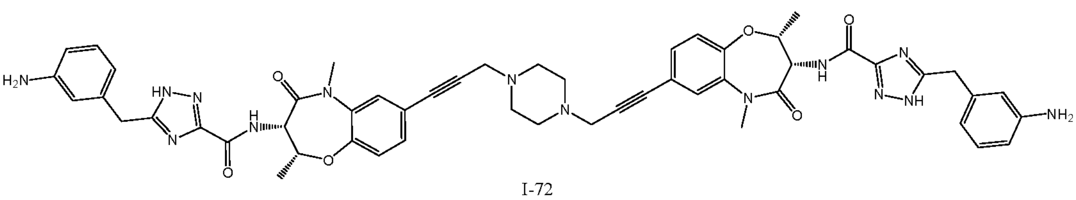

I-72

,

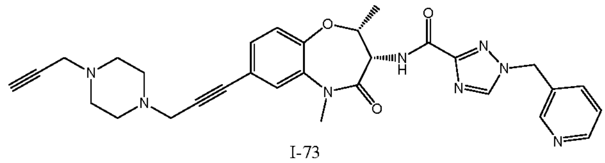

I-73

,

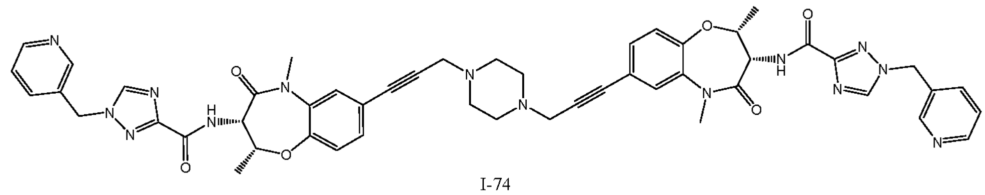

I-74

,

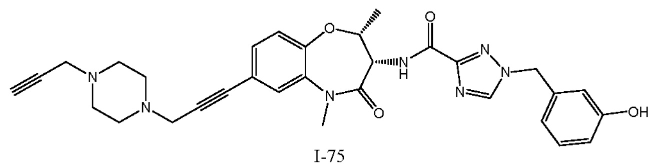

I-75

,

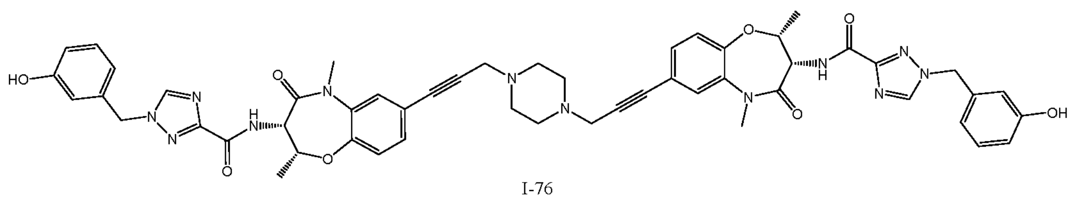

I-76

,

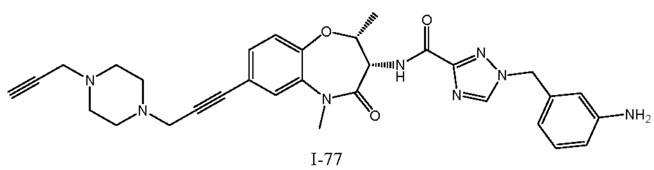

I-77

,

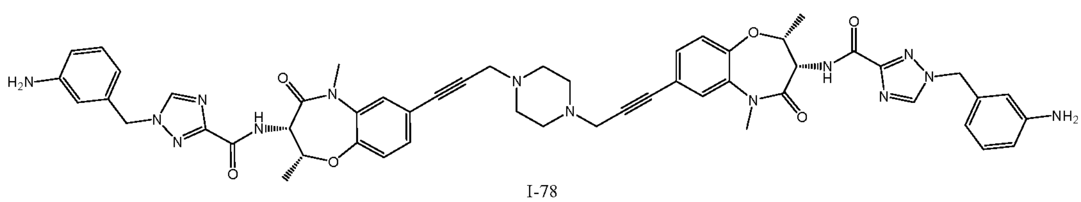

I-78

,

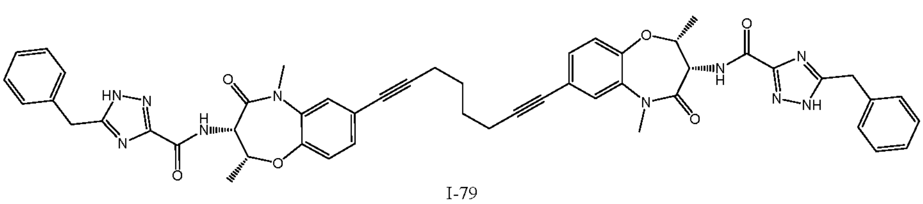

I-79

,

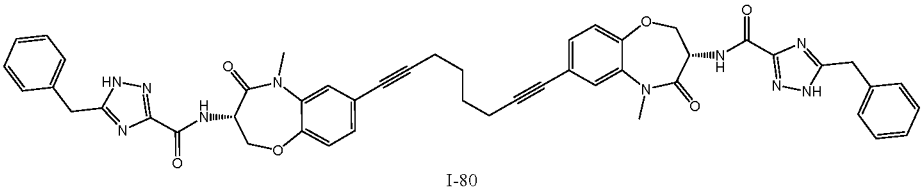

I-80

,

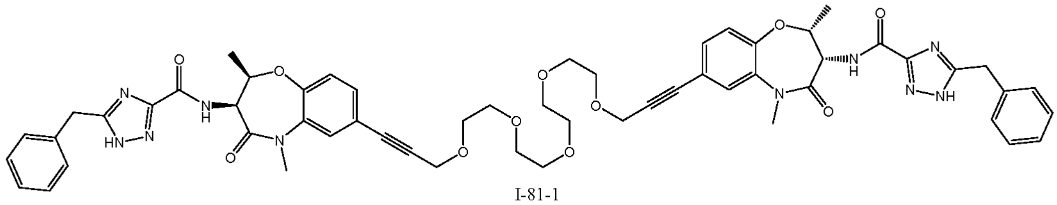

I-81-1

,

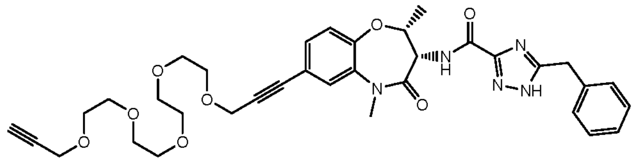

I-81-2

,

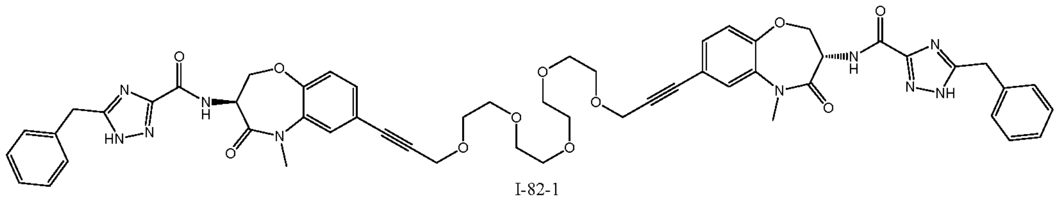

I-82-1

,

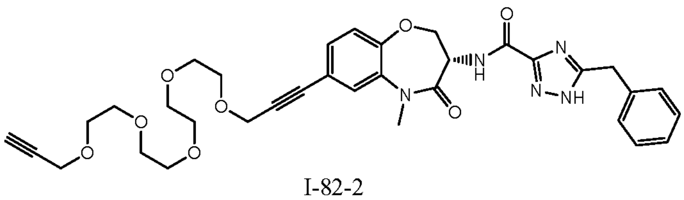

I-82-2

,

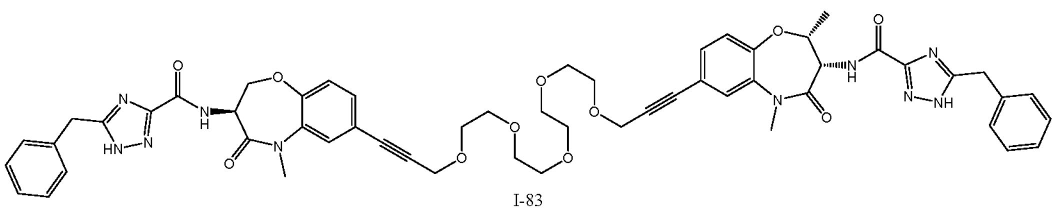

I-83

,

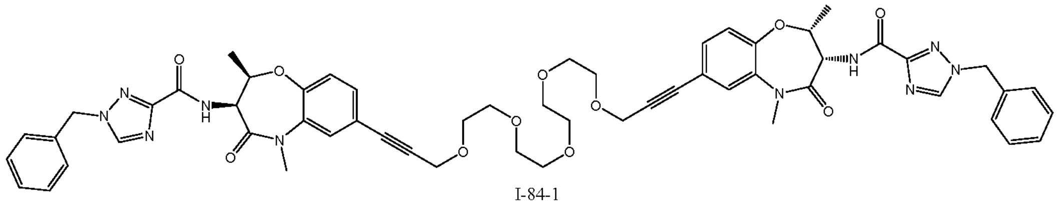

I-84-1

,

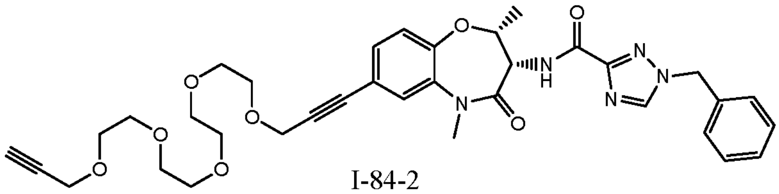
I-84-2
,

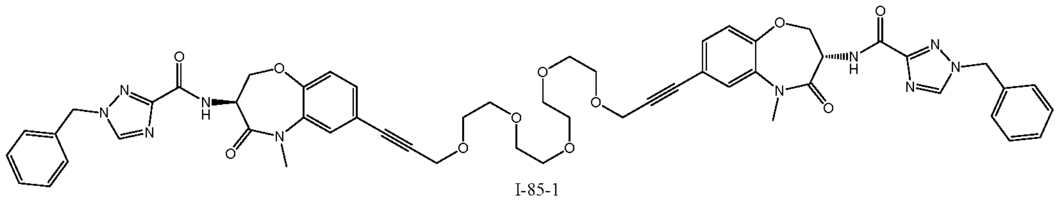
I-85-1
,

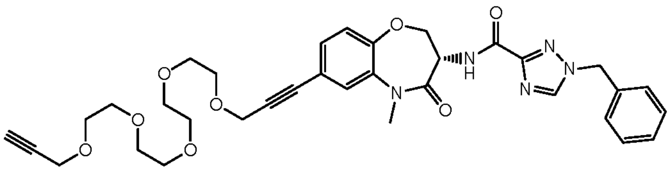
I-85-2
,

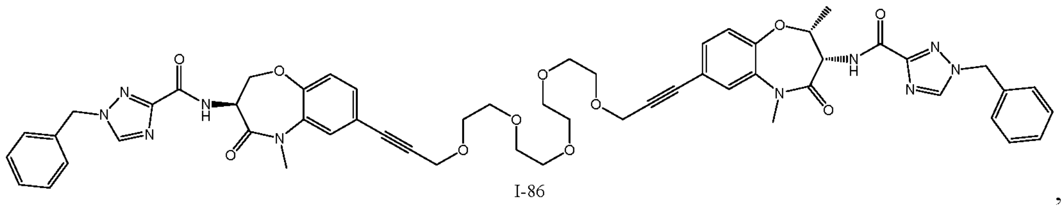
I-86
,

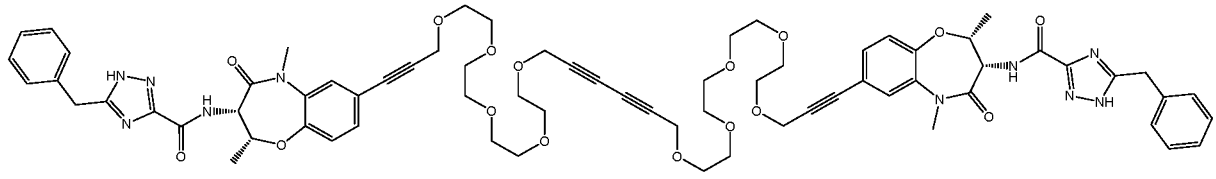
I-87
,

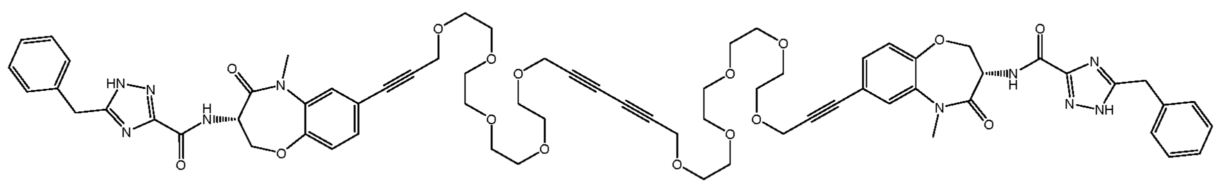
I-88
,

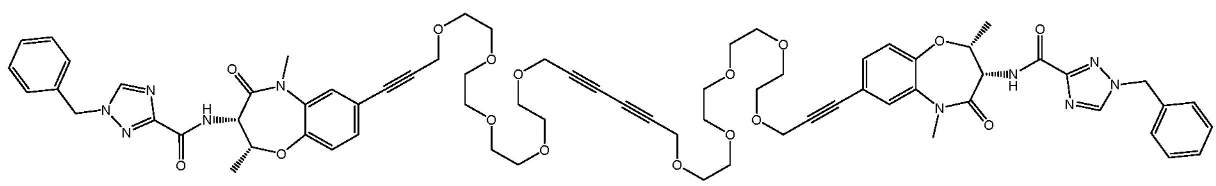
I-89
,

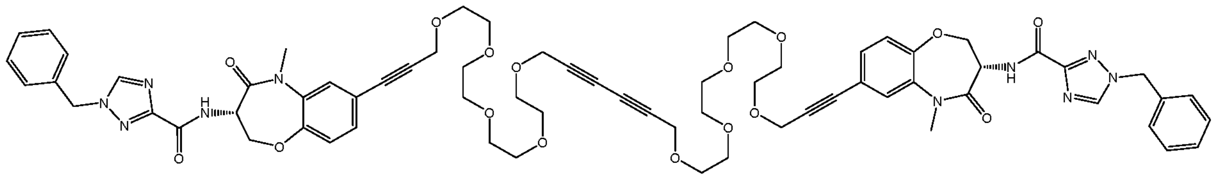

I-90

,

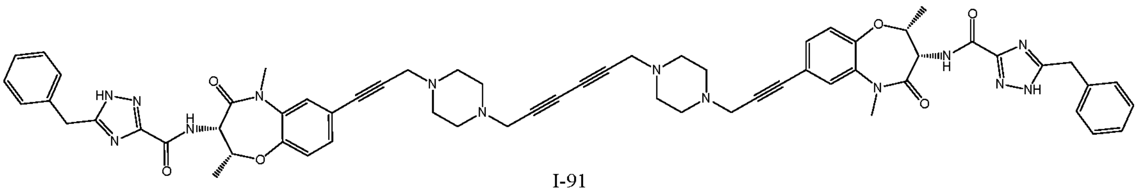

I-91

,

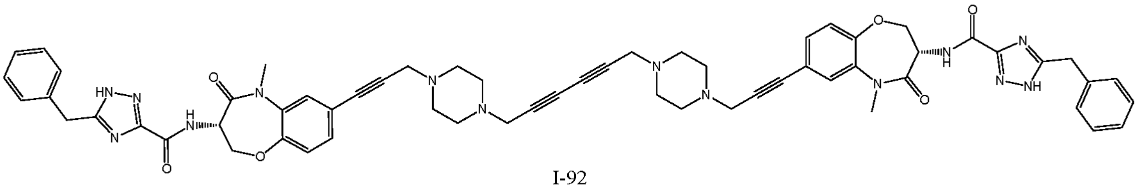

I-92

,

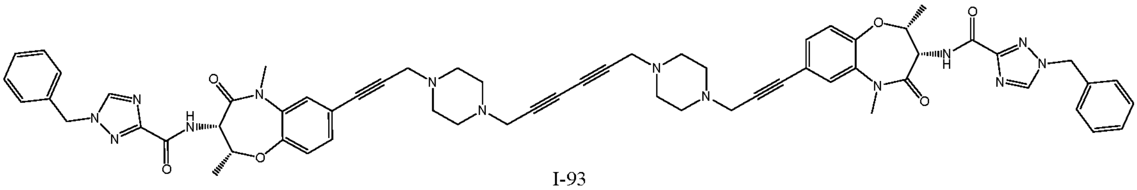

I-93

,

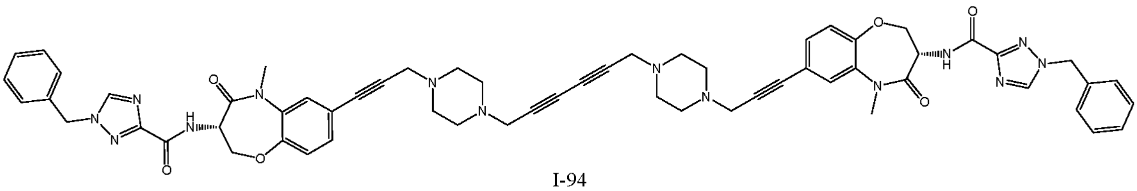

I-94

,

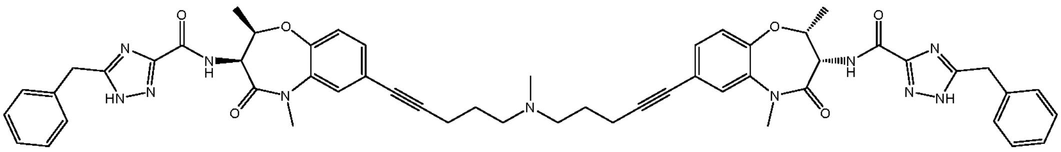

I-95

,

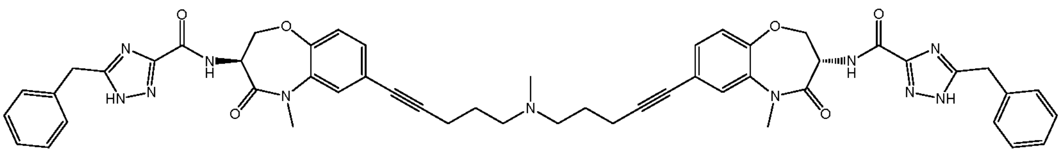

I-96

,

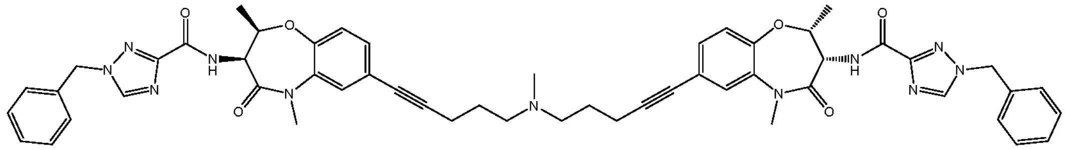

I-97

,

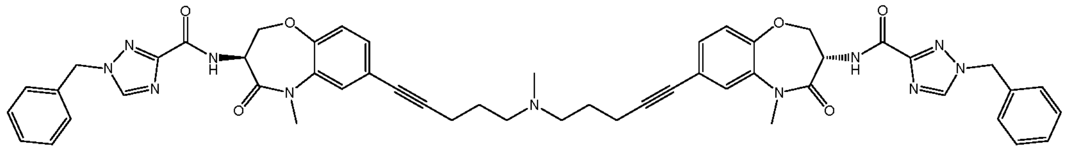

I-98

,

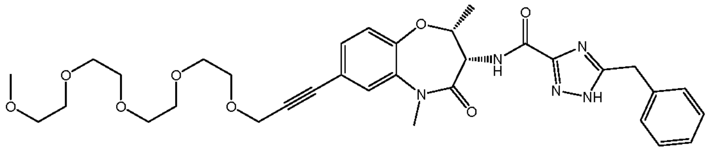

I-99

,

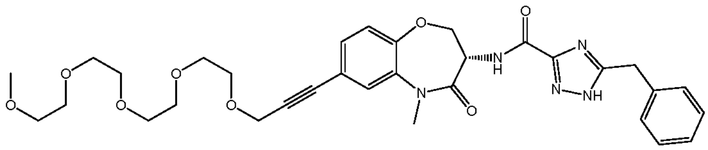

I-100

,

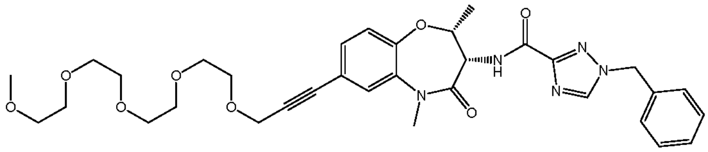

I-101

,

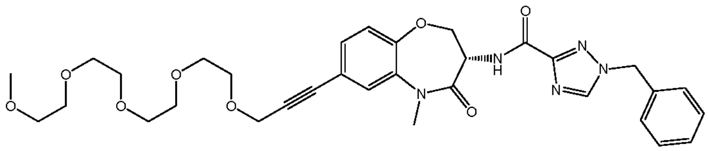

I-102

,

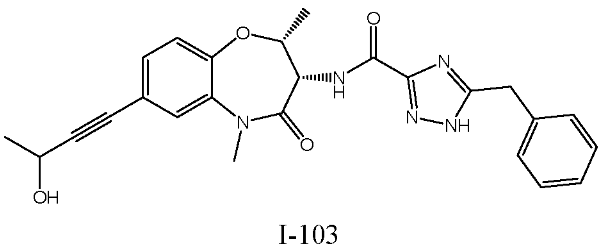

I-103

,

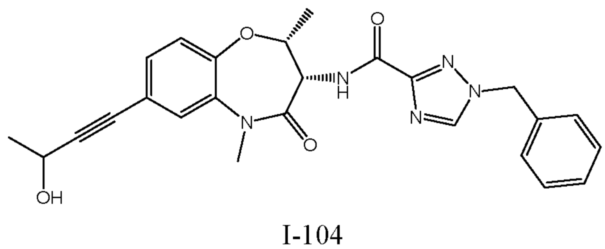

I-104

,

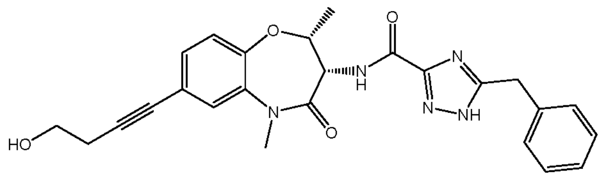
I-105 ,

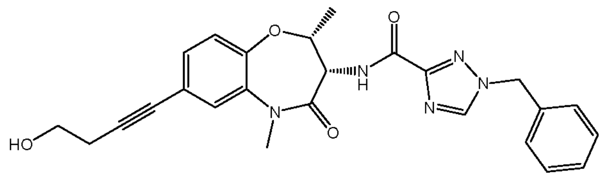
I-106 ,

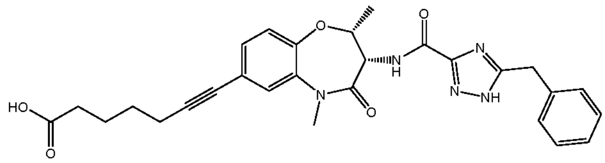
I-107 ,

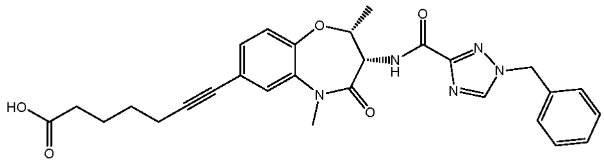
I-108 ,

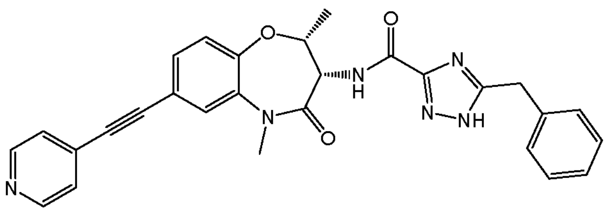
I-109 ,

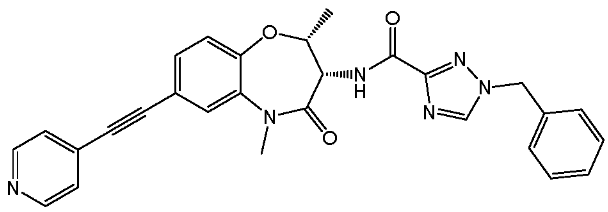
I-110 ,

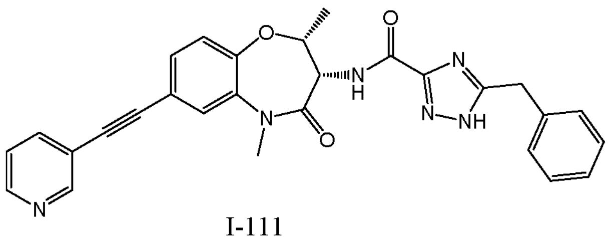
I-111 ,

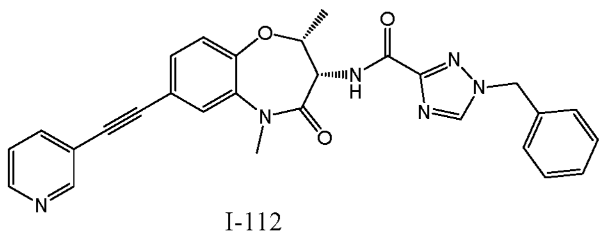
I-112 ,

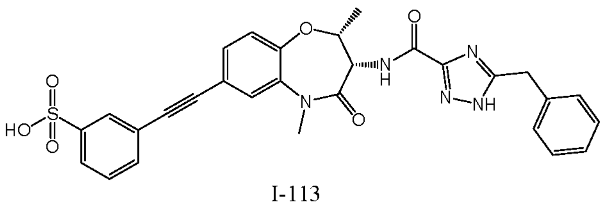
I-113 ,

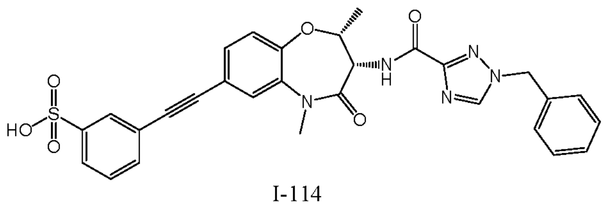
I-114 ,

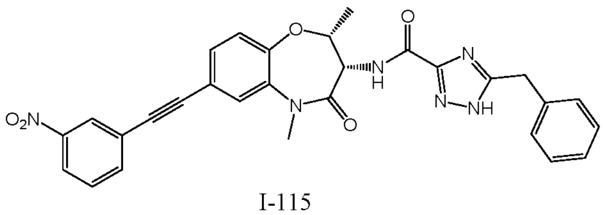
I-115 ,

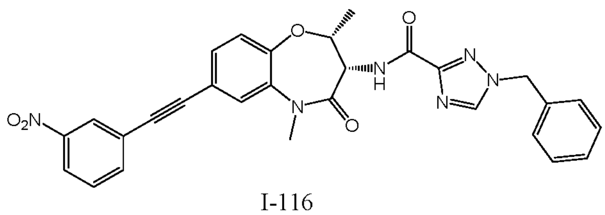
I-116 ,

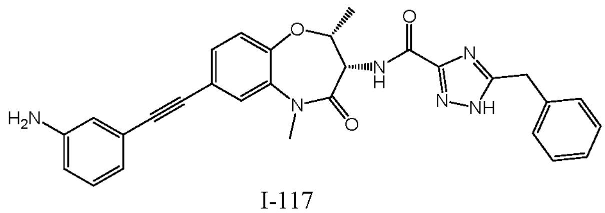

I-117 ,

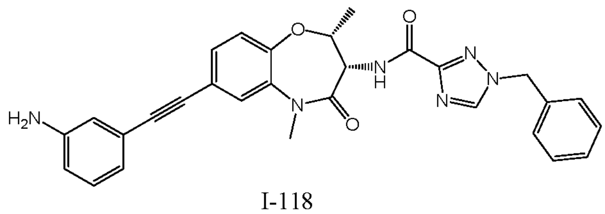

I-118 ,

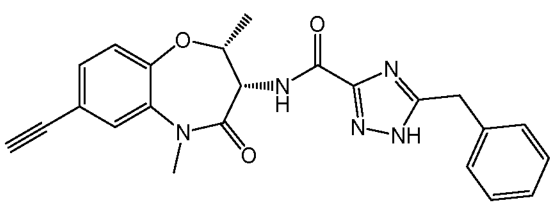

I-119 ,

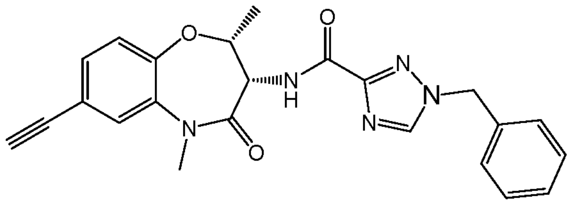

I-120 ,

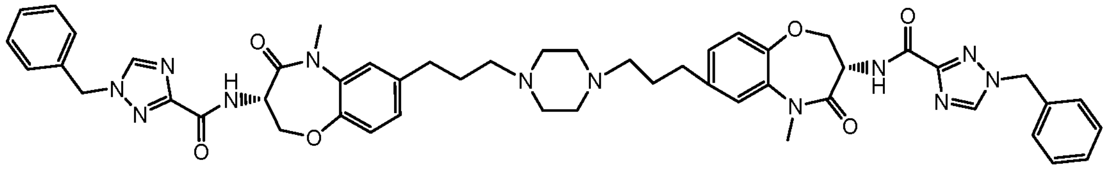

I-121 ,

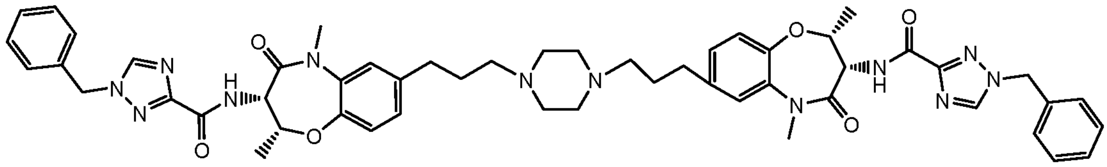

I-122 ,

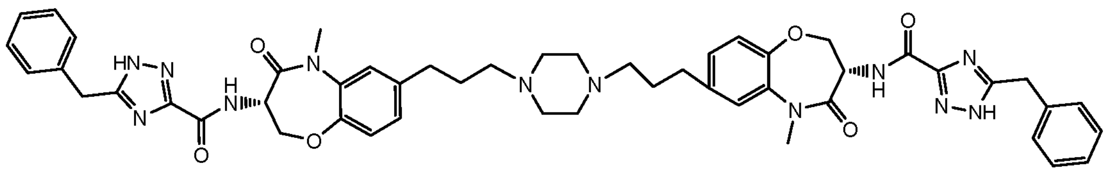

I-123 ,

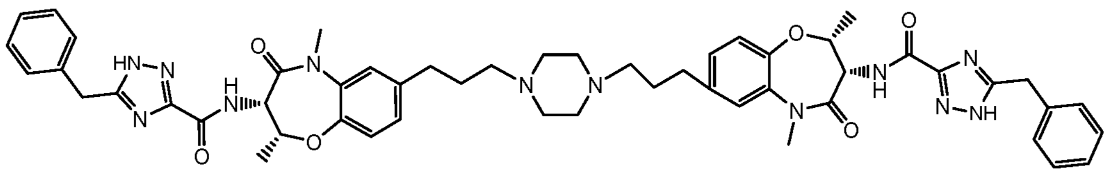

I-124 .

**12.** A compound according to any one of claims 1 to 11, or a salt thereof, or a stereoisomer thereof, or a solvate thereof, or a prodrug thereof for use in the manufacture of receptor-interacting protein (RIP) inhibitors; preferably, the RIP inhibitor is receptor-interacting protein 1 (RIP1) inhibitors.

**13.** A compound according to any one of claims 1 to 11, or a salt thereof, or a stereoisomer thereof, or a solvate thereof, or a prodrug thereof for use in the manufacture of medicaments for anti-inflammation, anti-tumor, prevention and/or treatment of psoriasis;

preferably, the anti-tumor medicaments are those for the treatment of pancreatic cancer, colon cancer, lung cancer, liver cancer, breast cancer, and lymphatic cancer;
and/or, the anti-inflammatory medicaments are those for preventing and/or treating rheumatoid arthritis, ulcerative colitis, and atopic dermatitis.

**14.** A medicament, **characterized in that** it is manufactured from a compound according to any one of claims 1 to 11, or a salt thereof, or a stereoisomer thereof, as active ingredients, in combination with pharmaceutically acceptable excipients or auxiliary ingredients.

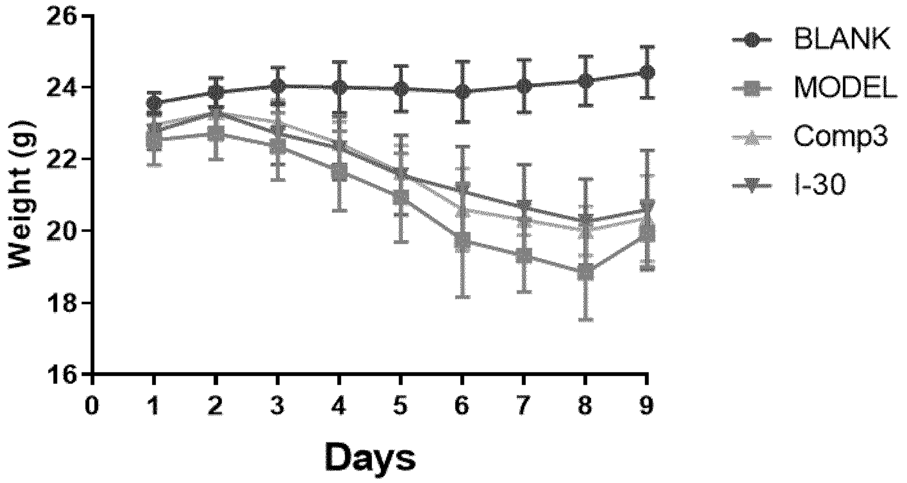


Figure 1

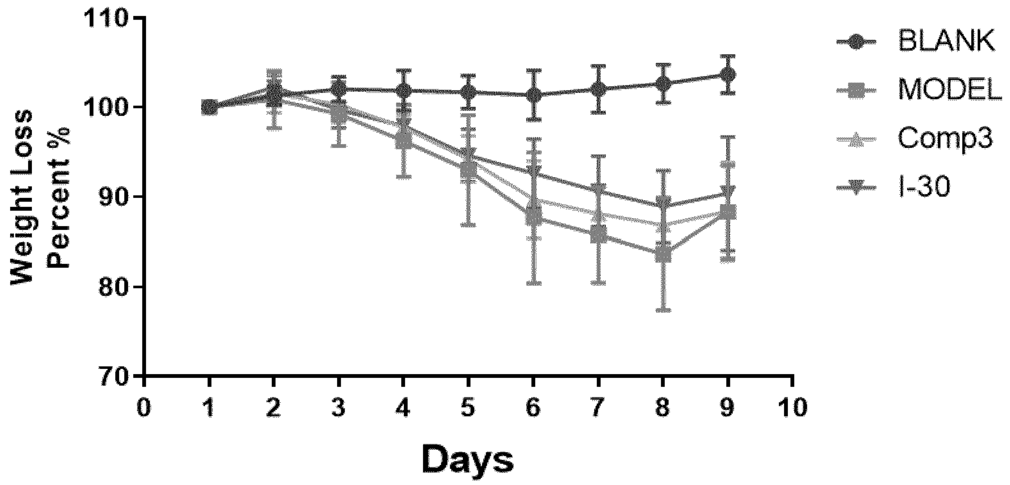


Figure 2

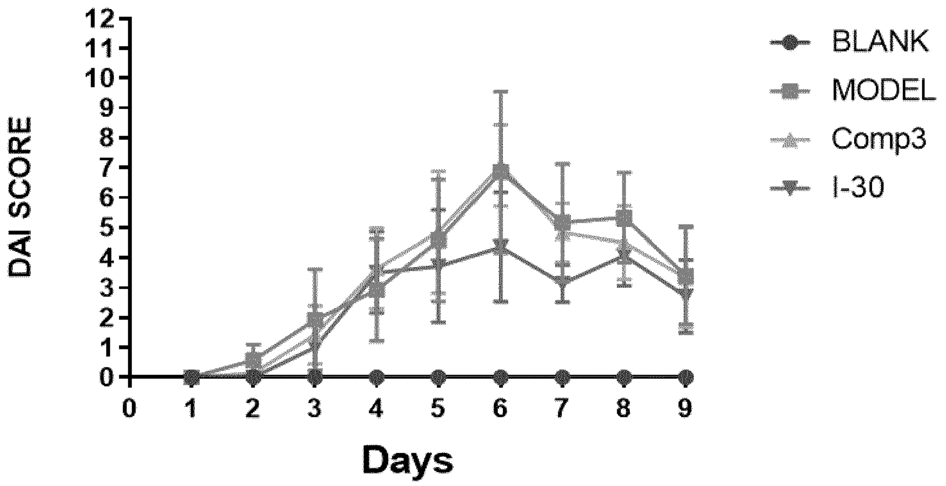


Figure 3

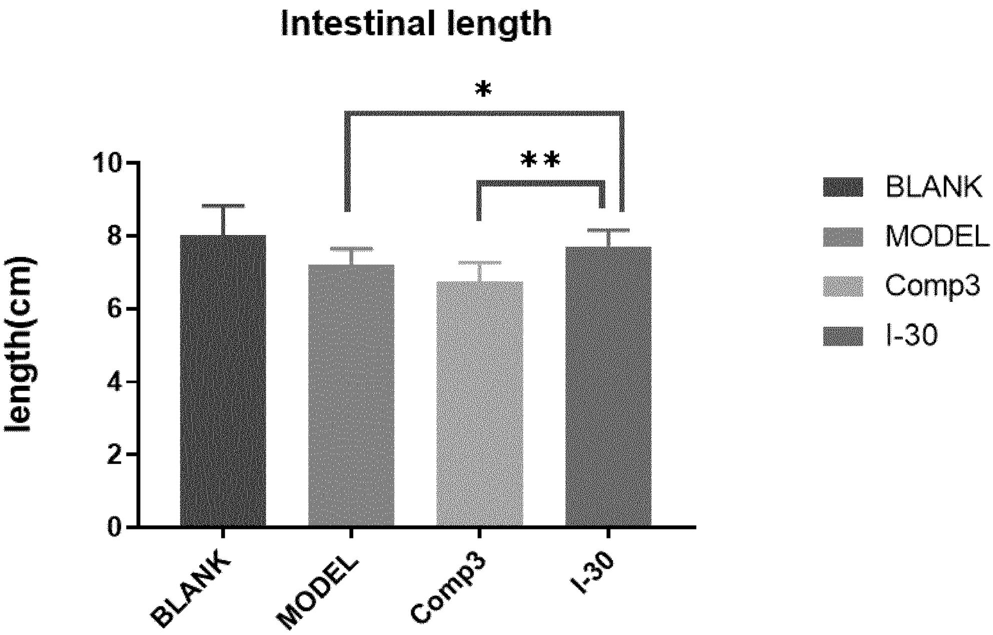


Figure 4

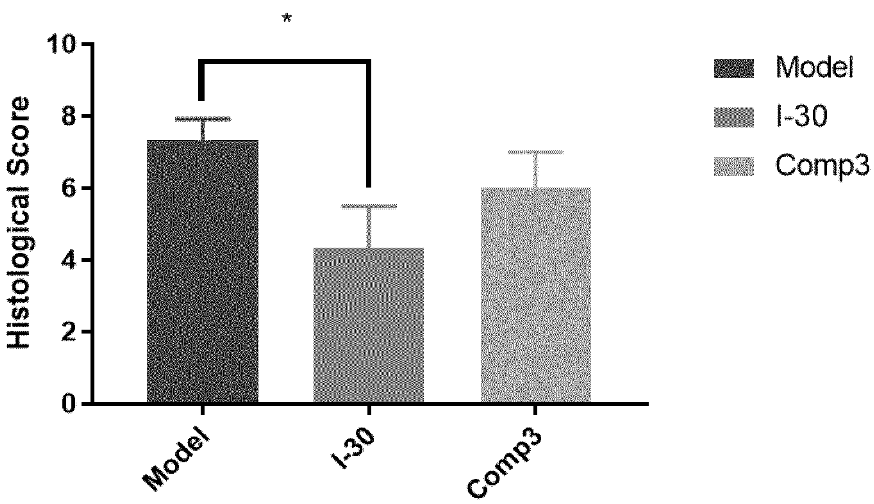


Figure 5

## INTERNATIONAL SEARCH REPORT

International application No.
**PCT/CN2022/093320**

**A. CLASSIFICATION OF SUBJECT MATTER**

C07D 413/14(2006.01)i; A61K 31/553(2006.01)i; A61P 35/00(2006.01)i; A61P 37/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; CNKI; CNABS; STNext; Web of Science: 成都贝诺科成生物科技有限公司, 受体相互作用蛋白, 炎, 肿瘤, receptor interaction protein, RIP, RIP1, inflam+, tumor, cancer

**C. DOCUMENTS CONSIDERED TO BE RELEVANT**

| Category* | Citation of document, with indication, where appropriate, of the relevant passages | Relevant to claim No. |
|---|---|---|
| X | CN 109071504 A (DENALI THERAPEUTICS INC.) 21 December 2018 (2018-12-21) description, abstract, description, table 1-table 3, and paragraphs 990-1060 | 1, 10-14 |
| X | CN 112368278 A (RIGEL PHARMACEUTICALS, INC.) 12 February 2021 (2021-02-12) description, abstract, claims 1-18, and description, paragraphs 393-404 | 1, 10-14 |
| X | WO 2020088194 A1 (SHANGHAI INSTITUTE OF MATERIA MEDICA, CHINESE ACADEMY OF SCIENCES) 07 May 2020 (2020-05-07) abstract, and claims 1-10 | 1, 10-14 |
| X | WO 2021046407 A1 (RIGEL PHARMACEUTICALS INC. et al.) 11 March 2021 (2021-03-11) the abstract, and claims 1-43 | 1, 10-14 |
| A | WO 2021046382 A1 (RIGEL PHARMACEUTICALS INC. et al.) 11 March 2021 (2021-03-11) description, abstract, and claims 1-45 | 1-14 |
| A | WO 2021092336 A1 (RIGEL PHARMACEUTICALS INC. et al.) 14 May 2021 (2021-05-14) the abstract, and claims 1-30 | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 July 2022** | **17 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/093320**

| Patent document cited in search report | Publication date (day/month/year) | Patent family member(s) | Publication date (day/month/year) |
|---|---|---|---|
| CN 109071504 A | 21 December 2018 | WO 2017136727 A2 | 10 August 2017 |
| | | KR 20180114910 A | 19 October 2018 |
| | | EA 201891620 A1 | 28 February 2019 |
| | | SG 10201913587 W A | 27 February 2020 |
| | | MX 2018009448 A | 21 November 2018 |
| | | US 2018022759 A1 | 25 January 2018 |
| | | EP 3414239 A2 | 19 December 2018 |
| | | CO 2018008707 A2 | 31 August 2018 |
| | | DO P2018000175 A | 15 October 2018 |
| | | CL 2018002081 A1 | 14 December 2018 |
| | | TN 2018000276 A1 | 16 January 2020 |
| | | BR 112018015410 A2 | 18 December 2018 |
| | | IL 260674 A | 31 October 2021 |
| | | IL 287136 D0 | 01 December 2021 |
| | | JP 2019508407 A | 28 March 2019 |
| | | EC SP18066138 A | 30 September 2018 |
| | | US 2019112318 A1 | 18 April 2019 |
| | | MA 44007 A | 19 December 2018 |
| | | CA 3012832 A1 | 10 August 2017 |
| | | US 2017226127 A1 | 10 August 2017 |
| | | AU 2017213628 A1 | 23 August 2018 |
| | | CR 13.04.2018 A | 04 December 2018 |
| | | TW 201730160 A | 01 September 2017 |
| | | US 2020317691 A1 | 08 October 2020 |
| | | PH 12018501583 A1 | 08 April 2019 |
| | | US 2018099981 A1 | 12 April 2018 |
| | | JP 2022017447 A | 25 January 2022 |
| | | SG 11201806302 R A | 30 August 2018 |
| CN 112368278 A | 12 February 2021 | KR 20210005222 A | 13 January 2021 |
| | | US 2020407332 A1 | 31 December 2020 |
| | | PH 12020551847 A1 | 28 June 2021 |
| | | AU 2019262144 A1 | 26 November 2020 |
| | | CO 2020015156 A2 | 26 February 2021 |
| | | WO 2019213447 A1 | 07 November 2019 |
| | | MA 52488 A | 10 March 2021 |
| | | JP 2021523226 A | 02 September 2021 |
| | | EC SP20078326 A | 31 March 2021 |
| | | PE 20211383 A1 | 27 July 2021 |
| | | CL 2020002841 A1 | 24 May 2021 |
| | | DO P2020000198 A | 30 April 2021 |
| | | EA 202092580 A1 | 07 April 2021 |
| | | US 2021002237 A1 | 07 January 2021 |
| | | BR 112020022420 A2 | 02 March 2021 |
| | | CR 20200581 A | 11 May 2021 |
| | | ZA 202007486 B | 29 June 2022 |
| | | CA 3099037 A1 | 07 November 2019 |
| | | US 2021040053 A1 | 11 February 2021 |
| | | SG 11202010915 X A | 30 December 2020 |
| | | JO P20200278 A1 | 03 November 2019 |
| | | ZA 202108603 B | 30 March 2022 |

Form PCT/ISA/210 (patent family annex) (January 2015)

**INTERNATIONAL SEARCH REPORT**
**Information on patent family members**

International application No.
**PCT/CN2022/093320**

| Patent document cited in search report | Publication date (day/month/year) | Patent family member(s) | Publication date (day/month/year) |
|---|---|---|---|
| | | US 2021002236 A1 | 07 January 2021 |
| | | US 2021009537 A1 | 14 January 2021 |
| | | EP 3788044 A1 | 10 March 2021 |
| | | US 2019337907 A1 | 07 November 2019 |
| WO 2020088194 A1 | 07 May 2020 | JP 2021535190 A | 16 December 2021 |
| WO 2021046407 A1 | 11 March 2021 | WO 2021046382 A1 | 11 March 2021 |
| | | AU 2020343671 A1 | 17 March 2022 |
| | | IL 290779 D0 | 01 April 2022 |
| | | EP 4025568 A1 | 13 July 2022 |
| | | CA 3149963 A1 | 11 March 2021 |
| | | CA 3149900 A1 | 11 March 2021 |
| | | US 2021070744 A1 | 11 March 2021 |
| | | TW 202122388 A | 16 June 2021 |
| | | US 2021070735 A1 | 11 March 2021 |
| | | AU 2020341681 A1 | 17 March 2022 |
| | | KR 20220042204 A | 04 April 2022 |
| | | KR 20220042429 A | 05 April 2022 |
| | | US 2021070743 A1 | 11 March 2021 |
| | | EP 4025572 A1 | 13 July 2022 |
| | | TW 202122389 A | 16 June 2021 |
| | | IL 290780 D0 | 01 April 2022 |
| | | IL 291033 D0 | 01 May 2022 |
| | | EP 4025574 A1 | 13 July 2022 |
| | | AU 2020343681 A1 | 17 March 2022 |
| | | TW 202122391 A | 16 June 2021 |
| | | CA 3149926 A1 | 11 March 2021 |
| | | WO 2021046447 A1 | 11 March 2021 |
| | | CO 2022002630 A2 | 21 June 2022 |
| | | KR 20220042431 A | 05 April 2022 |
| WO 2021046382 A1 | 11 March 2021 | AU 2020343671 A1 | 17 March 2022 |
| | | IL 290779 D0 | 01 April 2022 |
| | | EP 4025568 A1 | 13 July 2022 |
| | | CA 3149963 A1 | 11 March 2021 |
| | | CA 3149900 A1 | 11 March 2021 |
| | | US 2021070744 A1 | 11 March 2021 |
| | | TW 202122388 A | 16 June 2021 |
| | | US 2021070735 A1 | 11 March 2021 |
| | | WO 2021046407 A1 | 11 March 2021 |
| | | AU 2020341681 A1 | 17 March 2022 |
| | | KR 20220042204 A | 04 April 2022 |
| | | KR 20220042429 A | 05 April 2022 |
| | | US 2021070743 A1 | 11 March 2021 |
| | | EP 4025572 A1 | 13 July 2022 |
| | | TW 202122389 A | 16 June 2021 |
| | | IL 290780 D0 | 01 April 2022 |
| | | IL 291033 D0 | 01 May 2022 |
| | | EP 4025574 A1 | 13 July 2022 |
| | | AU 2020343681 A1 | 17 March 2022 |
| | | TW 202122391 A | 16 June 2021 |
| | | CA 3149926 A1 | 11 March 2021 |

**INTERNATIONAL SEARCH REPORT**
**Information on patent family members**

International application No.
**PCT/CN2022/093320**

| Patent document cited in search report | Publication date (day/month/year) | Patent family member(s) | Publication date (day/month/year) |
|---|---|---|---|
| | | WO 2021046447 A1 | 11 March 2021 |
| | | CO 2022002630 A2 | 21 June 2022 |
| | | KR 20220042431 A | 05 April 2022 |
| WO 2021092336 A1 | 14 May 2021 | US 2021139494 A1 | 13 May 2021 |
| | | AU 2020378407 A1 | 02 June 2022 |
| | | KR 20220079919 A | 14 June 2022 |
| | | IL 292536 D0 | 01 June 2022 |
| | | CA 3156594 A1 | 14 May 2021 |
| | | TW 202132307 A | 01 September 2021 |

Form PCT/ISA/210 (patent family annex) (January 2015)